# EUROPEAN PATENT SPECIFICATION

(11) **EP 2 061 502 B1**
(45) Date of publication and mention of the grant of the patent: **24.04.2013**
(21) Application number: 07838232.2
(22) Date of filing: 14.09.2007
(51) Int. Cl.: A61K 39/21, C07K 14/16

(54) **CARBOHYDRATE-BASED VACCINES FOR HIV**
KOHLENHYDRAT-BASIERTE IMPFSTOFFE FÜR HIV
VACCIN POUR VIH A BASE DE GLUCIDES

(30) Priority: 14.09.2006 US 844355 P; 27.03.2007 US 907272 P
(43) Date of publication of application: 27.05.2009
(73) Proprietor: Duke University, Durham, NC 27708-0083 (US); University Of Kansas, Lawrence, KS 66045 (US)
(72) Inventor: HAYNES, Barton F., Durham, NC 27708-0083 (US); DESAIRE, Heather, Lawrence, KS 66045 (US); GO, Eden P., Lawrence, KS 66045 (US)
(74) Representative: Campbell, Patrick John Henry
(86) International application number: PCT/US2007/019996
(87) International publication number: WO 2008/033500

(56) References cited:
- WO-A2-2004/033663
- WO-A2-2004/050711
- WO-A2-2004/075850
- SINGH SUDDHAM ET AL: "Chemoenzymatic synthesis of high-mannose type HIV-1 Gp120 glycopeptides" BIOORGANIC & MEDICINAL CHEMISTRY LETTERS, PERGAMON, ELSEVIER SCIENCE, GB LNKD- DOI:10.1016/S0960-894X(02)01025-9, vol. 13, no. 3, 10 February 2003 (2003-02-10), pages 327-330, XP002306980 ISSN: 0960-894X
- WANG LAI-XI ET AL: "Binding of high-mannose-type oligosaccharides and synthetic oligomannose clusters to human antibody 2G12: Implications for HIV-1 vaccine design" CHEMISTRY AND BIOLOGY, CURRENT BIOLOGY, LONDON, GB LNKD- DOI:10.1016/S1074-5521(03)00299-0, vol. 11, no. 1, 1 January 2004 (2004-01-01), pages 127-134, XP002454621 ISSN: 1074-5521
- LI HENGGUANG ET AL: "DESIGN AND SYNTHESIS OF A TEMPLATE-ASSEMBLED OLIGOMANNOSE CLUSTER AS AN EPITOPE MIMIC FOR HUMAN HIV-NEUTRALIZING ANTIBODY 2G12" ORGANIC & BIOMOLECULAR CHEMISTRY, ROYAL SOCIETY OF CHEMISTRY, GB LNKD- DOI:10.1039/B314565D, vol. 2, no. 4, 1 January 2004 (2004-01-01) , pages 483-488, XP009074357 ISSN: 1477-0520
- DUDKIN V Y ET AL: "TOWARD FULLY SYNTHETIC CARBOHYDRATE-BASED HIV ANTIGEN DESIGN: ON THE CRITICAL ROLE OF BIVALENCY" JOURNAL OF THE AMERICAN CHEMICAL SOCIETY, AMERICAN CHEMICAL SOCIETY, NEW YORK, US LNKD- DOI:10.1021/JA047720G, vol. 126, no. 31, 1 January 2004 (2004-01-01), pages 9560-9562, XP002995531 ISSN: 0002-7863
- CALARESE DANIEL ET AL: "Toward a carbohydrate-based HIV-1 vaccine" WATER-SOLUBLE POLYMERS: SYNTHESIS, SOLUTION PROPERTIES AND APPLICATIONS, AMERICAN CHEMICAL SOCIETY, WASHINGTON, DC, US, 1 March 2004 (2004-03-01), pages 161-185, XP008084732 ISBN: 978-0-541-23408-9
- SAIFUDDIN ET AL.: 'Interaction of mannose-binding lectin with primary isolates of human immunodeficiency virus type 1' JOURNAL OF GENERAL VIROLOGY vol. 81, 2000, pages 949 - 955, XP008131332
- HAYNES ET AL.: 'Analysis of HIV-1 subtype B third variable region peptide motifs for induction of neutralizing antibodies against HIV-1 primary isolates' VIROLOGY vol. 345, 2006, pages 44 - 55, XP005252848
- NI ET AL.: 'Toward a Carbohydrate-Based HIV-1 Vaccine Synthesis and Immunological Studies of Oligomannose-Containing Glycoconjugates' BIOCONJUGATE CHEM. vol. 17, 2006, pages 493 - 500, XP009074356

## Description

### TECHNICAL FIELD

The present invention relates, in general, to human immunodeficiency virus (HIV), and in particular to a carbohydrate-based vaccine for HIV and to methods of making and using same.

### BACKGROUND

Development of a safe, practical and effective HIV-1 vaccine is one of the highest priorities of the global scientific community (Klausner et al, Science 5628:2036-2039 (2003), Esparza et al, Science Strategic Plan, DOI: 10.1371/journal.pmed.0020025, Policy Forum Vol. 2, February 2005)). While antiretroviral treatnient (ART) has dramatically prolonged the lives of HIV-1 infected patients, anti-retroviral therapy is not yet routinely available in 20 developing countries, and the global rate of spread of HIV-1 continues unabated. If no effective AIDS vaccine is developed by year 2010, the number of people infected world-wide with HIV-1 could exceed 60 million (Derived from Statistics in Global Summary of the AIDS Epidemic, "AIDS Epidemic Update" UNAIDS. World Health Organization, December 2005)).

In spite of more than 20 years of research, the types of immune responses needed to protect an immunized individual from HIV-1 infection are not known. It is known that CD8+ cytotoxic T cell responses can control HIV-1 replication to varying degrees in acute HIV-1 infection (AHI) (reviewed in Letvin, Ann. Rev. Med. 56:213-223 (2005), Gandhi and Walker, Ann. Rev. Med. 53:149-172 (2002)), and, when induced by immunogens, can control viral set point after SIV or SHIV challenges in non-human primates (Letvin, Ann. Rev. Med. 56:213-223 (2005)). Strong proliferative CD4+ T cell responses to HIV-1 proteins have been shown to correlate well with immune control of HIV-1 viral load (Gandhi and Walker, Ann. Rev. Med. 53:149-172 (2002)). Thus, it is highly desirable for an HIV-1 vaccine to induce robust CD4+ and CD8+ T cell responses in order to control virus replication and reduce the likelihood of subsequent transmission (Letvin, Ann. Rev. Med. 56:213-223 (2005), Gandhi and Walker, Ann. Rev. Med. 53:149-172 (2002), Mastro and Kitayapom, AIDS Res. Hum. Retroviruses 14 Suppl 3:S223-S227 (1998), Quinn et al, N. Engl. J. Med. 342:921-929 (2000)).

The potential for complete ("sterilizing") immunity from HIV-1 infection may depend on the presence of pre-existing neutralizing antibodies. It is known that neutralizing antibodies can prevent the acquisition of AIDS virus infection after intravenous, vaginal, rectal and oral virus challenge in nonhuman primates (Shibata et al, Nat. Med. 5:204-210 (1999), Mascola et al, J. Virol. 73:4009-4018 (1999), Mascola et al, Nat. Med. 6:207-210 (2000), Parren et al, J. Virol. 75:8340-8347 (2001), Ferrantelli et al, J. Infect. Dis. 189:2167-2173 (2004)). This level of protection is highly attractive for a vaccine against a virus such as HIV-1, which integrates genetically and forms latent viral reservoirs soon after infection. However, the diversity of transmitted HIV-1 genetic variants and the relative resistance of most HIV-1 primary isolates to most types of inducible neutralizing antibodies have posed major hurdles to current vaccine efforts. Moreover, the few rare broadly reactive neutralizing monoclonal antibodies (Mabs) that have been isolated from HIV-1 infected patients represent species of antibodies that are not able to be routinely induced in animals or uninfected humans by HIV-1 Env immunogens (reviewed in Burton et al, PNAS 102:14943-14948 (2005),Haynes et al, Human Antibodies, In press)).

The strategies for induction of neutralizing antibodies that have been successful for other infectious agent vaccines have thus far failed for HIV-1 vaccine development, including immunization with an outer envelope protein (gp120) (The rgp120 HIV Vaccine Study Group, J. Infect. Dis. 191:654-665 (2005), Gilbert et al, J. Infect. Dis. 192:974-983 (2005)), and immunization with a killed or inactivated virus (Levine et al, J. Acquir. Immune Defic. Syndr. Hum. Retrovirol. 11:351-364 (1996), Lifson et al, AIDS Res. & Human Retrovir. 20:772-787 (2004)). Live attenuated SIVs have indeed induced substantial protection to SIV challenge but attenuated SIV strains have reverted to wild-type *in vivo,* and have proven unsafe in neonatal monkeys (Whitney et al, Curr. Opin. Infect. Dis. 17:17-26 (2004), Koff et al, Nat. Immunol. 7:19-23 (2005)).

Vaccine research areas that are of particular importance include those related to understanding the host immune response to HIV-1 virions and HIV-1 infected cells at sites of mucosal transmission, and work on characterizing the transmitted virus. Recent work suggests that the variable loops of transmitted HIV-1 of certain clades (A and C), but not others (B), may be shorter and the transmitted viruses more neutralization-sensitive than chronic HIV-1 strains (Derdeyn et al, Science 303:2019-2022 (2004), Frost et al, J. Virol. 79:6523-6527 (2005), Chohan et al, J. Virol. 79:6528-6531 (2005)). Further, broadly neutralizing IgA antibodies have been reported in the genitourinary tracts of seropositive (Alfsen et al, J. Immunol. 166:6257-6265 (2001)) and highly exposed and uninfected subjects (Devito et al, J. Acquir. Immune Defic. Syndr. Hum. Retrovirol. 30:413-420 (2002)). If these observations can be shown to be a correlate of protective immunity for mucosal transmission of HIV-1, it will be critical to translate these findings to strategies for inducing protective IgA mucosal antibodies in humans. Moreover, although IgG is the most common antibody type in cervicovaginal secretions, little attention has been paid to the specificity and regulation of mucosal IgG anti-HIV-1 immune responses.

A major conundrum for HIV-1 vaccine developers has been that, in spite of the presence of epitopes on the HIV-1 envelope that are targets for broadly neutralizing HIV-1 human Mabs, these species of antibodies are not made following immunization with antigenic Env proteins, nor are they routinely produced after infection with HIV-1 (reviewed in Burton et al, PNAS 102:14943-14948 (2005),Haynes et al, Human Antibodies, In press)). Two major reasons for the this failure are the lack of current immunogens that mirror the native envelope structures needed to induce neutralizing antibodies, and the likely tolerant state of the host to some conserved HIV-1 envelope epitopes.

That there are rare human broadly neutralizing Mabs (Burton et al, PNAS 102:14943-14948 (2005)), that HIV-1 cellular responses can transiently and in some cases persistently control HIV-1 (Letvin, Ann. Rev. Med. 56:213-223 (2005), Gandhi and Walker, Ann. Rev. Med. 53:149-172 (2002)), and that high levels of passively administered Mabs can protect against chimeric simian-HIV immunodeficiency virus (SHIV) challenges (Shibata et al, Nat. Med. 5:204-210 (1999), Mascola et al, J. Virol. 73:4009-4018 (1999), Mascola et al, Nat. Med. 6:207-210 (2000), Parren et al, J. Virol. 75:8340-8347 (2001), Mascola, Vaccine 20:1922-1925 (2002), Mascola, Current Mol. Med. 3:209-216 (2003)), all give hope that a successful AIDS vaccine can be made.

HIV-1 has adapted the gp120 portion of Env to escape immune recognition by a number of mechanisms including glycan shielding (Wei et al, Nature 422:307-312 (2003)), mutation of variable regions (66-69), and conformation masking (Kwong et al, Nature 420:678-682 (2002)).

The outer face of the gp120 envelope protein is an immunologically "silent face" that is covered by N-linked glycans; up to 50% of the weight of gp120 is carbohydrate (Wei et al, Nature 422:307-312 (2003), Scanlan et al, J. Virol. 76:7306-21 (2002), Scanlan et al, Adv. Exp. Med. Biol. 535:205-218 (2003), Calarese et al, Proc. Natl. Acad. Sci. USA 102:13372-13377 (2005)). The number of glycosylation sites on gp120 remains approximately the same (~25 sites), but the sites shift or "evolve" in position over time as neutralizing antibodies are generated - a phenomenon termed "evolving glycan shield" (Wei et al, Nature 422:307-312 (2003)). A rare human Mab exists (2G12) that binds to Mana 1-2Man at the termini of both D1 and D3 arms of a variety of oligomannose carbohydrates on the HIV-1 trimer and broadly neutralizes HIV-1 (Scanlan et al, J. Virol. 76:7306-21 (2002), Scanlan et al, Adv. Exp. Med. Biol. 535:205-218 (2003), Calarese et al, Proc. Natl. Acad. Sci. USA 102:13372-13377 (2005)). There are two potential reasons why HIV-1 virion carbohydrates are poorly immunogenic. First, because HIV-1 has no glycosylation machinery of its own, the sugars to which 2G12 binds, like other virion carbohydrates, are similar to host carbohydrates and are likely regarded as "self" antigens (Scanlan et al, J. Virol. 76:7306-21 (2002)). A second reason for the poor immunogenicity of carbohydrates on HIV-1 is microheterogeneity, ie, a single protein sequence would be expected to express multiple carbohydrate forms leading to a dilution of an immune response (Scanlan et al, J. Virol. 76:7306-21 (2002)).

Yang et al have demonstrated that only one trimer per virion is needed to bind to host cells to mediate infection (Yang et al, J. Virol. 79:12132-12147 (2005)). Therefore, to prevent virion infection of CD4+, CCR5+ host cells, all trimers on each virion must be bound by at least one neutralizing antibody molecule. For induction of antibodies that will neutralize HIV-1, it is critical to learn how to induce high affinity, durable, and broadly reactive neutralizing antibodies that are present both systemically and at mucosal surfaces. Therefore, if specific carbohydrates on the surface of HIV Env can be made immunogenic, then HIV Env carbohydrates would become a viable target of a neutralizing antibody response. Similarly, the HIV Env carbohydrates would become components of a HIV vaccine.

WO 2004/050711 describes gp120 glycans and glycoconjugates thereof, and methods for the synthesis and use thereof. Singh et al (Bioorganic & Medicinal Chemistry Letters, 13;327-330 (2003)) describes a chemoenzymatic approach to the synthesis of HIV-1 gp120 glycopeptides containing Man₉, Man₆ and Man₅ moieties using an endo-β-*N*-acetyglucosaminidase from *Arthobacter.* Li et al (Organic & Biomolecular Chemistry, 2;483-488 (2004)) describes the design and synthesis of a template-assembled oligomannose cluster intended to provide an epitope mimic for the human anti-HIV antibody 2G12. Dudkin et al (Journal of the American Chemical Society, 126;9560-9562 (2004)) describes synthetic constructs mimicking the 2G12 carbohydrate epitope. Calarese et al (Water-soluble polymers; synthesis, solution properties and applications American Chemical Society, 161-185 (2004)) relates to methods for generating carbohydrate-based HIV-1 vaccines. WO 2004/033663 describes an oligosaccharide cluster that mimics the HIV carbohydrate antigen having affinity for the 2G12 antibody. Ni et al (Bioconjugate Chem, 17;493-500 (2006)) describes the synthesis of oligomannose-containing glycoconjugates that include either a carrier protein or a universal T-helper epitope peptide to provide an effective immunogen.

### SUMMARY OF THE INVENTION

In general, the present invention relates to HIV. More specifically, the invention relates to a composition comprising carbohydrates of the V1 or V2 region of HIV Env rendered immunogenic by derivatization and a carrier.

### BRIEF DESCRIPTION OF THE DRAWINGS

Figure 1. Protein sequence of an example envelope protein. The glycosylation sites are highlighted in red.
Figure 2. MALDI data using "standard" digestion conditions.
Figure 3. Nanospray FT-MS data using standard digestion conditions.
Figure 4. Comparison of standard digestion conditions and digestion conditions of the invention.
Figure 5. MALDI-MS data for one of the CON-S glycopeptide fractions. The inset is a screen shot of output from *GlycoPep DB.*
Figure 6. MALDI MS/MS data of a glycopeptide ion observed in Figure 5. Data is confirms the peptide portion of the glycopeptide.
Figure 7. MALDI MS data.
Figure 8. HPLC-MS data collected on the Fourier transform mass spectrometer.
Figure 9. Differences in glycosylation at the conserved site ...*LENVT...* before the V1 loop on CON-S and JR-FL are attributed to differences in the proteins' 3-dimensional structures *in vivo.*
Figure 10. Summary of glycosylation for CON-S on the V 1 and V2 loops. V1 is very heavily glycosylated with high mannose sugars. V2 has 2 potential glycosylation sites that are not utilized.
Figure 11. Summary of glycosylation for JRFL on the V 1 and V2 loops. Like CON-S, V1 is very heavily glycosylated with high mannose sugars. V2 uses more of its glycosylation sites, compared to CON-S.
Figure 12. CON-S glycosylation between V2 and V3. This part of the sequence is generally not exposed to glycosyltransferases while passing through the Golgi. High mannose carbohydrates may act as "internal chaperones" to help fold proteins. While the carbohydrate may be stabilizing the protein, the protein might also be sitting in a stable conformation, protecting the carbohydrate.
Figure 13. CON-S glycosylation near the V3 loop.
Figure 14. JRFL glycosylation near the V3 loop. JRFL is more heavily glycosylated at the beginning of the loop and more structural heterogeneity at the end of the loop.
Figure 15. CON-S glycosylation after V3. Of the 8 characterized sites, three had no glycosylation. The areas between the variable loops have a higher proportion of high-mannose containing carbohydrates, suggesting they were buried within the protein during Golgi processing. The sialic acid on the outside of V4 and V5 may help slow metabolic clearance.
Figure 16. Of the eight characterized sites, six were glycosylated. At every position, a high degree of variablility in the glycosylation was found. This indicates that the protein had a high degree of structural heterogeneity as it was being post-translationally processed.
Figures 17A and 17B. Sequence alignment of CON-S gp140 ΔCFI and JR-FL gp140ΔCF. Dashes indicate gaps in amino acid sequence and the location of the variable regions (V1-V5) are shown. Potential glycosylation sites are indicated with a dot with difference in potential glycosylation sites are boxed. Identified tryptic fragments are underlined.
Figures 18A and 18B. Schematic representation of the experimental approach of the glycosylation mapping and profiling.
Figures 19A and 19B. Representative (Fig. 19A) ESI-FTICR MS and (Fig. 19B) MALDI MS spectra of the glycopeptide fraction generated from the proteolytic digest of the envelope proteins. Inset: MS/MS spectra of the identified tryptic glycopeptides. Peptide portion was determined from the characteristic cross-ring cleavages, ^{0,2}X (in MALDI MS) and ⁰Y, (in ESI-FTICR MS) ions.

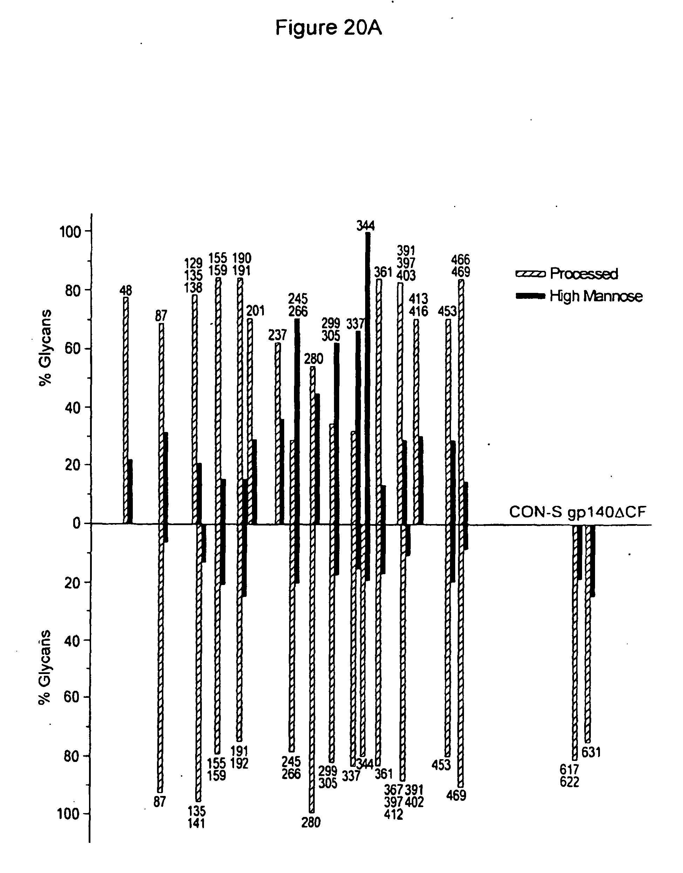

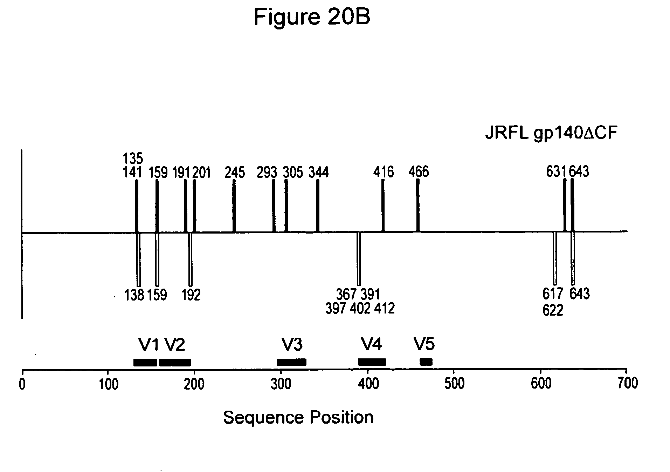

Figures 20A and 20B. Fig. 20A. Summary of glycan compositions (percent) present on the identified glycosylation site. Glycan compositions were broadly categorized into two classes (see text). Processed glycans include hybrid and complex type structures. Fig. 20B. Variably utilized and unutilized glycosylation sites for CON-S gp140 ΔCFI (top, sold black bars) JR-FL gp140 ΔCF (bottom, white filled bars). Note that numbers on top or bottom of the bars in Fig. 20A and Fig. 20B represents the glycosylation site/s. multiple numbers at the bottom of the bar in Fig. 20B denotes either of the glycosylation site is utilized.
Figures 21A and 21B. Fig. 21A. Representative MALDI MS spectrum of the deglycosylated glycopeptide fraction of CON-S gp140ΔCFI. Four tryptic peptides bearing potential glycosylation sites (see legend) were identified. Fig. 21B. MS/MS spectrum of one of the identified peptides in (Fig. 21A) bearing two potential glycosylation sites. Only one site is utilized as shown.
Figures 22A and 22B. (Fig. 22A) Sequence alignment of 60-residue segment found in the first conserved region of the envelope proteins. The sequence is 95% identical. Difference in amino acid residue is boxed. (Fig. 22B). Pictorial representation of the identified glycoforms. Note that the drawn structures are biologically relevant and isoforms exist.
Figure 23. Glycosylation of the tryptic glycopeptide at the beginning of the V3 region for JR-FL gp140 ΔCF and CON-S gp140 ΔCFI showing the utilized sites and identified glycan compositions.

### DETAILED DESCRIPTION OF THE INVENTION

The HIV Env V3 loop is an important target of HIV-1 neutralizing antibodies (Palker et al, Proc. Nat'1 Acad. Sci. USA 85:1932-1936 (1988), Haynes et al, Expert Review of Vaccines 5:347-363 (2006)). However, recent data suggest that the V3 loop is not available on the surface of many primary HIV strains (Bou-Habib et al, J Virol. 68:6006-13 (1994), Haynes et al, Virology 345:44-55 (2005)). Recent data (Zolla Pazner et al, AIDS VACCINE 2006 meeting, August 28, Sept 1, 2006, Amsterdam, Netherlands) show that the V1 and V2 loops of the HIV Env, when glycosylated, can cover the V3 loop and prevent neutralizing antibodies from binding to the V3 loop. Different strains (quasispecies) of HIV-1 have variable glycosylation sites (Zhang et al, Glycobiology 14:1229-1246 (2004)), thus, the sites of sugars on the surface of the Env "silent face" vary from quasispecies to quasispecies of HIV.

The present invention involves a strategy of identifying specific sugars at specific glycosylation sites in the V1 and V2 loops and derivatizing such carbohydrates to make them immunogenic. Producing an antibody response to the derivatized carbohydrates will force the virus to mutate that specific glycosylation site, thus eliminating the carbohydrate at that site, and thereby uncovering the V3 loop to make it available for anti-V3 antibodies to bind.

The present invention relates to a composition (e.g., a carbohydrate-based vaccine composition) comprising immunogenic (e.g., as a result of derivatization) carbohydrates and a carrier. In particular, the invention provides a composition comprising carbohydrates of the V1 or V2 region of HIV Env rendered immunogenic by derivatization and a carrier. Other composition components can include V3 peptides (such as peptide 62.19 clade B V3 (see, for example, PCT/US02/35625 and PCT/US04/005497; and U.S. Appln. Nos. 10/373,592 and 10/431,596) or whole consensus or wild type Env gp140s that induce anti-V3 antibodies (see, for example, PCT/US2004/030397; U.S. Appln. No. 10/572,638). In addition, other specific carbohydrates from other regions on the surface of the HIV Env trimer can also be identified, derivatized and utilized as further components of the composition.

Preparation of a composition of the invention involves the identification of carbohydrates of the V1, V2 region and derivatization those carbohydrates so as to render them immunogenic. Derivatization can be effected, for example, using various molecules, such as keyhole limpet hemocyanin, CRM197, or tetanus toxoid. Alternatively, HIV gag p24 helper regions such as the GTH1 sequence (YKRWIILGLNKIVRMYS), can be used to deriviatize the carbohydrate. (See Examples that follows.)

In accordance with the invention, all carbohydrates on the surface of HIV Env can be derivatized and used as a vaccine component. For example, all carbohydrates on an intact Env can be rendered immunogenic by virtue of having been derivatized with, for example, tetanus toxoid. Alternatively, all carbohydrates on HIV Env can be cleaved off, and then derivatized with, for example, tetanus toxoid (Wang et al, Vaccine 7: 1112-1117 (2003); Amir-Kroll et al, J. Immunol, 170: 6165-6171 (2003)), and utilized as an HIV vaccine component.

The Examples that follow include a detailed description of methods that can be used to identify glycosylation at specific sites in HIV envelope proteins.

Only one strain of HIV-1 Env has been previously characterized in a glycosylation site-specific fashion. That was gp120 expressed in CHO cells (Zhu et al, Biochemistry 39:11194-11204 (2000)). This form of the protein has proven ineffective as a vaccine in stage III clinical trials (The rgp120 HIV Vaccine Study Group, J. Infect. Dis. 191:654-665 (2005)). One explanation for this failure is that CHO cells produce glycosylation profiles that are different than glycosylation profiles observed in human T-cells. Therefore, the glycan shield in the failed vaccine did not adequately mimic the glycan shield of Env that is on the HIV-1 virus. It is well known that glycosylation in different cell lines or different species can produce different glycosylation patterns (Dalpathado et al, Biochemistry 45:8665-8673 (2006)), so one goal of the studies described in the Examples that follow was to express HIV-1 Env in cellular systems that result in glycosylation of HIV-1 that closely resembles that present on viral isolates.

In addition to the study referenced above, which identifies glycosylation at each of the glycosylation sites of HIV-1 Env, a number of other studies have been conducted that focus on characterizing carbohydrate structures of glycans released from HIV-1 Env isolated from CHO cells (Muzuochi et al, Biochemistry J. 254: 599-603 (1988)), human T cells (Adachi et al, J. Exp. Med. 167: 323-331 (1988), and human dendritic cells (Monzavi-Karbassi et al, Arch. Virol. 149: 75-91 (2003)). The structure of HIV Env carbohydrates that bind the broadly neutralizing antibody 2G12 have been extensively characterized (Scanlan et al, J. Virol. 76:7306-21 (2002), Scanlan et al, Adv. Exp. Med. Biol. 535:205-218 (2003), Calarese et al, Proc. Natl. Acad. Sci. USA 102:13372-13377 (2005), Wang et al Chem. and Biol. 11: 127-134 (2004)). Thus, several carbohydrate structures of HIV-1 Env have been identified, but, to date, no specific carbohydrate structures of the V1, V2 loop or other regions of the Env, have been identified that can be made immunogenic and the induced anti-carbohydrate antibodies neutralize HIV. However, monoclonal antibodies against several of these carbohydrate structures including LeY, A1 and sialy-Tn have been reported to neutralize HIV-1 showing, as with mab 2G12, proof of concept that anti-carbohydrate antibodies, if induced, could neutralize HIV (Hansen et al, J. Virol. 64: 2833-2840 (1990)). Moreover, HIV uses surface carbohydrates to bind and enter dendritic cells (Granelli-Piperno et al,. Current Biology 9:21-29 (1999); Turville et al, J. Virol. 79: 13519-13527 (2005)). Thus, anti-carbohydrate antibodies can inhibit HIV infectivity of most cell types involved in primary HIV infection.

Certain aspects of the invention can be described in greater detail in the non-limiting Examples that follows. (See also Burton et al, Nat. Immunol. 5:233-236 (2004), Cutalo et al, J. Am. Soc. Mass Spectrom. 15:1545-1555 (2004), Einfeld and Hunter, Proc. Natl. Acad. Sci. USA 85:8588-8692 (1988), Evans and Desrosiers, Immunol. Rev. 183:141-158 (2001), Fenouillet et al, Trends Biochem. Sci. 19:65-70 (1994), Flynn et al, J. Infect. Dis. 191:654-665 (2005), Gaschen et al, Science 296:2354-2360 (2002), Geyer et al, Eur. J. biochem. 193:855-862 (1990), Gilbert et al, J. Infect. Dis. 191:666-677 (2005), Hartley et al, AIDS Research and Human Retroviruses 21:171-189 (2005), Haynes and Montefiori Expert. Rev. Vaccines 5:579-595 (2006), Hebert et al J. Cell Biol. 139:613-623 (1997), Hemming et al, Arch. Virol. 134:335-344 (1994), Hemming et al, Arch. Virol. 141:2139-2151 (1996), Humbert and Dietrich, AIDS Rev. 8:51-59 (2006), Jeffs et al, J. Gen. Virol. 77:1403-1410 (1996), Johnson et al, J. Virol. 75:11426-11436 92001), Kothe et al, Virology 360:218-234 (2007), Kothe et al, Virology 352:438-449 (2006), Kwong, Nature 433:815-816 (2005), Kwong et al, Nature 393:648-659 (1998), Land and Braakman, Biochimie 83:783-790 (2001), Lee et al, Proc. Natol. Acad. Sci. USA 89:2213-2217 (1992), Letvin et al, Annu. Rev. Immunol. 20:73-99 (2002), Li et al, J. Virol. 67:584-588 (1993), Liedtke et al, Glycobiology 4:477-484 (1994), McMichael, Annu. Rev. Immunol. 24:227-255 (2006), Mizuochi et al, J. Biol. Chem. 265:8519-8524 (1990), Perrin et al, Virology 242:338-345 (1998), Poignard et al, Annu. Rev. Immunol. 19:253-274 (2001), Preston et al, Science 242:1168-1171 (1988), Reitter et al, Nat. Med. 4:679-684 (1998), Saunders et al, J. Virol. 79:9069-9080 (2005), Singh, Virol. J. 3:60 (2006), Wolk and Schreiber, Med. Microbiol. Immunol. 195:165-172 (2006), Wyatt et al, J. Virol. 67:4557-4565 (1993), Yeh et al, Biochemistry 32:11087-11099 (1993), Zolla-Pazner, Nat. Rev. Immunol. 4:199-210 (2004).)

### EXAMPLE I

### Experimental Details

### Glycoprotein Digestion

Glycoprotein digestion has three basic components, denaturing the protein, reducing and alkylating cysteines, and protease digestion. Each step is described below.

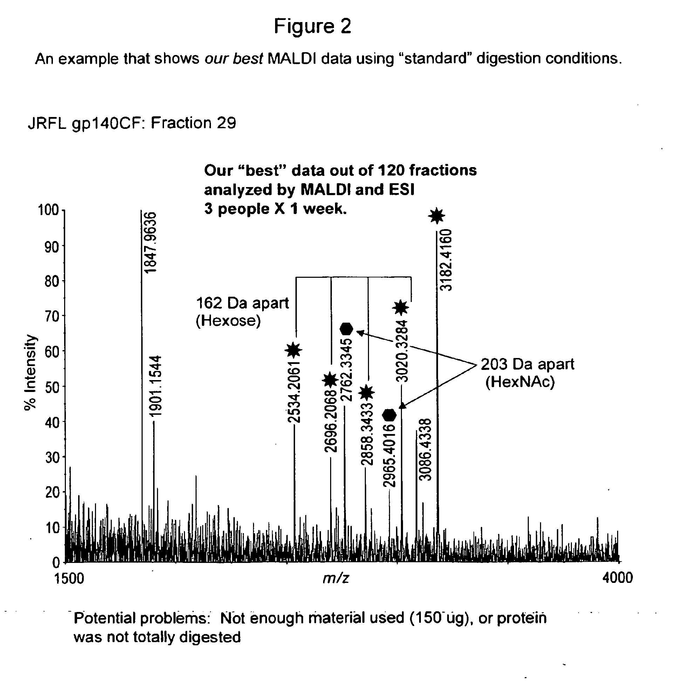

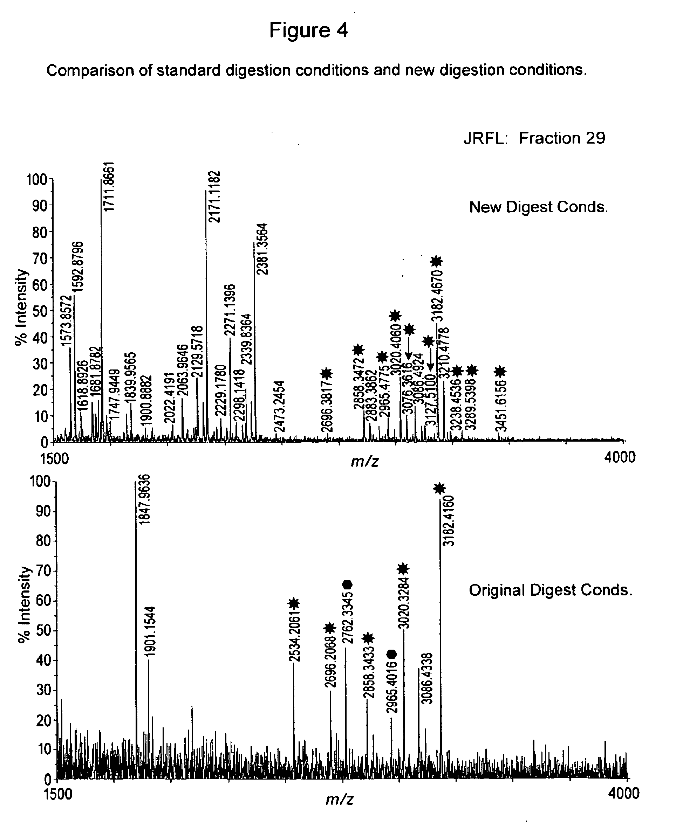

The denaturing conditions can be optimized by controlling the amount or type of buffer added, the excipients added -- such as EDTA, acetonitrile, or urea - -or by choosing to boil the protein. Two examples of denaturing conditions utilized are: (A) the protein solution is combined with aqueous ammonium bicarbonate buffer (pH 8.0), boiled for 20 minutes, and further diluted with acetonitrile, until the final acetonitrile concentration is 30% [these were the conditions in Fig. 2]; and (B) the glycoprotein is combined with 6 M urea, 2 mM EDTA, and tris buffer (pH 7.5) (see Fig. 4) .

After denaturing, the protein can be reduced and alkylated. This process can also be optimized by varying the reagent concentrations or reaction times. Two examples of reduction and alkylation conditions include: (A) reducing the cysteines with 10 mM DTT (dithiothreitol) at 60°C for 30 minutes, followed by an alkylation step, adding 25 mM IAA (indole acetic acid) and reacting at 37° C in the dark for 30 minutes; and (B) reducing the cysteines with 10 mM DTT at 60° C for 60 minutes, followed by an alkylation step, adding 25 mM IAA and reacting at 37° C in the dark for 60 minutes.

Digestion occurs after reduction and alkylation, and it can be achieved using a variety of enzymes. Trypsin is the most common enzyme used for these purposes, but other enzymes, like pronase, proteinase K, Lys-C, etc, can also be used, either individually or in combinations. Typical tryptic digestion conditions include digesting the protein at 37°C with trypsin at a protein:enzyme ratio of 50:1 (w/w) overnight, or digesting at 37°C with trypsin at a protein:enzyme ratio of 30:1 (w/w) overnight, followed by a second digestion under the same conditions

### Sample preparation for MS analysis.

After digestion is complete, additional sample preparation can be performed before MS analysis. For example, the sample can be fractionated by HPLC in an "offline" approach, where individual fractions are collected, and further processed prior to analysis. Alternatively, "online" HPLC-MS can be used, where MS analysis directly follows the separation step. In the "offline" approach, conditions can include using a C18 column with the dimensions, 4.6 id. × 150mm, and running a gradient of increasing acetonitrile (ACN) with time (where solvent A is H₂O with 0.1% formic acid, and solvent B is ACN with 0.1% formic acid) at a flow rate of 1 mL/minute. Under these conditions, a small portion of the digestion solution (20-30 µL) can be injected, and fractions can be collected every minute. For a sixty-minute chromatographic run, 60 fractions are collected (see Zhu et al, Biochemistry 39:11194-11204 (2000)) These fractions can be subsequently dried and reconstituted in 10 µL H₂O, prior to MS analysis. Alternatively, the final concentration of each fraction can be enhanced by drying each fraction and re-collecting a second round of fractions in the same vials, followed by a second drying step. The separation of glycopeptides can be optimized by changing the gradient, column type, mobile phase compositions, or temperature of the separation.

### MS analysis

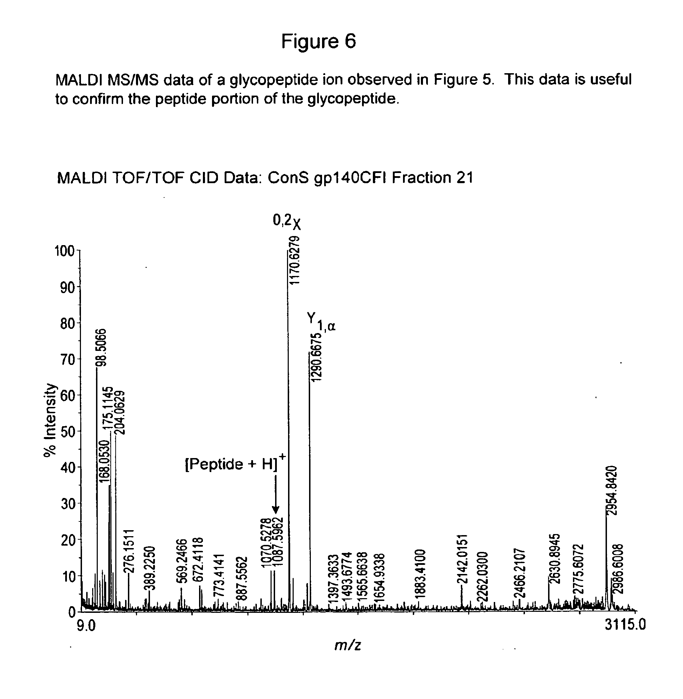

Mass spectrometric analysis of glycopeptides can be accomplished with a variety of instrumentation, for example, a MALDI TOF-TOF mass spectrometer (Proteomics 4700, by Applied Biosystems) and a ESI-Fourier Transform mass spectrometer (an LTQ-FTMS by Thermo.) In any case, the instruments can be calibrated prior to data acquisition. For MALDI-MS analysis, the offline sample preparation method can be used, and the aqueous samples can be spotted onto a MALDI target, along with an appropriate matrix, and MS data can be acquired in the reflectron mode, which optimizes mass accuracy. Figs. 2, 4, 5, and 7 contain examples of MALDI-MS data collected using the "offline" approach. Any peak detected that may be a glycopeptide can be further characterized by performing an MS/MS analysis. In this experiment, the precursor ion is selected, fragmented, and the tandem mass spectrometry data is recorded. Data from an example MS/MS experiment is shown in Fig. 6.

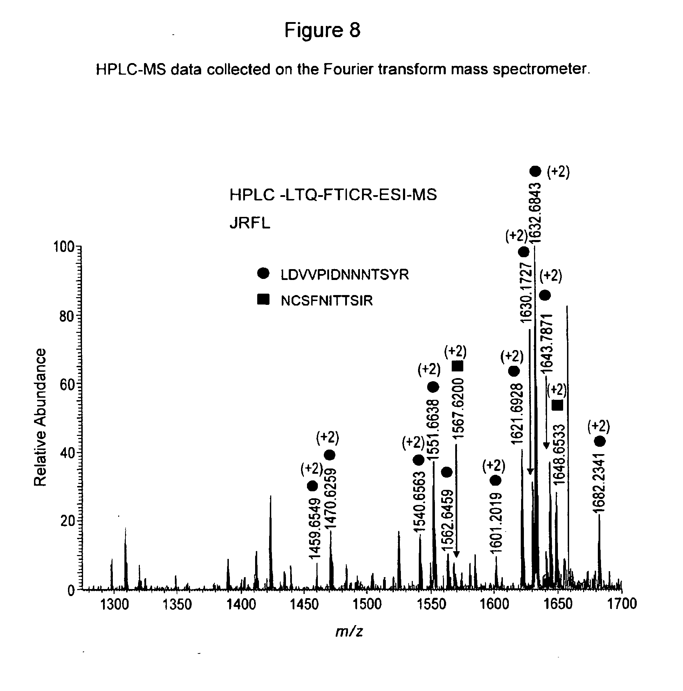

MS data can also be acquired using the LTQ-FTMS, using either online or offline sample preparation methods. In the offline case, the sample is loaded into a nanospray tip for nano-ESI-MS. MS/MS data of any peak that is suspected to be a glycopeptide is acquired, if possible. In the "online" approach MS analysis occurs in parallel with the HPLC separation. In these experiments, a capillary HPLC column is typically used, HPLC conditions can be optimized to provide maximal separation of glycopeptides, and high resolution MS data can be acquired in real-time. An example of MS data acquired in this fashion is in Fig. 8. In addition, automated MS/MS analysis is typically performed, using the data-dependent scanning features of the instrument.

### Data interpretation.

After the mass spectral data is acquired, it is used to discern the carbohydrate moieties attached to each glycosylation site on the glycoprotein of interest. The general strategy for assigning peaks to glycopeptide compositions involves: (1) identifying peaks that are most probably glycopeptides, (2) using the MS/MS data of these peaks (when available) to assign the peptide composition of each glycopeptide, and (3) assigning the full glycopeptide compositions with the aid of *GlycoPep DB,* which is a web-based algorithm designed and maintained at the University of Kansas.

Identifying mass spectral peaks that are most probably from glycopeptides can be done using a variety of strategies. Generally, these peaks are greater than 2000 Da, so one strategy is to attempt to assign any peak that is above this mass threshold. Another approach is to look for a pair or series of ions in the spectra that are separated by the mass of a common monosaccharide unit. For example, hexose is 162 Da, so when pairs of ions are present that are 162 Da apart, these ions can be assumed to be glycopeptides whose mass differs by the mass of one hexose unit. Examples of this approach are shown in Figs. 2, 5, 7, and 8. MS/MS data is also useful in confirming an ion is a glycopeptide. One way to use MS/MS data to verify an ion is a glycopeptide is to look for a pair of large ions that are 120 or 266 Da apart. These two ions correspond to two facile fragmentation products that are commonly generated during tandem mass spectrometry. This approach is shown in Fig. 6. Alternative methods of identifying probable glycopeptide ions based on MS data can also be used.

After a given mass spectral peak is identified as a likely glycopeptide candidate, MS/MS data can be used to assign the peptide composition of the glycopeptide. MALDI TOF-TOF analysis is particularly beneficial for this because the two ions needed to assign the glycopeptide, which are identifiable as a pair of ions that are 120 or 266 Da apart, are typically large ions in the spectrum. Once this pair of ions is identified, they are assigned as the ^{0,2}X and Y_{l,a} ions, for the glycopeptide, and the mass of the peptide is readily calculated, based on that assignment. (See Fig. 6).

After the peptide portion of the glycopeptide is assigned, the final step in the analysis is to use information about the peptide to simplify assigning the carbohydrate portion of the ion. *GlycoPep DB* can be used to do this assignment. *GlycoPepDB* uses the input peptide sequence (or peptide mass) along with its database of previously-characterized carbohydrates to identify mass matches for each peak input from the mass spectra.

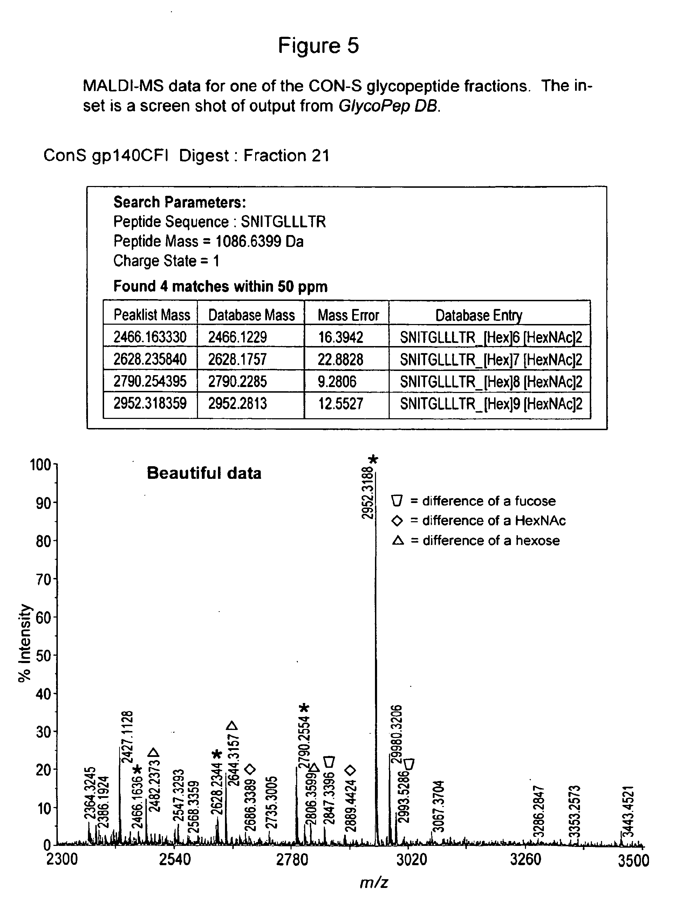

If MS/MS data are not available, it is still possible to obtain peptide compositions from *GlycoPep DB,* using only high resolution MS data. In this case, the user inputs, in an iterative fashion, any possible peptide mass or peptide sequence that could be part of a glycopeptide from the glycoprotein being analyzed. In these cases, *GlycoPep DB* provides all matched glycopeptide compositions that are consistent with the high resolution MS data provided. A screenshot of output from *GlycoPep DB* is shown in Fig. 5. It shows that several peaks in the mass spectrum in Fig. 5 are glycopeptides containing the same peptide sequence. While it is theoretically possible to assign the mass spectra of glycopeptides generated from HIV envelope proteins using other tools, other approaches lead to a higher incidence of incorrect assignments, and require significantly more analysis time.

### Results

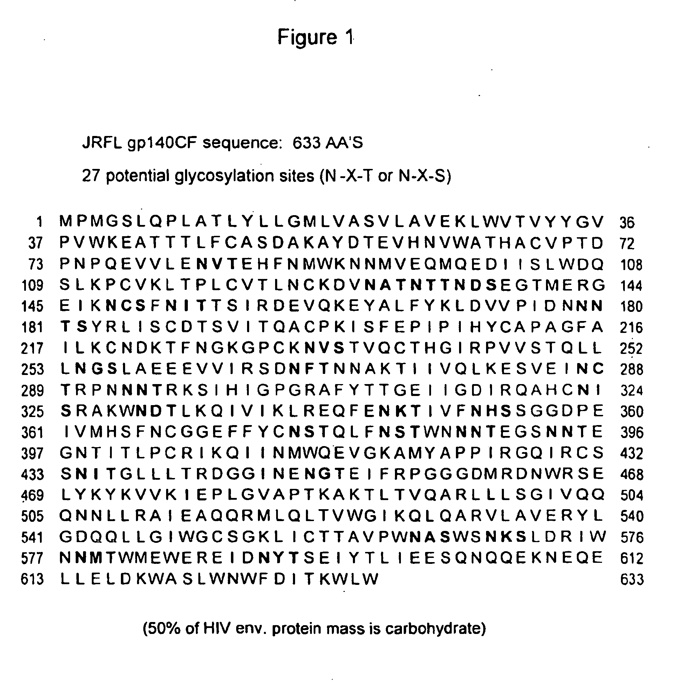

HIV envelope glycoproteins are heavily glycosylated. For example, the JRFL gp140 CF sequence (Fig. 1) has 27 different potential N-linked glycosylation sites (these are shown in red) Identifying these glycan structures represents an important component of several vaccine development strategies.

Since the glycosylation on HIV Env can vary from one glycosylation site to the next, one important aspect of targeting the glycans for vaccine development is being able to identify which structures are present at each attachment site of the protein. Because of the complexity of this protein, novel analytical strategies are needed to accomplish this task. The methods described herein can be used successfully to identify glycosylation at specific sites in HIV envelope proteins. They differ from previous analyses of other glycoproteins in that: (1) the digestion conditions are specifically tailored to provide stringent conditions that are necessary for digestion of this membrane-soluble protein, while simultaneously enhancing the signal of poorly ionizing glycopeptides, (2) the analysis steps are designed to ensure that peaks are not mis-assigned - this is a particularly serious concern with HIV envelope proteins because the sheer number of glycosylation sites leads to an almost-overwhelming number of possible MS peak assignments, therefore, it is critical to have strategies in place to help rule out all "wrong" peak assignments that happen to be the correct mass.

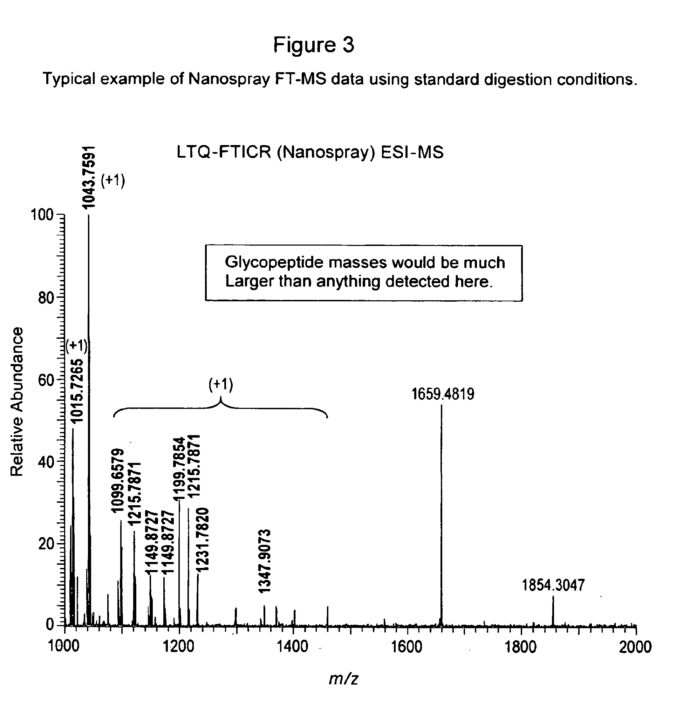

Figs. 2-4 demonstrate the importance of using custom-designed digestion conditions. The conditions used to produce the data in Fig. 2 are standard digestion conditions that are applicable to a wide range of glycoproteins. For HIV envelope proteins, these conditions produce poor signal-to-noise spectra, with few identifiable glycopeptide ions (Fig. 3 demonstrates that the spectral quality is not improved if these experiments are performed on a different type of mass spectrometer). Fig. 4 contrasts these "standard" conditions with another set of conditions that are custom-designed for HIV envelope protein analysis. In Fig. 4, the "new digest conditions" produce much cleaner spectra with more identifiable glycopeptide ions.

Figs. 5 and 6 demonstrate the data analysis approach. After obtaining high-quality, high resolution MS data of the HIV envelope glycopeptides, mass spectral peaks that correspond to glycopeptide ions are identified. In Fig. 5, these are identifiable because the ions are greater than 2000 Da in mass, and they appear in a series in the spectra, where the mass difference among each of the ions in the series differs by the mass of a monosaccharide unit. For example, peaks with an asterisk above them differ by the mass of a hexose monosaccharide unit, 162 Da.

After identifying likely glycopeptide peaks, MS/MS data can be acquired for each ion. An example of this data is shown in Fig. 6. This data is used to verify the peptide portion of the glycopeptide, as described above. This verification process greatly reduces the incidences of inaccurate peak assignments. The importance of assigning this peptide portion was demonstrated using unrelated glycoproteins (Irungu et al, Anal. Chem. 78:1181-1190 (2006)). After the peptide verification is complete, the carbohydrate portion of the glycopeptide can be assigned using *GlycoPep DB,* a web-interfaced algorithm written by my group. Example output of the *GlycoPep DB* algorithm is shown in the in-set in Fig. 5. Use of *GlycoPep DB* also reduces the incidences of incorrect glycopeptide assignments. While individual pieces of this approach have been demonstrated before, the present approach represents a novel global strategy of assigning glycopeptide peaks, where each step of the strategy is designed to limit all possibility of incorrect mass assignments.

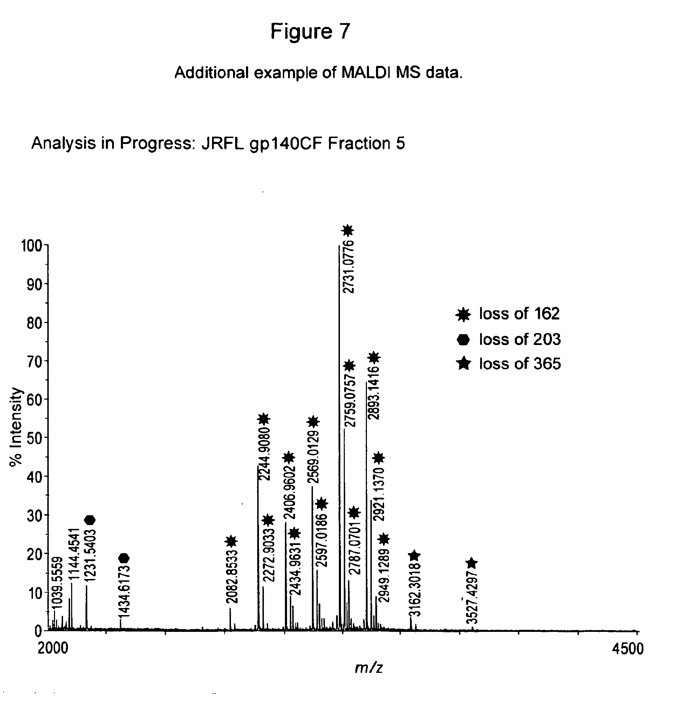

Figs. 7 and 8 represent additional examples of mass spectra of glycopeptide ions of HIV envelope glycoproteins. Fig. 7 was acquired using the same conditions used to generate date in Figs. 5 and 6, while Fig. 8 was acquired using the same digestion procedure but an alternate mass spectral detection strategy. In this case, online HPLC-MS analysis was completed using a different type of mass spectrometer, an FT-MS, as the detection method. The detection strategies described herein, MALDI MS and online HPLC-MS, are both widely used techniques. Both strategies are currently being implemented because they may provide complementary information.

The methods described above differ from other related analyses (e.g., Zhu et al, Biochemistry 39:11194-11204 (2000)), primarily from the standpoint of the data analysis methods used (differences also exist in the sample preparation methods, although these changes are "incremental"). In terms of data analysis, the prior work relied on a combination of tryptic digestion, exoglycosidase reaction with neuraminidase, and glycan release using PNGase F to assign the glycopeptides, and most structures were assigned with a mass accuracy threshold of +/- 0.5% (Zhu et al, Biochemistry 39:11194-11204 (2000)), which is quite poor by today's standards. The main elements that are different in the present approach include: PNGase F and neuraminidase are not required; mass accuracy is 100 times better; each glycopeptide is confirmed by MS/MS experiments; and the biological precedence for all glycan compositions is verified using tools like GlycoPepDB. These changes are necessary to ensure that peaks are not mis-assigned. It has recently been demonstrated (Irungu et al, Anal. Chem. 78:1181-1190 (2006)) that data analysis methods like those used in prior gp120 analysis, where the peptide composition is not independently verified for each assigned peak, can lead to incorrect or ambiguous assignments. The data analysis method described above represents the first example for any glycopeprotein analysis, including all HIV envelope glycoproteins, where three tools (low mass error, independent peptide confirmation, and GlycoPepDB) are combined into a single strategy focused on ensuring the best possible glycopeptide assignments.

### EXAMPLE II

Data for the glycosylation site-specific analysis for CON-S and JR-FL are presented in Tables 1-4. Each row in the Tables represents a unique ion that was identified using mass spectral analysis. Tables 1 and 2 represent data acquired via HPLC fractionation followed by MALDI-MS; and the data in Tables 3 and 4 are from online HPLC-MS analysis using a linear ion trap-Fourier transform mass spectrometer.

Each ion in the Table had to meet several criteria before it was considered to be "properly assigned." First, the mass error of the assigned ion had to be a reasonable value for the specific ion of interest and the instrument used. Mass accuracy was calculated based on the mass of the monoisotopic peak. "Reasonable" mass error was generally less than 160 ppm on the MALDI TOF-TOF and less than 100 ppm on the FT-MS (except when the isotopic peaks could not be resolved, and mass accuracy could not, therefore, be calculated based on the first isotopic peak.) "Typical" mass errors were much lower than the maximum threshold. They were usually in the 20 ppm mass range on the MALDI and 10 ppm range on the FT-MS.

The second criterion was that the assigned carbohydrate had to be biologically relevant. This criteron was generally achieved because the program GlycoPep DB was typically used to generate candidate assignments. All the structures in the GlycoPep DB database have been previously reported in the literature, and so they are biologically relevant. On the rare occasion when a spectrum was assigned manually, only biologically relevant glycosylation assignments were considered.

The final criterion for assuring the accuracy of the assigned peaks was that the peptide portion of each glycopeptide had to be independently confirmed. This confirmation could be achieved in a variety of ways. For the MALDI data, the peptide could typically be confirmed using an MS/MS experiment on the glycopeptide ion. During this experiment, two ions would generally be present, the [^{0,2}X] and [Peptide + H] ions, and they would confirm the mass of the peptide. This information is not required, nor is it possible to obtain, for every ion in the spectrum. Since ions containing the same peptide sequence generally elute in the same time span, the criteria for validating the assignment for any given ion was that the peptide portion of the glycopeptide of interest had to be confirmed present for at least one other ion present in the mass spectrum that corresponded to the fraction of interest.

Occasionally, the ion signal would be too small to perform the MS/MS analysis, or if the peptide was multiply glycosylated, MS/MS data would provide inconclusive results. In these cases, the peptide portion of the glycopeptide could be confirmed in an alternate approach, such as deglycosylating the glycopeptides using PNGase on the glycopeptide fraction. This enzyme cleaves the carbohydrates on the glycopeptide and releases the nonglycosylated peptides present in that fraction, and mass spectral analysis can be used to confirm the identity of the resulting peptides.

For the online LC-MS data, the ion that corresponds to the peptide portion of the glycopeptide is either [Peptide + HexNAc], or if the carbohydrate was fucosylated, the ion would typically be [Peptide + HexNAc + fucose]. Additionally, MS/MS data of the glycopeptides in the LC-MS experiment typically provided additional information about the carbohydrate portion of the glycopeptide, which could also be used to validate the ion's assignment, if the information about the peptide mass was not directly obtainable.

### EXAMPLE III

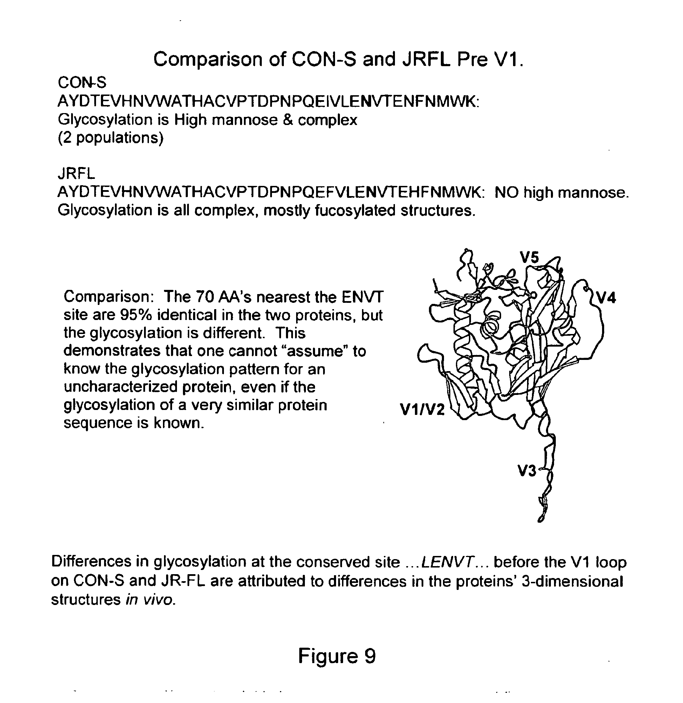

Glycosylation patterns are influenced by the three-dimensional structure of the protein. Comparison of the data for CON-S and JR-FL at the common glycosylation site before the V1 loop (Fig. 9) show that even though the sequence is highly conserved between the two proteins, the glycosylation is varied. This illustrates that glycosylation is not a direct result of primary sequence. Likewise, the difference in glycosylation cannot be attributed to a different cell line, a different purification method, or a different analysis, because both proteins underwent the same conditions. Therefore, glycosylation is most likely due to differences in the 3-dimensional environment around the glycosylation site, as the protein travels through the Golgi apparatus (where its glycosylation is modified) (Fig. 9).

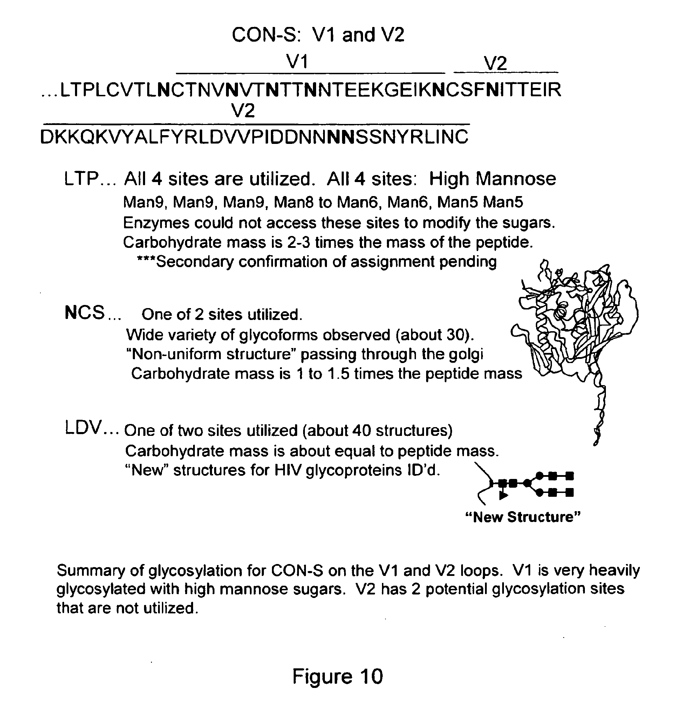

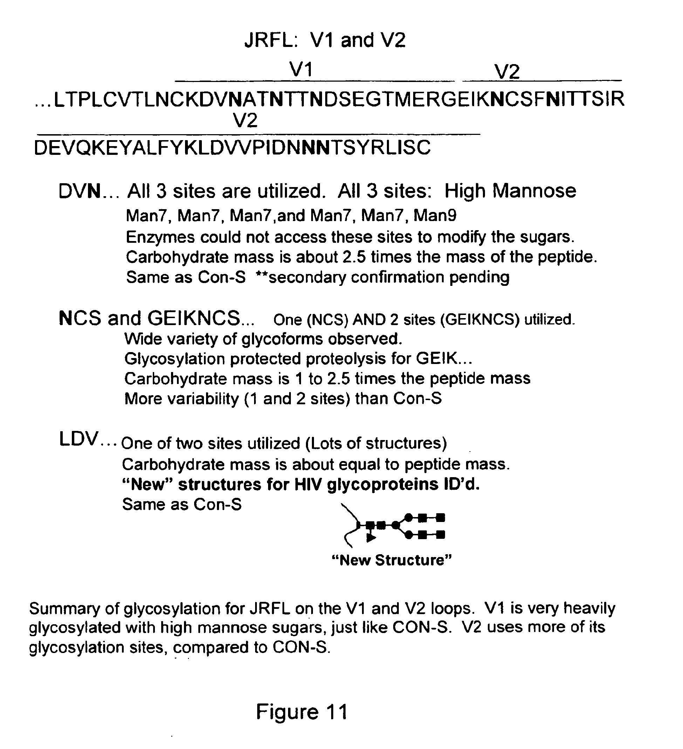

The V1 loop on both proteins contains glycosylation sites with high mannose carbohydrates. This implies that the glycosylation remodeling enzymes in the Golgi apparatus could not access these sugars while the protein was being post-translationally modified. This is one of the most heavily glycosylated regions of the entire protein, with the sugar mass being 2 to 3 times the peptide mass (Figs. 10 and 11).

The V2 loop shows variation between CON-S and JRFL. CON-S has only one site on each of the V2 peptides occupied by glycosylation - which means that one site on each of these peptides is left unoccupied (Fig. 10). (Additional validation tests are being undertaken.) In contrast, JRFL sometimes has both glycosylation sites on the first tryptic peptide of the V2 sequence occupied (Fig. 11). Both JRFL and CON-S show similar glycosylation patterns for the second glycosylated peptide in the V2 region. Here, numerous structures were obtained. Some of these glycans are not previously reported for HIV-containing glycopeptides. Those structures have 4HexNAc's adjoined to the trimannose core, and they are present in fucosylated and nonfucosylated forms (Figs. 10 and 11 and the mass assignment Tables). Since the area at the end of the V2 loop has so many different types of glycans present, this observation would be consistent with a lack of a single, conserved tertiary structure in the protein at this point, since proteins with well-defined tertiary structure generally afford more homogenous glycosylation (See Mir-Shekari et al, J. Biol. Chem., 272:4027-4036 (1997) and references cited therein for examples.)

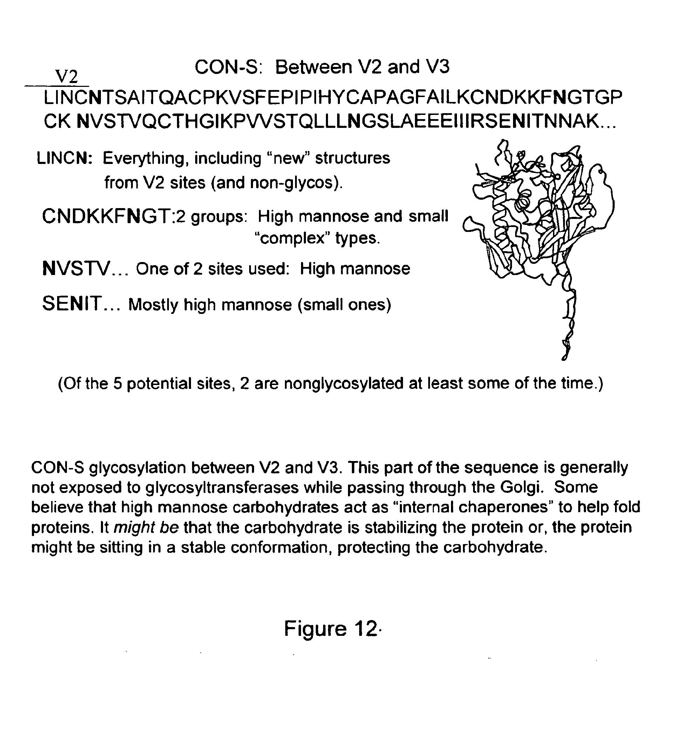

At the very end of the V2 loop in CON-S, many different types of glycoforms were detected, and a portion of the protein was nonglycosylated, demonstrating a very high level of structural heterogeneity (Fig. 12). This contrasts sharply with the protein sequence at the center of the region between V2 and V3 on CON-S. The tryptic peptide in this part. NVSTVQ..., had one site that was not utilized and the second site had only high-mannose containing carbohydrates (Fig. 12). This means that these glycosylation sites were not accessible to enzymes that add and modify carbohydrates. This could be explained by either the carbohydrate acting as an internal chaperone, and folding the protein around it (Jitsuhara et al, J. Biochem. 132:803-811 (2002)); or perhaps the protein was simply folded in such a way as to conceal the glycosylation site. In either case, the glycosylation sites were clearly occluded by protein, because they were not extensively modified by glycosyltransferase enzymes. This rationale is well-accepted for explaining the presence of high-mannose containing carbohydrates on glycoproteins from mammalian systems (Mir-Shekari et al, J. Biol. Chem. 272:4027-4036 (1997)). The other tryptic peptides between V2 and V3 generally follow the same trend: They have a high degree of high mannose containing carbohydrates on them (Fig. 12).

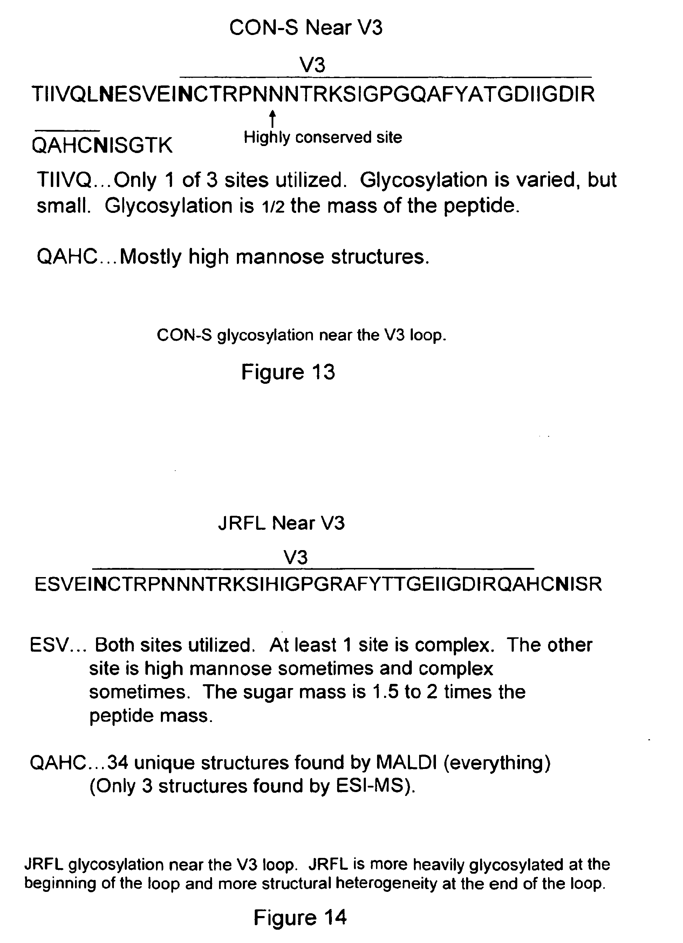

A summary of the glycosylation on the tryptic peptides that comprise the V3 loop is presented on Fig. 13 (for CON-S) and Fig. 14 (for JRFL). The most important observation about this data is that for CON-S, the beginning of the V3 loop contains open glycosylation sites and generally small glycoforms present at the only occupied site. In contrast, JRFL contains glycosylation at both its glycosylation sites at the beginning of the V3 loop. As a result, it seems most likely that it would be easier to induce V3-directed antibodies using CON-S compared to JRFL. For JRFL, the sugar mass is 1.5 to 2 times the mass of its corresponding tryptic peptide (at the beginning of V3); while for CON-S, the sugar mass is half the peptide mass. Clearly, the peptide is more accessible to antibodies in CON-S.

At the end of the V3 loop, a variety of glycoforms are observed in both proteins. Generally, CON-S contains a higher proportion of high mannose structures, while JRFL contains over 30 different glycoforms. This data indirectly indicates that this portion of the CON-S protein is more ordered, and the glycosylation site is generally occluded as the protein is traveling through the Golgi. Conversly, JRFL contains such a high degree of variability in its glycosylation, the only explanation for this is that this portion of the protein does not adopt a consistent structural epitope as it is being post-translationally processed.

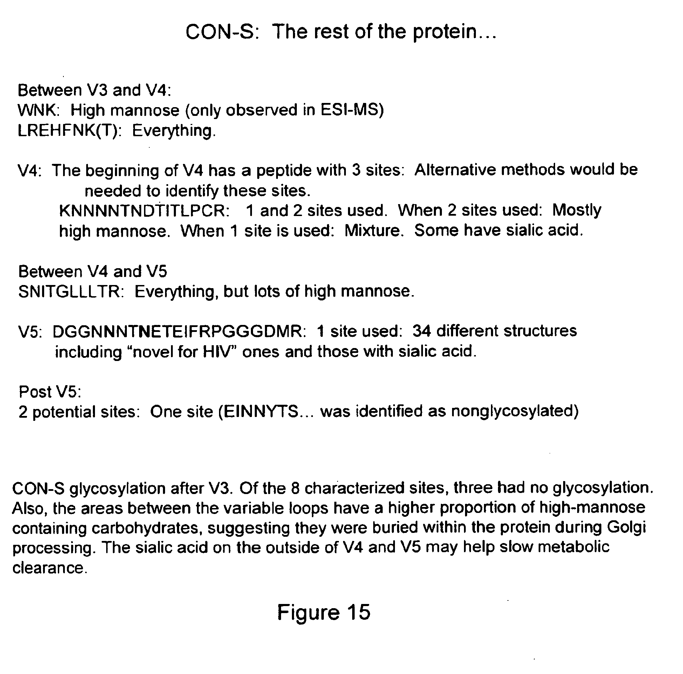

The glycosylation for the rest of CON-S is summarized on Fig. 15. Of the eight remaining characterized sites, 3 of them are nonglycosylated at least some of the time. This is significant because it demonstrates that one cannot assume glycosylation is present on these proteins whenever the consensus sequence N-X-T/S (where X is not proline) is present. Additionally, this data shows that some regions of the protein, particularly between the variable loops, contain high levels of high-mannose containing carbohydrates. This indicates that these sites were buried within the protein as it was being post-translationally modified.

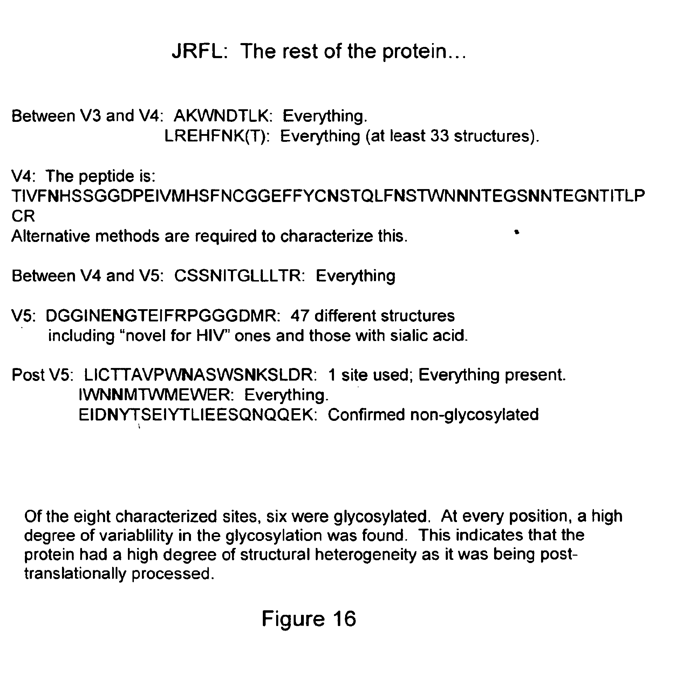

The glycosylation for the rest of JRFL is summarized on Fig. 16. The remarkable observation about this data is that specific trends in glycosylation were not observed. Instead, virtually every type of glycosylation was present at virtually every site. This strongly suggests that a high degree of variability exists in the tertiary structure of the protein. Otherwise, the glycosylation would follow more "typical" patterns that have been previously described, where occluded sites typically contain high-mannose sugars and exposed sites contain fully processed, complex carbohydrates. Since none of these glycosylation sites shows a preponderance of any given type of carbohydrate, the most probable explanation for the data is that a large degree of structural heterogeneity existed, as the protein was passing through the Golgi. Of the eight characterized sites in this part of the protein, two did not contain glycosylation.

This data directly demonstrates that in several cases for both proteins, potential glycosylation sites were not utilized. In fact, 9 of the 27 characterized sites on CON-S were nonglycosylated, at least some of the time. For JRFL, 18 sites were characterized, and of those, 4 were nonglycosylated at least some of the time.

Additionally, trends in glycosylation revealed differences in the proteins' local secondary structure around the glycosylation sites. Most notably, JRFL did not display a consistent trend in glycosylation for almost all of its sites. Instead, it contained minimally processed, high mannose, type carbohydrates, along with extensively processed complex carbohydrates, at every characterized site past V3. By contrast, CON-S showed more "traditional" glycosylation, where interior regions, like those between V2 and V3, between V3 and V4, and between V4 and V5, showed a higher preponderance of minimally processed glycans. These structures indicate that the glycosylation sites on CON-S were occluded during post-translational modification. By contrast, JRFL *did not* have the same conserved structural motifs occluding these glycosylation sites, as indicated by its large variability in glycosylation. It contains a more promiscuous secondary structure.

The correlation between glycosylation type and the glycosylation sites' immediate environment has been previously established in the literature, but this is the first time that the information flow is occurring backwards. That is, the glycosylation data is used to infer information about the surrounding secondary structure of the glycosylation sites. The information obtained from this process *provides in vivo* information about the structural heterogeneity of the protein, and the accessability of the area immediately surrounding the glycosylation sites, as the protein travels through the Golgi. It is possible that CON-S is a better immunogen than JRFL because it has more conserved structural motifs. Perhaps these conserved structural features are necessary to provide conformational epitopes that effectively elicit neutralizing antibodies.

### EXAMPLE IV

### Experimental Details

*Materials and reagents.* All reagents were obtained in high purity from Sigma-Aldrich except when noted otherwise. Ammonium bicarbonate (NH₄HCO₃), trizma hydrochloride, trizma base, ethylenediaminetetraacetic acid (EDTA), phosphate buffered saline solution (PBS), acetic acid, HPLC grade acetonitrile (CH₃CN) and methanol (CH₃OH) 2,5-dihydroxybenzoic acid (DHB), urea, α-cyano-4-hydroxycinnamic acid (CHCA), iodoacetamide (IAA), Sepharose® CL-4B, butanol, ethanol, dithiothreitol (DTT), trifluoroacetic acid (TFA), and formic acid were purchased from Sigma (St. Louis, MO). Water was purified using a Millipore Direct-Q3 Water Purification System (Billerica, MA). Sequencing grade trypsin (Tp) and N-Glycosidase F (PNGase F) from *Elizabethkingia meningosepticum* were obtained from Promega (Madison, WI) and Calbiochem (San Diego, CA), respectively.

*Expression and purification of envelope glycoproteins (JR-FL and CON-S).* All Env proteins were expressed and purified from the Duke Human Vaccine Research Institute in Durham N.C. The Env proteins were constructed, expressed and purified using the method described in literature (Gao et al, J. Virol. 79:1154-1163 (2005), Liao et al, Virology 353:268-282 (2006)).

*Tryptic digestion.* Samples containing 300 µg aliquots of the HIV-1 Env proteins, JR-FL gp140ΔCF and CON-S gp140ΔCFI, were denatured with 6M urea in 100 mM tris buffer (pH 7.5) containing 3 mM EDTA. The proteins were reduced and alkylated with 15 mM DTT at RT for an hour and 40 mM IAA at RT in the dark for another hour, respectively. The samples were brought to a final concentration of 50 mM DTT to neutralize excess IAA. Proteins were then digested at 37°C with trypsin at a protein:enzyme ratio of 30:1 (w/w) overnight, followed by a second trypsin digestion under the same conditions. The resulting HIV Env protein digest mixture was either subjected to off-line reversed-phase high performance liquid chromatography (RP-HPLC) fractionation for MALDI analysis or RP-HPLC ESI-FT MS analysis.

*Offline RP-HPLC fractionation.* A 20 µL aliquot of the HIV Env protein digest mixture was injected onto a C18 column (150 mm × 4.6 mm, 5 µm size column particle, Alltech, Deerfield, IL). Mobile phases utilized for the fractionation were 99.9% deionized H₂O + 0.1% formic acid B: 99.9 % CH₃CN + 0.1% formic acid. The following CH₃CN/H₂O multistep gradient was used to elute the glycopeptides from the column at a flow rate of 1 mL/min: 5% mobile phase B for 3 min, followed a linear increase to 40% B in 15 min, 15 min hold at 40% B then a linear increase to 85% B in 20 min. The column was then held at 85% B for 7 minutes before re-equilibration. Fractions were collected every minute for 60 minutes. The HPLC fractions were dried in a centrivap (Labconco Corporation, Kansas City, MO) and reconstituted with 10 µL H₂O. A total of 60 fractions were analyzed by MALDI-MS.

*N-deglycosylation.* HIV glycopeptide enriched fractions were deglycosylated using N-Glycosidase F (CalBioChem) utilizing the protocol recommended by the manufacturer. Briefly, solution containing 500 units/mL of N-Glycosidase F was prepared by 100 µL of deionized H₂O to 50 units of lyophilized enzyme in a vial. Glycans were released in each glycopeptide enriched fraction by adding 25 µL of 20 mM NH₄HCO₃ (pH = 8) and 4 µL ofN-Glycosidase F solution. The reaction was incubated overnight at 37°C and was stopped the following day by heating the sample to 100°C. The resulting solution was subsequently analyzed by MALDI-MS.

*Mass spectrometry and liquid chromatography.* MALDI MS and MS/MS experiments were performed on an Applied Biosystems 4700 Proteomics Analyzer mass spectrometer (Foster City, CA) operated in the positive ion mode. Samples were prepared by mixing equal volumes of the analyte and matrix solutions in a microcentrifuge tube, then immediately deposited on a MALDI plate, and allowed to dry in air. The matrix solution was prepared by mixing equal volumes of saturated solutions of CHCA in 50% CH₃CN in H₂O with 0.1% TFA and DHB in 50% CH₃CN in H₂O. Samples were irradiated with an ND-YAG laser (355 nm) operated at 200 Hz. This instrument was equipped with automated and multisampling capabilities for rapid sample analysis. Mass spectra were acquired in both reflectron and linear ion modes and were generated by averaging 3200 individual laser shots into a single spectrum. Each spectrum was accumulated from 80 shots at 40 different locations within the MALDI spot. The laser intensity was optimized to obtain adequate signal-to-noise (S/N) ratio and resolution for each sample. MALDI MS/MS data were acquired using a collision energy of 1 kV with nitrogen as collision gas.

LC/ESI-FTICR MS and MS/MS experiments were performed using a hybrid linear ion-trap (LIT) Fourier transform ion cyclotron resonance mass spectrometer (LTQ-FT, ThermoElectron, San Jose, CA) directly coupled to Dionex UltiMate capillary LC system (Sunnyvale, CA) equipped with FAMOS well plate autosampler. Mobile phases utilized for the experiment consisted of buffer A: 99.9% deionized H₂O + 0.1% formic acid and buffer B: 99.9 % CH₃CN + 0.1% formic acid which were pumped at a flow rate of 5 µL/min. Samples were injected into C18 PepMap™ 300 column (300 µm i.d. x 15 cm, 300 A, LC Packings, Sunnyvale, CA). After loading 5 µL of sample, glycopeptides were eluted at a flow rate of 5 µL/min using the same gradient described above. A short wash and blank run were performed between every sample to ensure that there was no sample carry-over. The hybrid LIT-FT-MS was operated in a data-dependent scanning mode with scan event details as follows: A full FT-MS scan within the mass range m/z 800-2000 followed by three data dependent MS/MS scans of the three most intense glycopeptide molecular ions from the full MS scan were sequentially and dynamically selected for subsequent collision-induced dissociation (CID) in the LTQ linear ion trap using a normalized collision energy of 35% and a 3 min dynamic exclusion window. If the data dependent MS/MS scan detects a neutral loss corresponding to monosaccharide units (hexose or HexNAc), an MS³ scan event is triggered for the parent ion. The temperature for the capillary of the ion source was set at 200 °C, ESI voltage of 4.0 kV, scanning in the positive ion mode.

*Glycopeptide identification.* Tandem mass spectra generated from MALDI MS and ESI-MS analyses were analyzed using GlycoPep DB (Go et al, Anal. Chem. 79:1708-1713 (2007)) and GlycoPep ID (Irungu et al, Anal. Chem. 79:3065-3074 (2007)) to elucidate the HIV Env protein tryptic glycopeptide composition. The details of the glycopeptide compositional analysis have been described previously. Briefly, the peptide portion is determined using the collision induced dissociation (CID) data. For MALDI MS analysis, CID data from glycopeptide enriched fractions were inspected manually to identify the characteristic signature fragment ions of the cross-ring cleavage, ^{0,2}X ion. This ion was used to verify the peptide portion of the glycopeptide. Once the peptide was identified, the peak list of the MS data from the fraction in which the peptide sequence was ascertained is searched against the all carbohydrate entries in the GlycoPep DB database. The query result generates all plausible glycopeptide compositions which are subsequently refined and verified by evaluating the MS and MS/MS data. The compositional assignment for ESI FTICR-MS data is realized using GlycoPep ID to determine the peptide portion of the glycopeptide and GlycoPep DB for glycopeptide composition. Identification of peptide portion is facilitated using GlycoPep ID in which a peptide prediction table is generated from a theoretical digest of the glycoprotein of interest with their corresponding sequence and *m*/*z* values as well as a list of predicted *m*/*z's* of the cross-ring cleavages, ^{0,2}X or ⁰Y₁ ions.

### Results

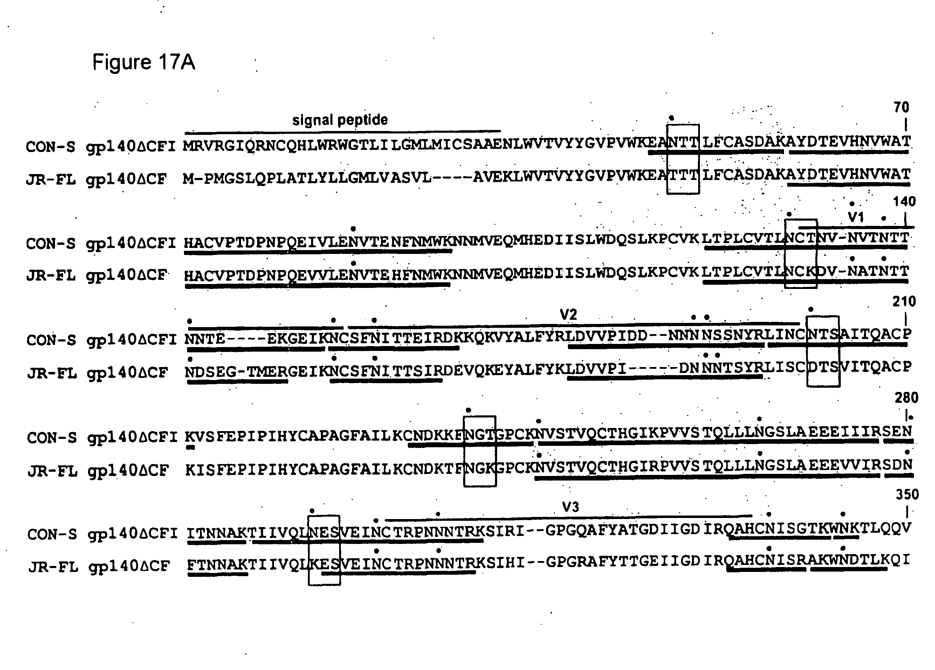

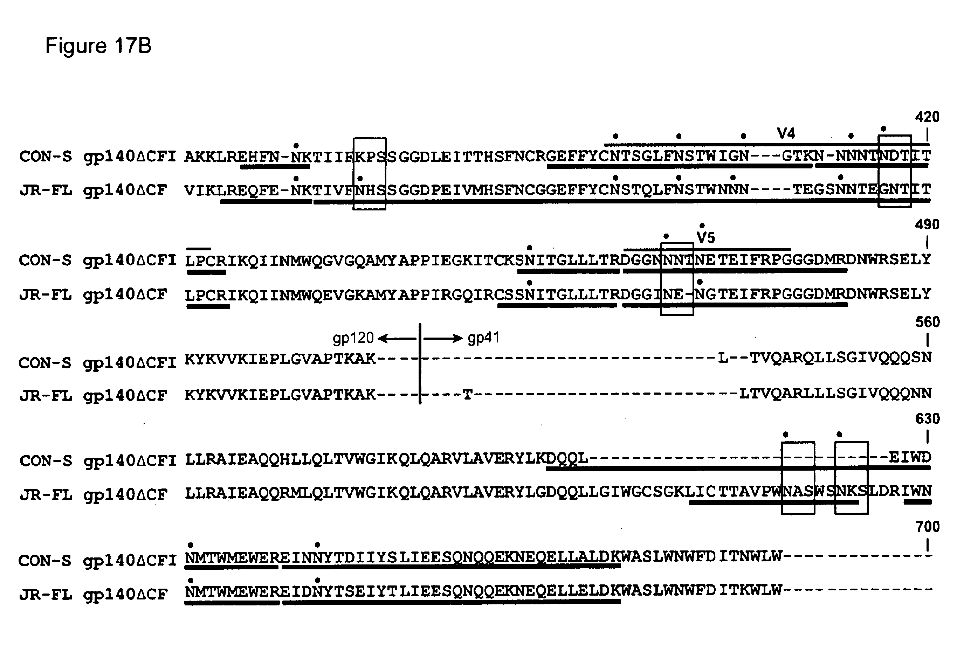

*CON-S and JR-FL Envelope Glycoproteins.* Modified forms of the synthetic Env immunogen, CON-S, representing the group M and the wild-type clade B Env protein, JR-FL were used in this study due to the marked improvement in immunogenicity and the protein's ability to express soluble oligomeric protein (Chakrabarti et al, J. Virol. 76:5357-5368 (2002), Gao et al, J. Virol. 79:1154-1163 (2005), Liao et al, Virology 353:268-282 (2006)). Based on the full length sequence of gp 160 of the reference HIV strain, HXB2, the gene construct of the modified form of CON-S (gp140ΔCFI) was constructed with three internal deletions that included the gp120/gp41 proteolytic cleavage site (C, residues 510-511), fusion domain of gp41 (F, residues 512-527), and the region between two heptad repeats (residues 546-579, 628-655) in the immunodominant region (I) and shortened variable loops whereas the modified form of JR-FL (gp140ΔCF) lacks the gp120/gp41 proteolytic cleavage site (C) and the fusion domain (F) of gp41 ((Chakrabarti et al, J. Virol. 76:5357-5368 (2002)). Both of the Env immunogen were lectin purified and were propagated in rVV (Gao et al, J. Virol. 79:1154-1163 (2005), Liao et al, Virology 353:268-282 (2006)). The full sequence alignment of CON-S gp140ΔCFI and JR-FL gp140ΔCF is shown in Figure 17 with the potential glycosylation sites shown in red (see "dotted" sites). The protein sequence of CON-S gp140ΔCFI and JR-FL gp140ΔCF is 81% identical. As can be seen in Figure 17, the Env proteins differ in nine potential glycosylation sites. Missing potential glycosylation sites in JR-FL gp140ΔCF but present in CON-S gp140ΔCFI and vice versa are shown in boxes. Glycosylation analysis using N-glycosite (http:// www.hiv.lanl.gov/) reveals 31 and 27 potential glycosylation sites for CON-S gp140ΔCFI and JR-FL gp140ΔCF, respectively. Throughout the text, CON-S gp140ΔCFI and JR-FL gp140ΔCF are referred as CON-S and JR-FL, respectively.

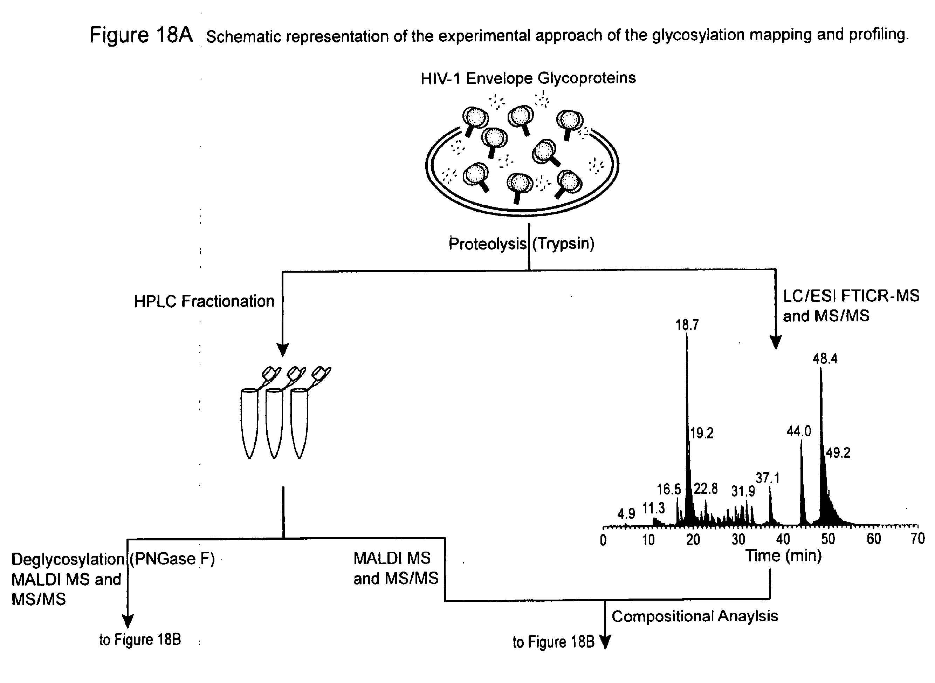

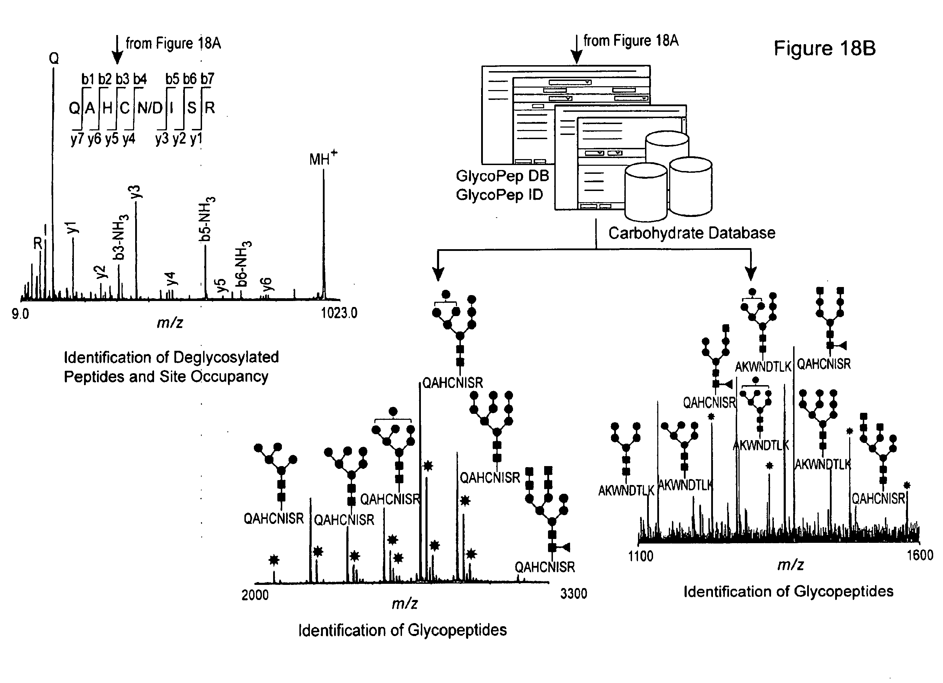

*Glycosylation Mapping by Mass Spectrometry.* The global mapping and comprehensive identification of glycosylation using glycopeptide-based MS analysis is perhaps one of the most challenging tasks in glycoproteomics. This difficulty mainly stems from the low ionization efficiency of glycopeptides, the extensive glycan heterogeneity at each glycosylation site, the relatively lower glycopeptide concentration compared to peptides, and the complexity of data analysis. Central to any successful MS analysis is the design and choice of sample preparative aides. Recently, thorough evaluation was performed of several chromatographic and enrichment methods used for glycopeptide-based MS analysis. They were optimized to markedly improve the glycopeptide coverage (Zhang et al, in submission (2007)). Accordingly, an optimized sample preparation method was tailored that is well-suited for the analysis of the extensively glycosylated recombinant HIV Env proteins, CON-S and JR-FL. Figure 18 provides a schematic representation of the experimental template used in this study. The approach was to integrate both MALDI-MS and LC/ESI FTICR-MS analyses to obtain a global profile of the glycosylation and distinguish differences in glycosylation profile between two Env immunogens.

CON-S and JR-FL both have ~600 amino acid residues. The reduced Env proteins have 15 cysteine residues, which were alkylated at the protein level by reacting the cysteine residues with IAA producing carbamidomethylated Env proteins. The carbamidomethylated Env proteins were subjected to all operations typical of an in-solution trypsin digestion generating about 100 possible tryptic fragments when allowing for up to one internal trypsin cleavage site (single missed cleavage). After digestion with trypsin, two separate aliquots of the glycoprotein digest were either subjected to HPLC fractionation for MALDI-MS analysis or LC/ESI FTICR-MS analysis (Figure 18). For both MS experiments, glycopeptides were separated within an 80-minute gradient. Out of the 60 HPLC fractions collected for each sample for MALDI analysis, there were 36 and 38 fractions that contained glycopeptides for JR-FL and CON-S, respectively. These fractions were subjected to MS/MS analysis to deduce the glycopeptide composition present in each fraction. A portion of each glycopeptide fraction was treated further with PNGase F to validate the peptide assignment and determine which of the potential glycosylation sites were occupied (Figure 18). Glycopeptide analysis using LC/ESI-FTICR-MS and MS/MS analysis was performed using data dependent acquisition mode with the hybrid LTQ linear ion trap. Glycopeptides were identified by locating clusters of peaks whose characteristic mass difference corresponds to the masses of the monosaccharide units (hexose, HexNAc, etc.) in the ESI FTICR-MS data. All of the 31 and 27 potential glycosylation sites for CON-S and JR-FL have been identified from the analysis (Table 5).

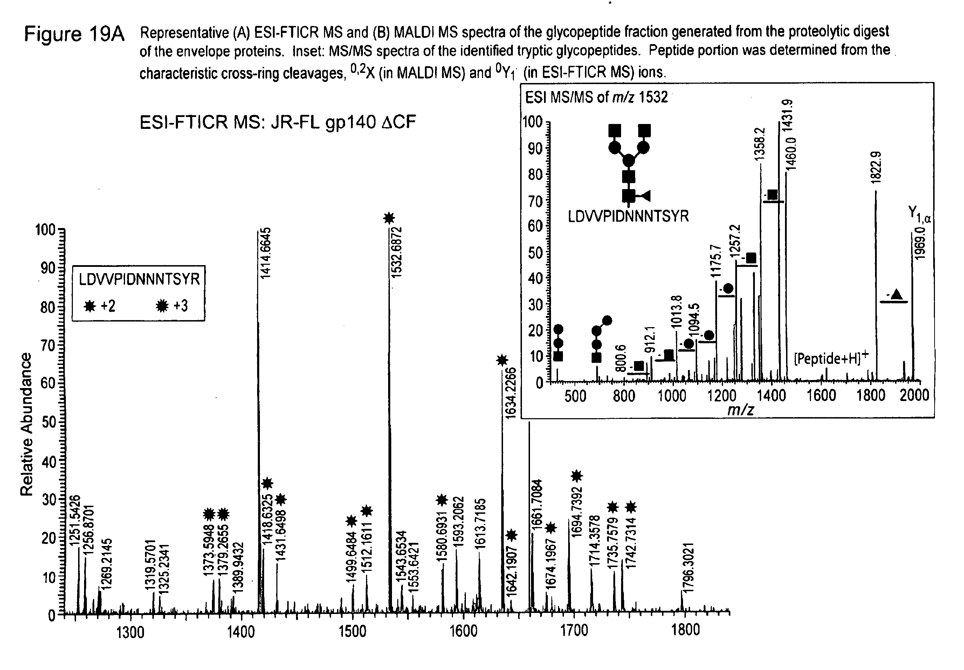

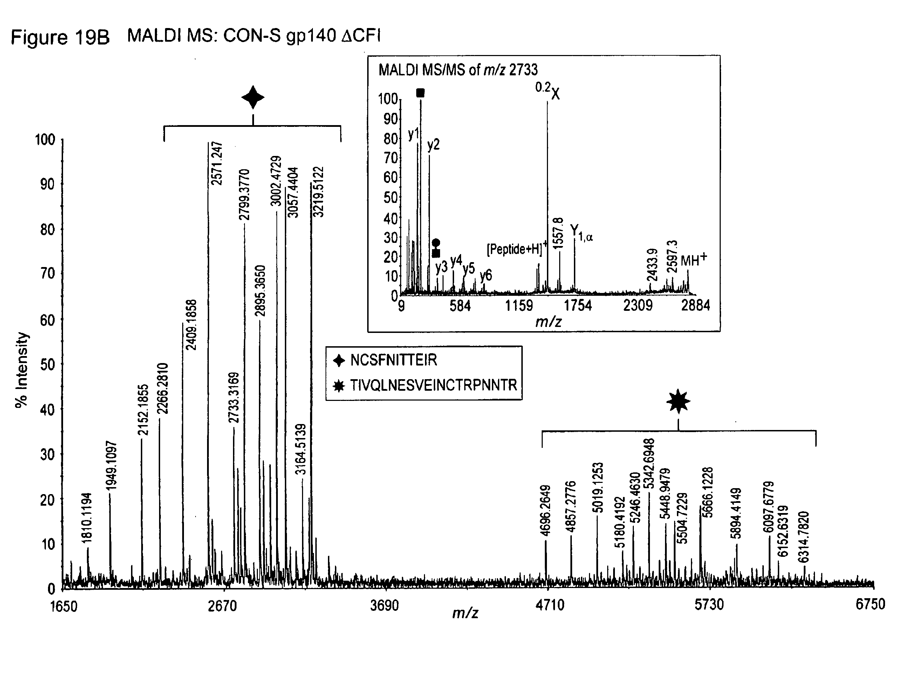

*Glycosylation Profiles of CON-S and JR-FL.* A total of 19 and 16 tryptic glycopeptides with both single and multiple glycosylation sites were identified for CON-S and JR-FL, respectively (see Figure 17). The glycosylation profiles, which include the glycan motif and site occupancy, were compared for CON-S and JR-FL. A partial list of the glycopeptide compositions is shown in Table 6 and the complete list is found in Tables 7 and 8. Representative MALDI MS and LC/ESI-FTICR MS and the corresponding tandem MS spectra of the glycopeptide fractions shown in Figure 19 depict the tryptic glycopeptides in the V2 and V3 regions for JR-FL (Figure 19A) and CON-S (Figure 19B), respectively. Glycopeptide compositions were deduced after identifying the peptide portion of the glycopeptide from MS/MS data (Figure 19 inset) and the glycan portion was verified using the mass list from each spectrum. Compositional analysis generated ~500 putative identifications for each immunogen per MS technique used. The query results were refined by evaluating the MS spectra manually and further confirming the assignments with all the available MS/MS data. Overall, ~400 unique glycopeptide compositions per immunogen have been identified consisting of high mannose, hybrid, and complex type N-linked glycans. This high level of coverage of glycosylation heterogeneity is unprecedented.

To differentiate the glycosylation motif between the synthetic and wild-type Env immunogen and facilitate analysis, the glycoforms were broadly categorized into two groups- namely high mannose and processed glycans. High mannose glycans consist of mannose-containing structures with 5-9 mannose sugars whereas processed glycans include all hybrid and complex type structures. The processed glycans were counted according to the following criteria: hexose (Hex) ≥3 and N-acetylglucosamine (HexNAc) ≥ 4 or Hex ≥ 4 and HexNAc ≥ 3. Figure 20A shows a summary of the glycosylation data populating each site. A closer look indicates that the CON-S and JR-FL differ in their site occupancy and the glycosylation pattern particularly surrounding the immunodominant V3 loop. To elucidate how these two differences in glycosylation profile can affect the immunogenicity of the Env proteins, a detailed comparison of the glycosylation of the Env proteins was made and its implication to vaccine design are discussed below.

### A. Open Glycosylation on CON-S and JR-FL

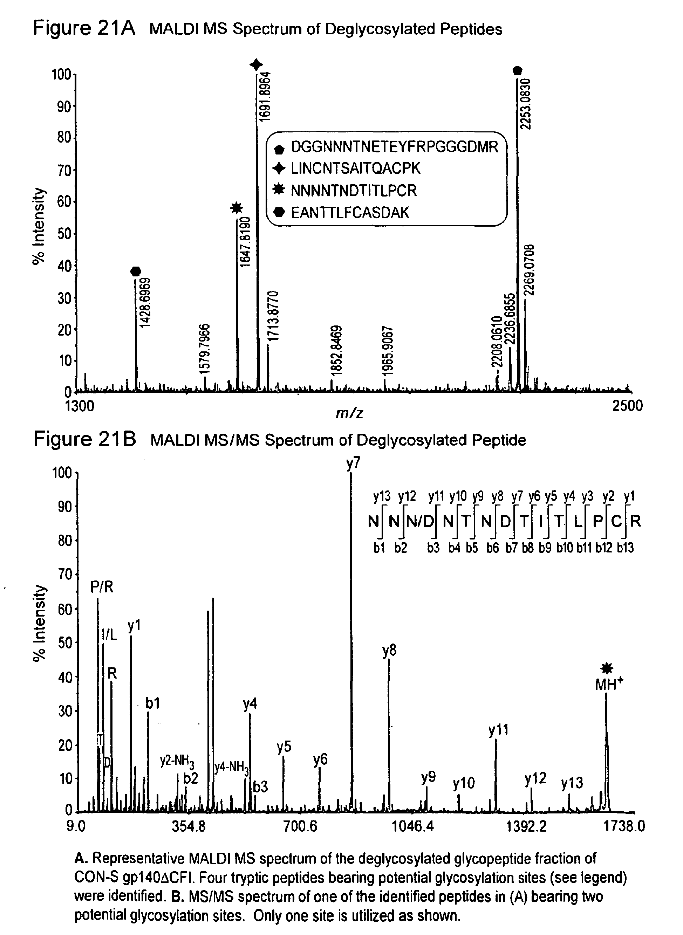

The identified glycopeptides isolated from the tryptic digests of CON-S and JR-FL accounted for 31 and 27 potential glycosylation sites, respectively. While glycopeptide mass mapping experiment allowed for the identification of glycopeptides, it can not directly predict the site occupancy. Several identified glycopeptides contain more than one glycosylation site (see Table 5). To identify which sites were occupied on these glycopeptides, the glycopeptide were enzymatically deglycosylated to determine the site occupancy (Morelle et al, Proteomics 6:3993-4015 (2006), Morelle and Michalski, Nat. Protoc. 2:1585-1602 (2007), Tarentino et al, Biochemistry 24:4665-4671 (1985), Tarentino and Plummer, Methods Enzymol. 230:44-57 (1994)). The enzymatic release of glycans converts asparagine in the NXT/S to aspartic acid resulting in a mass shift of 1 Da per site. Figure 21A contains representative MS data of a deglycosylated glycopeptide fraction for CON-S. Four deglycosylated glycopeptides exhibiting a mass increment of 1 Da each were detected, indicating that each of these glycopeptides contains one utilized glycosylation site. Tandem MS analyses of these peptides were performed to validate the peptide sequence as well as to determine site utilization for peptides with multiple glycosylation sites. MS/MS data (Figure 21B) of the tryptic peptide, NNN⁴¹³NTN⁴¹⁶DTITLPCR, in the V4 loop shows which of the two potential glycosylation sites is occupied. The N⁴¹³NT site is occupied, as evidenced by the fact that this asparagine has been converted to aspartic acid. The second site, N⁴¹⁶DT, must be unutilized, since the MS/MS data clearly indicates that this residue is still an asparagine, after PNGase F treatment. Analysis of another glycopeptide fraction containing this peptide shows that both N⁴¹³T and N⁴¹⁶DT are utilized. Thus, N⁴¹⁶DT is variably occupied as both glycosylated and non-glycosylated asparagine (N⁴¹⁶) were identified by MALDI MS from PNGase F treated glycopeptide fractions. Overall, the results revealed that out of the 31 potential glycosylation sites detected for CON-S (Table 5), glycosylation sites at N 141 in the C1-V1 region, N 191 in the V1/V2 region, N631 and N643 in the transmembrane region were not utilized at any time and nine sites which include N135 in C1-V1 region, N 159 and N201 in the V1/V2 region, N245 and N293 in the conserved region 2 (C2), N305 in the V3 region, N344 in the conserved region 3 (C3), N416 in the V4 loop, and N466 in the V5 loop were variably utilized (Figure 20B, top). For JR-FL, the sites at N138 in the V1 region, N192 in the V1/V2 region, and two sites at N617 (or 622) N643 in the transmembrane region were not utilized and the site at N 159 was variably utilized (Figure 20B, bottom). It should be noted that for the long peptide portion in the V4 region, two sites are occupied out of five potential glycosylation sites. It was not possible to determine which sites were occupied by MS/MS experiment due to the inherent low ionization efficiency of this high mass species. Table 5 shows the list of identified tryptic glycopeptides with open and variable glycosylation sites for both CON-S and JR-FL.

The observed variation in the degree of glycosylation occupancy for both immunogens can be attributed to several factors that commonly affect the efficiency of protein glycosylation. It is known that the extent of protein glycosylation is governed by the primary structure of X in the consensus glycosylation site, NXT/S, the amino acid residue flanking the NXT/S, the structural conformation of the local environment surrounding NXT/S, and the availability of the array of key glycosylating enzymes during glycan biosynthesis/processing (Jones et al, Biochem. Biophys. Acta 1726:121-137 (2005), Kornfeld and Kornfeld, Annu. Rev. Biochem. 54:631-664 (1985), Petrescu et al, Glycobiology 14:103-114 (2004)). Close examination of the amino acid sequence surrounding NXT/S for each glycopeptide in Table 5 indicates that the heterogeneity in site occupancy can be attributed to the conformation of local environment surrounding NXT/S and the amino acid at position X. For instance, the absence of glycosylation on the second asparagine residue for the glycopeptide, LDVVPIDDNNN¹⁹⁰N¹⁹¹SSNYR, in the V2 region for CON-S is most likely due to steric occlusion. The same trend is observed for a similar glycopeptide on JR-FL. After the transfer of N-linked glycans on N190 (CON-S) or N191 (JR-FL), the glycan addition to N191 or N192 is not favorable due to steric hindrance. Inefficient glycosylation at N135, N141, N159, N245, N293, N305, N416, N466, and N631 for CON-S and N138 and N159 for JR-FL may also be due to steric hindrance of occupied glycosylation sites in close proximity. Glycosylation efficiency is also regulated by the presence of large hydrophobic residues (W, L, F, and Y) and negatively charged residues (E and D) at position X in the consensus, NXT/S (Mellquist et al, Biochemistry 37:6833-6837 (1998), Shakin-Eshleman et al, J. Biol. Chem. 271:6363-6366 (1996)). Thus, the lack of glycosylation at N643 for both CON-S and JR-FL located at the transmembrane region is likely influenced by the presence of tyrosine (Y) at the X position. Inefficient glycosylation at the variably occupied site at N293 before the V3 region and N466 in the V5 region for CON-S is most likely influenced by the presence of glutamic acid (E) at the X position.

In addition to the primary sequence effecting glycosylation site occupancy, it is also quite possible that the cell line used to express the protein contributes to the number of open glycosylation sites in CON-S and JR-FL. Previous analyses, which have shown that all the potential glycosylation sites on Env are occupied, were conducted on proteins expressed in CHO cells (Leonard et al, J. Biol. Chem. 265:10373-10382 (1990), Zhu et al, Biochemistry 39:11194-11204 (2000)). In contrast, JR-FL and CON-S were both expressed in 293-T cells. Other glycoproteins have been described that contain dramatically different glycosylation patterns, when the proteins are obtained from different mammalian species (Dalpathado et al, Biochemistry 45:8665-8673 (2006), Liedtke et al, Glycoconj. 14:785-793 (1997)). If the differences in cell line used contribute to the differences in glycosylation site occupancy in Env, this would imply that changing cell lines for an immunogen could very likely effect its glycosylation profile and, as a result, its immunogenicity.

The existence of open and variable glycosylation can directly affect the immunogenicity of the Env proteins. Indeed, the susceptibility of the virus towards antibody neutralization is dependent on whether the unutilized sites could either effectively or ineffectively expose key protein epitopes. An increase in antibody neutralization was observed when the glycosylation sites in the V3 regions and near the receptor binding region were deglycosylated (Koch et al, Virology 313:387-400 (2003), Kolchinsky et al, J. Virol. 75:3435-3443 (2001)). The results show that there are more open sites surrounding the V1/V2, C2, V3 regions for CON-S, compared to the less effective immunogen, JR-FL.

### B. Glycosylation and Protein Structure

In addition to determining whether or not the glycosylation sites are occupied, glycosylation analysis also provides some insight into the three dimensional (3D) conformation of the protein, and its structural heterogeneity in *vivo.* Given that the appended carbohydrates could either be heavily processed or not depending on the site's accessibility to key glycosylating enzymes, the variability in glycosylation processing provides a probe of the protein's local structure at the glycosylation sites. High mannose glycans indicate minimal processing and a more protected local 3D structure, whereas fully processed glycans indicate that the glycosylation site was readily accessible to glycosyltransferase enzymes in the Golgi. In fact, high mannose glycans have been previously shown to help stabilize the local 3D conformation of proteins (Jitsuhara et al, J. Biochem. (Tokyo) 132:803-811 (2002), Nishimura et al, J. Biochem. (Tokyo) 123:516-520 (1998), Petrescu et al, Clycobiology 14:103-114 (2004)). Of course, Env immunogens must be able to adapt stable 3D conformations so they can provide conserved structural epitopes that elicit strong immunological response. To probe differences in protein structure between JR-FL, a poor immunogen, and CON-S, a more effective immunogen, two key regions/segments are highlighted where glycosylation is different between the two proteins.

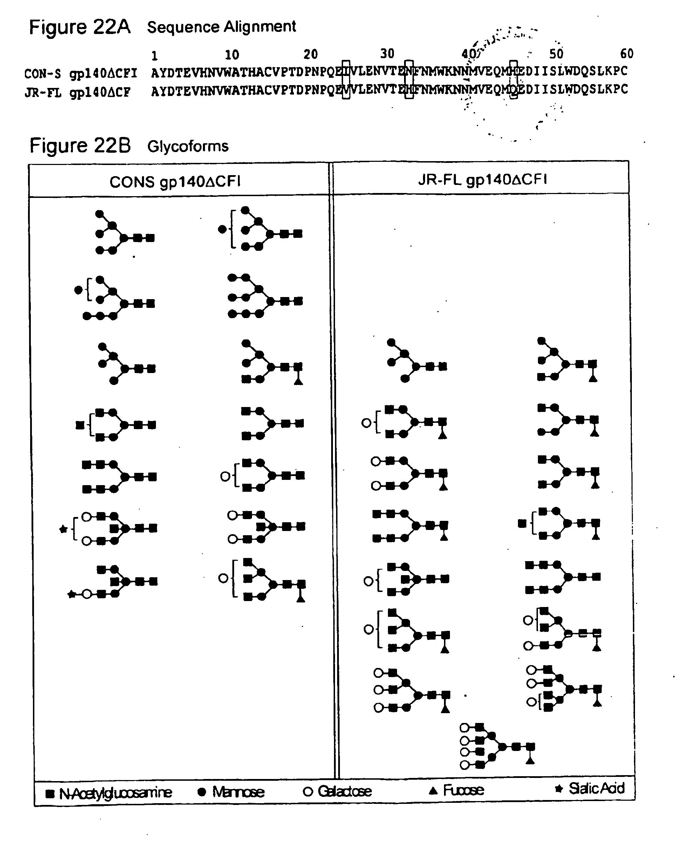

The first region where the difference in glycosylation was observed is the region before V1. The glycosylation site is located in the first conserved (C1) region. The sequence alignment of the 60-residue segment before and after the glycosylation site of CON-S and JR-FL is 95% identical (Figure 22A). Since the two sites have identical sequence, one would reasonably expect that these sites are equally exposed and accessible to glycosylation enzymes. However, the glycosylation data clearly shows different glycan motifs on CON-S and JR-FL (Figure 22B). CON-S glycans consist of high mannose structures with 5-9 mannose (Man) sugars and processed glycans which are mostly complex type structures with some degree of sialylation and a minimal amount of fucosylation. In contrast, JR-FL glycans consist of a single high mannose structure (Man5) and greater number processed glycoforms, most of which have been fucosylated more than CON-S. It should be noted that both immunogens are rVV expressed proteins and have undergone the same sample processing prior to MS analysis, so the expression and analysis conditions cannot be used to explain the glycosylation differences detected, and neither can the primary sequence. The results suggest that the difference in glycosylation is dictated by the structural conformation of the local environment surrounding the glycosylation site. Certain conformations would be more favorable for processing - in this case, JR-FL's higher degree of processing indicates that it must have a different 3D structure at this site, compared to CON-S. The relatively low accessibility of the same glycosylation site for CON-S evidenced by the presence of more high mannose structures implies that this site is partially buried in the folded protein. Thus, it is likely that this particular region for CON-S has lower protein flexibility allowing more stable 3D conformation in this region. This hypothesis is supported by the precedent that the extent of glycosylation of a particular site reflects the particular local conformation of the protein surrounding the glycosylation site (Rudd and Dwek, Crit. Rev. Biochem. Mol. Biol. 32:1-100 (1997), Scanlan et al, Nature 446:1038-1045 (2007)).

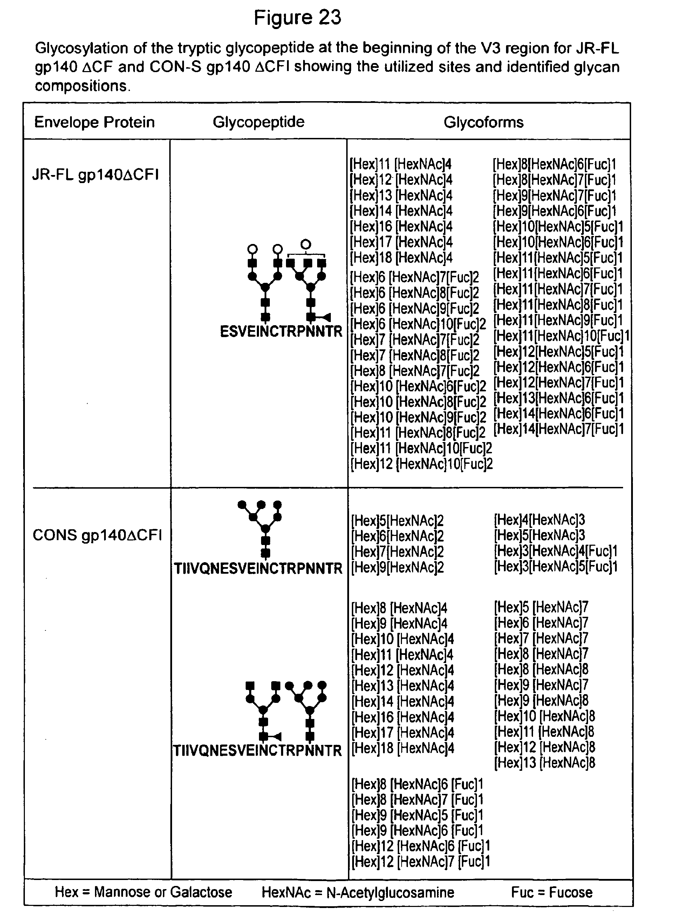

The next region where CON-S and JR-FL differ in glycosylation is the region surrounding the V3 loop (Figure 20). The V3 loop spans 35-residue segment connected by a disulfide bond and this region is known to be a determinant for HIV tropism and receptor binding. This region is glycosylated before and after the loop. The data shows that the sites at N245, N266, N293 and N299 in the C2 region and N305 in the V3 loop for CON-S are variably unoccupied as discussed in the preceding section. When glycosylated, these sites were generally occupied by high mannose, hybrid type glycans, and smaller complex type structures (Figure 23). In contrast, corresponding sites in JR-FL are fully glycosylated with larger processed glycoforms. The mere presence of a large population of high mannose glycans on these sites for CON-S gp140ΔCFI suggests that these sites went through very minimal processing (i.e. glucose trimming) before the protein traveled to the Golgi. These sites are either completely or partially buried within the protein backbone after folding and thereby less accessible to glycosyltransferase enzymes for processing in the Golgi. With more high mannose structures on these regions, the protein flexibility is also reduced (Rudd and Dwek, Crit. Rev. Biochem. Mol. Biol. 32:1-100 (1997), Scanlan et al, Nature 446:1038-1045 (2007)). While the V3 region of CON-S is less accessible to glycosyltransferase enzymes, this degree of inaccessibility could ultimately be a reason why the V3 region of CON-S elicits more neutralizing antibodies, compared to JR-FL. Since JR-FL is more accessible to glycosyltransferase enzymes, its glycosylation is substantially larger than the glycosylation on CON-S. As a result of JR-FL's heavier glycosylation both in the number of sites occupied and in the size of the glycans present, the protein sequence in this local area is covered more effectively by glycans, masking key epitopes from antibodies. In contrast, the protein sequence in the V3 loop of CON-S is generally less shielded and key epitopes are more accessible to antibodies.

*Implications to vaccine design.* The first step in understanding how glycosylation influences Env's immunogenicity is to define its global glycosylation profile. Distinguishing the differences in glycosylation provides insights on how the glycan profiles affect the functional conformation of the protein, necessary for eliciting potent immune response. While the overall degree glycosylation within isolates and across clades is conserved to maintain the glycan shield, glycosylation continues to evolve to effectively mask underlying epitopes and perhaps eliminate non-self glycosylation patterns generated by the host cell glycosylation machinery to evade immune recognition (Pashov et al, Curr. Pharm. Des. 13:185-201 (2007), Scanlan et al, Nature 446:1038-1045 (2007)). Therefore, the design of an effective Env protein-based immunogen as a vaccine component should require a detailed analysis of the differences in glycosylation profile between immunogens to improve vaccine development.

In an effort to accomplish this goal, the glycosylation of synthetic Env protein CON-S representing group M with shortened variable loops derived from clade C and the wild-type clade B JR-FL have been characterized and a comparative study of their glycosylation profile performed. In the context of amino acid sequence of these two Env proteins, they differ in nine potential glycosylation sites. Such differences could affect glycosylation efficiency and how proteins fold in general. While the difference in amino acid sequence between Env's provides an avenue to understand protein structure and glycosylation, defining the specific glycosylation footprint at each site provides additional insights as to why one vaccine candidate is more effective than the other. The analysis shows a substantial difference in glycosylation in terms of the degree and the type glycosylation pattern between CON-S and JR-FL. This difference can be correlated to differences in protein structure and ultimately immunogenicity. The results indicated that the more immunogenic Env protein has more unutilized sites surrounding the V1, V2, C2, V3 regions and high mannose structures as well as smaller processed type glycoforms in the C2 and the immunodominant V3 and V4 regions.

An effective Env immunogen must have a low degree of structural heterogeneity to allow better neutralization of underlying structural epitopes and the glycosylation of CON-S suggests that its structure is more highly conserved than JR-FL. Specifically, CON-S contains a higher degree high mannose glycan in the C2 domain and V4 region, along with minimally processed glycoforms and high mannose structures in the V3 loop. This result is reflective of the presence of more occluded glycosylation sites surrounding the C2, V3, and V4 regions. Since the high mannose glycoforms are known to reduce protein flexibility. These glycans are likely to promote protein stability and preserve specific protein configuration in these regions. In addition, the presence of more unutilized potential glycosylation sites surrounding these regions indicates that the key protein epitopes in this region are more exposed, which would assist in eliciting antibody response. From the data obtained thus far, the glycosylation features that appear to add to CON-S's enhanced immunogenicity include the number of open glycosylation sites, and the regions containing high-mannose glycans in the early part of the sequence, which correlate to a more well-conserved protein structure. These key findings are consistent with recent immunology data on CON-S gp140ΔCFI where high titers of antibodies were elicited when used in DNA vaccine in small animal models (Liao et al, Virology 353:268-282 (2006)). Further study and refinement of the correlation between glycosylation and immunogenicity will provide the opportunity to enable identification of certain glycosylation footprint of Env proteins that will promote the induction of antibodies to a broad spectrum of HIV-1 isolates. Such study is a step towards improving HIV vaccine design/development. Other synthetic as well as wild-type Env immunogens are being characterized and the glycosylation pattern correlated with immunologic activity in small animal models.

In conclusion, glycopeptide-based mass mapping approach was used to characterize the glycosylation of two Env protein vaccine candidates in a glycosylation site-specific fashion. The results show that the two Env proteins have different glycosylation site occupancy and different characteristic set of glycan motifs populating each glycosylation site. CON-S and JR-FL are the first two proteins shown to contain unoccupied potential glycosylation sites in the Env, and CON-S has a particularly high level of unoccupied sites: 19/31 are unoccupied at least some of the time. The open sites could be present in these proteins in part because a 293-T cell line was used as the expression system for both CON-S and JR-FL. Additionally, the higher level of unoccupied sites on CON-S, compared to JRFL could be due, in part, to its unique primary sequence.

Unraveling the differences in glycosylation provided important biological insights why CON-S may be more immunogenic than JR-FL. The characteristic features of CON-S include more unoccupied sites and sites that are populated with smaller glycoforms and/or high mannose structures. Such a glycosylation pattern would render better accessibility of antigenic epitopes to neutralizing antibodies. Together with immunological data, glycosylation site-specific analysis is an avenue of research that can provide information in directing antibody response.

**Table 1: MALDI-MS Glycopeptide Assignments For JR-FL**

| F# | Experimental | Theoretical | Error | ^{0,2}X | [Peptide + II] | Peptide Sequence | mod | position | Glycan |
|---|---|---|---|---|---|---|---|---|---|
| 3-6 | 2147.9563 | 2147.8656 | 42.2 | | | QAHCNISR | PyroQ,U | 319-326 | \|Hex\|2\|HexNAc\|4 |
| | 1860.7773 | 1860.7538 | 12.6 | | | QAHCNISR | PyroQ | 319-316 | \|Hex\|3\|HexNAc\|2 |
| | 2063.8450 | 2063.8332 | 5.7 | | | QAHCNISR | PyroQ | 319-326 | \|Hex\|3\|HexNAc\|3 |
| | 2080.8608 | 2080.8598 | 0.5 | | | QAHCNISR | | 319-326 | \|Hex\|3\|HexAc\|3 |
| | 2209.8904 | 2209.8911 | 0.3 | | | QAHCNISR | PyroQ | 319-326 | \|Hex\|3\|HexNAc\|3\|Fuc\|1 |
| | 2226.9297 | 2226.9177 | 5.4 | | | QAHCNISR | | 319-326 | \|Hex\|3\|Hex\|NAc\|3\|Fuc\|1 |
| | 2237.9001 | 2237.8860 | 6.3 | | | QAHCNISR | For,PyroQ | 319-326 | \|Hex\|3\|Hex\|NAc\|3\|Fuc\|1 |
| | 2254.9163 | 2254.9126 | 1.6 | | | QAHCNISR | For | 319-326 | \|Hex\|3\|Hex\|NAc\|3\|Fuc\|1 |
| | 2266.9385 | 2266.9125 | 11.5 | | | QAHCNISR | PyroQ | 319-326 | \|Hex\|3\|Hex\|NAc\|4 |
| | 2412.9746 | 2412.9705 | 1.7 | | | QAHCNISR | PyroQ | 319-326 | \|Hex\|3\|Hex\|NAc\|4\|Fuc\|1 |
| | 2419.9922 | 2429.9971 | 2.0 | | | QAHCNISR | | 319.326 | \|Hex\|3\|Hex\|NAc\|4\|Fuc\|1 |
| | 2440.9709 | 2440.9654 | 2.3 | | | QAHCNISR | For,PyroQ | 319-326 | \|Hex\|3\|Hex\|NAc\|4\|Fuc\|1 |
| | 2437.9893 | 1457.9920 | 1.0 | 1068.5095 | 985.5095 | QAHCNISR | For | 319-326 | \|Hex\|3\|Hex\|NAc\|4\|Fuc\|1 |
| | 2473.0239 | 2473.0019 | 8.5 | | | QAHCNISR | U | 319-326 | \|\|Hex\|3\|Hex\|NAc\|4\|Fuc\|1 |
| | 2469.9956 | 2469.9920 | 1.5 | | | QAHCNISR | PyroQ | 319-326 | \|Hex\|3\|Hex\|NAc\|5 |
| | 2616.0513 | 2616.0499 | 0.5 | | | QAHCNISR | PyroQ | 319-326 | \|Hex\|3\|Hex\|NAc\|5\|Fuc\|1 |
| | 2633.0708 | 2633.0765 | 2.2 | 1068.5217 | 985.5217 | QAHCNISR | | 319-326 | \|Hex\|3\|Hex\|NAc\|5\|Fuc\|1 |
| | 2644.0269 | 2644.0448 | 6.8 | | | QAHCNISR | For,PyroQ | 319-326 | \|Hex\|3\|Hex\|NAc\|5\|Fuc\|1 |
| | 2661.0991 | 2661.0714 | 10.4 | | | QAHCNISR | For | 319-326 | \|Hex\|3\|Hex\|NAc\|5\|Fuc\|1 |
| | 2732.9641 | 2733.1038 | 51.1 | | | QAHCNISR | u | 319-326 | \|Hex\|3\|Hex\|NAc\|6 |
| | 2819.1650 | 2819.1293 | 12.7 | | | QAHCNISR | PyroQ | 319-326 | \|Hex\|3\|HexINAc\|6\|Fuc\|1 |
| | 2039.8585 | 2039.8333 | 12.4 | | | QAHCNISR | | 319-326 | \|Hex\|4\|HexNAc\|2 |
| | 2225.9060 | 2223.8860 | 9.0 | | | QAHCNISR | PyroQ | 319-326 | \|Hex\|4\|HexNAc\|3 |
| | 2371.9438 | 2371.9439 | 0.0 | | | QAHCNISR | PyroQ | 319-326 | \|Hex\|4\|HexNAc\|3\|Fuc\|1 |
| | 2388.9639 | 2388.9705 | 2.8 | | | QAHCNISR | | 319-326 | \|Hex\|4\|HexNAc\|3\|Fuc\|1 |
| | 2399.9746 | 2399.9388 | 14.9 | | | QAHCNISR | For,PyroQ | 319-326 | \|Hex\|4\|HexNAc\|3\|Fuc\|1 |
| | 2416.9871 | 2416.9654 | 9.0 | | | QAHCNISR | For | 319-326 | \|Hex\|4\|HexNAc\|3\|Fuc\|1 |
| | 2533.9917 | 2534.0080 | 6.4 | | | QAHCNISR | | 319-326 | \|Hex\|4\|HexNAc\|3\|NeuNac\|1 |
| | 2592.0489 | 2592.0499 | 0.4 | | | QAHCNISR | | 319.316 | \|Hex\|4\|HexNAc\|3\|Fuc\|1 |
| | 2632.0984 | 2632.0448 | 20.4 | | | QAHCNISR | PyroQ | 319-326 | [Hex]4[HexNAc]5 |
| | 2778.1162 | 2778.1027 | 4.9 | | | QAHCNISR | PyroQ | 319-326 | [Hex]4[HexNAc]5Fuc1 |
| | 2195.124 | 2795.1293 | 1.9 | 1068.5785 | 985.5785 | QAHCNISR | | 319-326 | [Hex]4[HexNAc]5[Fuc]1 |
| | 2806.1675 | 2806.0975 | 24.9 | | | QAHCNISR | For,PyroQ | 319-326 | [Hex]4[HexNAc]5[Fuc]1 |
| | 2184.8643 | 2184.8594 | 2.2 | 1051.5658 | 968.5658 | QAHCNISR | PyroQ | 319-326 | [Hex]5[HexNAc]2 |
| | 2101.8879 | 2201.8860 | 0.9 | 1068.4609 | 985.4609 | QAHCNISR | | 319-326 | [Hex]5[HexNAc]2 |
| | 2212.8665 | 2212.8543 | 5.5 | | | QAHCNISR | For,PyroQ | 319-326 | [Hex]5[HexNAc]2 |
| | 2229.8896 | 2229.8809 | 3.9 | | | QAHCNISR | For | 319-326 | [Hex]5[HexNAc]2 |
| | 2244.9014 | 2244.8918 | 4.3 | | | QAHCNISR | U | 319-326 | [Hex]5[HexNAc]2 |
| | 2272.8921 | 2171.8867 | 2.4 | | | QAHCNISR | U,For | 319-326 | [Hex]5[HexNAc]2 |
| | 2387.9365 | 2387.9388 | 1.0 | | | QAHCNISR | PyroQ | 319-326 | [Hex]5[HexNAc]3 |
| | 2404.9954 | 2404.9654 | 12.5 | | | QAHCNISR | | 319-326 | [Hex]5[HexNAc]3 |
| | 2433.0063 | 2432.9603 | 18.9 | | | QAHCNISR | For | 319-326 | [Hex]5[HexNAc]3 |
| | 2447.9729 | 7447.9712 | 0.7 | | | QAHCNISR | U | 319-326 | [Hex]5[HexNAc]3 |
| | 2534.0139 | 2533.9967 | 6.8 | | | QAHCNISR | PyroQ | 319-326 | [Hex]5[HexNAc]3[Fuc]1 |
| | 2551.0503 | 2551.0233 | 10.6 | | | QAHCNISR | | 319-326 | [Hex]5[HexNAc]3[Fuc]1 |
| | 2579.0405 | 2579.0182 | 8.6 | | | QAHCNISR | For | 319-326 | [Hex]5[HexNAc]3[Fuc]1 |
| | 2394.0916 | 2394.0291 | 24.1 | | | QAHCNISR | u | 319-326 | [Hex]5[HexNAc]3[Fuc]1 |
| | 2591.0437 | 2591.0182 | 9.8 | | | QAHCNISR | PyroQ | 319-326 | [Hex]5[HexNAc]4 |
| | 2737.0818 | 2737.0761 | 2.1 | 1051.4860 | 968.4860 | QAHCNISR | PyroQ | 319-326 | [Hex]5[HexNAc]4[Fuc]1 |
| | 2754.0999 | 2754.1027 | 1.0 | | | QAHCNISR | | 319-326 | [Hex]5[HexNAc]4[Fuc]1 |
| | 2765.0752 | 2765.0710 | 1.5 | | | QAHCNISR | For,Pyro | 319-326 | [Hex]5[HexNAc]4[Fuc]1 |
| | 2794.1653 | 2794.0976 | 24.2 | | | QAHCNISR | PyroQ | 319-326 | [Hex]5[HexNAc]5 |
| | 2837.1743 | 2837.1034 | 25.0 | | | QAHCNISR | For.U | 319-326 | [Hex]5[HexNAc]5 |
| | 2940.1963 | 2940.1555 | 13.9 | 1051.5258 | 968.5258 | QAHCNISR | PyroQ | 319-326 | [Hex]5[HexNAc]5[Fuc]1 |
| | 2957.1902 | 2957.1821 | 2.7 | | | QAHCNISR | | 319-326 | [Hex]5[HexNAc]5[Fuc]1 |
| | 2983.1865 | 2985.1770 | 3.2 | | | QAHCNISR | For | 319-326 | [Hex]5[HexNAc]5[Fuc]1 |
| | 3000.2456 | 3000.1879 | 19.2 | | | QAHCNISR | u | 319-326 | [Hex]5[HexNAc]5[Fuc]1 |
| | 2346.9089 | 2346.9123 | 1.4 | 1051.4539 | 968.4539 | QAHCNISR | PyroQ | 319-326 | [Hex]6[HexNAc]2 |
| | 2363.9373 | 2363.9389 | 0.7 | 1051.4402 | 968.4402 | QAHCNISR | | 319-326 | [Hex]6[HexNAc]2 |
| | 2406.9661 | 2406.9447 | 8.9 | 1111.541 | 1028.5410 | QAHCNISR | U | 319-326 | [Hex]6[HexNAc]2 |
| | 2434.9368 | 2434.9396 | 1.1 | | | QAHCNISR | U,For | 319-326 | [Hex]6[HexNAc]2 |
| | 2549.9931 | 2549.9916 | 0.6 | | | QAHCNISR | PyroQ | 319-326 | [Hex]6[HexNAc]3 |
| | 2567.0396 | 2567.0182 | 8.3 | | | QAHCNISR | | 319-326 | [Hex]6[HexNAc]3 |
| | 2610.0247 | 2610.0240 | 0.3 | | | QAHCNISR | U | 319-326 | [Hex]6[HexNAc]3 |
| | 2696.1089 | 2696.0495 | 22.0 | | | QAHCNISR | PyroQ | 319-326 | [Hex]6[HexNAc]3[Fuc]1 |
| | 2741.0996 | 2741.0710 | 10.4 | | | QAHCNISR | For | 319-326 | [Hex]6[HexNAc]3[Fuc]1 |
| | 2956.2246 | 2956.1504 | 25.1 | | | QAHCNISR | PyroQ | 319-326 | [Hex]6[HexNAc]5 |
| | 3102.2200 | 3102.2083 | 3.8 | 1051.4325 | 968.4325 | QAHCNISR | PyroQ | 319-326 | [Hex]6[HexNAc]5[Fuc]1 |
| | 3119.2397 | 3119.2349 | 1.6 | 1068.5348 | 985.5348 | QAHCNISR | | 319-326 | [Hex]6[HexNAc]5[Fuc]1 |
| | 2508.9653 | 2508.9651 | 0.1 | 1068.4847 | 985.4847 | QAHCNISR | PyroQ | 319-326 | [Hex]7[HexNAc]2 |
| | 2525.9885 | 2525.9917 | 1.3 | 1068.4807 | 985.4807 | QAHCNISR | | 319-326 | [Hex]7[HexNAc]2 |
| | 2569.0071 | 2568.9975 | 3.7 | 1111.5365 | 1028.5365 | QAHCNISR | U | 319-326 | [Hex]7[HexNAc]2 |
| | 2597.0090 | 2596.9924 | 6.4 | 1111.5713 | 1028.5713 | QAHCNISR | U,For | 319-326 | [Hex]7[HexNAc]2 |
| | 3467.5456 | 3467.3405 | 59.2 | 1051.7153 | 968.7153 | QAHCNISR | PyroQ | 319-326 | [Hex]7[HexNAc]6[Fuc]1 |
| | 2671.0217 | 2671.0179 | 1.4 | 1051.5985 | 968.5985 | QAHCNISR | PyroQ | 319-326 | [Hex]8[HexNAc]2 |
| | 2688.0483 | 2688.0445 | 1.4 | 1068.5270 | 985.5270 | QAHCNISR | | 319-326 | [Hex]8[HexNAc]2 |
| | 2699.0173 | 2699.0128 | 1.7 | 1068.4771 | 985.4771 | QAHCNISR | For,PyroQ | 319-326 | [Hex]8[HexNAc]2 |
| | 2716.0508 | 2716.0394 | 4.2 | 1051.4662 | | QAHCNISR | For | 319-326 | [Hex]8[HexNAc]2 |
| | 2731.0652 | 2731.0503 | 5.5 | 1111.5343 | 1028.5343 | QAHCNISR | U | 319-326 | [Hex]8[HexNAc]2 |
| | 2759.0601 | 2759.0452 | 5.4 | 1111.5834 | 1028.5834 | QAHCNISR | U,For | 319-326 | [Hex]8[HexNAc]2 |
| | 3077.3538 | 3077.1766 | 37.6 | | | QAHCNISR | PyroQ | 319-326 | [Hex]8[HexNAc]4 |
| | 2833.0745 | 2833.0707 | 1.3 | 1051.5906 | 968.5906 | QAHCNISR | PyroQ | 319-326 | [Hex]9[HexNAc]2 |
| | 2850.1138 | 2850.0973 | 5.8 | 1051.4762 | 968.4762 | QAHCNISR | | 319-326 | [Hex]9[HexNAc]2 |
| | 2861.0684 | 2861.0656 | 1.0 | 1051.7351 | 968.7351 | QAHCNISR | For,PyroQ | 319-326 | [Hex]9[HexNAc]2 |
| | 2878.0955 | 2878.0922 | 1.1 | 1051.5214 | | QAHCNISR | For | 319-326 | [Hex]9[HexNAc]2 |
| | 2893.1174 | 2893.1031 | 4.9 | 1111.543 | 1028.5430 | QAHCNISR | U | 319-326 | [Hex]9[HexNAc]2 |
| | 2921.1191 | 2921.098 | 7.2 | 1111.5675 | 1028.5675 | QAHCNISR | U,For | 319-326 | [Hex]9[HexNAc]2 |
| | | | | | | | | | |
| 7-8 | 2346.0696 | 2346.0343 | 15.0 | | | LREQFENK | | 340-347 | [Hex]2[HexNAc][Fuc]1 |
| 12-13 | 2101.9580 | 2101.9283 | 14.1 | | | LREQFENK | | 340-347 | [Hex]3[HexNAc]2[Fuc]1 |
| | 2158.9602 | 2158.9498 | 4.8 | | | LREQFENK | | 340-347 | [Hex]3[HexNAc]3 |
| | 2305.0049 | 2305.0082 | 1.4 | 1146.7590 | 1063.7590 | LREQFENK | | 340-347 | [Hex]3[HexNAc]3[Fuc]1 |
| | 2333.0093 | 2333.0026 | 2.9 | | | LREQFENK | For | 340-347 | [Hex]3[HexNAc]3[Fuc]1 |
| | 2348.0476 | 2348.0140 | 14.3 | | | LREQFENK | U | 340-347 | [Hex]3[HexNAc]3[Fuc]1 |
| | 2362.0161 | 2362.0291 | 5.5 | | | LREQFENK | | 340-347 | [Hex]3[HexNAc]4 |
| | 2508.0801 | 2508.0870 | 2.8 | 1146.6277 | 1063.6277 | LREQFENK | | 340-347 | [Hex]3[HexNAc]4[Fuc]1 |
| | 2522.2243 | 2522.1028 | 48.2 | 1160.8904 | 1077.8904 | LREQFENK | methyl | 340-347 | [Hex]3[HexNAc]4[Fuc]1 |
| | 2536.1326 | 2536.0819 | 20.0 | | | LREQFENK | For | 340-347 | [Hex]3[HexNAc]4[Fuc]1 |
| | 2551.1365 | 2551.0928 | 17.1 | 1189.5271 | 1106.5271 | LREQFENK | U | 340-347 | [Hex]3[HexNAc]4[Fuc]1 |
| | 2565.1423 | 2565.1086 | 13.1 | | | LREQFENK | | 340-347 | [Hex]3[HexNAc] |
| | 2711.1494 | 2711.1664 | 6.3 | 1146.8489 | 1063.8489 | LREQFENK | | 340-347 | [Hex]3[HexNAc]5[Fuc]1 |
| | 2725.3162 | 2725.1821 | 49.2 | 1160.6283 | 1077.6283 | LREQFENK | methyl | 340-347 | [Hex]3[HexNAc]5[Fuc]1 |
| | 2739.1438 | 2739.1614 | 6.4 | | | LREQFENK | For | 340-347 | [Hex]3[HexNAc]5[Fuc]1 |
| | 2754.1232 | 2754.1722 | 17.8 | 1189.7161 | 1106.7161 | LREQFENK | U | 340-347 | [Hex]3[HexNAc]5[Fuc]1 |
| | 2914.2271 | 2914.2459 | 6.5 | | | LREQFENK | | 340-347 | [Hex]3[HexNAc]6[Fuc]1 |
| | 2928.3909 | 2928.2615 | 44.2 | 1160.5890 | 1077.5990 | LREQFENK | methyl | 340-347 | [Hex]3[HexNAc]6[Fuc]1 |
| | 2957.2390 | 2957.2517 | 4.3 | | | LREQFENK | U | 340-347 | [Hex]3[HexNAc]6[Fuc]1 |
| | 2117.9783 | 2117.9233 | 26.0 | | | LREQFENK | | 340-347 | [Hex]4[HexNAc]2 |
| | 2321.0354 | 2321.0026 | 14.1 | | | LREQFENK | | 340-347 | [Hex]4[HexNAc]3 |
| | 2467.0479 | 2467.0605 | 5.1 | | | LREQFENK | | 340-347 | [Hex]4[HexNAc]3[Fuc]1 |
| | 2495.1211 | 2495.0554 | 26.3 | | | LREQFENK | For | 340-347 | [Hex]4[HexNAc]3[Fuc]1 |
| | 2310.1135 | 2510.0663 | 18.8 | | | LREQFENK | U | 340-347 | [Hex]4[HexNAc]3[Fuc]1 |
| | 2670.2852 | 2670.1399 | 54.4 | 1146.5914 | 1063.5914 | LREQFENK | | 340-347 | [Hex]4[HexNAc]4[Fuc]1 |
| | 2694.3022 | 2684.1556 | 54.6 | 1160.5767 | 1077.5767 | LREQFENK | methyl | 340-347 | [Hex]4[HexNAc]4[Fuc]1 |
| | 2711.2012 | 2713.1462 | 20.3 | 1189.5264 | 1106.5264 | LREQFENK | U | 340-347 | [Hex]4[HexNAc]4[Fuc]1 |
| | 2873.2473 | 2873.2191 | 9.8 | | | LREQFENK | | 340-347 | [Hex]4[HexNAc]5[Fuc]1 |
| | 2901.1711 | 2901.2141 | 14.8 | | | LREQFENK | For | 340-347 | [Hex]4[HexNAc]5[Fuc]1 |
| | 2916.1899 | 2916.2250 | 12.0 | 1189.6660 | 1106.6660 | LREQFENK | U | 340-347 | [Hex]4[HexNAc]5[Fuc]1 |
| | 3119.3691 | 3119.3049 | 20.6 | | | LREQFENK | u | 340-347 | [Hex]4[HexNAc]6[Fuc]1 |
| | 2280.0222 | 2279.9760 | 20.3 | 1146.5656 | 1063.5656 | LREQFENK | | 340-347 | [Hex]5[HexNAc]2 |
| | 2308.0676 | 2307.9709 | 41.9 | | | LREQFENK | For | 340-347 | [Hex]5[HexNAc]2 |
| | 2323.0325 | 2322.9818 | 21.8 | | | LREQFENK | u | 340-347 | [Hex]5[HexNAc]2 |
| | 2469.1606 | 2469.0403 | 48.7 | | | LREQFENK | U | 340-347 | [Hex]5[HexNAc]2[Fuc]1 |
| | 2483.1245 | 2483.0554 | 27.8 | | | LREQFENK | | 340-347 | [Hex]5[HexNAc]3 |
| | 2629.1079 | 2629.1133 | 2.1 | | | LREQFENK | | 340-347 | [Hex]5[HexNAc]3[Fuc]1 |
| | 2657.1960 | 2657.1082 | 33.0 | | | LREQFENK | For | 340-347 | [Hex]5[HexNAc]3[Fuc]1 |
| | 2672.1677 | 2672.1191 | 18.2 | | | LREQFENK | U | 340-347 | [Hex]5[HexNAc]3[Fuc]1 |
| | 2832.1821 | 2832.1927 | 3.7 | 1146.6254 | 1063.6254 | LREQFENK | | 340-347 | [Hex]5[HexNAc]3[Fuc]1 |
| | 2860.1958 | 2860.1876 | 2.9 | | | LREQFENK | For | 340-347 | [Hex]5[HexNAc]4[Fuc]1 |
| | 2875.2493 | 2875.1985 | 17.7 | 1189.5570 | 1106.5570 | LREQFENK | U | 340-347 | [Hex]5[HexNAc]4[Fuc]1 |
| | 3035.2500 | 3035.2721 | 7.3 | 1146.6147 | 1063.6147 | LREQFENK | | 340-347 | [Hex]5[HexNAc]5[Fuc]1 |
| | 3063.3503 | 3063.2670 | 27.2 | | | LREQFENK | For | 340-347 | [Hex]5[HexNAc]5[Fuc]1 |
| | 3078.3340 | 3078.2779 | 18.2 | | | LREQFENK | U | 340-347 | [Hex]5[HexNAc]5[Fuc]1 |
| | 2442.0735 | 2442.0289 | 18.3 | | | LREQFENK | | 340-347 | [Hex]6[HexNAc]2 |
| | 2485.0767 | 2485.0347 | 16.9 | | | LREQFENK | U | 340-347 | [Hex]6[HexNAc]2 |
| | 2645.1660 | 2645.1081 | 21.9 | 1146.5852 | | LREQFENK | | 340-347 | [Hex]6[HexNAc] |
| | 2834.1987 | 2834.1719 | 9.5 | | | LREQFENK | U | 340-347 | [Hex]6[HexNAc]3[Fuc]1 |
| | 2791.1648 | 2791.1927 | 10.0 | | | LREQFENK | | 340-347 | [Hex]6[HexNAc]3[Fuc]1 |
| | 3197.3093 | 3197.3249 | 4.9 | 1146.6859 | 1063.6859 | LREQFENK | | 340-347 | [Hex]6[HexNAc]5[Fuc]1 |
| | 3225.3354 | 3225.3198 | 4.8 | | | LREQFENK | For | 340-347 | [Hex]6[HexNAc]5[Fuc]1 |
| | 3240.2872 | 3240.3312 | 13.6 | | | LREQFENK | U | 340-347 | [Hex]6[HexNAc]5[Fuc]1 |
| | 3282.4448 | 3282.3413 | 31.5 | | | LREQFENK | For | 340-347 | [Hex]6[HexNAc] |
| | 3400.3762 | 3400.4048 | 8.4 | 1146.6462 | 1063.6462 | LREQFENK | | 340-347 | [Hex]6[HexNAc]6[Fuc]1 |
| | 2604.1636 | 2604.0817 | 31.5 | 1146.5667 | 1063.5667 | LREQFENK | | 340-347 | [Hex]7[HexNAc]2 |
| | 2647.1235 | 2647.0875 | 13.6 | | | LREQFENK | U | 340-347 | [Hex]7[HexNAc]2 |
| | 3416.5291 | 3416.3992 | 38.0 | | | LREQFENK | | 340-347 | [Hex]7[HexNAc]6 |
| | 3562.4316 | 3562.4571 | 7.2 | 1146.6810 | 1063.6810 | LREQFENK | | 340-347 | [Hex]7[HexNAc]6[Fuc]1 |
| | 3605.4634 | 3605.4629 | 0.1 | 1189.8983 | 1106.8983 | LREQFENK | U | 340-347 | [Hex]7[HexNAc]6[Fuc]1 |
| | 2766.1726 | 2766.1345 | 13.8 | | | LREQFENK | C13? | 340-347 | [Hex]8[HexNAc]2 |
| | 2809.1265 | 2809.1403 | 4.9 | 1189.7231 | 1106.7231 | LREQFENK | U | 340-347 | [Hex]8[HexNAc]2 |
| | 2928.2351 | 2928.1873 | 16.3 | | | LREQFENK | C13? | 340-347 | [Hex]9[HexNAc]2 |
| | 2942.3828 | 2942.2031 | 61.1 | | | LREQFENK | methyl | 340-347 | [Hex]9[HexNAc]2 |
| | 2956.3018 | 2956.1822 | 40.5 | | | LREQFENK | For | 340-347 | [Hex]9[HexNAc]2 |
| | 2971.1519 | 2971.1931 | 13.9 | 1189.7788 | 1106.7788 | LREQFENK | U | 340-347 | [Hex]9[HexNAc]2 |
| | 3090.4238 | 3090.2401 | 59.4 | | | LREQFENK | | 340-347 | [Hex]10[HexNAc]2 |
| | | | | | | | | | |
| 9-12 | 2013.8879 | 2013.9010 | 6.5 | | | AKWNDTLK | | 327-334 | [Hex]3[HexNAc]2[Fuc]1 |
| | 2216.9792 | 2216.9804 | 0.5 | | | AKWNDTLK | | 327-334 | [Hex]3[HexNAc]3[Fuc]1 |
| | 2420.0789 | 2420.0597 | 7.9 | 1058.5187 | 975.5187 | AKWNDTLK | | 327-334 | [Hex]3[HexNAc]4[Fuc]1 |
| | 2623.1213 | 2623.1392 | 6.8 | | | AKWNDTLK | | 327-334 | [Hex]3[HexNAc]5[Fuc]1 |
| | 2233.0652 | 2232.9753 | 40.3 | | | AKWNDTLK | | 327-334 | [Hex]3[HexNAc]3 |
| | 2379.0356 | 2379.0332 | 1.0 | | | AKWNDTLK | | 327-334 | [Hex]4[HexNAc]3[Fuc]1 |
| | 2785.2178 | 2785.1919 | 9.3 | | | AKWNDTLK | | 327-334 | [Hex]4[HexNAc]5[Fuc]1 |
| | 2191.9480 | 2191.9487 | 0.3 | 1058.8015 | 975.8015 | AKWNDTLK | | 327-334 | [Hex]5[HexNAc]2 |
| | 2395.0344 | 2395.0281 | 2.6 | | | AKWMDTLK | | 327-334 | [Hex]5[HexNAc]3 |
| | 2541.1064 | 2541.0860 | 8.0 | 1058.5221 | 975.5221 | AKWNDTLK | | 327-334 | [Hex]5[HexNAc]3[Fuc]1 |
| | 2598.0649 | 2598.1075 | 16.4 | | | AKWNDTLK | | 327-334 | [Hex]5[HexNAc]4 |
| | 2744.2117 | 2744.1654 | 16.9 | 1058.4429 | 975.4816 | AKWNDTLK | | 327-334 | [Hex]5[HexNAc]4[Fuc]1 |
| | 2947.2251 | 2947.2448 | 6.7 | | | AKWNDTLK | | 327-334 | [Hex]5[HexNAc]5[Fuc]1 |
| | 2353.9919 | 2354.0016 | 4.1 | 1058.7585 | 975.7585 | AKWNDTLK | | 327-334 | [Hex]6[HexNAc]2 |
| | 2382.0110 | 1381.9965 | 6.1 | | | AKWNDTLK | For | 327-334 | [Hex]6[HexNAc]2 |
| | 2557.1262 | 2557.0809 | 17.7 | | | AKWNDTLK | | 327-334 | [Hex]6[HexNAc]3 |
| | 2707.1860 | 2703.1388 | 17.5 | | | AKWNDTLK | | 327-334 | [Hex]6[HexNAc]3[Fuc]1 |
| | 2516.0298 | 2516.0544 | 9.8 | 1058.7723 | 975.7723 | AKWNDTLK | | 317-334 | [Hex]7[HexNAc]2 |
| | 2678.0605 | 2678.1072 | 17.4 | 1058.7169 | 975.7169 | AKWNDTLK | | 327-334 | [Hex]8[HexNAc]2 |
| | 2840.3750 | 2840.1600 | 75.7 | 1058.7704 | 975.7704 | AKWNDTLK | | 327-334 | [Hex]9[HexNAc]2 |
| | | | | | | | | | |
| 9 | 4464.9632 | 4464.7973 | 37.2 | | | ESVEIN*C*TRPNNNTR | | 282-296 | [Hex]8[HexNAc]6[Fuc]1 |
| | 4693.1523 | 4692.9084 | 52.0 | | | ESVEIN*C*TRPNNNTR | | 282-296 | [Hex]6[HexNAc]8[Fuc]2 |
| | 4627.2714 | 4626.8501 | 91.1 | | | ESVEIN*C*TRPNNNTR | | 282-296 | [Hex]9[HexNAc]6[Fuc]1 |
| | 4747.8504 | 4747.8764 | 5.5 | | | ESVEIN*C*TRPNNNTR | | 282-296 | [Hex]11[HexNAc]5[Fuc]1 |
| | 4788.6450 | 4788.9031 | 53.9 | | | ESVEIN*C*TRPNNNTR | | 282-296 | [Hex]10[HexNAc]6[Fuc]1 |
| | 4855.7248 | 4854.9612 | 157.3 | | | ESVEIN*C*TRPNNNTR | | 282-296 | [Hex]7[HexNAc]8[Fuc]2 |
| | 4896.2964 | 4895.9878 | 63.0 | | | ESVEIN*C*TRPNNNTR | | 282-296 | [Hex]6[HexNAc]9[Fuc]2 |
| | 4951.0351 | 4950.9558 | 16.0 | | | ESVEIN*C*RRPNNNTR | | 282-296 | [Hex]11[HexNAc]6[Fuc]1 |
| | 5113.2030 | 5113.0086 | 38.0 | | | ESVEIN*C*TRPNNNTR | | 282-296 | [Hex]12[HexNAc]6[Fuc]1 |
| | | | | | | | | | |
| 14 | 5485.4434 | | | | | GEIKNCSFNITTSIRDEVQK | | 144-158 | [Hex]12[HexNAc]5[Fuc]1 |
| | 5687.6182 | | | | | GEIKNCSFNITTSIRDEVQK | | 144-158 | [Hex]12[HexNAc]6[Fuc]1 |
| | 5891.7075 | | | | | GEIKNCSFNITTSIRDEVQK | | 144-158 | [Hex]12[HexNAc]7[Fuc]1 |
| | 6094.5786 | | | | | GEIKNCSPNITTSIRDEVQK | | 144-158 | [Hex]12[HexNAc]8[Fuc]1 |
| | | | | | | | | | |
| 17-20 | 3333.5847 | 3333.4059 | 53.6 | | | DGGINENGTEIFRPGGGDMR | | 443-462 | [Hex]3[HexNAc]3[Fuc]1 |
| | 3536.4839 | 3536.4851 | 0.4 | 2174.813 | 2091.8183 | DGGINENGTEIFRPGGGDMR | | 443-462 | [Hex]3[HexNAc]4[Fuc]1 |
| | 3552.6560 | 3552.4801 | 49.5 | | | DGGINENGTEIFRPGGGDMR | mox | 443-462 | [Hex]3[HexNAc]4[Fuc]1 |
| | 3593.7019 | 3593.5068 | 54.3 | | | DGGINENGTEIFRPGGGDMR | | 443-462 | [Hex]3[HexNAc]5 |
| | 3739.5930 | 3739.5647 | 7.6 | 2175.0710 | 2092.0710 | DGGINENGTEIFRPGGGDMR | | 443-462 | [Hex]3[HexNAc]5[Fuc]1 |
| | 3812.7542 | 3812.5811 | 45.4 | | | DGGINENGTEIFRPGGGDMR | mox | 443-462 | [Hex]3[HexNAc]6 |
| | 3942.6719 | 3942.6440 | 7.1 | 2174.9634 | 2091.9634 | DGGINENGTEIFRPGGGDMR | | 443-462 | [Hex]3[HexNAc]6[Fuc]1 |
| | 3292.5959 | 3292.3793 | 65.8 | | | DGGINENGTEIFRPGGGDMR | | 443-462 | [Hex]4[HexNAc]2[Fuc]1 |
| | 3495.4312 | 3495.4587 | 7.9 | | | DGGINENGTEIFRPGGGDMR | | 443-462 | [Hex]4[HexNAc]3[Fuc]1 |
| | 3698.5691 | 3698.5381 | 8.4 | | | DGGINENGTEIFRPGGGDMR | | 443-462 | [Hex]4[HexNAc]4[Fuc]1 |
| | 3901.6543 | 3901.6543 | | | | DGGINENGTEIFRPGGGDMR | | 443-462 | [Hex]4[HexNAc]5[Fuc]1 |
| | 4104.9648 | 4104.6943 | 65.9 | 2174.6272 | 2091.6272 | DGGINENGTEIFRPGGGDMR | | 443-462 | [Hex]4[HexNAc]6[Fuc]1 |
| | 3308.5476 | 3308.3742 | 52.4 | | | DGGINENGTEIFRPGGGDMR | | 443-462 | [Hex]5[HexNAc]2 |
| | 3657.7288 | 3657.5115 | 59.4 | | | DGGINENGTEIFRPGGGDMR | | 443-462 | [Hex]5[HexNAc]3[Fuc]1 |
| | 3860.6372 | 3860.5909 | 12.0 | 2174.9124 | 2091.9114 | DGGINENGTEIFRPGGGDMR | | 443-462 | [Hex]5[HexNAc]4[Fuc]1 |
| | 3917.8411 | 3917.6124 | 58.4 | | | DGGINENGTEIFRPGGGDMR | | 443-462 | [Hex]5[HexNAc]5 |
| | 4063.6707 | 4063.6703 | 0.1 | 2175.0156 | 2092.0156 | DGGINENGTEIFRPGGGDMR | | 443-462 | [Hex]5[HexNAc]5[Fuc]1 |
| | 4267.0376 | 4266.7502 | 67.4 | | | DGGINENGTEIFRPGGGDMR | | 443-462 | [Hex]5[HexNAc]6[Fuc]1 |
| | 4225.7739 | 4225.7231 | 12.0 | 2174.9670 | 2091.9670 | DGGINENGTEIFRPGGGDMR | | 443-462 | [Hex]6[HexNAc]5[Fuc]1 |
| | 4428.9495 | 4428.8030 | 33.1 | 2174.8556 | 2091.8556 | DGGINENGTEIFRPGGGDMR | | 443-462 | [Hex]6[HexNAc]6[Fuc]1 |
| | 3632.3776 | | | 2174.7842 | 2091.7842 | DGGINENCTEIFRPGGGDMR | | 443-462 | [Hex]7[HexNAc]2 |
| | 4590.9377 | 4590.8553 | 17.9 | 2175.1523 | 2092.1523 | DGGINENGTEIFRPGGGDMR | | 443-462 | [Hex]7[HexNAc]6[Fuc]1 |
| | 4794.4761 | | | | | DGGINENGTEIFRPGGGDMR | | 443-462 | [Hex]7[HexNAc]7[Fuc]1 |
| | 3794.5559 | 3794.5327 | 6.1 | 2174.9795 | 2091.9795 | DGGINENGTEIFRPGGGDMR | | 443-462 | [Hex]8[HexNAc]2 |
| | 3956.6758 | 3956.5855 | 22.8 | | | DGGINENGTEIFRPGGGDMR | | 443-462 | [Hex]9[HexNAc]2 |
| | 4146.9858 | | | | | DGGINENGTEIFRPGGGDMR | For | 443-462 | [Hex]10[HexNAc]2 |
| | | | | | | | | | |
| 18-20 | 2861.2158 | 2861.2567 | 14.3 | 1702.8928 | 1619.8928 | LDVVPIDNNNTSYR | | 171-184 | [Hex]3[HexNAc]3[Fuc]1 |
| | 3064.3611 | 3064.3361 | 8.2 | 1702.8866 | 1619.8866 | LDVVPIDNNNTSYR | 171.184 | 171-184 | [Hex]3[HexNAc]4[Fuc]1 |
| | 3092.2859 | 3092.3309 | 14.6 | | | LDVVPIDNNNTSYR | For | 171-184 | [Hex]3[HexNAc]4[Fuc]1 |
| | 3267.4178 | 3267.4155 | 0.7 | 1702.7805 | 1619-7805 | LDVVPIDNNNTSYR | | 171-184 | [Hex]3[HexNAc]5[Fuc]1 |
| | 3295.6944 | 3295.4104 | 83.1 | | | LDVVPIDNNNTSYR | For | 171-184 | [Hex]3[HexNAc]5[Fuc]1 |
| | 3470.4568 | 3470.4949 | 11.0 | 1702.8885 | 1619.8885 | LDVVPIDNNNTSYR | | 171-184 | [Hex]3[HexNAc]6[Fuc]1 |
| | 3023.2849 | 3023.3095 | 8.1 | | | LDVVPIDNNNTSYR | | 171-184 | [Hex]4[HexNAc]3[Fuc]1 |
| | 3226.3701 | 3226.3889 | 5.8 | 1702.7338 | 1619.7338 | LDVVPIDNNNTSYR | | 171-184 | [Hex]4[HexNAc]4[Fuc]1 |
| | 2836.1949 | 2836.1250 | 10.6 | 1702.8431 | 1619.8431 | LDVVPIDNNNTSYR | | 171-184 | [Hex]5[HexNAc] |
| | 3185.5374 | 3185.3623 | 55.0 | | | LDVVPIDNNNTSYR | | 171-184 | [Hex]5[HexNAc]3[Fuc]1 |
| | 3228.5425 | 3228.3681 | 54.0 | | | LDVVPIDNNNTSYR | U | 171-184 | [Hex]5[HexNAc]3[Fuc]1 |
| | 3431.5073 | 3431.4475 | 17.4 | | | LDVVPIDNNNTSYR | U | 171-184 | [Hex]5[HexNAc]4[Fuc]1 |
| | 3388.4583 | 3388.4417 | 4.9 | 1702.8772 | 1619.8772 | LDVVPIDNNNTSYR | | 171-184 | [Hex]5[HexNAc]4[Fuc]1 |
| | 3956.6758 | 3956.6533 | 5.7 | | | LDVVPIDNNNTSYR | | 171-184 | [Hex]6[HexNAc]6[Fuc]1 |
| | | | | | | | | | |
| 21,22 | 3153.3216 | 3153.3778 | 17.8 | | | NCSFNITTSIRDEVQK | | 148-158 | [Hex]3[HexNAc]3[Fuc]1 |
| | 3356.4980 | 3356.4598 | 11.4 | 1994.8033 | 1911.8033 | NCSFNITTSIRDEVQK | | 148-158 | [Hex]3[HexNAc]4[Fuc]1 |
| | 3559.5686 | 3559.5362 | 9.1 | 1994.9463 | 1911.9463 | NCSFNITTSIRDEVQK | | 148-158 | [Hex]3[HexNAc]6 |
| | 3315.4333 | 3315.4302 | 0.9 | 1994.9707 | 1911.9707 | NCSFNITTSIRDEVQK | | 148-158 | [Hex]4[HexNAc]3[Fuc]1 |
| | 3518.5340 | 3518.5096 | 12.6 | 1995-0107 | 1912-0107 | NCSFNITTSIRDEVQK | | 148-158 | [Hex]4[HexNAc]4[Fuc]1 |
| | 3721.7263 | 3721.5889 | 36.9 | | | NCSFNITTSIRDEVQK | | 148-158 | [Hex]4[HexNAc]5[Fuc]1 |
| | 3128.3633 | 3128.3457 | 5.6 | 1994.8428 | 1911.8428 | NCSFNITTSIRDEVQK | | 148.158 | [Hex]5[HexNAc]2 |
| | 3762.7498 | 3762.5414 | 55.4 | | | NCSFNITTSIRDEVQK | | 148-158 | [Hex]5[HexNAc]3 |
| | 3477.5159 | 3477.4831 | 9.4 | | | NCSFNITTSIRDEVQK | | 148-158 | [Hex]5[HexNAc]4 |
| | 3680.6418 | 3680.5624 | 21.6 | | | NCSFNITTSIRDEVQK | | 148-158 | [Hex]5[HexNAc]4[Fuc]1 |
| | 3883.5893 | 3883.6418 | 13.5 | | | NCSFNITTSIRDEVQK | | 148-158 | [Hex]5[HexNAc]5[Fuc]1 |
| | 3290.4221 | 3290.3986 | 7.1 | 1994.7664 | 1911.7664 | NCSFNITTSIRDEVQK | | 148-158 | [Hex]6[HexNAc]2 |
| | 4045.9458 | 4045.6946 | 62.1 | | | NCSFNITTSIRDEVQK | | 148-158 | [Hex]6[HexNAc]5[Fuc]1 |
| | 3452.4651 | 3452.4514 | 4.0 | 1994.8760 | 1911.8760 | NCSFNITTSIRDEVQK | | 148-158 | [Hex]7[HexNAc]2 |
| | 3614.5623 | 3614.5042 | 16.1 | 1994.7569 | 1911.7569 | NCSFNITTSIRDEVQK | | 148-158 | [Hex]8[HexNAc] |
| | 3776.6552 | 3776.5570 | 26.0 | 1995.0573 | 1912.0573 | NCSFNITTSIRDEVQK | | 148-158 | [Hex]9[HexNAc]2 |
| | | | | | | | | | |
| 24-25 | 2559.1650 | 2559.1377 | 10.7 | | | CSSNITGLLLTR | PyroC | 431-442 | [Hex]3[HexNAc]3[Fuc]1 |
| 29-30 | 2576.1475 | 2576.1643 | 6.5 | | | CSSNITGLLLTR | | 431-442 | [Hex]3[HexNAc]3[Fuc]1 |
| | 2762.2170 | 2762.2110 | 0.0 | 1400.7405 | 1317.7405 | CSSNITGLLLTR | PyroC | 431-442 | [Hex]3[HexNAc]3[Fuc]1 |
| | 2179.2349 | 2779.2439 | 3.2 | 1400.5940 | 1317.5940 | CSSNITGLLLTR | | 431-442 | [Hex]3[HexNAc]4[Fuc]1 |
| | 2965.2952 | 2965.2965 | 0.4 | | | CSSNITGLLLTR | PyroC | 431-442 | [Hex]3[HexNAc]5[Fuc]1 |
| | 2982.3120 | 2982.3231 | 3.7 | | | CSSNITGLLLTR | | 431-442 | [Hex]3[HexNAc]5[Fuc]1 |
| | 2721.1565 | 2721.1905 | 12.5 | 1400.8679 | 1317.8679 | CSSNITGLLLTR | PyroC | 431-442 | [Hex]4[HexNAc]3[Fuc]1 |
| | 2924.2830 | 2924.2699 | 4.5 | | | CSSNITGLLLTR | | 431-442 | [Hex]4[HexNAc]4[Fuc]1 |
| | 2941.2930 | 2941.2965 | 1.2 | | | CSSNITGLLLTR | | 431-442 | [Hex]4[HexNAc]4[Fuc]1 |
| | 3127.4250 | 3127.3493 | 24.2 | | | CSSNITGLLLTR | PyroC | 431-442 | [Hex]4[HexNAc]5[Fuc]1 |
| | 2534.0940 | 2534.1060 | 4.7 | 1400.7428 | 1317.7428 | CSSNITGLLLTR | PyroC | 431-442 | [Hex]5[HexNAc] |
| | 2551.1184 | 2551.1326 | 5.6 | 1417.7903 | 1334.7903 | CSSNITGLLLTR | | 431-442 | [Hex]5[HexNAc] |
| | 2737.1940 | 2737.1854 | 3.1 | 1400.9066 | 1317.9066 | CSSNITGLLLTR | PyroC | 431-442 | [Hex]5[HexNAc]3 |
| | 2754.2078 | 2754.2120 | 1.5 | | | CSSNITGLLLTR | | 431-442 | [Hex]5[HexNAc]3 |
| | 2883.2478 | 2883.2433 | 1.6 | | | CSSNITGLLLTR | PyroC | 431-442 | [Hex]5[HexNAc]3[Fuc]1 |
| | 3086.4109 | 3086.3227 | 28.6 | 1400.8037 | 1317.8037 | CSSNITGLLLTR | PyroC | 431-442 | [Hex]5[HexNAc]4[Fuc]1 |
| | 3103.3445 | 3103.3493 | 1.5 | | | CSSNITGLLLTR | | 431-442 | [Hex]5[HexNAc]4[Fuc]1 |
| | 2696.1577 | 2696.1589 | 0.4 | 1400.7422 | 1317.7422 | CSSNITGLLLTR | PyroC | 431-442 | [Hex]6[HexNAc]2 |
| | 2713.1985 | 2713.1855 | 4.8 | 1417.8252 | 1334.8252 | CSSNITGLLLTR | | 431-442 | [Hex]6[HexNAc]2 |
| | 2859.3179 | 2859.2439 | 25.9 | | | CSSNITGLLLTR | Pyro,For | 431-442 | [Hex]6[HexNAc]2[Fuc]1 |
| | 2899.2617 | 2899.2382 | 8.1 | | | CSSNITGLLLTR | PyroC | 431-442 | [Hex]6[HexNAc]3 |
| | 3306.4746 | 3306.4287 | 13.9 | | | CSSNITGLLLTR | | 431-442 | [Hex]5[HexNAc]5[Fuc]1 |
| | 3451.4156 | 3451.4549 | 6.0 | 1400.7539 | 1317.7539 | CSSNITGLLLTR | | 431-442 | [Hex]6[HexNAc]5[Fuc]1 |
| | 2858.2073 | 2858.2117 | 1.5 | 1400.7268 | 1317.7268 | CSSNITGLLLTR | Pyro | 431-442 | [Hex]7[HexNAc]2 |
| | 2875.2400 | 2875.2383 | 0.6 | | | CSSNITGLLLTR | | 431-442 | [Hex]7[HexNAc]2 |
| | 3010.1744 | 3020.2645 | 3.3 | 1400.6576 | 1317.6576 | CSSNITGLLLTR | PyroC | 431-442 | [Hex]8[HexNAc]2 |
| | 3037.2922 | 3037.2911 | 0.4 | 1417.6700 | 1334.6700 | CSSNITGLLLTR | | 431-442 | [Hex]8[HexNAc]2 |
| | 3182.3301 | 3182.3173 | 4.0 | 1400.7017 | 1317.7017 | CSSNITGLLLTR | PyroC | 431-442 | [Hex]9[HexNAc]2 |
| | 3199.3481 | 3199.3439 | 1.3 | 1417.3895 | 1334.3895 | CSSNITGLLLTR | | 431-442 | [Hex]9[HexNAc]2 |
| | 3210.3374 | 3210.3122 | 7.8 | | | CSSNITGLLLTR | Pyro,For | 431-442 | [Hex]9[HexNAc]2 |
| | 3227.3622 | 3227.3388 | 7.3 | | | CSSNITGLLLTR | For | 431-442 | [Hex]9[HexNAc]2 |
| | | | | | | | | | |
| 28-30 | 2937.3625 | 2937.1952 | 57.0 | | | IWNNMTWMEWER | | 575-586 | [Hex]3[HexNAc]3[Fuc]1 |
| | 3140.3572 | 3140.2745 | 26.3 | 1778.9164 | 1695.9164 | IWNNMTWMEWER | | 575-586 | [Hex]3[HexNAc]4[Fuc]1 |
| | 3546.4897 | 3546.4333 | 15.9 | 1778.9004 | 1695.9004 | IWNNMTWMEWER | | 575-586 | [Hex]3[HexNAc]6[Fuc]1 |
| | 3343.4673 | 3343.3539 | 33.9 | 1778.7937 | 1695.7937 | IWNNMTWMEWER | | 575-586 | [Hex]3[HexNAc]5[Fuc]1 |
| | 3156.4517 | 3156.2695 | 57.7 | | | IWNNMTWMEWER | | 575-586 | [Hex]4[HexNAc]4 |
| | 3188.4783 | 3188.2593 | 68.7 | | | IWNNMTWMEWER | mox | 575-586 | [Hex]4[HexNAc] |
| | 3359.4663 | 3359.3488 | 35.0 | | | IWNNMTWMEWER | | 575-586 | [Hex]4[HexNAc] |
| | 3391.5055 | 3391.3387 | | | | IWNNMTWMEWER | mox | 575-586 | [Hex]4[HexNAc] |
| | 3505.5205 | 3505.4067 | 32.5 | | | IWNNMTWMEWER | | 575-586 | [Hex]4[HexNAc]5[Fuc]1 |
| | 3708.6440 | 3708.4583 | 50.1 | 1778.7129 | 1695.7129 | IWNNMTWMEWER | | 575-586 | [Hex]4[HexNAc]6[Fuc]1 |
| | 2912.2561 | 2912.1635 | 31.8 | | | IWNNMTWMEWER | | 575-586 | [Hex]5[HexNAc]2 |
| | 3464.4841 | 3464.3802 | 30.0 | | | IWNNMTWMEWER | | 575-586 | [Hex]5[HexNAc]4[Fuc]1 |
| | 3667.4997 | 3667.4596 | 10.9 | 1779.0131 | 1696.0131 | IWNNMTWMEWER | | 575-586 | [Hex]5[HexNAc]5[Fuc]1 |
| | 3480.6052 | 3480.3323 | 78.4 | | | IWNNMTWMEWER | | 575-586 | [Hex]6[HexNAc] |
| | 3512.5930 | 3512.3221 | 77.1 | | | IWNNMTWMEWER | mox | 575-586 | [Hex]6[HexNAc]4 |
| | 3829.6699 | 3829.5124 | 41.1 | 1778.9711 | 1695.9711 | IWNNMTWMEWER | | 575-586 | [Hex]6[HexNAc]5[Fuc]1 |
| | 4032.5845 | 4032.5918 | 1.8 | 1779.0432 | 1696.0432 | IWNNMTWMEWER | | 575-586 | [Hex]6[HexNAc]6[Fuc]1 |
| | 3236.4509 | 3236.2692 | 56.1 | | | IWNNMTWMEWER | | 575-586 | [Hex]7[HexNAc]2 |
| | 4194.6055 | 4194.6446 | 9.3 | 1779.0536 | 1696.0536 | IWNNMTWMEWER | | 575-586 | [Hex]7[HexNAc]6[Fuc]1 |
| | 3398.4519 | 3398.3220 | 38.2 | | | IWNNMTWMEWER | | 575-586 | [Hex]8[HexNAc]2 |

**Table 2: MALDI-MS Glycopeptide Assignments For CON-S**

| F# | Experimental | Theoretical | Error | ^{0.1}X | [Peptide+II] | Peptide Sequence | mod | position | Glycan |
|---|---|---|---|---|---|---|---|---|---|
| 3 | 2004.8567 | 2004.7914 | 32.6 | 871.3885 | 788.3885 | EHFNNK | | 347-352 | [Hex]5[HexNAc]21 |
| | 2232.9470 | 2232.9024 | 20.0 | 871.3597 | 788.3597 | EHFNNK | | 347-352 | [Hex]3[HexNAc]4[Fuc]1 |
| | | | | | | | | | |
| 3-10 | 1961.9016 | 1961.8710 | 15.6 | 1140.6959 | 1057.6959 | LREHFNNK | For | | [Hex]2[HexNAc]2[Fuc]1 |
| | 2136.9360 | 2136.9560 | 9.4 | | | LREHFNNK | | 345-352 | [Hex]2[HexNAc]3[Fuc]1 |
| | 1949.8629 | 1949.8710 | 4.2 | 1140.8523 | 1057.8523 | LREHFNNK | | 345-352 | [Hex]3[HexNAc] |
| | 1977.8767 | 1977.8659 | 5.5 | 1140.6898 | 1057.6898 | LREHFNNK | For | 345-352 | [Hex]3[HexNAc]2 |
| | 1992.8917 | 1992.8768 | 7.5 | | | LREHFNNK | U | 345-352 | [Hex]3[HexNAc]2 |
| | 2095.9573 | 2095.9289 | 13.6 | | | LREHFNNK | | 345-352 | [Hex]3[HexNAc]2[Fuc]1 |
| | 2152.9226 | 2152.9504 | 12.9 | | | LREHFNNK | | 345-352 | [Hex]3[HexNAc]3 |
| | 2180.9934 | 2180.9453 | 22.1 | | | LREHFNNK | For | 345-352 | [Hex]3[HexNAc]3 |
| | 2195.9373 | 2195.9562 | 8.6 | | | LREHFNNK | U | 345-352 | [Hex]3[HexNAc]3 |
| | 2298.9907 | 2299.0083 | 7.7 | 1140.5957 | 1057.5957 | LREHFNNK | | 345-352 | [Hex]3[HexNAc]3[Fuc]1 |
| | 2327.0503 | 2327.0032 | 20.2 | 1140.6896 | 1057.5538 | LREHFNNK | For | 345-352 | [Hex]3[HexNAc]3[Fuc]1 |
| | 2342.0002 | 2342.0141 | 5.9 | 1183.6112 | 1100.6112 | LREHFNNK | U | 345-352 | [Hex]3[HexNAc]3[Fuc]1 |
| | 2356.0176 | 2356.0297 | 5.1 | | | LREHFNNK | | 345-352 | [Hex]3[HexNAc]4 |
| | 2383.9631 | 2384.0247 | 25.8 | | | LREHFNNK | For | 345-352 | [Hex]3[HexNAc]4 |
| | 2502.0662 | 2502.0876 | 8.6 | 1140.6412 | 1057.6412 | LREHFNNK | | 345-352 | [Hex]3[HexNAc]4[Fuc]1 |
| | 2530.1099 | 2530.0826 | 10.8 | 1140.6864 | 1057.5538 | LREHFNNK | For | 345-352 | [Hex]3[HexNAc]4[Fuc]1 |
| | 2545.1033 | 2545.0934 | 3.9 | 1183.6959 | 1100.6959 | LREHFNNK | U | 345-352 | [Hex]3[HexNAc]4[Fuc]1 |
| | 2559.1252 | 2559.1091 | 6.3 | 1140.5767 | 1057.5538 | LREHFNNK | | 345-352 | [Hex]3[HexNAc]5 |
| | 2602.0845 | 2602.1149 | 11.7 | | | LREHFNNK | U | 345-352 | [Hex]3[HexNAc]5 |
| | 2705.1394 | 2705.1670 | 10.2 | | | LREHFNNK | | 345-352 | [Hex]3[HexNAc]5[Fuc]1 |
| | 2733.1826 | 2733.1619 | 7.6 | | | LREHFNNK | For | 345-352 | [Hex]3[HexNAc]5[Fuc]1 |
| | 2748.1536 | 2748.1729 | 7.0 | 1183.9606 | 1100.9606 | LREHFNNK | U | 345-352 | [Hex]3[HexNAc]5[Fuc]1 |
| | 2908.2629 | 2908.2464 | 5.7 | 1140.7122 | 1057.5538 | LREHFNNK | | 345-352 | [Hex]3[HexNAc]6[Fuc]1 |
| | 2936.2362 | 2976.2414 | | | | LREHFNNK | For | 345-352 | [Hex]3[HexNAc]6[Fuc]1 |
| | 2951.2561 | 2951.2527 | 1.2 | 1183.6116 | 1100.6116 | LREHFNNK | U | 345-352 | [Hex]3[HexNAc]6[Fuc]1 |
| | 2111.9068 | 2111.9238 | 8.0 | 1140.5538 | 1057.5538 | LREHFNNK | | 345-352 | [Hex]4[HexNAc] |
| | 2139.9651 | 2139.9188 | 21.6 | 1140.6241 | 1057.5538 | LREHFNNK | For | 345-352 | [Hex]4[HexNAc]2 |
| | 2154.9404 | 2154.9296 | 5.0 | 1183.5651 | 1100.5651 | LREHFNNK | U | 345-352 | [Hex]4[HexNAc] |
| | 2258.0251 | 2257.9817 | 19.2 | | | LREHFNNK | | 345-352 | [Hex]4[HexNAc]2[Fuc]1 |
| | 2285.9465 | 2285.9766 | | | | LREHFNNK | For | 345-352 | [Hex]4[HexNAc]2[Fuc]1 |
| | 2314.9868 | 2315.0032 | 7.1 | | | LREHFNNK | | 345-352 | [Hex]4[HexNAc] |
| | 2343.0317 | 2342.9981 | 14.3 | | | LREHFNNK | For | 345-352 | [Hex]4[HexNAc]3 |
| | 2357.9973 | 2358.0090 | 5.0 | | | LREHFNNK | U | 345-352 | [Hex]4[HexNAc] |
| | 2461.0405 | 2461.0611 | 8.4 | | | LREHFNNK | | 345-352 | [Hex]4[HexNAc]3[Fuc]1 |
| | 2489.0342 | 2489.0560 | 8.8 | | | LREHFNNK | For | 345-352 | [Hex]4[HexNAc]3[Fuc]1 |
| | 2504.0708 | 2504.0669 | 1.6 | 1183.5569 | 1100.5569 | LREHFNNK | U | 345-352 | [Hex]4[HexNAc]3[Fuc]1 |
| | 2518.0513 | 2518.0826 | 12.4 | | | LREHFNNK | | 345-352 | [Hex]4[HexNAc] |
| | 2664.1150 | 2664.1405 | 9.6 | | | LREHFNNK | | 345-352 | [Hex]4[HexNAc]4[Fuc]1 |
| | 2692.1648 | 2692.1354 | 10.9 | 1168.6179 | 1085.6179 | LREHFNNK | For | 345-352 | [Hex]4[HexNAc]4[Fuc]1 |
| | 2707.1616 | 2707.1463 | 5.7 | 1183.5730 | 1100.5730 | LREHFNNK | U | 345-352 | [Hex]4[HexNAc]4[Fuc]1 |
| | 2721.1809 | 2721.1620 | 6.9 | | | LREHFNNK | | 345-352 | [Hex]4[HexNAc]6 |
| | 2867.2256 | 2867.2198 | 2.0 | 1140.651 | 1057.5538 | LREHFNNK | | 345-352 | [Hex]4[HexNAc]5[Fuc]1 |
| | 2895.2046 | 2895.2147 | 3.5 | | | LREHFNNK | For | 345-352 | [Hex]4[HexNAc]5[Fuc]1 |
| | 2910.2202 | 2910.2256 | 1.9 | 1183.5436 | 1100.5436 | LREHFNNK | U | 345-352 | [Hex]4[HexNAc]5[Fuc]1 |
| | 3070.3928 | 3070.2997 | 30.3 | 1140.6404 | 1057.6404 | LREHFNNK | | 345-352 | [Hex]4[HexNAc]6[Fuc]1 |
| | 2273.9646 | 2273.9766 | 5.3 | 1140.5774 | 1057.5774 | LREHFNNK | | 345-352 | [Hex]5[HexNAc]2 |
| | 2302.0171 | 2301.9715 | 19.8 | 1140.6028 | 1057.5538 | LREHFNNK | For | 345-352 | [Hex]5[HexNAc]2 |
| | 2316.9829 | 2316.9824 | 0.2 | | | LREHFNNK | u | 345-352 | [Hex]5[HexNAc]2 |
| | 2477.0398 | 2477.0560 | 6.5 | | | LREHFNNK | | 345-352 | [Hex]5[HexNAc]3 |
| | 2520.0627 | 2520.0618 | 0.4 | 1183.5493 | 1100.5493 | LREHFNNK | U | 345-352 | [Hex]5[HexNAc]3 |
| | 2623.0879 | 2623.1139 | 9.9 | | | LREHFNNK | | 345-352 | [Hex]5[HexNAc]3[Fuc]1 |
| | 2651.1321 | 2651.1088 | 8.8 | | | LREHFNNK | For | 345-352 | [Hex]5[HexNAc]3[Fuc]1 |
| | 2666.1113 | 2666.1197 | 3.2 | 1183.5592 | 1100.5592 | LREHFNNK | u | 345-352 | [Hex]5[HexNAc]3[Fuc]1 |
| | 2680.0977 | 2680.1354 | 14.1 | | | LREHFNNK | | 345-352 | [Hex]5[HexNAc]4 |
| | 2826.2041 | 2826.1933 | 3.8 | 1140.6008 | 1057.5538 | LREHFNNK | | 345-352 | [Hex]5[HexNAc]4[Fuc]1 |
| | 2854.1816 | 2854.1882 | 2.3 | | | LREHFNNK | For | 345-352 | [Hex]5[HexNAc]4[Fuc]1 |
| | 1869.2039 | 2869.1991 | 1.7 | 1183.5896 | 1100.5896 | LREHFNNK | U | 345-352 | [Hex]5[HexNAc]4[Fuc]1 |
| | 2883.0730 | 2883.2148 | 49.2 | | | LREHFNNK | | 345-352 | [Hex]5[HexNAc]5 |
| | 3029.2881 | 3029.2727 | 5.1 | 1140.6306 | 1057.5538 | LREHFNNK | | 345-352 | [Hex]5[HexNAc]5[Fuc]1 |
| | 3057.0841 | 3057.268 | 60.0 | | | LREHFNNK | For | 345-352 | [Hex]5[HexNAc]5[Fuc]1 |
| | 2436.0095 | 2436.0295 | 8.2 | | | LREHFNNK | | 345-352 | [Hex]6 [HexNAc]2 |
| | 2479.0408 | 2479.0353 | 2.2 | 1183.5679 | 1100.5679 | LREHFNNK | U | 345-352 | [Hex]6[HexNAc]2 |
| | 2639.1279 | 2639.1088 | 7.2 | | | LREHFNNK | | 345-352 | [Hex]6[HexNAc]3 |
| | 2682.0945 | 2682.1146 | 7.5 | | | LREHFNNK | U | 345-352 | [Hex]6[HexNAc]3 |
| | 2185.1487 | 2785.1667 | 6.5 | | | LREHFNNK | | 345-352 | [Hex]6[HexNAc]3[Fuc]1 |
| | 2813.1521 | 2813.1616 | 3.4 | | | LREHFNNK | For | 345-352 | [Hex]6[HexNAc]3[Fuc]1 |
| | 2828.1577 | 2828.1725 | 5.2 | | | LREHFNNK | U | 345-352 | [Hex]6[HexNAc]3[Fuc]1 |
| | 3191.2815 | 3191.3255 | 13.8 | | | LREHFNNK | | 345-352 | [Hex]6 [HexNAc]5[Fuc]1 |
| | 2598.1082 | 2598.0823 | 10.0 | 1140.5834 | 1057.5538 | LREHFNNK | | 345-352 | [Hex]7[HexNAc]2 |
| | 2626.0715 | 2626.0772 | 2.2 | | | LREHFNNK | For | 345-352 | [Hex]7[HexNAc]2 |
| | 2641.0942 | 2641.0881 | 2.3 | | | LREHFNNK | u | 345-352 | [Hex]7[HexNAc]2 |
| | 1801.0750 | 2801.1616 | 30.9 | | | LREHFNNK | | 345-352 | [Hex]7[HexNAc]3 |
| | 2829.2004 | 2829.1565 | 15.5 | | | LREHFNNK | For | 345-352 | [Hex]7[HexNAc]3 |
| | 3453.1436 | 3453.4056 | 75.9 | | | LREHFNNK | U | 345-352 | [Hex]7[HexNAc]6 |
| | 2760.1155 | 2760.1351 | 7.1 | 1140.6312 | 1057.6312 | LREHFNNK | | 345-352 | [Hex]8[HexNAc]2 |
| | 2738.2285 | 2788.1300 | 35.3 | | | LREHFNNK | For | 345-352 | [Hex]8[HexNAc]2 |
| | 2803.1436 | 2803.1409 | 1.0 | 1183.5778 | 1100.5778 | LREHFNNK | U | 345-352 | [Hex]8[HexNAc]2 |
| | 2963.2173 | 2963.215 | 0.8 | 1140.7018 | 1057.7018 | LREHFNNK | | 345-352 | [Hex]8[HexNAc]3 |
| | 2922.1624 | 2922.1879 | 8.7 | 1140.5961 | 1057.5961 | LREHFNNK | | 343-352 | [Hex]9[HexNAc]2 |
| | 2950.1631 | 1930.1828 | 6.7 | | | LREHFNNK | For | 345-352 | [Hex]9[HexNAc]2 |
| | 2965.1924 | 1965.1937 | 0.4 | 1183.6176 | 1100.6176 | LREHFNNK | U | 345-352 | [Hex]9[HexNAc]2 |
| | | | | | | | | | |
| 5 | 2044.8149 | 2044.8550 | 19.7 | 1073.3104 | 990.3304 | SENITNNAK | | 271-279 | [Hex]4[HexNAc]2 |
| | 2206.8865 | 2206.9080 | 9.7 | 1073.5948 | 990.5948 | SENITNNAK | | 271-279 | [Hex]5[HexNAc]2 |
| | | | | | | | | | |
| | 2096.7878 | 2096.8210 | 15.8 | 963.7707 | 880.7707 | FNGTGPCK | | 230-237 | [Hex]5[HexNAc]2 |
| | 2935.1594 | 2935.0800 | 27.1 | | | FNGTGPCK | | 230-237 | [Hex]10[HexNAc]2 |
| | 2905.1387 | 2905.1437 | 1.7 | | | FNGTGPCK | | 230-237 | [Hex]3[HexNAc]6[Fuc]2 |
| | | | | | | | | | |
| 6 | 2152.9331 | 2152.8698 | 35.6 | 1181.7933 | 1098.7933 | QAHCNISGTK | PyroQ | 314-333 | [Hex]4[HexNAc]2 |
| | 2502.1433 | 2502.0070 | 54.5 | 1181.5763 | 1098.5763 | QAHCNISGTK | PyroQ | 324-333 | [Hex]4[HexNAc]4 |
| | 2619.1443 | 2619.0609 | 31.8 | | | QAHCNISGTK | U | 314.333 | [Hex]4[HexNAc]4 |
| | 2705.2190 | 2705.0864 | 49.0 | 1181.2706 | 1098.2706 | QAHCNISGTK | PyroQ | 324-333 | [Hex]4[HexNAc]5 |
| | 2315.0049 | 2314.9225 | 35.6 | 1181.5394 | 1098.5394 | QAHCNISGTK | PyroQ | 324-333 | [Hex]5[HexNAc]2 |
| | 2342.9916 | 2342.9174 | 34.2 | 1181.6140 | 1098.6140 | QAHCIVISGTK | PyroQ,For | 324-333 | [Hex]5[HexNAc]2 |
| | 2375.0718 | 2374.9549 | 49.2 | | | QAHCNISGTK | U | 314.333 | [Hex]5[HexNAc]2 |
| | 2477.0840 | 2476.9754 | 43.8 | 1181.5067 | 1098.5067 | QAHCNISGTK | PyroQ | 324-333 | [Hex]6[HexNAc]2 |
| | 2505.1428 | 2504.9703 | 68.9 | | | QAHCNISGTK | PyroQ,For | 324-333 | [Hex]6[HexNAc]2 |
| | 2639.1426 | 2639.0282 | 43.3 | 1181.5585 | 1098.5585 | QAHCNISGTK | PyroQ | 324-333 | [Hex]7[HexNAc]2 |
| | 2801.1992 | 2801.0810 | 42.2 | | | QAHCNISGTK | PyroQ | 324-333 | [Hex]8[HexNAc]2 |
| | 2829.2283 | 2829.0759 | 53.9 | 1181.6649 | 1098.2706 | QAHCNISGTK | PyroQ,For | 314-333 | [Hex]8[HexNAc]2 |
| | 2963.2224 | 2963.1338 | 31.8 | 1181.6060 | 1098.606 | QAHCNISGTK | PyroQ | 324-333 | [Hex]9[HexNAc]2 |
| | 2991.2319 | 2991.1287 | 34.5 | 1181.2990 | 1098.2990 | QAHCNISGTK | PyroQ,For | 324-333 | [Hex]9[HexNAc]2 |
| | | | | | | | | | |
| 6-8 | 2688.2720 | 2688.0910 | 67.3 | 1591.1300 | 1508.1300 | CNDKKFNGTGPCK | PyroC,u | 225.237 | [Hex]2[HexNAc]4 |
| | 2485.1846 | 2485.0166 | 67.6 | | | CNDKKFNGTGPCK | PyroC,u | 225-237 | [Hex]2[HexNAc]5 |
| | 2400.9614 | 2400.9792 | 7.4 | | | CNDKKFNGTGPCK | PyroC | 225-237 | [Hex]3 [HexNAc]2 |
| | 2444.0171 | 2443.9850 | 13.1 | | | CNDKKFNGTGPCK | Pyroc,U | 225-237 | [Hex]3[HexNAc]2 |
| | 2750.1011 | 2750.1165 | 5.6 | 1591.6967 | 1508.8652 | CNDKKFNGTGPCK | PyroC | 225-237 | [Hex]3 [HexNAc]3[Fuc]1 |
| | 2953.3030 | 2953.1958 | 24.9 | 1591.7927 | 1508.7705 | CNDKKFNGTGPCK | PyroC | 225-237 | [Hex]3[HexNAc]4[Fuc]1 |
| | 3156.2390 | 3156.2752 | 11.5 | 1591.7273 | 1508.7273 | CNDKKFNGTGPCK | PyroC | 225-237 | [Hex]3[HexNAc]5[Fuc]1 |
| | 2563.0159 | 2563.0320 | 6.3 | | | CNDKKFNGTGPCK | PyroC | 225-237 | [Hex]4[HexNAc]2 |
| | 2606.0569 | 2606.0379 | 7.3 | | | CNDKKFNGTGPCK | | 225-237 | [Hex]4[HexNAc]2 |
| | 2912.0967 | 2912.1693 | 24.9 | 1591.7705 | 1508.7705 | CNDKKFNGTGPCK | PyroC | 225-237 | [Hex]4[HexNAc]3[Fuc]1 |
| | 2725.0557 | 2725.0848 | 10.7 | 1591.8652 | 1508.8652 | CNDKKFNGTGPCK | PyroC | 225-237 | [Hex]5[HexNAc]2 |
| | 2754.0549 | 2754.1114 | 20.5 | | | CNDKKFNGTGPCK | For | 225-237 | [Hex]4[HexNAc]2[Fuc]1 |
| | 3049.1704 | 3049.1905 | 6.6 | | | CNDKKFNGTGPCK | PyroC | 225-237 | [Hex]7 [HexNAc]2 |
| | 3211.2683 | 3211.2767 | 2.6 | 1591.9930 | 1508.993 | CNDKKFNGTGPCK | PyroC | 225-237 | [Hex]8[HexNAc]2 |
| | 3373.3042 | 3373.3295 | 7.5 | 1592.0395 | 1509.0395 | CNDKKFNGTGPCK | PyroC | 225-237 | [Hex]9[HexNAc]2 |
| | | | | | | | | | |
| 12 | 2929.3943 | 2929.2361 | 54.0 | | | NNNNTNDTITLPCR | | 397-410 | [Hex]2[HexNAc]4[Fuc]1 |
| 14-16 | 2539.0361 | 2539.0722 | 14.2 | 1729.9348 | 1646.9348 . | NNNNTNDTITLPCR | | 397-410 | [Hex]3[HexNAc]2 |
| | 2582.2520 | 2582.0780 | 67.4 | 1777.5357 | 1690.5357 | NNNNTNDTITLPCR | U | 397-410 | [Hex]3[HexNAc]2 |
| | 2888.4421 | 2888.2185 | 77.4 | 1719.8123 | 1646.8123 | NNNNTNDTITLPCR | | 397-410 | [Hex]3[HexNAc]3[Fuc]1 |
| | 3091.2617 | 3091.2888 | 8.8 | 1730.0999 | 1647.0999 | NNNNTNDTITLPCR | | 397-410 | [Hex]3[HexNAc]4[Fuc]1 |
| | 3119.5381 | 3119.2837 | 81.6 | 1729.8289 | 1646.8289 | NNNNTNDTITLPCR | For | 397-410 | [Hex]3[HexNAc]5[Fuc]1 |
| | 3294.3725 | 3294.3683 | 1.3 | 1730.0304 | 1647.0304 | NNNNTNDTITLPCR | | 397-410 | [Hex]3[HexNAc]5[Fuc]1 |
| | 2701.1055 | 2701.1251 | 7.3 | 1729.9556 | 1646.9556 | NNNNTNDTITLPCR | | 397-410 | [Hex]4[HexNAc]2 |
| | 2744.1780 | 2744.1308 | 17.2 | 1729.8444 | 1646.8444 | NNNNTNDTITLPCR | U | 397-410 | [Hex]4[HexNAc]2 |
| | 2904.2659 | 2904.2044 | 21.2 | 1729.9015 | 1646.9015 | NNNNTNDTITLPCR | | 397-410 | [Hex]4[HexNAc]3 |
| | 3050.3079 | 3050.2623 | 14.9 | 1729.8844 | 1646.8844 | NNNNTNDTITLPCR | | 397-410 | [Hex]4[HexNAc]3[Fuc]1 |
| | 3135.5369 | 3135.2787 | 82.4 | | | NNNNTNDTITLPCR | For | 397-410 | [Hex]4[HexNAc]4 |
| | 3253.4631 | 3253.3417 | 37.3 | 1729.9799 | 1646.9799 | NNNNTNDTITLPCR | | 397-410 | [Hex]4[HexNAc]4[Fuc]1 |
| | 3338.6038 | 3338.3581 | 73.6 | | | NNNNTNDTITLPCR | For | 397-410 | [Hex]4[HexNAc]5 |
| | 3456.3638 | 3456.4210 | 16.5 | | | NNNNTNDTITLPCR | | 397-410 | [Hex]4[HexNAc]5[Fuc]1 |
| | 2863.1807 | 2863.1778 | 1.0 | 1730.0854 | 1647.0854 | NNNNTNDTITLPCR | | 397-410 | [Hex]5[HexNAc]2 |
| | 3066.3567 | 3066.2572 | 32.4 | 1729.8693 | 1646.8693 | NNNNTNDTITLPCR | | 397-410 | [Hex]5[HexNAc]3 |
| | 3212.4602 | 3212.3151 | 45.2 | 1719.8853 | 1646.8853 | NNNNTNDTITLPCR | | 397-410 | [Hex]5[HexNAc]3[Fuc]1 |
| | 3312.3333 | 3312.3424 | 2.7 | | | NNNNTNDTITLPCR | U | 397-410 | [Hex]5[HexNAc]4 |
| | 3025.3657 | 3025.2307 | 44.6 | 1729.8638 | 1646.8638 | NNNNTNDTITLPCR | | 397-410 | [Hex]6[HexNAc]2 |
| | 3954.5527 | 3954.5795 | 6.8 | | | NNNNTNDTITLPCR | For | 397-410 | [Hex]6[HexNAc]5[Fuc]2 |
| | 3187.3948 | 3187.2835 | 34.9 | 1729.8529 | 1646.8529 | NNNNTNDTITLPCR | | 397-410 | [Hex]7[HexNAc]2 |
| | 3349.4900 | 3349.3363 | 45.9 | 1729.9173 | 1646.9173 | NNNNTNDTITLPCR | | 397-410 | [Hex]8[HexNAc]2 |
| | 3511.5010 | 3511.3891 | 31.9 | 1729.5841 | 1646.5841 | NNNNTNDTITLPCR | | 397-410 | [Hex]9[HexNAc]2 |
| | 4890.0397 | 4889.8654 | 35.6 | | | NNNNTNDTITLPCR | | 397-410 | [Hex]15[HexNAc]4 |
| | 5051.9805 | 5051.9182 | 12.3 | 3433.4656 | 3350.4656 | NNNNTNDTITLPCR | | 397-410 | [Hex]16[HexNAc]4 |
| | 5214.0788 | 5213.9710 | 20.7 | 3433.7410 | 3350.7410 | NNNNTNDTITLPCR | | 397-410 | [Hex]17[HexNAc]4 |
| | 5376.3584 | 5376.0239 | 62.2 | 3433.3542 | 3350.3542 | NNNNTNDTITLPCR | | 397-410 | [Hex]18[HexNAc]4 |
| | | | | | | | | | |
| 12 | 3143.2939 | 3143.2965 | 0.8 | | | DGGNNNTNETEIFRPGGGDMR | | 448-408 | [Hex]3[HexNAc]2 |
| 15-17 | 3492.4683 | 3492.4683 | 0.0 | | | DGGNNNTNETEIFRPGGGDMR | | 448-408 | [Hex]3[HexNAc]3[Fuc]1 |
| | 3695.5764 | 3695.5131 | 17.1 | | | DGGNNNTNETEIFRPGGGDMR | | 448408 | [Hex]3[HexNAc]4[Fuc]1 |
| | 3723.4619 | 3723.5080 | 12.4 | | | DGGNNNTNETEIFRPGGGDMR | For | 448-408 | [Hex]3[HexNAc]4[Fuc]1 |
| | 3695.5459 | 3695.5132 | 8.8 | 2316.9102 | 2233.9102 | DGGNNNTNETEIFRPGGGDMR | | 448-408 | [Hex]3[HexNAc]4[Fuc]1 |
| | 3898.6094 | 3898.5926 | 4.3 | 2316.2063 | 2233.2063 | DGGNNNTNETEIFRPGGGDMR | | 448-408 | [Hex]3[HexNAc]5[Fuc]1 |
| | 3926.6179 | 3926.5875 | 7.7 | 2333.9707 | 2250.9707 | DGGNNNTNETEFRPGGGDMR | For | 448-408 | [Hex]3[HexNAc]5[Fuc]1 |
| | 4119.8287 | 4129.6670 | | | | DGGNNNTNETEIFRPGCGDMR | For | 448-408 | [Hex]3[HexNAc]6[Fuc]1 |
| | 4322.8402 | 4222.6980 | | 2334.1511 | 2251.1511 | DGGNNNTNETEIFRPGGGDMR | | 448-408 | [Hex]3[HexNAc]5[Fuc]1 |
| | 4101.7837 | 4101.6723 | 27.2 | 2334.0486 | 1151.0486 | DGGNNNTNETEIFRPGGGDMR | | 448-408 | [Hex]3[HexNAc]6[Fuc]1 |
| | 4144.8853 | 4144.6778 | 50.1 | | | DGGNNNTNETEIFRPGGGDMR | U | 448-408 | [Hex]3[HexNAc]6[Fuc]1 |
| | 4304.3888 | | | | | DGGNNNTNETEIFRPGGGDMR | | 448-408 | [Hex]3[HexNAc]7[Fuc]1 |
| | 3289.3408 | 3289.3544 | 4.1 | 2317.9521 | 2134.9521 | DGGNNNTNETEIFRPGGGDMR | | 448-408 | [Hex]4[HexNAc]2 |
| | 3305.3665 | 3305.3493 | 5.2 | 2333.9445 | 2250.9445 | DGGNNNTNETEIFRPGGGDMR | | 448-408 | [Hex]4[HexNAc]2 |
| | 3654.5195 | 3654.4866 | 9.0 | | | DGGNNNTNETEIFRPGGGDMR | | 448-408 | [Hex]4[HexNAc]3[Fuc]1 |
| | 3711.5139 | 3711.5081 | 1.6 | | | DGGNNNTNETEIFRGGGDMR | | 448-408 | [Hex]4[HexNAc]4 |
| | 3857.5933 | 3857.5660 | 7.1 | 2333.947 | 2250.947 | DGGNNNTNETEIFRPGGGDMR | | 448-408 | [Hex]4[HexNAc]4 [Fuc]1 |
| | 3898.6182 | 3898.5926 | 6.6 | 2334.0938 | 2251.0938 | DGGNNNTNETEIFRPGGGDMR | | 448-408 | [Hex]3[HexNAc]5[Fuc]1 |
| | 3914.6484 | 3914.5875 | 15.6 | | | DGGNNNTNETEIFRPGGGDMR | | 448-408 | [Hex]4[HexNAc]5 |
| | 4060.7559 | 4060.6453 | 27.2 | 2334.0450 | 2251.045 | DGGNNNTNETEIFRPGGGDMR | | 448-408 | [Hex]4[HexNAc]5[Fuc]1 |
| | 4088.7886 | 4088.6403 | 36.3 | | | DGGNNNTNETEIFRPGGGDMR | For | 448-408 | [Hex]4[HexNAc]5[Fuc]1 |
| | 4263.7798 | 4263.725 | 12.9 | 2333.7738 | 2250.7738 | DGGNNNTNETEIFRPGGGDMR | | 448-408 | [Hex]4[HexNAc]6[Fuc]1 |
| | 3467.4426 | 3467.4021 | 11.7 | 2317.8085 | 2234.8085 | DGGNNNTNETEIFRPGGGDMR | | 448-408 | [Hex]5[HexNAc]2 |
| | 3816.5886 | 3916.5394 | 12.9 | | | DGGNNNTNETEIFRPGGGDMR | | 448-408 | [Hex]5[HexNAc]3[Fuc]1 |
| | 4019.6672 | 4019.6188 | 12.0 | 2333.8958 | 2250.8958 | DGGNNNTNETEIFRPGGGDMR | | 448-408 | [Hex]5[HexNAc]4[Fuc]1 |
| | 4425.8540 | | | 2334.3171 | 2251.3171 | DGGNNNTNETEIFRPGGGDMR | | 448-408 | [Hex]5[HexNAc]4[Fuc]1 |
| | 4222.7593 | 4222.6982 | 14.5 | 2334.5022 | 2251.5022 | DGGNNNTNETEIFRPGGGDMR | | 448-408 | [Hex]5[HexNAc]5[Fuc]1 |
| | 4425.8745 | | | 2333.8787 | 2250.8787 | DGGNNNTNETEIFRPGGGDMR | | 448-408 | [Hex]5[HexNAc]6[Fuc]1 |
| | 3629.5090 | 3629.4550 | 14.9 | | | DGGNNNTNETEIFRPGGGDMR | | 448-408 | [Hex]6[HexNAc]2 |
| | 4384.9868 | 4384.7510 | 53.8 | 2334.1511 | 2251.1511 | DGGNNNTNETEIFRPGGGDMR | | 448-408 | [Hex]6[HexNAc]5[Fuc]1 |
| | 3791.5891 | 3791.5078 | 21.4 | | | DGGNNNTNETEIFRPGGGDMR | | 448-408 | [Hex]7[HexNAc]2 |
| | 3953.6326 | 3953.5606 | 18.2 | | | DGGNNNTNETEIFRPGGGDMR | | 448-408 | [Hex]8[HexNAc]2 |
| | 4115.6401 | 4115.6134 | 6.5 | 2334.188 | 2251.188 | DGGNNNTNETEIFRPGGGDMR | | 448-408 | [Hex]9[HexNAc]2 |
| | 4305.9760 | 4305.6611 | 73.1 | | | DGGNNNTNETEIFRPGGGDMR | For | 448-408 | [Hex]10[HexNAc]2 |
| | | | | | | | | | |
| 17-19 | 3231.4648 | 3231.3803 | 26.1 | | | LDVVPIDDNNNNSSNYR | | 173-189 | [Hex]2[HexNAc]4[Fuc]1 |
| 23 | 2841.2253 | 2841.2164 | 3.1 | 2032.1564 | 1949.1564 | LDVVPIDDNNNNSSNYR | | 173-189 | [Hex]3[HexNAc]2 |
| | 3044.2886 | 3044.2958 | 2.4 | | | LDVVPIDDNNNNSSNYR | | 173-189 | [Hex]3[HexNAc]3 |
| | 3190.3647 | 3190.3537 | 3.4 | 2031.891 | 1948.891 | LDVVPIDDNNNNSSNYR | | 173-189 | [Hex]3[HexNAc]3[Fuc]1 |
| | 3247.4536 | 3247.3751 | 24.2 | | | LDVVPIDDNNNNSSNYR | | 173-189 | [Hex]3[HexNAc]4 |
| | 3393.4331 | 3393.4330 | 0.0 | 2032.0162 | 1949.0162 | LDVVPIDDNNNNSSNYR | | 173-189 | [Hex]3[HexNAc]4[Fuc]1 |
| | 3421.5024 | 3421.4279 | 21.8 | 2031.6831 | 1948.6831 | LDVVPIDDNNNNSSNYR | For | 173-189 | [Hex]3[HexNAc]4[Fuc]1 |
| | 3450.4529 | 3450.4546 | 0.5 | 2031.9046 | 1948.9046 | LDVVPIDDNNNNSSNYR | | 173-189 | [Hex]3[HexNAc]5 |
| | 3596.5557 | 3596.5125 | 12.0 | 2032.0199 | 1949.0199 | LDVVPIDDNNNNSSNYR | | 173-189 | [Hex]3[HexNAc]5[Fuc]1 |
| | 3624.5776 | 3624.5074 | 19.4 | 2031.9602 | 1948.9602 | LDVVPIDDNNNNSSNYR | For | 173-189 | [Hex]3[HexNAc]5[Fuc]1 |
| | 3799.6394 | 3799.5919 | 12.5 | | | LDVVPIDDNNNNSSNYR | | 173-189 | [Hex]3[HexNAc]6[Fuc]1 |
| | 3827.6697 | 3827.5868 | 21.7 | 2031.8813 | 1948.8813 | LDVVPIDDNNNNSSNYR | For | 173-189 | [Hex]3[HexNAc]6[Fuc]1 |
| | 3842.8455 | 3842.5977 | 64.5 | | | LDVVPIDDNNNNSSNYR | U | 173-189 | [Hex]3[HexNAc]6[Fuc]1 |
| | 4002.7573 | | | | | LDVVPIDDNNNNSSNYR | | 173-189 | [Hex]3[HexNAc]7[Fuc]1 |
| | 3003.2751 | 3003.2693 | 1.9 | 2031.9586 | 1948.9586 | LDVVPIDDNNNNSSNYR | | 173-189 | [Hex]4[HexNAc]2 |
| | 3206.2073 | 3206.3486 | 44.1 | | | LDVVPIDDNNNNSSNYR | | 173-189 | [Hex]4[HexNAc]3 |
| | 3352.3937 | 3352.4065 | 3.8 | 2031.5471 | 1948.5471 | LDVVPIDDNNNNSSNYR | | 173-189 | [Hex]4[HexNAc]3[Fuc]1 |
| | 3555.5718 | 3555.4859 | 24.2 | 2031.9476 | 1948.9476 | LDVVPIDDNNNNSSNYR | | 173-189 | [Hex]4[HexNAc]4[Fuc]1 |
| | 3758.6563 | 3758.5652 | 24.2 | 2031.9564 | 1948.9564 | LDVVPIDDNNNNSSNYR | | 173-189 | [Hex]4[HexNAc]5[Fuc]1 |
| | 3786.6106 | 3786.5602 | 13.3 | | | LDVVPIDDNNNNSSNYR | For | 173-189 | [Hex]4[HexNAc]5[Fuc]1 |
| | 3961.7151 | 3961.6451 | 17.7 | 2031.8823 | 1948.8823 | LDVVPIDDNNNNSSNYR | | 173-189 | [Hex]4[HexNAc]6[Fuc]1 |
| | 4164.7207 | | | 2031.9269 | 1948.9269 | LDVVPIDDNNNNSSNYR | | 173-189 | [Hex]4[HexNAc]7[Fuc]1 |
| | 3165.3523 | 3165.3220 | 9.6 | 2031.2015 | 1949.2015 | LDVVPIDDNNNNSSNYR | | 173-189 | [Hex]5[HexNAc]2 |
| | 3368.4202 | 3368.4014 | 5.6 | | | LDVVPIDDNNNNSSNYR | | 173-189 | [Hex]5[HexNAc]3 |
| | 3514.4844 | 3514.4593 | 7.1 | | | LDVVPIDDNNNNSSNYR | | 173-189 | [Hex]5[HexNAc]3[Fuc]1 |
| | 3717.5706 | 3717.5387 | 8.6 | 2031.1868 | 1948.1868 | LDVVPIDDNNNNSSNYR | | 173-189 | [Hex]5[HexNAc]4[Fuc]1 |
| | 3774.6531 | 3774.5602 | 24.6 | | | LDVVPIDDNNNNSSNYR | | 173-189 | [Hex]5[HexNAc]5 |
| | 3920.6768 | 3920.6181 | 15.0 | 2031.0206 | 1948.0206 | LDVVPIDDNNNNSSNYR | | 173-189 | [Hex]5[HexNAc]5[Fuc]1 |
| | 4123.7106 | 4123.6980 | 3.1 | 2032.0349 | 1949.0349 | LDVVPIDDNNNNSSNYR | | 173-189 | [Hex]5[HexNAc]6[Fuc]1 |
| | 4326.8469 | | | 2032.03 | 1949.03 | LDVVPIDDNNNNSSNYR | | 173-189 | [Hex]5[HexNAc]7[Fuc]1 |
| | 3327.3921 | 3327.3749 | 5.2 | 2031.7514 | 1948.7514 | LDVVPIDDNNNNSSNYR | | 173-189 | [Hex]6[HexNAc]2 |
| | 4082.7095 | 4082.6709 | | | | LDVVPIDDNNNNSSNYR | | 173-189 | [Hex]6[HexNAc]5[Fuc]1 |
| | 4285.8389 | 4285.8031 | 8.4 | 2031.6776 | 1948.6776 | LDVVPIDDNNNNSSNYR | | 173-189 | [Hex]6[HexNAc]6[Fuc]1 |
| | 4488.9243 | | | 2031.9003 | 1948.9003 | LDVVPIDDNNNNSSNYR | | 173.189 | [Hex]6[HexNAc]7[Fuc]1 |
| | 3489.4756 | 3489.4277 | 13.7 | 2031.4802 | 1948.4802 | LDVVPIDDNNNNSSNYR | | 173-189 | [Hex]7[HexNAc]2 |
| | 3532.6579 | 3532.4335 | | 2075.1130 | 1992.1130 | LDVVPIDDNNNNSSNYR | U | 173-189 | [Hex]7[HexNAc]2 |
| | 3692.4224 | 3692.5070 | 22.9 | | | LDVVPIDDNNNNSSNYR | | 173-189 | [Hex]7[HexNAc]3 |
| | 4447.8613 | 4447.8031 | 13.1 | 2031.8382 | 1948.8382 | LDVVPIDDNNNNSSNYR | | 173-189 | [Hex]7[HexNAc]6 [Fuc]1 |
| | 4650.9059 | 4650.8825 | 5.0 | 2031.9338 | 1948.9338 | LDVVPIDDNNNNSSNYR | | 173.189 | [Hex]7[HexNAc]7[Fuc]1 |
| | 3651.5273 | 3651.4805 | 12.8 | 2031.7455 | 1948.7455 | LDVVPIDDNNNNSSNYR | | 173.189 | [Hex]8[HexNAc]2 |
| | 3679.5537 | 3679.4753 | 21.3 | 2031.7537 | 1948.7537 | LDVVPIDDNNNNSSNYR | For | 173-189 | [Hex]8 [HexNAc]2 |
| | 3694.2814 | 3694.4863 | | | | LDWPIDDNNNNSSNYR | U | 173-189 | [Hex]8[HexNAc]2 |
| | 3813.6113 | 3813.5333 | 20.5 | | | LDVVPIDDNNNNSSNYR | | 173-189 | [Hex]9[HexNAc]2 |
| | 3841.6394 | 3841.5282 | 28.9 | 2031.7725 | 1948.7725 | LDVVPIDDNNNNSSNYR | For | 173-189 | [Hex]9 [HexNAc]2 |
| | 4165.8022 | 4165.6339 | 40.4 | | | LDVVPIDDNNNNSSNYR | For | 173-189 | [Hex]11[HexNAc]2 |
| | 4327.9448 | | | | | LDVVPIDDNNNNSSNYR | For | 173-189 | [Hex]12[HexNAc]2 |
| | | | | | | | | | |
| 19-23 | 2247.1045 | 2246.9592 | 64.7 | | | NCSFNITTEIR | | 150-160 | [Hex]3[HexNac]2 |
| | 2290.1731 | 2289.9650 | | | | NCSFNITTEIR | U | 150-160 | [Hex]3[HexNAc]2 |
| | 2393.0872 | 2393.0171 | 29.3 | | | NCSFNITTEIR | | 150-160 | [Hex]3[HexNAc]2[Fuc]1 |
| | 2450.0596 | 2450.0386 | 8.6 | | | NCSFNTTEIR | | 150-160 | [Hex]3[HexNAc]3 |
| | 2596.0635 | 2596.0965 | 12.7 | | | NCSFNITTEIR | | 150-160 | [Hex]3[HexNAc]3[Fuc]1 |
| | 2653.1636 | 2653.1179 | 17.2 | | | NCSFNITTEIR | | 150-160 | [Hex]3[HexNAc[4 |
| | 2799.2029 | 2799.1758 | 9.7 | 1437.9518 | 1354.9518 | NCSFNITTEIR | | 150-160 | [Hex]3[HexNAc]4[Fuc]1 |
| | 2842.1602 | 2842.1816 | 7.5 | | | NCSFNITTEIR | U | 150-160 | [Hex]3[HexNAc]4[Fuc]1 |
| | 2856.2510 | 2856.1974 | 18.8 | | | NCSFNITTEIR | | 150-160 | [Hex]3[HexNAc]5 |
| | 3002.2961 | 3002.2552 | 13.6 | 1437.6120 | 1354.6120 | NCSFNITTEIR | | 150-160 | [Hex]3[HexNAc]5[Fuc]1 |
| 3045.2581 | 3045.2581 | 3045.2611 | 1.0 | | | NCSFNITTEIR | U | 150-160 | [Hex]3[HexNAc]5[Fuc]1 |
| | 3059.3750 | 3059.2767 | 32.1 | | | NCSFNITTEIR | | 150-160 | [Hex]3[HexNAc]6 |
| | 3205.4419 | 3205.3347 | 33.4 | | | NCSFNITTEIR | | 150-160 | [Hex]3[HexNAc]6[Fuc]1 |
| | 3205.3550 | 3205.3347 | 6.3 | 1437.8108 | 1354.8108 | NCSFNITTEIR | | 150-160 | [Hex]3[HexNAc]6[Fuc]1 |
| | 2409.0415 | 2409.0120 | 12.2 | 1437.7975 | 1354.7975 | NCSFNITTEIR | | 150-160 | [Hex]4[HexNAc]2 |
| | 2612.1174 | 2612.0914 | 10.0 | 1437.8113 | 1354.8113 | NCSFNITTEIR | | 150-160 | [Hex]4[HexNAc]3 |
| | 2758.1653 | 2758.1493 | 5.8 | | | NCSFNITTEIR | | 150-160 | [Hex]4[HexNAc]3[Fuc]1 |
| | 2801.1345 | 2801.1551 | 7.4 | | | NCSFNITTEIR | U | 150-160 | [Hex]4[HexNAc]3[Fuc]1 |
| | 2815.2466 | 2815.1705 | 27.0 | | | NCSFNITTEIR | | 150-160 | [Hex]4[HexNAc]4 [Hex]4[NAc]4 |
| | 2961.2813 | 2961.2287 | 17.8 | 1437.7058 | 1354.7058 | NCSFNITTEIR | | 150-160 | [Hex]4[HexNac]4[Fuc]1 |
| | 3004.3835 | 3004.2345 | 49.6 | 1480.7999 | 1397.7999 | NCSFNITTEIR | U | 150-160 | [Hex]4 [HexNAc]4[Fuc]1 |
| | 3164.3542 | 3164.3080 | 14.6 | 1437.7386 | 1354.7386 | NCSFNITTEIR | | 150-160 | [Hex]4[HexNAc]5[Fuc]1 |
| | 3207.3638 | 3207.3138 | 15.6 | | | NCSFNITTEIR | U | 150-160 | [Hex]4[HexNAc]5[Fuc]1 |
| | 2571.0828 | 2571.0648 | 7.0 | 1437.9241 | 1354.9241 | NCSFNITTEIR | | 150-160 | [Hex]5[HexNAc]2 |
| | 2614.0593 | 2614.0706 | 4.3 | 1480.8892 | 1397.8892 | NCSFNITTEIR | U | 150-160 | [Hex]5[HexNAc]2 |
| | 2774.1431 | 2774.1442 | 0.4 | | | NCSFNITTEIR | | 150-160 | [Hex]5 [HexNAc]3 |
| | 2817.2344 | 2817.1500 | 30.0 | 1480.7618 | 1397.7618 | NCSFNITTEIR | U | 150-160 | [Hex]5[HexNAc]3 |
| | 2920.1741 | 2920.2021 | 9.6 | | | NCSFNITTEIR | | 150-160 | [Hex]5[HexNAc]3[Fuc]1 |
| | 2963.3796 | 2963.2079 | 57.9 | 1480.7709 | 1397.7709 | NCSFNTTEIR | U | 150-160 | [Hex]5[HexNAc]3[Fuc]1 |
| | 2571.0828 | | | | | NCSFNITTEIR | | 150-160 | [Hex]5[HexNA]4 |
| | 3003.4358 | | | | | NCSFNITTEIR | | 150-160 | [Hex]5[HexNAc]4[Fuc]1 |
| | 3123.3137 | 3123.2815 | 10.3 | | | NCSFNITTEIR | | 150-160 | [Hex]5[HexNAc]4[Fuc]1 |
| | 3166.3899 | 3166.2873 | 32.4 | 1480.8305 | 1397.8035 | NCSFNITTEIR | U | 50-160 | [Hex]5 [HexNAc]4[Fuc]1 |
| | 3326.4493 | | | | | NCSFNITTEIR | | 150-160 | [Hex]5[HexNAc]5[Fuc]1 |
| | 3369.4958 | 3369.3667 | 38.3 | | | NCSFNITTEIR | U | 150-160 | [Hex]5[HexNAc]5[Fuc]1 |
| | 2733.1489 | 2733.1177 | 11.4 | 1437.7366 | 1354.7366 | NCSFNITTEIR | | 150-160 | [Hex]6[HexNAc]2 |
| | 2776.1262 | 2776.1235 | 1.0 | | | NCSFNITTEIR | U | 150-160 | [Hex]6[HexNAc]2 |
| | 2936.2502 | 2936.1970 | 18.1 | | | NCSFNITTEIR | | 150-160 | [Hex]6[HexNAc]3 |
| | 3691.6216 | 3691.4931 | | | | NCSFNITTEIR | | 150-160 | [Hex]6[HexNAc[6]Fuc]1 |
| | 2895.2109 | 2895.1705 | 14.0 | 1437.9220 | 1354.922 | NCSFNITTEIR | | 150-160 | [Hex]7[HexNAc]2 |
| | 2938.2834 | 2938.1763 | 36.5 | 1480.7330 | 1397.7330 | NCSFNITTEIR | U | 50-160 | [Hex]7[HexNAc]2 |
| | 3057.2703 | 3057.2233 | 15.4 | 1437.7136 | 1354.7136 | NCSFNITTEIR | | 150-160 | [Hex]8[HexNAc]2 |
| | 3085.4431 | 3085.2182 | 72.9 | | | NCSFNITTEIR | For | 150-160 | [Hex]8[HexNAc]2 |
| | 3100.2202 | 3100.2291 | 2.9 | | | NCSFNITTEIR | U | 150-160 | [Hex]8[HexNAc]2 |
| | 3219.3145 | 3219.2761 | 11.9 | 1437.7230 | 1354.7230 | NCSFNITTEIR | | 150-160 | [Hex]9[HexNAc]2 |
| | 3262.2795 | 3262.2819 | 0.7 | | | NCSFNITTEIR | U | 150-160 | [Hex]9[HexNAc]2 |
| | | | | | | | | | |
| 20 | 2142.0044 | 2142.0173 | 6.0 | | | SNITGLLLTR | | 438-447 | [Hex]4[HexNAc]2 |
| 22-24 | 2304.0483 | 2304.0272 | 9.2 | 1170.9198 | 1087.9198 | SNITGLLLTR | | 438-447 | [Hex]5[HexNAc]2 |
| 26-28 | 2466.0999 | 2466.1229 | 9.3 | 1170.9374 | 1081.9374 | SNITGLLLTR | | 438-447 | [Hex]6[HexNAc]2 |
| | 2532.4939 | 2532.1810 | 123.6 | | | SNITGLLLTR | | 438-447 | [Hex]3[HexNAc]4[Fuc]1 |
| | 2938.6240 | 2938.3399 | 96.7 | | | SNITGLLLTR | | 438-447 | [Hex]3[HexNAc]6[Fuc]1 |
| | 2631.7939 | 2632.2084 | 157.5 | | | SNITGLLLTR | U | 438-447 | [Hex]3[HexNAc]5 |
| | 2735.2043 | 2735.2605 | 20.5 | | | SNITGLLLTR | | 438-447 | [Hex]3[HexNAc]5[Fuc]1 |
| | 2751.4282 | 2751.2554 | 62.8 | 1213.7461 | 1130.7461 | SNITGLLLTR | | 438-447 | [Hex]4[HexNAc]5 |
| | 2304.0483 | 2304.0272 | 9.2 | 1170.9198 | 1087.9198 | SNITGLLLTR | | 438-447 | [Hex]5[HexNAc]2 |
| | 2347.2393 | 2347.0758 | 69.7 | | | SNITGLLLTR | U | 438-447 | [Hex]5[HexNAc]2 |
| | 2915.7188 | 2915.2874 | | | | SNITGLLLTR | U | 438-447 | [Hex]6[HexNAc]4 |
| | 2628.1599 | 2628.1757 | 6.0 | 1170.9219 | 1087.9219 | SNITGLLLTR | | 438-447 | [Hex]7[HexNAc]2 |
| | 2671.3274 | 2671.1815 | 54.6 | | | SNITGLLLTR | U | 438-447 | [Hex]7[HexNAc]2 |
| | 2790.2070 | 2790.2285 | 7.7 | 1170.9211 | 1087.9211 | SNITGLLLTR | | 438-447 | [Hex]8[HexNac]2 |
| | 2818.4807 | 2818.2333 | 87.8 | | | SNITGLLLTR | For | 438-447 | [Hex]8 [HexNAc]2 |
| | 2833.2844 | 2833.234 | 17.7 | 1213.9207 | 1087.9221 | SNITGLLLTR | U | 38-447 | [Hex]8[HexNAc]2 |
| | 2952.2661 | 2952.2813 | 5.1 | 1170.9221 | 1087.9221 | SNITGLLLTR | | 438-447 | [Hex]9[HexNAc]2 |
| | 2980.5190 | 2980.2762 | 81.5 | | | SNITGLLLTR | For | 438-447 | [Hex]9[HexNAc]2 |
| | 2995.3093 | 2995.2871 | 7.4 | 1213.9792 | 1130.9792 | SNITGLLLTR | U | 438-447 | [Hex]9[HexNAc]2 |
| | | | | | | | | | |
| 23 | 3477.7258 | 3477.6272 | 28.4 | | | TIIVQLNESVEINCTRPNNNTR | | 280-301 | [Hex]3[HexNAc]2 |
| | 4030.0671 | 4029.8438 | 55.4 | 2668.5552 | 2585.5552 | TIIVQLNESVEINCTRPNNNTR | | 280-301 | [Hex]3[HexNAc]4[Fuc]1 |
| | 4233.1758 | 4232.9232 | 59.7 | 2668.4062 | 2585.4062 | TIIVQLNESVEINCTRPNNNTR | | 280-301 | [Hex]3[HexNAc]5[Fuc]1 |
| | 3639.7512 | 3639.6800 | 19.6 | | | TIIVQLNESVEINCTRPNNNTR | | 280-301 | [Hex]4[HexNAc]2 |
| | 3842.6460 | 3842.7594 | 29.5 | | | TIIVQLNESVEINCTRPNNNTR | | 280-301 | [Hex]4[HexNAc]3 |
| | 3801.6996 | 3801.7328 | 8.7 | | | TIIVQLNESVEINCTRPNNNTR | | 280-301 | [Hex]5[HexNAc]2 |
| | 4126.0322 | 4125.8385 | 46.9 | | | TIIVQLNESVEINCTRPNNNTR | | 280-301 | [Hex]7[HexNAc]2 |
| | | | | | | | | | |
| 31,32 | 4511.4214 | 4511.1628 | 35.2 | | | NVSTVQCTIIGIKPVVSTQLLLNGSLAEEEIII R | | 238-270 | [Hex]3[HexNAc]2 |
| 33 | 5063.5541 | 5063.4864 | 13.4 | 3701.2331 | 3618.2331 | NVSTVQCTHGIKPVVSTQLLLNGSLAEEEIII R | | 238-270 | [Hex]3[HexNAc]4[Fuc]1 |
| | 5266.7388 | 5266.5658 | 32.8 | 3701.7438 | 3618.7438 | NVSTVQCTHGIKPVVSTQLLLNGSLAEEEIII R | | 238-270 | [Hex]3[HexNAc]5[Fuc]1 |
| | 4673.3067 | 4673.3226 | 3.4 | | | NVSTVQCTIIGIKPVVSTQLLLNGSLACEEIII R | | 238-270 | [Hex]4[HexNAc]2 |
| | 4835.4373 | 4835.3754 | 12.8 | 3701.6550 | 3618.6550 | NVSTVQCTHGIKPVVSTQLLLNGSLAEEEIIIR | | 238-270 | [Hex]3[HexNAc]2 |
| | 5159.6187 | 5159.4811 | 26.7 | 3702.1625 | 3619.1615 | NVSTVQCTHGIKPVVSTQLLLNGSLAEEEIII R | | 238-270 | [Hex]7[HexNAc]2 |
| | 5321.7019 | 5321.5339 | 31.6 | | | NVSTVQCTHGIKPVVSTQLLLNGSLAEEEIII R | | 238-270 | [Hex]8 [HexNAc]2 |
| | 5483.6548 | 5483.5867 | 12.4 | | | NVSTVQCTHGIKPVVSTQLLLNGSLAEEEIII R | | 238-270 | [Hex]9[HexNAc]2 |
| | 6375.6035 | | | | | NVSTVQCTHGIKPVVSTQLLLNGSLAEEEIII R | | 238-270 | [Hex]12 [HexNAc]2 |
| | | | | | | | | | |
| 35 | 3590.7429 | 3590.493 | 69.6 | 1808.5692 | 1725.5692 | NCSFNITTEIRDKK | | 150-163 | [Hex]9[HexNAc]2 |
| | | | | | | | | | |
| 36 | 3373.7151 | | | | | NCSFNITTEIRDKK | | 150-163 | [Hex]3[HexNAc]5[Fuc]1 |
| | 2942.4314 | | | 1808.0563 | 1725.0563 | NCSFNITTEIRDKK | | 150-163 | [Hex]5[HexNAc]2 |
| | 3266.5955 | | | | | NCSFNITTEIRDKK | | 150-163 | [Hex]7[HexNAc]2 |
| | 3428.6143 | | | 1808.5538 | 1725.5538 | NCSFNITEIRDKK | | 150-163 | [Hex]8[HexNAc]2 |
| | 3590.7109 | | | 1808.8792 | 1725.8792 | NCSFNITTEIRDKK | | 150-163 | [Hex]9[HexNAc]2 |

**Table 3: LC-MS Glycopeptide Assignments For JR-FL**

| F# | Charge State | Experimental | Theoretical | Error | Y₁ | [Peptide + II] | Peptide Sequence | Mod | Position | Glycan |
|---|---|---|---|---|---|---|---|---|---|---|
| 4-6 | 2+ | 1153.0135 | 1153.0075 | 5.2 | | 1063.5537 | LREQFENK | | 340-363 | [Hex]3[HexNAc]3[Fuc]1 |
| | 2+ | 1254.5502 | 1254.5417 | 6.8 | | 1063.5537 | LREQFENK | | 340-357 | [Hex]3[HexNAc]4[Fuc] |
| | 2+ | 1356.0872 | 1356.0869 | 0.2 | 1266.7 | 1063.5537 | LREQFENK | | 340-354 | [Hex]3[HexNAc]5[Fuc]1 |
| | 2+ | 1457.6187 | 1457.6266 | 5.4 | | 1063.5537 | LREQFENK | | 340-363 | [Hex]3[HexNAc]6[Fuc]1 |
| | 2+ | 1234.0360 | 1234.0339 | 1.7 | | 1063.5537 | LREQFENK | | 340-363 | [Hex]4[HexNAc]3[Fuc]1 |
| | 2+ | 1437.1078 | 1437.1132 | 3.8 | | 1063.5537 | LREQFENK | | 340-357 | [Hex]4[HexNAc]5[Fuc]1 |
| | 2+ | 1315.0671 | 1315.0603 | 5.2 | | 1063.5537 | LREQFENK | | 340-354 | [Hex]5[HexNAc]3[Fuc]1 |
| | 2+ | 1416.6016 | 1416.600 | 1.1 | 1266.6 | 1063.5537 | LREQFENK | | 340-363 | [Hex]5[HexNAc]4[Fuc]1 |
| | 2+ | 1562.1387 | 1562.1477 | 5.8 | | 1063.5537 | LREQFENK | | 340-351 | [Hex]5[HexNAc]4[Fuc]1[NeuNAc]1 |
| | 2+ | 1518.1695 | 1518.1397 | 19.6 | | 1063.5537 | LREQFENK | | 340-363 | [Hex]5[HexNAc]5[Fuc]1 |
| | 2+ | 1140.4962 | 1140.4917 | 3.9 | | 1063.5537 | LREQFENK | | 340-357 | Hex]5[HexNAc]2 |
| | 2+ | 1302.5497 | 1302.5445 | 4.0 | | 1063.5537 | LREQFENK | | 340-354 | [Hex]7[HexNAc]2 |
| | 2+ | 1383.5724 | 1383.5709 | 1.1 | 1266.6 | 1063.5537 | LREQFENK | | 340-363 | [Hex][8]HexNAc]2 |
| | 2+ | 1464.6025 | 1464.5973 | 3.6 | 1266.6 | 1063.5537 | LREQFENK | | 340-352 | [Hex]9[HexNAc]2 |
| | | | | | | | | | | |
| 11-13 | 2+ | 1108.9988 | 1108.9938 | 4.5 | | 975.5264 | AKWNDTLK | | 327-334 | [Hex]3[HexNAc]3[Fuc]1 |
| 14-16 | 2+ | 1232.0435 | 1232.0364 | 5.8 | | 1018.5322 | AKWMDTLK | U | 327-334 | [Hex]3[HexNac]1 |
| | 2+ | 1210.5391 | 1210.5335 | 4.6 | | 975.5264 | AKWNDTLK | | 327-334 | [Hex]3[HexNac]Fuc]1 |
| | 2+ | 1304.5245 | 1304.5552 | 23.5 | | 1018.5322 | AKWNDTLK | U | 327-334 | [Hex]3[HexNac]1 |
| | 2+ | 1333.5862 | 1333.5761 | 7.5 | | 1018.5322 | AKWNDTLK | U | 327-334 | [Hex]3[HexNAc]4[NeuNAc]1 [Hex]3[HexNAc]5[Fuc]1 |
| | 2+ | 1312.0836 | 1312.0732 | 7.9 | | 975.5264 | AKWNDTLK | | 327-334 | [Hex]3[HexNAc]5[Fuc]1 |
| | 3+ | 875.0563 | 875.05126 | 5.7 | | 975.5264 | AKWNDTLK | | 327-334 | [Hex]3[HexNAc]5[Fuc]1 |
| | 2+ | 1413.6098 | 1413.6129 | 2.3 | | 975.5264 | AKWNDTLK | | 327-334 | [Hex]3[HexNAc]6[Fuc]1 |
| | 3+ | 942.7483 | 942.7444 | 4.1 | | 975.5264 | AKWNDTLK | | 327-334 | [Hex]3[HexNAc]6[Fuc]1 |
| | 2+ | 1218.5409 | 1218.5310 | 8.1 | | 975.5264 | AKWNDTLK | | 327-334 | [Hex]4[HexNAc]4 |
| | 2+ | 1211.5251 | 1211.5231 | 1.7 | | 1018.5322 | AKWNDTLK | U | 327-334 | [Hex]4[HexNAc]3[Fuc]1 |
| | 2+ | 1357.0678 | 1357.0708 | 2.3 | | 1018.5322 | AKWNDTLK | U | 327-334 | [Hex]4[HexNAc]3[Fuc]1NeuNAc]1 |
| | 2+ | 1385.574 | 1385.5816 | 5.5 | | 1018.5322 | AKWNDTLK | U | 327-334 | [Hex]4[hexNac]4[NeuNac]1 |
| | 2+ | 1393.1149 | 1393.0996 | 11.0 | | 975.5264 | AKWNDTLK | | 327-334 | [Hex]4[HexNAc]5[Fuc]1 |
| | 3+ | 929.0752 | 929.06883 | 6.9 | | 975.5264 | AKWNDTLK | | 327-334 | [Hex]4[HexNAc]5[Fuc]1 |
| | 2+ | 1096.4889 | 1096.4780 | 9.9 | | 975.5264 | AKWNDTLK | | 327-334 | [Hex]5[HexNAc]2 |
| | 2+ | 1117.9962 | 1117.9809 | 13.7 | 1221.6 | 1018.5322 | AKWNDTLK | U | 27-334 | [Hex]5[HexNAc]2 |
| | 2+ | 1292.5602 | 1292.5495 | 8.3 | | 1018.5322 | AKWNDTLK | U | 27-334 | [Hex]5[HexNac]3[Fuc]1 |
| | 2+ | 1271.0563 | 1271.0466 | 7.6 | | 975.5264 | AKWNDTLK | | 327-334 | [Hex]5[HexNAc]3[Fuc]1 |
| | 3+ | 847.7045 | 847.7002 | 5.1 | | 975.5264 | AKWNDTLK | | 327-334 | [Hex]5[HexNAc]3[Fuc]1 |
| | 2+ | 1372.5957 | 1372.5863 | 6.8 | | 975.5264 | AKWNDTLK | | 327-334 | [Hex]5[HexNAc]4[Fuc]1 |
| | 3+ | 915.3974 | 915.39333 | 4.5 | | 975.5264 | AKWNDTLK | | 327-334 | [Hex]5[HexNAc[4]Fuc]1 |
| | 2+ | 1518.1420 | 1518.1340 | 5.2 | | 975.5264 | AKWNDTLK | | 327-334 | [Hex]5[HexNAc]4[Fuc]1[NeuNAc]1 |
| | 3+ | 1012.4247 | 1012.4251 | 0.4 | | 975.5264 | AKWNDTLK | | 327-334 | [Hex]5[HexNAc]4[Fuc]1[NeuNAc]1 |
| | 3+ | 983.0925 | 983.08646 | 6.1 | | 975.5264 | AKWNDTLK | | 327-334 | [Hex]5[HexNAc]5[Fuc]1 |
| | 2+ | 1177.5095 | 1177.5044 | 4.3 | 975.5/11 78.7 | 975.5264 | AKWNDTLK | | 327-334 | [Hex]6[HexNAc]2 |
| | 2+ | 1199.0081 | 1199.0073 | 0.6 | | 1018.5322 | AKWIVDTLK | U | 327-334 | [Hex]6 [HexNAc]2 |
| | 2+ | 1293.0577 | 1293.0415 | 12.5 | | 1003.5213 | AKWNDTLK | FOR | 327-334 | [Hex]6[HexNAc]3 |
| | 2+ | 1279.052 | 1279.0441 | 6.2 | | 975.5264 | AKWNDTLK | | 327-334 | [Hex]6[HexNAc]3 |
| | 3+ | 853.0334 | 853.03183 | 1.9 | | 975.5264 | AKWNDTLK | | 327-334 | [Hex]6[HexNAc[3 |
| | 2+ | 1352.0792 | 1352.0730 | 4.6 | | 975.5264 | AKWNDTLK | | 327-334 | [Hex]6[HexNAc]3[Fuc]1 |
| | 3+ | 901.7213 | 901.7178 | 3.9 | | 975.5264 | AKWNDTLK | | 327-334 | [Hex]6[HexNAc]3[Fuc]1 |
| | 2+ | 1424.6169 | 1424.5918 | 17.6 | | 975.5264 | AKWNDTLK | | 327-334 | [Hex]6[HexNAc]3[NeuNA]1 |
| | 2+ | 1424.6169 | 1424.5918 | 17.6 | | 975.5264 | AKWNDTLK | | 327-334 | [Hex]6[HexNAc]3[NeuNAc]1 |
| | 3+ | 950.0626 | 950.06363 | 1.1 | | 975.5264 | AKWNDTLK | | 327-334 | [Hex]6[HexNAc]3[NeuNAc]1 |
| | 2+ | 1394.5787 | 1394.5812 | 1.8 | | 1003.5213 | AKWNDTLK | FO R | 327-334 | [Hex]6[HexNAc]4 |
| | 2+ | 1258.5395 | 1258.5308 | 6.9 | 1178.6 | 975.5264 | AKWNDTLK | | 327-334 | [Hex]7[HexNAc]2 |
| | 3+ | 839.3596 | 839.35633 | 3.9 | 1178.7 | 975.5264 | AKWNDTLK | | 327-334 | [Hex]7[HexNAc]2 |
| | 2+ | 1280.0359 | 1280.0337 | 1.6 | | 1018.5322 | AKWDDTLK | U | 327-334 | [Hex]7[HexNAc]2 |
| | 2+ | 1339.5667 | 1339.5572 | 7.1 | 1178.6 | 975.5264 | AKWNDTLK | | 327-334 | [Hex]8[HexNAc]2 |
| | 3+ | 893.3783 | 893.37393 | 4.9 | 1178.2 | 975.5264 | AKWNDTLK | | 327-334 | [Hex]8[HexNAc]2 |
| | 2+ | 1361.0668 | 1361.0601 | 4.8 | | 1018.5322 | AKWNDTLK | U | 327-334 | [Hex]8[HexNAc]2 |
| | 3+ | 893.3776 | 893.37393 | 4.1 | | 975.5264 | AKWNDTLK | | 327-334 | [Hex]8[HexNAc]2 |
| | 2+ | 1420.5936 | 1420.5836 | 7.0 | 1178.7 | 975.5264 | AKWNDTLK | | 327-334 | [Hex]9[HexNAc]2 |
| | 2+ | 1442.0962 | 1442.0865 | 6.7 | | 1018.5322 | AKWNDTLK | U | 327-334 | [Hex]9[HexNAc]2 |
| | 3+ | 947.3965 | 947.39153 | 5.3 | 1178.6 | 975.5264 | AKWNDTLK | | 327-334 | [Hex]9[HexNAc]2 |
| | | | | | | | | | | |
| 15-16 | 2+ | 1276.0527 | 1276.0153 | 29.3 | | 985.4637 | QAHCNISR | | 319-326 | [Hex]5[HexNAc]3[Fuc]1 |
| | 2+ | 1377.5928 | 1377.555 | 27.4 | | 985.4637 | QAHCNISR | | 319-326 | [Hex]5[HexNAc]4[Fuc]1 |
| | 2+ | 1487.0876 | 1487.0921 | 3.0 | | 985.4637 | QAHCNISR | | 319-326 | [Hex]6[HexNAc]5 |
| | | | | | | | | | | |
| 18-19 | 2+ | 1431.1325 | 1431.1320 | 0.3 | | 1619.8027 | LDVVPIDNNNTSYR | | 171-184 | [Hex]3[HexNAc]3[Fuc]1 |
| | 2+ | 1532.6792 | 1532.6716 | 4.9 | 1824.0 | 1619.8027 | LDVVPIDNNNTSYR | | 171-184 | [Hex]3[HexNAc]4[Fuc]1 |
| | 3+ | 1022.1259 | 1022.1168 | 8.8 | | 1619.8027 | LDVVPIDNNNTSYR | | 171-184 | [Hex]3[HexNAc]4[Fuc]1 |
| | 2+ | 1634.2266 | 1634.2114 | 9.3 | 1823.9 | 1619.8027 | LDVVPIDNNNTSYR | | 171-184 | [Hex]3[HexNAc]5[Fuc]1 |
| | 3+ | 1089.8197 | 1089.8100 | 8.9 | | 1619.8027 | LDVVPIDNNNTSYR | | 171-184 | [Hex]3[HexNAc]5[Fuc]1 |
| | 2+ | 1735.7579 | 1735.7511 | 3.9 | | 1619.8027 | LDVVPIDNNNTSYR | | 171-184 | [Hex]3[HexNAc]6[Fuc]1 |
| | | 1512.1611 | 1512.1584 | 1.8 | | 1619.8027 | LDVVPIDNNNTSYR | | 171-184 | [Hex]4[HexNAc]3[Fuc]1 |
| | 2+ | 1642.1907 | 1642.2088 | 11.0 | | 1619.8027 | LDVVPIDNNNTSYR | | 171-184 | [Hex]4[HexNAc]5 |
| | 3+ | 1337.8800 | 1337.8912 | 8.4 | | 1619.8027 | LDVVPIDNNNTSYR | | 171-184 | [Hex]4[HexNAc]5[Fuc]1[NeuNAc]2 |
| | 2+ | 1418.6325 | 1418.6161 | 11.5 | | 1619.8027 | LDVVPIDNNNTSYR | | 171-184 | [Hex]5[HexNAc]2 |
| | 2+ | 1593.2062 | 1597.1848 | 13.4 | | 1619.8027 | LDVVPIDNNNTSYR | | 171-184 | [Hex]5[HexNAc]3[Fuc]1 |
| | 3+ | 1062.4683 | 1062.4589 | 8.8 | | 1619.8027 | LDVVPIDNNNTSYR | | 171-184 | [Hex]5[HexNAc]3[Fuc]1 |
| | 2+ | 1694.7371 | 1694.7245 | 7.4 | 1823.9 | 1619.8027 | LDVVPIDNNNTSYR | | 171-184 | [Hex]5[HexNAc]4[Fuc]1 |
| | 3+ | 1197.8553 | 1197.8452 | 8.4 | | 1619.8027 | LDVVPIDNNNTSYR | | 171-184 | [Hex]5[HexNAc]5[Fuc]1 |
| | 2+ | 1499.6545 | 1499.6426 | 7.9 | | 1619.8027 | LDVVPIDNNNTSYR | | 171-184 | [Hex]6[HexNAc]2 |
| | 2+ | 1674.1967 | 1674.2112 | 8.7 | | 1619.8017 | LVVVPIDNNNTSYR | | 171-184 | [Hex]6[HexNAc]3[Fuc]1 |
| | 3+ | 1270.8585 | 1170.8700 | 9.0 | | 1619.8027 | LDVVPIDNNNTSYR | | 171-184 | [Hex]6[HexNAc]6 |
| | 3+ | 1319.5512 | 1319.5559 | 3.6 | | 1619.8027 | LDVVPIDNNNTSYR | | 171.184 | [Hex]6[HexNAc]6[Fuc]1 |
| | 2+ | 1580.6923 | 1590.6690 | 14.7 | | 1619.8027 | LDVVPIDNNNTSYR | | 171-184 | [Hex]7[HexNAc]2 |
| | 3+ | 1373.571 | 1373.5735 | 1.9 | | 1619.8027 | LDVVPIDNNNTSYR | | 171-184 | [Hex]7[HexNAc]6[Fuc]1 |
| | 2+ | 1661.7095 | 1661.6954 | 8.5 | | 1619.8027 | LDVVPIDNNNTSYR | | 171-184 | [Hex]8[HexNAc]2 |
| | 3+ | 1468.2872 | 1468.265 | 15.1 | | 1619.8027 | LDVVPIDNNNTSYR | | 171-184 | [Hex]8[HexNAc]5[Fuc]1[NeuGe]1 |
| | 2+ | 1742.7289 | 1742.7218 | 4.1 | | 1619.8027 | LDVVPIDNNNTSYR | | 171-184 | [Hex]9[HexNAc]2 |
| | | | | | | | | | | |
| 18-20 | 3+ | 1111.8127 | 1111.8068 | 5.3 | | 2091.9519 | DGGINENGTEIFRPGGGDMR | | 443-462 | [Hex]3[HexNAc]3[Fuc]1 |
| | 3+ | 1169.4996 | 1169.4843 | 13.1 | | 2091.9519 | DGGINENGTEIFRPGCGDMR | | 443-462 | [Hex]3[HexNAc]3[NeuNAc]1 |
| | 3+ | 1179.5052 | 1179.4999 | 4.5 | 1221.4 | 2091.9519 | DGGINENGTEIFRPGGGDMR | | 443-462 | [Hex]3[HexNAc]4[Fuc]1 |
| | 3+ | 1198.4897 | 1198.5071 | 14.5 | | 2091.9519 | DGGINENGTEIFRPGGGDMR | | 443-462 | [Hex]3[HexNAc]5 |
| | 3+ | 1247.1980 | 1247.1931 | 3.9 | | 2091.9519 | DGGINENGTEIFRPGGGDMR | | 443-462 | [Hex]3[HexNAc]5[Fuc]1 |
| | 3+ | 1314.8981 | 1314.8862 | 9.1 | | 2091.9519 | DGGINENGTEIFRPGGGDMR | | 443-462 | [Hex]3[HexNAc]6[Fuc]1 |
| | 3+ | 1368.9120 | 1368.9038 | 6.0 | | 2107.9468 | DGGINENGTEIFRPGGGDMR | OX | 43-462 | [Hex]3[HexNAc]6[Fuc]2 |
| | 3+ | 1184.8352 | 1184.8316 | 3.0 | | 2091.9519 | DGGINENGTEIFRPGGGDMR | | 443-462 | [Hex]4[HexNAc]4 |
| | 3+ | 1107.4579 | 1107.4629 | 4.5 | | 2119.9468 | DGGINENGTEIFRPGGGDMR | FOR | 443-462 | [Hex]4[HexNAc]2[Fuc]1 |
| | 3+ | 1165.8293 | 1165.8244 | 4.2 | | 2091.9519 | DGGINENGTEIFRPGGGDMR | | 443-462 | [Hex]4[HexNAc]3[Fuc]1 |
| | 3+ | 1261.8588 | 1262.8560 | 2.1 | | 2091.9519 | DGGINENGTEIFRPGGGDMR | | 443-462 | [Hex]4[HexNAc]3[Fuc]1[NeuNAc]1 |
| | 3+ | 1330.5602 | 1330.5493 | 8.1 | | 2091.9519 | DGGINENGTEIFRPGGGDMR | | 443-462 | [Hex]4[HexNAc]4[Fuc]1[NeuNAc]1 |
| | 3+ | 1252.5317 | 1252.5247 | 5.6 | | 2091.9519 | DGGINENGTEIFRPGCCDMR | | 443-462 | [Hex]4[HexNAc]5 |
| | 3+ | 1301.2222 | 1301.2106 | 8.8 | 1221.4 | 2091.9519 | DGGINENGTEIFRPGGGDMR | | 443-462 | [Hex]4[HexNAc]5[Fuc]1 |
| | 3+ | 1398.2534 | 1398.2425 | 7.8 | | 2091.9519 | DGGINENGTEIFRPGGGDMR | | 443-462 | [Hex]4[HexNAc]5[Fuc]1[NeuNAc]1 |
| | 3+ | 1417.2489 | 1417.2496 | 0.6 | | 2091.9519 | DGGINENGTEIFRPGCGDMR | | 443-462 | [Hex]4[HexNAc]6[Fuc]1[NeuNAc]1 |
| | 3+ | 1465.9532 | 1465.9356 | 12.0 | | 2091.9519 | DGGINENGTEIFRPGGGDMR | | 443-462 | [Hex]4[HexNAc]6[Fuc]1[NeuNAc]1 |
| | 3+ | 1171.1581 | 1171.1560 | 1.7 | | 2091.9519 | DGGINENGTEIFRPGGGDMR | | 443-462 | [Hex]5[HexNAc]3 |
| | 3+ | 1268.2010 | 1268.1878 | 10.3 | | 2091.9519 | DGGINENGTEIFRPGGGDMR | | 443-462 | [Hex]5[HexNAc]3[NeuNAc]1 |
| | 3+ | 1103.4695 | 1103.4629 | 6.0 | | 2091.9519 | DGGINENGTEIFRPGGGDMR | | 443-462 | [Hex]5[HexNAc]2 |
| | 3+ | 1119.8537 | 1219.8420 | 9.6 | | 2091.9519 | DGGINENGTEIFRPGGGDMR | | 443-462 | [Hex]5[HexNAc]3[Fuc]1 |
| | 3+ | 1287.5434 | 1287.5351 | 6.4 | | 2091.9519 | DGGINENGTEIFRPGGGDMR | | 443-462 | [Hex]5[HexNAc]5[Fuc]1 |
| | 3+ | 1384.5786 | 1384.5669 | 8.4 | | 2091.9519 | DGGINENGTEIFRPGGGDMR | | 443-462 | [Hex]5[HexNAc]4[Fuc]1[NeuNAc]1 |
| | 3+ | 1481.6149 | 1481.5987 | 10.9 | 11148.4/1221 | 2091.9519 | DGGINENGTEIFRPGGGDMR | | 443-462 | [Hex]5[HexNAc]4[Fuc]1[NeuNAc]1 |
| | 3+ | 1306.5448 | 1306.5423 | 1.9 | | 2091.9519 | DGGINENGTEIFRPGGGDMR | | 443-462 | [Hex]5[HexNAc]5 |
| | 3+ | 1355.2366 | 1355.2283 | 6.1 | 1221.4 | 2091.9519 | DGGINENGTEIFRPGGGDMR | | 443-462 | [Hex]5[HexNAc]5[Fuc]1 |
| | 3+ | 1452.2683 | 1452.2601 | 5.6 | | 2091.9519 | DGGINENGTEIFRPGGGDMR | | 443-462 | [Hex]5[HexNAc]5[Fuc]1[NeuNAc]1 |
| | 3+ | 1403.5745 | 1403.5741 | 0.3 | | 2091.9519 | DGGINENGTEIFRPGGGDMR | | 443-462 | [Hex]5[HexNAc]5[NeuNAc]1 |
| | 3+ | 1333.2109 | 1333.1946 | 12.2 | | 2091.9519 | DGGINENGTEIFRPGGGDMR | | 443-462 | [Hex]5[HexNAc]5[SO3]1 |
| | 3+ | 1157.4879 | 1157.4805 | 6.3 | | 2091.9519 | DGGINENGTEIFRPGGGDMR | | 443-462 | [Hex]6[HexNAc]2 |
| | 3+ | 1360.5682 | 1360.5599 | 6.1 | | 2091.9519 | DGGINENGTEIFRPGGGDMR | | 443-462 | [Hex]6[HexNAc]5 |
| | 3+ | 1215.1788 | 1225.1736 | 4.2 | | 2091.9519 | DGGINENGTEIFRPGGGDMR | | 443-462 | [Hex]6[HexNAc]3 |
| | 3+ | 1273.8745 | 1273.8596 | 11.7 | | 2091.9519 | DGGINENGTEIFRPGGGDMR | | 443-462 | [Hex]6[HexNAc]3[Fuc]1 |
| | 3+ | 1322.2201 | 1322.2054 | 11.0 | | 2091.9519 | DGGINENGTEIFRPGGGDMR | | 443-462 | [Hex]6[HexNAc]3[NeuNAc]1 |
| | 3+ | 1389.9101 | 1389.8986 | 8.3 | | 2091.9519 | DGGINENCTEIFRPGGGDMR | | 443-462 | [Hex]6[HexNAc]4[NeuNAc]1 |
| | 3+ | 1409.1587 | 1409.2459 | 9.1 | | 2091.9519 | DGGINENGTEIFRPGGGDMR | | 443-462 | [Hex]6[HexNAc]5[Fuc]1 |
| | 3+ | 1311.6288 | 1511:6108 | 11.9 | | 2091.9519 | DGGINENGTEIFRPGGGDMR | | 443-462 | [Hex]6[HexNAc]5[Fuc]1[NeuGc]1 |
| | 3+ | 1506.2964 | 1506.2777 | 12.4 | | 2091.9519 | DGGINENGTEIFRPGGGDMR | | 443-462 | [Hex]6[HexNAc]5[Fuc]1[NeuNAc]1 |
| | 3+ | 1603.3206 | 1603.3095 | 6.9 | 1149.1/1 222.0 | 2091.9519 | DGGINENGTEIFRPGGGDMR | | 443-462 | [Hex]6[HexNAc]5[Fuc]1[NeuNAc]2 |
| | 3+ | 1428.2686 | 1428.2530 | 10.9 | | 2091.9519 | DGGINENGTEIFRPGGGDMR | | 443-462 | [Hex]6[HexNAc]6 |
| | 3+ | 1476.9544 | 1476.9390 | 10.4 | 1221.4 | 2091.9519 | DGGINENGTEIFRPGGGDMR | | 443-462 | [Hex]6[HexNAc]6[Fuc]1 |
| | 3+ | 1525.277 | 1525.2848 | 5.2 | | 2091.9519 | DGGINENGTEIFRPGGGDMR | | 443-462 | [Hex]6[HexNAc]6[NeuNAc]1 |
| | 3+ | 1628.0097 | 1627.9884 | 13.1 | | 2091.9519 | DGGINENGTEIFRPGGGDMR | | 443-461 | [Hex]7[HexNAc]6[Fuc]1[NeuNAc]1 |
| | 3+ | 1211.5025 | 1211.4981 | 3.5 | | 2091.9519 | DGGINENGTEIFRPGGGDMR | | 443-462 | [Hex]7[HexNAc]2 |
| | 3+ | 1279.1816 | 1279.1912 | 7.6 | | 2091.9519 | DGGINENGTEIFRPGGGDMR | | 443-462 | [Hex]7[HexNAc]3 |
| | 3+ | 1530.9761 | 1530.9566 | 12.7 | 1221.0 | 2091.9519 | DGGINENGTEIFRPGGGDMR | | 443-462 | [Hex]7[HexNAc]6[Fuc]1 |
| | 3+ | 1633.3537 | 1633.3215 | 19.7 | | 2019.9519 | DGGINENGTEIFRPGGGDMR | | 443-462 | [Hex]7[HexNAc]6[Fuc]1[NeuGc]1 |
| | 3+ | 1725.045 | 1725.0202 | 14.4 | 1221.32 | 2091.9519 | DGGINENGTEIFRPGGGDMR | | 443-462 | [Hex]7[HexNAc]6[Fuc]1[NeuNAc]2 |
| | 3+ | 1270.8585 | 1270.8474 | 8.7 | | 2107.9468 | DGGINENGTEIFRPGGGDMR | OX | 43-462 | [Hex]8[HexNAc]2 |
| | 3+ | 1265.5192 | 1265.5157 | 2.7 | | 2091.9519 | DGGINENGTEIFRPGGGDMR | | 443-462 | [Hex]8[HexNAc]2 |
| | 3+ | 1319.5459 | 1319.5333 | 9.5 | | 2091.9519 | DGGINENGTEIFRPGGGDMR | | 443-462 | [Hex]9[HexNAc]2 |
| | 3+ | 1373.5437 | 1373.5509 | 5.3 | | 2091.9519 | DGGINENGTEIFRPGGGDMR | | 443-462 | [Hex]10[HexNAc]2 |
| | | | | | | | | | | |
| 20-21 | 3+ | 1119.4877 | 1119.4904 | 2.4 | | 1911.9234 | NCSFNITTSIRDEVQK | | 148-158 | [Hex]3[HexNAc]4[Fuc]1 |
| | 3+ | 1187.1908 | 1187.1836 | 6.1 | 1131.0 | 1911.9234 | NCSFNITTSIRDEVQK | | 148-158 | [Hex]3[HexNAc]5[Fuc]1 |
| | 3+ | 1120.1566 | 1120.1502 | 5.7 | | 1954.9292 | NCSFNITTSIRDEVQK | U | 48-158 | [Hex]4[HexNAc]3[Fuc]1 |
| | 3+ | 1241.1953 | 1241.2012 | 4.8 | | 1911.9234 | NCSFNITRSIRDEVQK | | 148-158 | [Hex]4[HexNAc]5[Fuc]1 |
| | 3+ | 1227.5392 | 1227.5256 | 11.0 | 1058.4/1 131.4 | 1911.9234 | NCSFNITTSIRDEVQK | | 148-158 | [Hex]5[HexNAc]4[Fuc]1 |
| | 3+ | 1246.5354 | 1246.5328 | 2.1 | | 1911.9234 | NCSFNITTSIRDEVQK | | 148.158 | [Hex]5[HexNAc]5 |
| | 3+ | 1295.2152 | 1295.2188 | 2.8 | | 1911.9234 | NCSFNITTSIRDEVQK | | 148-158 | [Hex]5[HexNAc]5[Fuc]1 |
| | 3+ | 1392.2512 | 1392.2506 | 0.4 | | 1911.9234 | NCSFNITTSIRDEVQK | | 148-158 | [Hex]5[HexNAc]5[Fuc]1[NeuNAc]1 |
| | 3+ | 1262.2046 | 1262.1960 | 6.8 | | 1911.9234 | NCSFNITTSIRDEVQK | | 148-158 | [Hex]6[HexNAc]3[NeuNAc]1 |
| | 3+ | 1259.5146 | 1259.5096 | 4.0 | | 1911.9234 | NCSNITSIRDEVQK | | 148-158 | [Hex]6[HexNAc]4[SO3]1 |
| | 3+ | 1205.5102 | 1205.5063 | 3.2 | 1058.3 | 1911.9234 | NCSFNITTSIRDEVQK | | 148-158 | [Hex]8[HexNAc]2 |
| | 3+ | 1259.5146 | 1259.5239 | 7.4 | | 1911.9134 | NCSFNITTSIRDEVQK | | 148-158 | [Hex]9[HexNAc]2 |
| | 3+ | 1367.5514 | 1367.5591 | 5.6 | | 1911.9234 | NCSFNITTSIRDEVQK | | 148-158 | [Hex]11[HexNAc]2 |
| | | | | | | | | | | |
| 21-24 | 2+ | 1215.5448 | 1215.5569 | 10.0 | | 1334.7103 | CSSNITGLLLTR | | 431-442 | [Hex]3[HexNAc]3 |
| | 2+ | 1288.5958 | 1288.5858 | 7.8 | | 1334.7103 | CSSNITGLLLTR | | 431-442 | [Hex]3[HexNAc]3[Fuc]1 |
| | 2+ | 1317.1068 | 1317.0965 | 7.8 | | 1334.7103 | CSSNITGLLLTR | | 431-442 | [Hex]3[HexNAc]4 |
| | 2+ | 1390.1364 | 1390.1255 | 7.8 | 1537.8/1 683.9 | 1334.7103 | CSSNITGLLLTR | | 431-442 | [Hex]3[HexNAc]4[Fuc]1 |
| | 3+ | 927.0917 | 927.0861 | 6.0 | | 1334.7103 | CSSNITGLLLTR | | 431-442 | [Hex]3[HexNAc]4[Fuc]1 |
| | 2+ | 1390.1245 | 1390.1255 | 0.7 | | 1334.7103 | CSSNITGLLLTR | | 431-442 | [Hex]3[HexNAc]4[Fuc]1 |
| | 2+ | 1381.6240 | 1381.6122 | 8.6 | | 1260.6623 | CSSNITGLLLTR | PyroC | 431-442 | [Hex]3[HexNAc]4[Fuc]1 |
| | 2+ | 1491.6762 | 1491.6652 | 7.4 | 1537.8/1 683.8 | 1334.7103 | CSSNITGLLLTR | | 431-442 | [Hex]3[HexNAc]5[Fuc]1 |
| | 3+ | 994.7863 | 994.7792 | 7.1 | | 1334.7103 | CSSNITGLLLTR | | 431.442 | [Hex]3[HexNAc]5[Fuc] |
| | 2+ | 1296.5939 | 1296.5833 | 8.2 | | 1334.7103 | CSSNITGLLLTR | | 431-442 | [Hex]4[HexNAc]3 |
| | 2+ | 1369.6179 | 1369.6122 | 4.2 | | 1334.7103 | CSSNITGLLLTR | | 431.442 | [Hex]4[HexNAc]3[Fuc]1 |
| | 3+ | 1078.1394 | 1078.1355 | 3.6 | | 1334.7103 | CSSNITGLLLTR | | 431-442 | [Hex]4[HexNAc]4[Fuc]1[NeuNAc]1 |
| | 2+ | 1572.7100 | 1572.6916 | 11.7 | | 1334.7103 | CSSNITGLLLTR | | 431-442 | [Hex]4[HexNAc]5[Fuc]1 |
| | 2+ | 1377.6164 | 1377.6097 | 4.9 | | 1334.7103 | CSSNITGLLLTR | | 431-442 | [Hex]5[HexNAc]3 |
| | 2+ | 1276.0779 | 1276.0700 | 6.2 | | 1334.7103 | CSSNITGLLLTR | | 431-442 | [Hex]5[HexNAc]2 |
| | 2+ | 1267.5668 | 1267.5567 | 8.0 | | 1260.6623 | CSSNITGLLLTR | PyroC | 431-442 | [Hex]5[HexNAc]2 |
| | 2+ | 1450.6507 | 1450.6386 | 8.3 | | 1334.7103 | CSSNITGLLLTR | | 431-442 | [Hex]5[HexNAc]3[Fuc]1 |
| | 2+ | 1479.1699 | 1479.1494 | 13.9 | | 1334.7103 | CSSNITGLLLTR | | 431-442 | [Hex]5[HexNAc]4 |
| | 2+ | 1552.1921 | 1552.1783 | 8.9 | | 1334.7103 | CSSNITGLLLTR | | 431-442 | [Hex]5[HexNAc]4[Fuc]1 |
| | 3+ | 1035.1253 | 1035.1213 | 3.9 | | 1334.7103 | CSSNITGLLLTR | | 431-442 | [Hex]5[HexNAc]4[Fuc]1 |
| | 2+ | 1697.7391 | 1697.7260 | 7.7 | | 1334.7103 | CSSNITGLLLTR | | 431-442 | [Hex]5[HexNAc]4[Fuc]1[NeuNAc]1 |
| | 3+ | 1132.1535 | 1132.1531 | 0.4 | | 1334.7103 | CSSNITGLLLTR | | 431-442 | [Hex]5[HexNAc]4[Fuc]1[NeuNAc]1 |
| | 2+ | 1653.7465 | 1653.7180 | 17.2 | | 1334.7103 | CSSNITGLLLTR | | 431-442 | [Hex]5[HexNAc]5[Fuc]1 |
| | 2+ | 1357.1039 | 1357.0964 | 5.5 | | 1334.7103 | CSSNITGLLLTR | | 431-442 | [Hex]6[HexNAc]2 |
| | 2+ | 1458.6430 | 1458.6361 | 4.7 | | 1334.7103 | CSSNITGLLLTR | | 431-442 | [Hex]6[HexNAc]3 |
| | 2+ | 1531.6806 | 1531.6650 | 10.2 | | 1334.7103 | CSSNITGLLLTR | | 431.442 | [Hex]6[HexNAc]3[Fuc]1 |
| | 2+ | 1604.1660 | 1604.1838 | 11.1 | | 1334.7103 | CSSNITGLLLTR | | 431-442 | [Hex]6[HexNAc]3[NeuNAc]1 |
| | 2+ | 1438.1321 | 1438.1228 | 6.5 | | 1334.7103 | CSSNITGLLLTR | | 431-442 | [Hex]7[HexNAc]2 |
| | 3+ | 1013.0974 | 1013.1019 | 4.4 | | 1334.7103 | CSSNITGLLLTR | | 431-442 | [Hex]8[HexNAc]2 |
| | 2+ | 1519.1635 | 1519.1492 | 9.4 | | 1334.7103 | CSSNITGLLLTR | | 431-442 | [Hex]8[HexNAc]2 |
| | 2+ | 1510.6585 | 1510.6359 | 15.0 | | 1260.6623 | CSSNITGLLLTR | PyroC | 431-442 | [Hex]8[HexNAc]2 |
| | 3+ | 1067.1183 | 1067.1195 | 1.1 | | 1334.7103 | CSSNITGLLLTR | | 443-462 | [Hex]9[HexNAc]2 |
| | 2+ | 1600.1891 | 1600.1756 | 8.4 | 1537.8 | 1334.7103 | CSSNITGLLLTR | | 443-462 | [Hex]9[HexNAc]2 |
| | | | | | | | | | | |
| 23-24 | 2+ | 1545.1895 | 1545.1863 | 2.1 | | 1847.9113 | LICTTAVPWNASWSNK | | 555-570 | [Hex]3[HexNAc]3[Fuc]1 |
| | 2+ | 1646.7348 | 1646.7260 | 5.3 | 1026.2/1 099 | 1847.9113 | LICTTAVPWMASWSNK | | 555-570 | [Hex]3[HexNAc]4[Fuc]1 |
| | 3+ | 1098.1575 | 1098.1531 | 4.0 | 1025.9 | 1847.9113 | LICTTAVPWNASWSNK | | 555-570 | [Hex]3[HexNAc]4[Fuc]1 |
| | 2+ | 1748.2660 | 1748.2657 | 0.2 | | 1847.9113 | LICTTAVPWNASWSNK | | 555-570 | [Hex]3[HexNAc]5[Fuc]1 |
| | 3+ | 1165.8545 | 1165.8462 | 7.1 | | 1847.9113 | LICTTAVPWNASWSNK | | 555-570 | [Hex]3[HexNAc]5[Fuc]1 |
| | 3+ | 1233.5400 | 1233.5394 | 0.5 | | 1847.9113 | LICTTAVPWNASWSNK | | 555-570 | [Hex]3[HexNAc]6[Fuc]1 |
| | 3+ | 1238.1741 | 1238.1579 | 13.1 | | 1847.9113 | LICTTAVPWNASWSNK | | 555-570 | [Hex]3[HexNAc]6[S03]2 |
| | 2+ | 1626.2139 | 1626.2127 | 6.9 | | 1847.9113 | LICTTAVPWNASWSNK | | 555-570 | [Hex]4[HexNAc]3[Fuc]1 |
| | 3+ | 1084.4862 | 1084.4776 | 7.9 | | 1847.9113 | LICTTAVPWNASWSNK | | 555-570 | [Hex]4[HexNAc]3[Fuc]1 |
| | 3+ | 1181.4943 | 1181.5094 | 12.8 | | 1847.9113 | LICTTAVPWNASWSNK | | 555-570 | [Hex]4[HexNAc]3[Fuc]1[NeuNAc]1 |
| | 3+ | 1219.8777 | 1219.8638 | 11.4 | | 1847.9113 | LICTTAVPWNASWSNK | | 555-570 | [Hex]4[HexNAc]5[Fuc]1 |
| | 3+ | 1384.5927 | 1384.5888 | 2.8 | | 1841.9113 | LICTTAVPWNASWSNK | | 555-570 | [Hex]4[HexNAc]6[Fuc]1[NeuNAc]1 |
| | 2+ | 1531.6864 | 1532.6705 | 10.4 | | 1847.9113 | LICTTAVPWNASWSNK | | 555-570 | [Hex]5[HexNAc]2 |
| | 2+ | 1808.7661 | 1808.7788 | 7.0 | | 1847.9113 | LICTTAVPWNASWSNK | | 555-570 | [Hex]5[HexNAc]4[Fuc]1 |
| | 3+ | 1206.1957 | 1206.1883 | 6.1 | 1026.1/1 099.0 | 1847.9113 | LICTTAVPWNASWSNK | | 555-570 | [Hex]5[HexNAc]4[Fuc]1 |
| | 3+ | 1303.2216 | 1303.2201 | 1.2 | 1099.2/1 026 | 1847.9113 | LICTTAVPWNASWSNK | | 555-570 | [Hex]5[HexNAc]4[Fuc]1[NeuNAc]1 |
| | 3+ | 1273.8855 | 1273.8814 | 3.2 | | 1847.9113 | LICTTAVPWNASWSNK | | 555-570 | [Hex]5[HexNAc]5[Fuc]1 |
| | 2+ | 1613.6986 | 1613.6969 | 1.1 | | 1847.9113 | LICTTAVPWNASWSNK | | 555-570 | [Hex]6[HexNAc]2 |
| | 3+ | 1076.1353 | 1076.1337 | 1.5 | | 1847.9113 | LICTTAVPWNASWSNK | | 555-570 | [Hex]6[HexNAc]2 |
| | 3+ | 1191.5289 | 1192.5128 | 13.5 | | 1847.9113 | LICTTAVPWNASWSNK | | 555-570 | [Hex]6[HexNAc]3[Fuc]1 |
| | 3+ | 1238.1741 | 1238.1722 | 1.5 | | 1847.9113 | LICTTAVPWNASWSNK | | 555-570 | [Hex]6[HexNAc]4[SO3]1 |
| | 3+ | 1327.8999 | 1327.8990 | 0.7 | | 1847.9113 | LICTTAVPWNASWSNK | | 555-570 | [Hex]6[HexNAc]5[Fuc]1 |
| | 2+ | 1694.7447 | 1694.7233 | 12.6 | | 1847.9113 | LICTTAVPWNASWSN | | 555-570 | [Hex]7[HexNAc]2 |
| | 3+ | 1314.2352 | 1314.2235 | 8.9 | | 1847.9113 | LICTTAVPWNASWSNK | | 555-570 | [Hex]7[HexNAc]4[Fuc]1 |
| | 2+ | 1775.7544 | 1775.7497 | 2.6 | | 1847.9113 | LICTTAVPWNASWSNK | | 555-570 | [Hex]8[HexNAc]2 |
| | 3+ | 1184.1662 | 1184.1689 | 2.3 | | 1847.9113 | LICTTAVPWNASWSNK | | 555-570 | [Hex]8[HexNAc]2 |
| | 3+ | 1238.1741 | 1238.1865 | 10.0 | | 1847.9113 | LICTTAVPWNASWSNK | | 555-570 | [Hex]9[HexNAc]2 |
| | | | | | | | | | | |
| | 3+ | 1079.4228 | 1079.4279 | 4.8 | | 1695.7412 | IWNNMTWMEWER | | 575-586 | [Hex]7[HexNAc]2 |
| | | | | | | | | | | |
| 27-28 | 4+ | 1481.4264 | 1481.4040 | 15.1 | 1562.3 (+3) | | AYDTEVHNVWATHACVPTDPN PQEVVLENVTEHFNMWK | | 54-91 | [Hex]4[HexNAc]4[Fuc]1 |
| 30-31 | 4+ | 1532.1911 | 1532.1738 | 11.3 | 1561.7 (+3) | | AYDTEVHNVWATHACVPTDPN PQEVVLENVTEHFNMWK | | 54-91 | [Hex]5[HexNAc]2 |
| | 5+ | 1225.9579 | 1225.9405 | 14.2 | | | AYDTEVHNVWATHACVPTDPN PQEVVLENVTEHFNMWK | | 54-91 | [Hex]5[HexNAc]3[Fuc]1 |
| | 4+ | 1546.4609 | 1546.4292 | 20.5 | | | AYDTEVHNVWATHACVPTDPN PQEVVLENVTEHFNMWK | | 54-91 | [Hex]5[HexNAc]3[Fuc]1 |
| | 4+ | 1582.9747 | 1582.9437 | 19.6 | | | AYDTEVHNVWATHACVPTDPN PQEVVLENVTEHFNMWK | | 54-91 | [Hex]5[HexNAc]4[Fuc]1 |
| | 4+ | 1471.1580 | 1471.1473 | 7.3 | | | AYDTEVHNVWATHACVPTDPN PQEVVLENVTEHFNMWK | | 54-91 | [Hex]5[HexNAc]4[Fuc]1 |
| | 5+ | 1229.1332 | 1229.1395 | 5.1 | | | AYDTEVIINVWATHACVPTDPN PQEVVLENVTEHFNMWK | | 54-91 | [Hex]6[HexNAc]6[Fuc]1 |
| | 5+ | 1250.1566 | 1250.1458 | 8.6 | | | AYDTEVHNVWATHACVPTDPN PQEVVLENVTEHFNMWK | | 54-91 | |
| | 4+ | 1613.2335 | 1613.2002 | 20.6 | | | AYDTEVHNVWATHACVPTDPN PQEVVLENVTEHFNMWK | | 54-91 | \|Hex\|3\|Hex\|NAc\|3\|Fuc\|1 |
| | 5+ | 1266.5685 | 1266.5564 | 9.6 | | | AYDTEVIINVWATHACVPTDPN PQEVVLENVTEHFNMWK | | 54-91 | \|Hex\|3\|HexNAc\|3\|Fuc\|1 |
| | 5+ | 1258.3565 | 1258.3511 | 4.3 | | | AYDTEVHNVWATHACVPTDPN PQEVVLENVTEHFNMWK | | 54-91 | \|Hex\|3\|HexNAc\|6 |
| | 5+ | 1269.3493 | 1269.3275 | 17.2 | | | AYDTEVHNVWATHACVPTDPN PQEVVLENVTEHFNMWK | | 54-91 | \|Hex\|4\|HexNAc\|3\|Fuc\|1 |
| | 5+ | 1290.7546 | 1290.7616 | 5.4 | | | AYDTEVHNVWATHACVPTDPN PQEVVLENVTEHFNMWK | | 54-91 | \|Hex\|4\|HexNAc\|4\|Fuc\|1 |
| | 5+ | 1323.1835 | 1323.1722 | 8.5 | | | AYDTEVHNVWATHACVPTDPN PQEVVLENVTEHFNMWK | | 54-91 | \|Hex\|4\|HexNAc\|5\|Fuc\|1 |
| | 5+ | 1396.2154 | 1396.1986 | 12.0 | | | AYDTEVHNVWATHACVPTDPN PQEVVLENVTEHFNMWK | | 54-91 | \|Hex\|5\|HexNAc\|Fuc\|2 |
| | | | | | | | | | | |
| | | | | | | | | | | |
| 30-31 | 5+ | 1364.0199 | 1363.8970 | 90.1 | | | DVNATNTTNDSEGTMER | ox | 127-143 | 3 sites: Man7, Man9, Man9 |
| | 5+ | 1299.2039 | 1299.0758 | 98.6 | | | DVNATNTTNDSEGTMER | ox | 127-143 | 3 sites: Man7, Man7, Man7 |
| | | | | | | | | | | |
| 32 | | 1337.6222 | 1337.6335 | 8.4 | | | EIDNYTSEIYTLIEESQNQQEK | | | Non-glycosylated |
| | | | | | | | | | | |
| | | | | | | | | | | |
| | | | | | | | | | | |
| | | | | | | | | | | |

**Table 4: LC-MS Glycopeptide Assignments For CON-S**

| F# | Charge | Experimental | Theoretical | Error | Y₁ | \|Peptide + II\| | Peptide Sequence | Mod | Position | Glycan |
|---|---|---|---|---|---|---|---|---|---|---|
| 1-4 | 2+ | 1156.4487 | 1156.4539 | 4.50 | | 851.3722 | FNGTGPCK | FOR | 230-237 | \|Hex\|4\|HexNAc\|4\| |
| | 2+ | 1587.6075 | 1587.6072 | 0.19 | | 823.3773 | FNGTGPCK | | 230-237 | \|Hex\|5\|HexNAc\|4\|Fuc\|1\|NeuNAc\|2 |
| | 2+ | 1291.9885 | 1291.9934 | 3.79 | 1083.5 | 880.3987 | FNGTGPCK | | 230-237 | \|Hex\|8\|HexNAc\|2 |
| | 2+ | 1373.0222 | 1373.0198 | 1.75 | 1083.5 | 880.3987 | FNGTGPCK | | 230-237 | \|Hex\|9\|HexNAc\|2 |
| | | | | | | | | | | |
| 1-2 | 2+ | 1116.9586 | 1116.9549 | 3.31 | | 788.3691 | EHFNNK | | 347-352 | \|Hex\|3\|HexNAc\|4\|Fuc\|1 |
| | 2+ | 1218.5059 | 1218.4946 | 9.27 | | 788.3691 | EHFNNK | | 347-352 | \|Hex\|5\|HexNAc\|5\|Fuc\|1 |
| | 2+ | 1164.9294 | 1164.9308 | 1.12 | | 788.3691 | EHFNNK | | 347-352 | \|Hex\|4\|HexNAc\|4\|SO3\|1 |
| | 2+ | 1002.9005 | 1002.8994 | 1.10 | 991.57 | 788.3691 | EHFNNK | | 347-352 | \|Hex\|5\|HexNAc\|2 |
| | 2+ | 1177.4673 | 1177.4680 | 0.59 | | 788.3691 | EHFNNK | | 347-352 | \|Hex\|5\|HexNAc\|3\|Fuc\|1 |
| | 2+ | 1245.9790 | 1245.9786 | 0.32 | | 788.3691 | EHFNNK | | 347-352 | \|Hex\|8\|HexNAc\|2 |
| | 2+ | 1327.0049 | 1327.0050 | 0.08 | | 788.3691 | EHFNNK | | 347-352 | \|Hex\|9\|HexNAc\|2 |
| | | | | | | | | | | |
| 3-4 | 2+ | 1251.5432 | 1251.5475 | 3.44 | 631.0/12 61.6 | 1057.5543 | LREHFNNK | | 345-352 | \|Hex\|3\|HexNAc\|4\|Fuc\|1 |
| | 2+ | 1353.0867 | 1353.0872 | 0.37 | | 1057.5543 | LREHFNNK | | 345-352 | \|Hex\|3\|HexNAc\|5\|Fuc\|1 |
| | 2+ | 1231.0430 | 1231.0342 | 7.15 | | 1057.5543 | LREHFNNK | | 345-352 | \|Hex\|4\|HexNAc\|3\|Fuc\|1 |
| | 2+ | 1137.4981 | 1137.4920 | 5.36 | | 1057.5543 | LREHFNNK | | 345-352 | \|Hex\|5\|HexNAc\|2 |
| | 2+ | 1312.0724 | 1312.0606 | 8.99 | | 1057.5543 | LREHFNNK | | 345-352 | \|Hex\|5\|HexNAc\|3\|Fuc\|1 |
| | 2+ | 1218.5217 | 1218.5184 | 2.71 | | 1057.5543 | LREHFNNK | | 345-352 | \|Hex\|6\|HexNAc\|2 |
| | 2+ | 1380.5756 | 1380.5712 | 3.19 | | 1057.5543 | LREHFNNK | | 345-352 | \|Hex\|8\|HexNAc\|2 |
| | 2+ | 1461.5983 | 1461.5976 | 0.48 | | 1057.5543 | LREHFNNK | | 345-352 | \|Hex\|9\|HexNAc\|2 |
| | | | | | | | | | | |
| 2-4 | 2+ | 1166.4753 | 1166.4781 | 2.49 | | 1115.5268 | QAHCNISGTK | | 324-333 | \|Hex\|5\|HexNAc\|2 |
| | 2+ | 1157.9581 | 1157.9649 | 5.87 | 1301.5 | 1098.5002 | QAHCNISGTK | Pyro C | 324-333 | \|Hex\|5\|HexNAc\|2 |
| | 2+ | 1587.6075 | 1587.6184 | 6.87 | | 1098.5002 | QAHCNISGTK | PyroC | 324-333 | \|Hex\|6\|HexNAc\|4\|Fuc\|1 |
| | 2+ | 1409.5582 | 1409.5574 | 0.49 | | 1115.5268 | QAHCNISGTK | | 324-333 | \|Hex\|8\|HexNAc\|2 |
| | 3+ | 940.0417 | 940.0407 | 1.06 | 1318.5 | 1115.5268 | QAHCNISGTK | | 324-333 | \|Hex\|8\|HexNAc\|2 |
| | 2+ | 1401.0501 | 1401.0442 | 4.21 | | 1098.5002 | QAHCNISGTK | Pyro C | 324-333 | \|Hex\|8\|HexNAc\|2 |
| | 2+ | 1490.5868 | 1490.5839 | 2.01 | 1318.6 | 1115.5268 | QAHCNISGTK | | 324-333 | \|Hex\|9\|HexNAc\|2 |
| | 3+ | 994.0609 | 994.0583 | 2.62 | 660.02/1 318.6 | 1115.5268 | QAHCNISGTK | | 324-333 | \|Hex\|9\|HexNAc\|2 |
| | 2+ | 1482.0715 | 1482.0706 | 0.61 | 1301.6 | 1098.5002 | QAHCNISGTK | Pyro C | 324-333 | \|Hex\|9\|HexNAc\|2 |
| | | | | | | | | | | |
| 18-19 | 3+ | 1218.8466 | 1218.8505 | 3.20 | | 1543.7440 | QAHCNISGTKWNK | | 324-336 | \|Hex\|3\|HexNAc\|8 |
| | 3+ | 1305.1849 | 1305.1852 | 0.23 | 1726.5 | 1571.7389 | QAHCNISGTKWNK | FOR | 324-336 | \|Hex\|6\|HexNAc\|3\|Fuc\|1\|NeuGe\|2 |
| | | | | | | | | | | |
| 2-3 | 2+ | 1319.5503 | 1319.5529 | 1.97 | 1339.4 | 990.4856 | SENITNNAK | | 271-279 | \|Hex\|3\|HexNAc\|5\|Fuc\|1 |
| | 2+ | 1205.5023 | 1205.4973 | 4.15 | | 990.4856 | SENITNNAK | | 271-279 | \|Hex\|5\|HexNAc\|3 |
| | 2+ | 1372.5452 | 1372.5479 | 1.97 | | 1033.4914 | SENITNNAK | U | 271-279 | \|Hex\|5\|HexNAc\|3\|NeuNAc\|1 |
| | 2+ | 1103.9567 | 1103.9576 | 0.82 | 1193.5 | 990.4856 | SENITNNAK | | 271-279 | \|Hex\|5\|HexNAc\|2 |
| | 2+ | 1184.9899 | 1184.9841 | 4.89 | | 990.4856 | SENITNNAK | | 271-279 | \|Hex\|6\|HexNAc\|2 |
| | 2+ | 1409.5882 | 1409.5663 | 15.54 | | 1033.4914 | SENITNNAK | U | 271-279 | \|Hex\|6\|HexNAc\|4 |
| | 2+ | 1584.1332 | 1584.1350 | 1.14 | | 1033.4914 | SENITNNAK | U | 271-279 | \|Hex\|6\|HexNAc\|5\|Fuc\|1 |
| | 2+ | 1266.0109 | 1266.0105 | 0.39 | 1193.5 | 990.4856 | SENITNNAK | | 271-279 | \|Hex\|7\|HexNAc\|2 |
| | 2+ | 1490.5868 | 1490.5927 | 3.96 | | 1033.4914 | SENITNNAK | U | 271-279 | \|Hex\|7\|HexNAc\|4 |
| 3-4 | 2+ | 1347.0394 | 1347.0369 | 1.86 | 1193.5 | 990.4856 | SENITNNAK | | 271-279 | \|Hex\|8\|HexNAc\|2 |
| | 2+ | 1428.0684 | 1428.0633 | 3.57 | | 990.4856 | SENITNNAK | | 271-279 | \|Hex\|9\|HexNAc\|2 |
| | | | | | | | | | | |
| 3 | 2+ | 1442.5788 | 1442.6577 | 54.69 | 1546.7 | 1343.7801 | WNKTLQQVAKK | | 334-344 | \|Hex\|7\|HexNAc\|2 |
| | 2+ | 1156.4409 | 1156.4383 | 2.33 | 650.33 | 447.2356 | WNK | | 334-336 | \|Hex\|9\|HexNAc\|2 |
| | | | | | | | | | | |
| 4-5 | 2+ | 1160.4872 | 1160.4858 | 1.21 | | 1427.6476 | EANTTLFCASDAK | | 46-58 | \|Hex\|3\|HexNAc\|2 |
| | 2+ | 1261.0140 | 1262.0255 | 9.11 | | 1427.6476 | EANTTLFCASDAK | | 46-58 | \|Hex\|3\|HexNAc\|3 |
| | 2+ | 1335.0390 | 1335.0545 | 11.61 | 1630.7/1 776.6 | 1427.6476 | EANTTLFCASDAK | | 46-58 | \|Hex\|3\|HexNAc\|3\|Fuc\|1 |
| | 2+ | 1363.5621 | 1363.5652 | 2.27 | | 1427.6476 | EANTTLFCASDAK | | 46-58 | \|Hex\|3\|HexNAc\|4 |
| | 2+ | 1436.5968 | 1436.5941 | 1.88 | 1776.7/1 630.6 | 1427.6476 | EANTTLFCASDAK | | 46-58 | \|Hex\|3\|HexNAc\|4\|Fuc\|1 |
| | 2+ | 1465.1025 | 1465.1049 | 1.64 | | 1427.6476 | EANTTLFCASDAK | | 46-58 | \|Hex\|3\|HexNAc\|5 |
| | 2+ | 1538.1244 | 1538.1339 | 6.18 | | 1427.6476 | EANTTLFCASDAK | | 46-58 | \|Hex\|3\|HexNAc\|5\|Fuc\|1 |
| | 2+ | 1639.6930 | 1639.6736 | 11.83 | | 1427.6476 | EANTTLFCASDAK | | 46-58 | \|Hex\|3\|HexNAc\|6\|Fuc\|1 |
| | 2+ | 1241.5155 | 1241.5123 | 2.58 | | 1427.6476 | EANTTLFCASDAK | | 46-58 | \|Hex\|4\|HexNAc\|2 |
| | 2+ | 1343.0704 | 1343.0519 | 13.77 | | 1427.6476 | EANTTLFCASDAK | | 46-58 | \|Hex\|4\|HexNAc\|3 |
| | 2+ | 1416.0768 | 1416.0809 | 2.90 | 1630.7/1 776.7 | 1427.6476 | EANTTLFCASDAK | | 46-58 | \|Hex\|4\|HexNAc\|3\|Fuc\|1 |
| | 2+ | 1424.0856 | 1424.0783 | 5.13 | 1630.7 | 1427.6476 | EANTTLFCASDAK | | 46-58 | \|Hex\|5\|HexNAc\|3 |
| | 2+ | 1322.5446 | 1322.5386 | 4.54 | 1630.7 | 1427.6476 | EANTTLFCASDAK | | 46-58 | \|Hex\|5\|HexNAc\|2 |
| | 2+ | 1497.1163 | 1497.1073 | 6.01 | | 1427.6476 | EANTTLFCASDAK | | 46-58 | \|Hex\|5\|HexNAc\|3\|Fuc\|1 |
| | 2+ | 1464.0842 | 1464.0567 | 18.78 | | 1427.6476 | EANTTLFCASDAK | | 46-58 | \|Hex\|5\|HexNAc\|3\|SO3\|1 |
| | 2+ | 1525.6765 | 1525.6180 | 12.13 | | 1427.6476 | EANTTLFCASDAK | | 46-58 | \|Hex\|5\|HexNAc\|4 |
| | 2+ | 1403.5756 | 1403.5651 | 7.48 | 1630.7 | 1427.6476 | EANTTLFCASDAK | | 46-58 | \|Hex\|6\|HexNAc\|2 |
| | 2+ | 1505.1039 | 1505.1047 | 0.53 | 1630.6 | 1427.6476 | EANTTLFCASDAK | | 46-58 | \|Hex\|6\|HexNAc\|3 |
| | 2+ | 1578.1262 | 1578.1337 | 4.75 | | 1427.6476 | EANTTLFCASDAK | | 46-58 | \|Hex\|6\|HexNAc\|3\|Fuc\|1 |
| | 2+ | 1606.6709 | 1606.6444 | 16.49 | | 1427.6476 | EANTTLFCASDAK | | 46-58 | \|Hex\|4\|HexNAc\|4 |
| | 2+ | 1484.6057 | 1484.5915 | 9.56 | 1630.9 | 1427.6476 | EANTTLFCASDAK | | 46-58 | \|Hex\|7\|HexNAc\|2 |
| | 2+ | 1565.6242 | 1565.6179 | 4.02 | 1630.7 | 1427.6476 | EANTTLFCASDAK | | 46-58 | \|Hex\|8\|HexNAc\|2 |
| | 2+ | 1646.6523 | 1646.6443 | 4.86 | 1630.7 | 1427.6476 | EANTTLFCASDAK | | 46-58 | \|Hex\|9\|HexNAc\|2 |
| | 2+ | 1436.5968 | 1436.5942 | 1.81 | | 1370.6262 | EANTTLFCASDAK | | 46-58 | \|Hex\|3\|HexNAc\|5 |
| | 2+ | 1538.1244 | 1538.1339 | 6.18 | | 1370.6262 | EANTTLFCASDAK | | 46-58 | \|Hex\|3\|HexNAc\|6 |
| | 2+ | 1416.0768 | 1416.0809 | 2.90 | | 1370.6262 | EANTTLFCASDAK | | 46-58 | \|Hex\|4\|HexNAc\|4 |
| | 2+ | 1517.6222 | 1517.6206 | 1.05 | 1630.7/1 776.7 | 1370.6262 | EANTTLFCASDAK | | 46-58 | \|Hex\|4\|HexNAc\|5 |
| | 2+ | 1686.6788 | 1686.6631 | 9.31 | | 1370.6262 | EANTTLFCASDAK | | 46-58 | \|Hex\|5\|HexNAc\|3\|NeuNAc\|2 |
| | 2+ | 1497.1163 | 1497.1073 | 6.01 | | 1370.6262 | EANTTLFCASDAK | | 46-58 | \|Hex\|5\|HexNAc\|4 |
| | 3+ | 1086.7249 | 1086.7223 | 2.39 | | 1370.6262 | EANTTLFCASDAK | | 46-58 | \|Hex\|8\|HexNAc\|3\|Fuc\|1\|NeuGe\|2 |
| | 2+ | 1578.1262 | 1578.1337 | 4.75 | | 1370.6262 | EANTTLFCASDAK | | 46-58 | \|Hex\|6\|HexNAc\|4 |
| | 2+ | 1659.1649 | 1659.1601 | 2.89 | | 1370.6262 | EANTTLFCASDAK | | 46-58 | \|Hex\|7\|HexNAc\|4 |
| | | | | | | | | | | |
| | | | | | | | | | | |
| 4-5 | 2+ | 1535.1362 | 1535.1326 | 2.35 | | 1690.8256 | LINCNTSAITQACPK | | 190-204 | \|Hex\|3\|HexNAc\|4\|SO3\|1 |
| 15-16 | 2+ | 1616.1596 | 1616.1590 | 0.37 | | 1690.8256 | LINCNTSAITQACPK | | 190-204 | \|Hex\|4\|HexNAc\|4\|SO3\|1 |
| 18-20 | 2+ | 1628.7059 | 1628.6963 | 5.96 | | 1690.8256 | LINCNTSAITQACPK | | 190-204 | \|Hex\|5\|HexNAc\|3\|Fuc\|1 |
| | 2+ | 1730.2230 | 1730.2360 | 7.51 | | 1690.8256 | LINCNTSAITQACPK | | 190-204 | \|Hex\|5\|HexNAc\|4\|Fuc\|1 |
| | 2+ | 1535.1362 | 1535.1541 | 11.66 | | 1690.8256 | LINCNTSAITQACPK | | 190-204 | \|Hex\|6\|HexNAc\|2 |
| | 2+ | 1616.1596 | 1616.1805 | 12.93 | | 1690.8256 | LINCNTSAITQACPK | | 190-204 | \|Hex\|7\|HexNAc\|2 |
| | | | | | | | | | | |
| | 2+ | 1292.0772 | 1292.0748 | 1.86 | 1893.8/9 47.7 | 1690.8256 | LINCNTSAITQACPK | | 190-204 | \|Hex\|3\|HexNAc\|2 |
| | 2+ | 1393.6180 | 1393.6145 | 2.51 | 1894.6/9 48.2 | 1690.8256 | LINCNTSAITQACPK | | 190-204 | \|Hex\|3\|HexNAc\|3 |
| | 2+ | 1466.6413 | 1466.6435 | 1.50 | | 1690.8256 | LINCNTSAITQACPK | | 190-204 | \|Hex\|3\|HexNAc\|3\|Fuc\|1 |
| | 2+ | 1495.1548 | 1495.1542 | 0.40 | | 1690.8256 | LINCNTSAITQACPK | | 190-204 | \|Hex\|3\|HexNAc\|4 |
| | 2+ | 1568.1874 | 1568.1831 | 2.74 | 1893.9 | 1690.8256 | LINCNTSAITQACPK | | 190-204 | \|Hex\|3\|HexNAc\|4\|Fuc\|1 |
| | 2+ | 1669.7298 | 1669.7229 | 4.13 | 1893.8 | 1690.8256 | LINCNTSAITQACPK | | 190-204 | \|Hex\|3\|HexNAc\|5\|Fuc\|1 |
| | 2+ | 1771.2772 | 1771.2626 | 8.24 | | 1690.8256 | LINCNTSATTQACPK | | 190-204 | \|Hex\|3\|HexNAc\|6\|Fuc\|1 |
| | 2+ | 1373.1031 | 1373.1013 | 1.31 | 1893.8/9 47.7 | 1690.8256 | LINCNTSATTPQACPK | | 190-204 | \|Hex\|4\|HexNAc\|2 |
| | 2+ | 1474.6313 | 1474.6409 | 6.51 | 1893.9/9 48 | 1690.8256 | LINCNTSAITQACPK | | 190-204 | \|Hex\|4\|HexNAc\|3 |
| | 2+ | 1547.6695 | 1547.6699 | 0.26 | | 1690.8256 | LINCNTSAITQACPK | | 190-204 | \|Hex\|4\|HexNAc\|3\|Fuc\|1 |
| | 2+ | 1750.7448 | 1750.7492 | 2.51 | | 1690.8256 | LINCNTSAITQACPK | | 190-204 | \|Hex\|8\|HexNAc\|5\|Fuc\|1 |
| | 3+ | 1361.5569 | 1361.5655 | 6.32 | | 1690.8256 | LINCNTSAITQACPK | | 190-204 | \|Hex\|4\|HexNAc\|5\|Fuc\|1\|NeuNAc\|2 |
| | 2+ | 1555.6774 | 1555.6673 | 6.49 | | 1690.8256 | LINCNTSAITPQACPK | | 190-204 | \|Hex\|5\|HexNAc\|3 |
| | 2+ | 1454.1300 | 1454.1276 | 1.65 | 1893.8/9 47.7 | 1690.8256 | LINCNTSAITQACPK | | 190-204 | \|Hex\|5\|HexNAc\|2 |
| | 2+ | 1628.7037 | 1628.6963 | 4.60 | 1893.8 | 1690.8256 | LINCNTSAITQACPK | | 190-204 | \|Hex\|5\|HexNAc\|3\|Fuc\|1 |
| | 2+ | 1730.2303 | 1730.2360 | 3.29 | | 1690.8256 | LINCNTSAITQACPK | | 190-204 | \|Hex\|5\|HexNAc\|4\|Fuc\|1 |
| | 3+ | 1347.8951 | 1347.8900 | 3.78 | | 1690.8256 | LINCNTSAITQACPK | | 190-204 | \|Hex\|5\|HexNAc\|4\|Fuc\|1NeuNAc\|2 |
| | 2+ | 1831.7700 | 1831.7757 | 3.11 | | 1690.8256 | LINCNTSAITQACPK | | 190-204 | \|Hex\|5\|HexNAc\|5\|Fuc\|1 |
| | 2+ | 1535.1602 | 1535.1541 | 3.97 | | 1690.8256 | LINCNTSAITQACPK | | 190-204 | \|Hex\|6\|HexNAc\|2 |
| | 2+ | 1636.6929 | 1636.6937 | 0.49 | | 1690.8256 | LINCNTSAITQACPK | | | \|Hex\|6\|HexNAc\|3 |
| | 2+ | 1709.7191 | 1709.7227 | 2.11 | | 1690.8256 | LINCNTSAITQACPK | | 190-204 | \|Hex\|6\|HexNAc\|3\|Fuc\|1 |
| | 2+ | 1616.1805 | 1616.1805 | 0.00 | 1893.9/9 47.6 | 1690.8256 | LINCNTSAITQACPK | | 190-204 | \|Hex\|7\|HexNAc\|2 |
| | 2+ | 1697.2128 | 1697.2069 | 3.48 | | 1690.8256 | LINCNTSAITQACPK | | 190-204 | \|Hex\|8\|HexNAc\|2 |
| | 2+ | 1778.2159 | 1778.2333 | 9.79 | | 1690.8256 | LINCNTSAITQACPK | | 190-204 | \|Hex\|9\|HexNAc\|2 |
| | 3+ | 1239.8433 | 1239.8422 | 0.89 | | 1690.8256 | LINCNTSAITQACPK | | 190-204 | \|Hex\|10\|HexNAc\|2 |
| | | | | | | | | | | |
| 15-17 | 2+ | 1270.0439 | 1270.0398 | 3.23 | | 1646.7555 | NNNNTNDTITLPCR | | 397-410 | \|Hex\|3\|HexNAc\|2 |
| 20-21 | 2+ | 1444.5985 | 1444.6084 | 6.85 | | 1646.7555 | NNNNTNDTITLPCR | | 397-410 | \|Hex\|3\|HexNAc\|3\|Fuc\|1 |
| 30-31 | 2+ | 1546.1551 | 1546.1481 | 4.53 | | 1646.7555 | NNNNTNDTITLPCR | | 397-410 | \|Hex\|3\|HexNAc\|4\|Fuc\|1 |
| | 2+ | 1618.6795 | 1618.6669 | 7.78 | | 1646.7555 | NNNNTNDTITLPCR | | 397-410 | \|Hex\|3\|HexNAc\|4\|NeuNAc\|1 |
| | 2+ | 1647.6877 | 1647.6878 | 0.06 | | 1646.7555 | NNNNTNDTITLPCR | | 397-410 | \|Hex\|3\|HexNAc\|5\|Fuc\|1 |
| | 2+ | 1351.0603 | 1351.0662 | 4.37 | 925.7/18 49.8 | 1646.7555 | NNNNTNDTITLPCR | | 397-410 | \|Hex\|4\|HexNAc\|2 |
| | 2+ | 1424.0871 | 1424.0951 | 5.62 | | 1646.7555 | NNNNTNDTITLPCR | | 397-410 | \|Hex\|4\|HexNAc\|2\|Fuc\|1 |
| | 2+ | 1452.6028 | 1452.6059 | 2.13 | | 1646.7555 | NNNNTNDTITLPCR | | 397-410 | \|Hex\|4\|HexNAc\|3 |
| | 2+ | 1525.6392 | 1525.6348 | 2.88 | | 1646.7555 | NNNNTNDTITLPCR | | 397-410 | \|Hex\|4\|HexNAc\|3\|Fuc\|1 |
| | 2+ | 1655.6876 | 1655.6853 | 1.39 | | 1646.7555 | NNNNTNDTITLPCR | | 397-410 | \|Hex\|4\|HexNAc\|5 |
| | 2+ | 1728.7193 | 1728.7142 | 2.95 | | 1646.7555 | NNNNTNDTITLPCR | | 397-410 | \|Hex\|4\|HexNAc\|5\|Fuc\|1 |
| | 3+ | 1346.8753 | 1346.8755 | 0.15 | | 1646.7555 | NNNNTNDTITLPCR | | 397-410 | \|Hex\|4\|HexNAc\|5\|Fuc\|1\|NeuNAc\|2 |
| | 3+ | 1268.8394 | 1268.8509 | 9.06 | 1849.8 | 1646.7555 | NNNNTNDTITLPCR | | 397-410 | \|Hex\|4\|HexNAc\|6\|Fuc\|1\|NeuNAc\|1 |
| | 2+ | 1533.6464 | 1533.6323 | 9.19 | | 1646.7555 | NNNNTNDTITLPCR | | 397-410 | \|Hex\|5\|HexNAc\|3 |
| | 2+ | 1432.0954 | 1432.0926 | 1.96 | | 1646.7555 | NNNNTNDTITLPCR | | 397-410 | \|Hex\|5\|HexNAc\|2 |
| | 3+ | 1071.4507 | 1071.4432 | 7.00 | | 1646.7555 | NNNNTNDTITLPCR | | 397-410 | \|Hex\|5\|HexNAc\|3\|Fuc\|1 |
| | 2+ | 1606.6708 | 1606.6612 | 5.98 | | 1646.7555 | NNNNTNDTITLPCR | | 397-410 | \|Hex\|5\|HexNAc\|3\|Fuc\|1 |
| | 2+ | 1635.1774 | 1635.1720 | 3.30 | | 1646.7555 | NNNNTNDTITLPCR | | 397-410 | [Hex]5[HexNAc]4 |
| | 3+ | 1120.1207 | 1110.1291 | 7.59 | 1849 | 1589.7341 | NNNNTNDTITLPCR | | 397-410 | [Hex]5[HexNAc]4[Fuc]1 |
| | 2+ | 1708.1051 | 1708.2009 | 2.52 | | 1646.7555 | NNNNTNDTITLPCR | | 397-410 | [Hex]5[HexNAc]4[Fuc]1 |
| | 3+ | 1206.8378 | 1206.8295 | 6.88 | | 1646.7555 | NNNNTNDTITLPCR | | 397-410 | [Hex]5[HexNAc]5[Fuc]1 |
| | 2+ | 1513.1252 | 1513.1190 | 4.10 | | 1646.7555 | NNNNTNDTITLPCR | | 397-410 | [Hex]6[HexNAc]2 |
| | | 1527.1224 | 1517.1165 | 3.86 | | 1646.7555 | NNNNTNDTITLPCR | FOR | 397-410 | [Hex]6[HexNAc]2 |
| | 3+ | 1260.8585 | 1260.8471 | 9.04 | | 1646.7555 | NNNNTNDTITLCR | | 397-410 | [Hex]6[HexNAc]5[Fuc]1 |
| | 3+ | 1309.5245 | 1309.5331 | 6.57 | | 1589.7341 | NNNNTNDTITLPCR | | 397-410 | [Hex]6[HexNAc]6[Fuc]1 |
| | 2+ | 1594.1540 | 1594.1454 | 5.39 | | 1646.7553 | NNNNTNDTITLPCR | | 397-410 | [Hex]7[HexNAc]2 |
| | 3+ | 1314.8659 | 1314.8647 | 0.91 | | 1589.7341 | NNNNTNDTITLPCR | | 397-410 | [Hex]7[HexNAc]6 |
| | 3+ | 1382.5632 | 1382.5578 | 3.91 | | 1646.7555 | NNNNTNDTITLPCR | | 397-410 | [Hex]7[HexNAc]6[Fuc]1 |
| | 3+ | 1117.1245 | 1117.1170 | 6.71 | | 1646.7555 | NNNNTNDTITLPCR | | 397-410 | [Hex]8[HexNAc]2 |
| | 2+ | 1675.1782 | 1675.1718 | 3.82 | | 1646.7555 | NNNNTNDTITLPCR | | 397-410 | [Hex]8[HexNAc]2 |
| | 3+ | 1171.1453 | 1171.1346 | 9.14 | | 1646.7555 | NNNNTNDTITLPCR | | 397-410 | [Hex]9[HexNAc]2 |
| | 2+ | 1756.2094 | 1756.1982 | 6.38 | | 1646.7555 | NNNNTNDTITLPCR | | 397-410 | [Hex]9[HexNAc]2 |
| | 3+ | 1279.1832 | 1279.1698 | 10.48 | | 1646.7555 | NNNNTNDTITLPCR | | 397-410 | [Hex]11[HexNAc]2 |
| | | | | | | | | | | |
| 18-20 | 3+ | 1232.5150 | 1232.5092 | 4.71 | | 2250.9798 | DGGNNNTNETEIFRPGGGDM R | | 448-408 | [Hex]3[HexNAc]4[Fuc]1 |
| | 3+ | 1300.2075 | 1300.2024 | 3.92 | | 2250.9798 | DGGNNNTNETEIFRPGGGDM R | | 448-408 | [Hex]3[HexNAc]5[Fuc]1 |
| | 3+ | 1367.8986 | 1367.8955 | 2.27 | | 2250.9798 | DGGNNNTNETEIFRPGGGDM R | | 448-408 | [Hex]3[HexNAc]6[Fuc]1 |
| | 3+ | 1102.4567 | 1102.4547 | 1.81 | 1227.9 | 2250.9798 | DGGNNNTNETEIFRPGGGDM R | | 448-408 | [Hex]4[HexNAc]2 |
| | 3+ | 1218.8466 | 1218.8337 | 10.58 | | 2250.9798 | DGGNNNTNETEIFRPGGGDM R | | 448-408 | [Hex]4[HexNAc]3[Fuc]1 |
| | 3+ | 1305.5118 | 1305.5340 | 9.34 | | 2250.9798 | DGGNNNTNETEIFRPGGGDM | | 448-408 | [Hex]4[HexNAc]5 |
| | 3+ | 1354.2230 | 1354.2200 | 2.22 | | 2150.9798 | DGGNNNTNETEIFRPGGGDM R | | 448-408 | [Hex]4[HexNAc]5[Fuc]1 |
| | 3+ | 1451.2552 | 1451.2518 | 2.34 | 1227.5 | 2250.9798 | DGGNNNTNETEIFRPGGGDM R | | 448-408 | [Hex]4[HexNAc]5[Fuc]1[NeuNAc]1 |
| | 3+ | 1518.9468 | 1518.9449 | 1.25 | | 2250.9798 | DGGNNNTNETEJFRPGGGDM R | | 448-408 | [Hex]4[HexNAc]6[Fuc]1[NeuNAc]1 |
| | 3+ | 1156.4763 | 1156.4722 | 3.55 | 1227.9 | 2250.9798 | DGGNNNTNETEIFRPGGGDM | | 448-408 | [Hex]5[HexNAc]2 |
| | | | | | | | R | | | |
| | 3+ | 1272.8628 | 1272.8513 | 9.03 | | 2250.9798 | DGGNNNTNETEIFRPGGGDM R | | 448-408 | [Hex]5[HexNAc]3[Fuc]1 |
| | 3+ | 1408.2524 | 1408.2376 | 10.51 | | 2250.9798 | DGGNNNTNETEIFRPGGGDM R | | 448-408 | [Hex]5[HexNAc]25[Fuc]1 |
| | 3+ | 1505.2666 | 1505.2694 | 1.86 | | 2250.9798 | DGGNNNTNETEIFRPGGGDM | | 448-408 | [Hex]5[HexNAc]5[Fuc]1[NeuNAc]1 |
| | 3+ | 1210.4972 | 1210.4899 | 6.03 | | 2250.9798 | DGGNNNTNETEIFRPGGGDM R | | 448-408 | [Hex]6[HexNAc]2 |
| | 3+ | 1326.8643 | 1326.8689 | 3.47 | | 2250.9798 | DGGNNNTNETEIFRPGGGDM R | | 448-408 | [Hex]6[HexNAc]3[Fuc]1 |
| | 3+ | 1375.2253 | 1375.2148 | 7.64 | | 2250.9798 | DGGNNNTNETEIFRPGGGDM R | | 448-408 | [Hex]6[HexNAc]3[NeuNAc]1 |
| | 3+ | 1437.5853 | 1437.5763 | 6.26 | | 2234.9849 | DGGNNNTNETEIFRPGGGDM R | | 448-408 | [Hex]6[HexNAc]4[NeuNAc]1 |
| | 3+ | 1462.2479 | 1462.2552 | 4.99 | | 2250.9798 | DGGNNNTNETEIFRPGGGDM R | | 448-408 | [Hex]6[HexNAc]5[Fuc]1 |
| | 3+ | 1559.3010 | 1559.2870 | 8.98 | | 2250.9798 | DGGNNNTNETEIFRPGGGDM R | | 448-408 | [Hex]6[HexNAc]5[Fuc]1[NeuNAc]1 |
| | 3+ | 1264.5123 | 1264.5075 | 3.80 | | 2250.9798 | DGGNNNTNETEIFRPGGGDM R | | 448-408 | [Hex]7[HexNAc]2 |
| | 3+ | 1372.5427 | 1372.5427 | 0.00 | 1227.9 | 2250.9798 | DGGNNNTNETEIFRPGGGDM R | | 448-408 | [Hex]9[HexNAc]2 |
| | 3+ | 1480.5706 | 1480.5779 | 4.93 | | 2250.9798 | DGGNNNTNETEIFRPGGGDM R | | 448-408 | [Hex]11[HexNAc]2 |
| | | | | | | | | | | |
| 20-21 | 2+ | 1522.6397 | 1522.6516 | 7.82 | | 1948.8997 | LDVVPIDDNNNNSSNYR | | 173-189 | [Hex]3[HexNAc]3 |
| | 2+ | 1595.6768 | 1595.6805 | 2.32 | | 1948.8997 | LDVVPIDDNNNNSSNYR | | 173.189 | [Hex]3[HexNAc]3[Fuc]1 |
| | 2+ | 1697.2305 | 1697.2202 | 6.07 | 1076.8 | 1948.8997 | LDVVPIDDNNNNSSNYR | | 173-189 | [Hex]3[HexNAc]4[Fuc]1 |
| | 2+ | 1798.7723 | 1798.7599 | 6.89 | | 1948.8997 | LDVVPIDDNNNNSSNYR | | 173-189 | [Hex]3[HexNAc]5[Fuc]1 |
| | 3+ | 1199.5139 | 1199.5090 | 4.09 | | 1948.8997 | LDVVPIDDNNNNSSNYR | | 173-189 | [Hex]3[HexNAc]5[Fuc]1 |
| | 2+ | 1900.3074 | 1900.2996 | 4.10 | | 1948.8997 | LDVVPIDDNNNNSSNYR | | 173-189 | [Hex]3[HexNAc]6[Fuc]1 |
| | 2+ | 1502.1424 | 1502.1383 | 2.73 | 1076.8 | 1948.8997 | LDVVPIDDNNNNSSNYR | | 173-189 | [Hex]4[HexNAc]2 |
| | 2+ | 1603.6862 | 1603.6780 | 5.11 | | 1948.8997 | LDVVPIDDNNNNSSNYR | | 173-189 | [Hex]4[HexNAc]3 |
| | 2+ | 1676.7151 | 1676.7069 | 4.89 | | 1948.8997 | LDVVPIDDNNNNSSNYR | | 173-189 | [Hex]4[HexNAc]3[Fuc]1 |
| | 2+ | 1879.7935 | 1879.7863 | 3.83 | 1076.8 | 1948.8997 | LDVVPIDDNNNNSSNYR | | 173-189 | [Hex]4[HexNAc]5[Fuc]1 |
| | 2+ | 1684.7036 | 1684.7044 | 0.42 | | 1948.8997 | LDVVPIDDNNNNSSNYR | | 173-189 | [Hex]5[HexNAc]3 |
| | 2+ | 1583.1708 | 1583.1647 | 3.85 | | 1948.8997 | LDVVPIDDNNNNSSNYR | | 173-189 | [Hex]5[HexNAc]2 |
| | 2+ | 1757.7375 | 1757.7333 | 2.39 | | 1948.8997 | LDVVPIDDNNNNSSNYR | | 173-189 | [Hex]5[HexNAc]3[Fuc]1 |
| | 3+ | 1172.1578 | 1172.1580 | 0.17 | | 1948.8997 | LDVVPIDDNNNNSSNYR | | 173-189 | [Hex]5[HexNAc]3[Fuc]1 |
| | 2+ | 1960.7955 | 1960.8127 | 8.77 | | 1948.8997 | LDVVPIDDNNNNSSNYR | | 173-189 | [Hex]5[HexNAc]5[Fuc]1 |
| | 2+ | 1664.1902 | 1664.1911 | 0.54 | 1077.2 | 1948.8997 | LDVVPIDDNNNNSSNYR | | 173-189 | [Hex]6[HexNAc]2 |
| | 2+ | 1838.7486 | 1838.7597 | 6.04 | | 1948.8997 | LDVVPIDDNNNNSSNYR | | 173-189 | [Hex]6[HexNAc]3[Fuc]1 |
| | 3+ | 1274.5348 | 1274.5214 | 10.51 | | 1948.8997 | LDVVPIDDNNNNSSNYR | | 173.189 | [Hex]6[HexNAc]3[NeuNAc]1 |
| | 2+ | 1745.2247 | 1745.2175 | 4.13 | 1076.6 | 1948.8997 | LDVVPIDDNNNNSSNYR | | 173-189 | [Hex]7[HexNAc]2 |
| | 2+ | 1826.2494 | 1826.2439 | 3.01 | 1076.7 | 1948.8997 | LDVVPIDDNNNNSSNYR | | 173-189 | [Hex]8[HexNAc]2 |
| | 3+ | 1353.2122 | 1353.2179 | 4.21 | | 1948.8997 | LDVVPIDDNNNNSSNYR | | 173-189 | [Hex]8[HexNAc]4 |
| | 2+ | 1907.2739 | 1907.2703 | 1.89 | | 1948.8997 | LDVVPIDDNNNNSSNYR | | 173-189 | [Hex]9[HexNAc]2 |
| | | | | | | | | | | |
| 23-27 | 2+ | 1123.9866 | 1123.9833 | 2.94 | | 1354.6425 | NCSFNITTEIR | | 150-160 | [Hex]3[HexNAc]2 |
| 34-35 | 2+ | 1298.5541 | 1298.5519 | 1.69 | 1703.8/1 557.7 | 1354.6425 | NCSFNITTEIR | | 150-160 | [Hex]3[HexNAc]3[Fuc]1 |
| | 2+ | 1327.0679 | 1327.0626 | 3.99 | | 1354.6425 | NCSFNITTEIR | | 150-160 | [Hex]3[HexNAc]4 |
| | 2+ | 1400.1004 | 1400.0916 | 6.29 | | 1354.6425 | NCSFNITTEIR | | 150-160 | [Hex]3[HexNAc]4[Fuc]1 |
| | 2+ | 1501.6420 | 1501.6313 | 7.13 | | 1354.6425 | NCSFNITTEIR | | 150-160 | [Hex]3[HexNAc]5[Fuc]1 |
| | 2+ | 1603.1831 | 1603.1710 | 7.55 | 1703/15 57 | 1354.6425 | NCSFNITTEIR | | 150-160 | [Hex]3[HexNAc]6[Fuc]1 |
| | 2+ | 1306.5547 | 1306.5494 | 4.06 | 1557 | 1354.6423 | NCSFNITTEIR | | 150-160 | [Hex]4[HexNAc]3 |
| | 2+ | 1379.5812 | 1379.5783 | 2.10 | | 1354.6425 | NCSFNITTEIR | | 150-160 | [Hex]4[HexNAc]3[Fuc]1 |
| | 2+ | 1408.0902 | 1408.0891 | 0.78 | | 1354.6425 | NCSFNITTEIR | | 150-160 | [Hex]4[HexNAc]4 |
| | 2+ | 1422.0813 | 1422.0865 | 3.66 | | 1382.6374 | NCSFNITTEIR | FOR | 150-160 | [Hex]4[HexNAc]4 |
| | 2+ | 1481.1246 | 1481.1180 | 4.46 | 1703.8/1 557.7 | 1354.6425 | NCSFNITTEIR | | 150-160 | [Hex]4[HexNAc]4[Fuc]1 |
| | 2+ | 1205.0137 | 1205.0097 | 3.40 | | 1354.6425 | NCSFNITTEIR | | 150-160 | [Hex]4[HexNAc]2 |
| | 2+ | 1582.6652 | 1582.6577 | 4.74 | | 1354.6425 | NCSFNITTEIR | | 150-160 | [Hex]4[HexNAc]5[Fuc]1 |
| | 2+ | 1728.2005 | 1728.2054 | 2.84 | | 1197.6111 | NCSFNITTEIR | | 150-160 | [Hex]4[HexNAc]5[Fuc]1[NeuNAc]1 |
| | 2+ | 1286.0405 | 1286.0361 | 3.42 | | 1354.6425 | NCSFNITTEIR | | 150-160 | [Hex]5[HexNAc]2 |
| | 2+ | 1387.5846 | 1387.5758 | 6.41 | 1557.7 | 1354.6425 | NCSFNITTEIR | | 150-160 | [Hex]5[HexNAc]3 |
| | 2+ | 1533.1154 | 1533.1235 | 5.28 | | | NCSFNITTEIR | | 150-160 | [Hex]5[HexNAc]3[NeuNAc]1 |
| | 2+ | 1707.6869 | 1707.6921 | 3.05 | | 1354.6425 | NCSFNITTEIR | | 150-160 | [Hex]5[HexNAc]4[Fuc]1[NeuNAc]1 |
| | 2+ | 1460.6116 | 1460.6047 | 4.72 | | 1354.6425 | NCSFNITTEIR | | 150-160 | [Hex]5[HexNAc]3[Fuc]1 |
| | 2+ | 1489.1111 | 1489.1155 | 2.95 | | 1354.6425 | NCSFNITTEIR | | 150-160 | [Hex]5[HexNAc]4 |
| | 2+ | 1503.1151 | 1503.1129 | 1.46 | | 1382.6374 | NCSFNITTEIR | FOR | 150-160 | [Hex]5[HexNAc]4 |
| | 2+ | 1707.6948 | 1707.6921 | 1.58 | | 1297.6211 | NCSFNITTEIR | | 150-160 | [Hex]5[HexNAc]4[Fuc]1[NeuNAc]1 |
| | 2+ | 1367.0661 | 1367.0625 | 2.63 | 1557.7 | 1354.6425 | NCSFNITTEIR | | 150-160 | [Hex]6[HexNAc]2 |
| | 2+ | 1468.6081 | 1468.6022 | 4.02 | | 1354.6425 | NCSFNITTEIR | | 150-160 | [Hex]6[HexNAc]3 |
| | 2+ | 1671.6941 | 1671.6816 | 7.48 | | 1354.6425 | NCSFNITTEIR | | 150-160 | [Hex]6[HexNAc]5 |
| | 2+ | 1671.6914 | 1671.6816 | 5.86 | | 1297.6211 | NCSFNITTEIR | | 150-160 | [Hex]6[HexNAc]5 |
| | 2+ | 1541.6194 | 1541.6311 | 7.59 | 1703.8/1 557.6 | 1354.6425 | NCSFNITTEIR | | 150-160 | [Hex]6[HexNAc]3[Fuc]1 |
| | 2+ | 1541.6262 | 1541.6311 | 3.18 | 1703.8/1 557 | 1354.6425 | NCSFNITTEIR | | 150-160 | [Hex]6[HexNAc]3[Fuc]1 |
| | 2+ | 1584.1572 | 1584.1393 | 11.30 | | 1382.6374 | NCSFNITTEIR | FOR | 150-160 | [Hex]6[HexNAc]4 |
| | 2+ | 1448.0986 | 1448.0889 | 6.70 | | 1354.6425 | NCSFNITTEIR | | 150-160 | [Hex]7[HexNAc]2 |
| | 2+ | 1563.6435 | 1563.6260 | 11.19 | | 1382.6374 | NCSFNITTEIR | FOR | 150-160 | [Hex]7[HexNAc]3 |
| | 2+ | 1636.6431 | 1636.6555 | 7.58 | | 1354.6425 | NCSFNITTEIR | | 150-160 | [Hex]7[HexNAc]3[Fuc]1 |
| | 2+ | 1636.6431 | 1636.6550 | 7.27 | | 1325.6160 | NCSFNITTEIR | FOR | 150-160 | [Hex]7[HexNAc]4 |
| | 2+ | 1529.1250 | 1529.1153 | 6.34 | | 1354.6425 | NCSFNITTEIR | | 150-160 | [Hex]8[HexNAc]2 |
| | 2+ | 1610.1519 | 1610.1417 | 6.33 | 1557.7 | 1354.6425 | NCSFNITTEIR | | 150-160 | [Hex]9[HexNAc]2 |
| | | | | | | | | | | |
| | | | | | | | | | | |
| 29-30 | 2+ | 1165.0431 | 1165.0545 | 9.78 | 1290.7 | 1087.6477 | SNITGLLLTR | | 438-447 | [Hex]3[HexNAc]3[Fuc]1 |
| 26-28 | 2+ | 1193.5674 | 1193.5652 | 1.84 | 1290.7 | 1087.6477 | SNITGLLLTR | | 438-447 | [Hex]8[HexNAc]4 |
| 14-15 | 2+ | 1266.5982 | 1266.5942 | 3.24 | | 1087.6477 | SNITGLLLTR | | 438-447 | [Hex]3[HexNAc]4[Fuc]1 |
| | 2+ | 1266.5962 | 1266.5942 | 1.66 | | 1087.6477 | SNITGLLLTR | | 438-447 | [Hex]3[HexNAc]4[Fuc]1 |
| | 2+ | 1295.1151 | 1295.1050 | 7.88 | | 1087.6477 | SNITGLLLTR | | 438-447 | [Hex]3[HexNAc]5 |
| | 2+ | 1368.1384 | 1368.1339 . | 3.29 | | 1087.6477 | SNITGLLLTR | | 438-447 | [Hex]3[HexNAc]5[Fuc]1 |
| | 2+ | 1368.1382 | 1368.1339 | 3.14 | | 1087.6477 | SNITGLLLTR | | 438-447 | [Hex]3[HexNAc]3[Fuc]1 |
| | 2+ | 1267.5791 | 1267.5838 | 3.71 | | 1130.6535 | SNITGLLLTR | U | 438-447 | [Hex]4[HexNAc]3[Fuc]1 |
| | 2+ | 1274.5985 | 1274.5917 | 5.34 | | 1087.6477 | SNITGLLLTR | | 438-447 | [Hex]4[HexNAc]4 |
| | 2+ | 1507.1642 | 1507.1658 | 1.06 | | 1115.6426 | SNITGLLLTR | FOR | 438-447 | [Hex]4[HexNAc]4[Fuc]1[NeuNAc]1 |
| | 2+ | 1528.6717 | 1528.6687 | 1.96 | | 1158.6484 | SNITGLLLTR | FOR, U | 438-447 | [Hex]4[HexNAc]4[Fuc]1[NeuNAc]1 |
| | 2+ | 1071.5125 | 1071.5123 | 0.19 | 1290.7 | 1087.6477 | SNITGLLLTR | | 438-447 | [Hex]4[HexNAc]2 |
| | 2+ | 1173.0544 | 1173.0520 | 2.05 | | 1087.6477 | SNITGLLLTR | | 438-447 | [Hex]4[HexNAc]3 |
| | 2+ | 1246.0837 | 1246.0809 | 2.25 | 1290 | 1087.6477 | SNITGLLLTR | | 438-447 | [Hex]4[HexNAc]3[Fuc]1 |
| | 2+ | 1489.1458 | 1489.1387 | 4.77 | | 1087.6477 | SNITGLLLTR | | 438-447 | [Hex]4[HexNAc]5[Fuc]1[SO3]1 |
| | 2+ | 1254.0812 | 1254.0784 | 2.23 | 1290.7 | 1087.6477 | SNITGLLLTR | | 438-447 | [Hex]5[HexNAc]3 |
| | 2+ | 1268.0810 | 1268.0758 | 4.10 | | 1115.6426 | SNITGLLLTR | FOR | 438-447 | [Hex]5[HexNAc]3 |
| | 2+ | 1327.1130 | 1327.1073 | 4.30 | | 1087.6477 | SNITGLLLTR | | 438-447 | [Hex]5[HexNAc]3[Fuc]1 |
| | 2+ | 1355.6198 | 1355.6181 | 1.25 | | 1087.6477 | SNITGLLLTR | | 438-447 | [Hex]5[HexNAc]4 |
| | 2+ | 1269.0798 | 1269.0655 | 11.27 | | 1158.6484 | SNITGLLLTR | FOR, U | 438-447 | [Hex]6[HexNAc]2 |
| | 2+ | 1559.6943 | 1559.6871 | 4.62 | | 1130.6535 | SNITGLLLTR | U | 438-447 | [Hex]6[HexNAc]5 |
| | 2+ | 1233.5696 | 1233.5651 | 2.84 | 1290.7 | 1087.6477 | SNITGLLLTR | | 438-447 | [Hex]6[HexNAc]2 |
| | 2+ | 1429.6647 | 1429.6366 | 19.66 | | 1130.6535 | SNITGLLLTR | U | 438-447 | [Hex]6[HexNAc]3[Fuc]1 |
| | 2+ | 1314.5969 | 1314.5915 | 4.11 | 1290.7 | 1087.6477 | SNITGLLLTR | | 438-447 | [Hex]7[HexNAc]2 |
| | 2+ | 1417.1249 | 1417.1208 | 2.89 | | 1130.6535 | SNITGLLLTR | U | 438-447 | [Hex]8[HexNAc]2 |
| | 2+ | 1395.6220 | 1395.6179 | 2.94 | 1290.7 | 1087.6477 | SNITGLLLTR | | 438-447 | [Hex]8[HexNAc]2 |
| | 2+ | 1498.1530 | 1498.1472 | 3.87 | | 1130.6535 | SNITGLLLTR | U | 438-447 | [Hex]9[HexNAc]2 |
| | 2+ | 1476.6450 | 1476.6443 | 0.47 | 1290.7 | 1087.6477 | SNITGLLLTR | | 438-447 | [Hex]9[HexNAc]2 |
| | | | | | | | | | | |
| 31-32 | 6+ | 1377.4800 | 1377.2066 | **** | | 2850.3613 | LTPLCVTLNCTNVNVTNTTNN TEEK | | 121-145 | 4 Sites: Man6, Man6, Man6, Man5 |
| | 6+ | 1404.6400 | 1404.2154 | **** | | 2850.3613 | LTPLCVTLNCTNVNVTNTTNN TEEK | | 121-145 | 4 Sites: Man6, Man6, Man6, Man6 |
| | 6+ | 1431.4400 | 1431.2242 | **** | | 2850.3613 | LTPLCVTLNCTNVNVTNTTNN TEEK | | 121-145 | 4 Sites: Man6, Man6, Man6, Man7 |
| | 6+ | 1458.3100 | 1458.2330 | **** | | 2850.3613 | LTPLCVTLNCTNVNVTNTTNN TEEK | | 121-145 | 4 Sites: Man6, Man6, Man7, Man7 |
| | 6+ | 1485.3400 | 1485.2418 | **** | | 2850.3613 | LTPLCVTLNCTNVNVTNTTNN TEEK | | 121-145 | 4 Sites: Man6, Man7, Man7, Man7 |
| | 6+ | 1512.4100 | 1512.2506 | **** | | 2850.3613 | LTPLCVTLNCTNVNVTNTTNN TEEK | | 121-145 | 4 Sites: Man7, Man7, Man7, Man7 |
| | 6+ | 1539.3200 | 1539.2594 | **** | | 2850.3613 | LTPLCVTLNCTNVNVTNTTNN TEEK | | 121-145 | 4 Sites: Man8, Man7, Man7, Man7 |
| | 6+ | 1566.3400 | 1566.2682 | **** | | 2850.3613 | LTPLCVTLNCTNVNVTNTTNN TEEK | | 121-145 | 4 Sites: Man8, Man8, Man7, Man7 |
| | 6+ | 1593.1500 | 1593.2770 | **** | | 2850.3613 | LTPLCVTLNCTNVNVTNTTNN TEEK | | 121-145 | 4 Sites: Man8, Man8, Man8, Man7 |
| | 6+ | 1620.3200 | 1620.2858 | **** | | 2850.3613 | LTPLCVTLNCTNVNVTNTTNN TEEK | | 121-145 | 4 Sites: Man08, Man8, Man8, Man8 |
| | 6+ | 1647.5100 | 1647.2946 | **** | | 2850.3613 | LTPLCVTLNCTNVNVTNTTNN TEEK | | 121-145 - | 4 Sites: Man9, Man8, Man8, Man8 |
| | 6+ | 1674.4000 | 1674.3034 | **** | | 2850.3613 | LTPLCVTLNCTNVNVTNTTNN TEEK | | 121-145 | 4 Sites: Man9, Man9, Man8, Man8 |
| | 6+ | 1701.1700 | 1701.3122 | **** | | 2850.3613 | LTPLCVTLNCTNVNVTNTTNN TEEK | | 121-145 | 4 Sites: Man9, Man9, Man9, Man8 |
| | 5+ | 1717.2900 | 1717.2675 | **** | | 2850.3613 | LTPLCVTLNCTNVNVTNTTNN TEEK | | 121-145 | 4 Sites: Man6, Man6, Man6, Man7 |
| | 5+ | 1749.5800 | 1749.6781 | **** | | 2850.3613 | LTPLCVTLNCTNVNVTNTTNN TEEK | | 121-145 | 4 Sites: Man6, Man6, Man7, Man7 |
| | 5+ | 1782.3700 | 1782.0886 | **** | | 2850.3613 | LTPLCVTLNCTNVNVTNTTNN TEEK | | 121-145 | 4 Sites: Man6, Man7, Man7, Man7 |
| | 5+ | 1814.4300 | 1814.4992 | **** | | 2850.3613 | LTPLCVTLNCTNVNVTNTTNN TEEK | | 121-145 | 4 Sites: Man7, Man7, Man7, Man7 |
| | 5+ | 1847.2000 | 1846.9097 | **** | | 2850.3613 | LTPLCVTLNCTNVNVTNTTNN TEEK | | 121-145 | 4 Sites: Man8, Man7, Mn7, Man7 |
| | 5+ | 1879.5000 | 1879.3203 | **** | | 2850.3613 | LTPLCVTLNCTNVNVTNTTNN TEEK | | 121-145 | 4 Sites: Man8, Man8, Man7, Man7 |
| | 5+ | 1911.4300 | 1911.7309 | **** | | 2850.3613 | LTPLCVTLNCTNVNVTNTTNN TEEK | | 121-145 | 4 Sites: Man8, Man8, Man8, Man7 |
| | 5+ | 1944.4900 | 1944.1414 | **** | | 2850.3613 | LTPLCVTLNCTNVNVTNTTNN TEEK | | 121-145 | 4 Sites: Man8, Man8, Man8, Man8 |
| | 5+ | 1976.9500 | 1976.5520 | **** | | 2850.3613 | LTPLCVTLNCTNVNVTNTTNN TEEK | | 121.145 | 4 Sites: Man9, Man8, Man8, Man8 |
| | | | | | | | | | | |
| 35-36 | 4+ | 1479.4066 | 1479.3881 | 8.54 | | 4412.0389 | AYDTEVHNVWATHACVPTDP NPQEIVLENVTENFNMWK | U,Ox | 59-96 | [Hex]3[HexNAc]3[Fuc]2 |
| | 4+ | 1428.3833 | 1428.3841 | 0.53 | | 1.0078 | AYDTEVHNVWATHACVPTDP NPQEIVLENVTENFNMWK | | 59-96 | [Hex]3[HexNAc]4 |
| | 4+ | 1479.1620 | 1479.1539 | 5.46 | | 4412.0389 | AYDTEVHNVWATHACVPTDP NPQEIVLENVTENFNMWK | | 59-96 | [Hex]3[HexNAc]5 |
| | 4+ | 1493.4064 | 1493.4038 | 2.18 | | | AYDTEVHNVWATHACVPTDP NPQEIVLENVTENFNMWK | | 59-96 | [Hex]3[HexNAc]5 |
| | 4+ | 1529.9330 | 1529.9238 | 6.03 | | | AYDTEVHNVWATHACVPTDP NPQEIVLENVTENFNMWK | | 59-96 | [Hex]3[HexNAc]6 |
| | 4+ | 1468.8833 | 1468.8973 | 9.51 | | | AYDTEVHNVWATHACVPTDP NPQEIVLENVTENFNMWK | | 59-96 | [Hex]4 [HexNAc]4 |
| | 4+ | 1519.6750 | 1519.6671 | 5.18 | | | AYDTEVHNVWATHACVPTDP NPQEIVLENVTENFNMWK | | 59-96 | [Hex]4[HexNAc]5 |
| | 4+ | 1570.4200 | 1570.4369 | 10.76 | | 4469.0603 | AYDTEVHNVWATHACVPTDP NPQEIVLENVTENFNMWK | | 59-96 | [Hex]4[HexNAc]5[Fuc]1 |
| | 4+ | 1592.3395 | 1592.4410 | 63.74 | | | AYDTEVHNVWATHACVPTDP NPQEIVLENVTENFNMWK | | 59-96 | [Hex]4[HexNAc]5[NeuNAc]1 |
| | 4+ | 1509.4340 | 1509.4105 | 15.59 | | 4412.0399 | AYDTEVHNVWATHACVPTDP NPQEIVLENVTENFNMWK | | 59-96 | [Hex]5[HexNAc]3[Fuc]1 |
| | 4+ | 1422.1360 | 1422.1261 | 6.96 | | | AYDTEVHNVWATHACVPTDP NPQEIVLENVTENFNMWK | | 59-96 | [Hex]5[HexNAc]2 |
| | 4+ | 1560.2100 | 1560.1803 | 19.02 | | | AYDTEVHNVWATHACVPTDP NPQEIVLENVTENFNMWK | | 59-96 | [Hex]5[HexNAc]5 |
| | 4+ | 1632.7999 | 1632.9542 | 94.49 | | | AYDTEVHNVWATHACVPTDP NPQEIVLENVTENFNMWK | | 59-96 | [Hex]5[HexNAc]5[NeuNAc]1 |
| 36-37 | 4+ | 1462.6240 | 1462.6394 | 10.53 | | | AYDTEVHNVWATHACVPTDP NPQEIVLENVTENFNMWK | | 59-96 | [Hex]6[HexNAc2 |
| | 4+ | 1503.1770 | 1503.1526 | 16.23 | | | AYDTEVHNVWATHACVPTDP NPQEIVLENVTENFNMWK | | 59-96 | [Hex]7[HexNAc]2 |
| | 4+ | 1543.6690 | 1543.6658 | 2.07 | | | AYDTEVHNVWATHACVPTDP NPQEIVLENVTENFNMWK | | 59-96 | [Hex]8[HexNAc]2 |
| 36-37 | 4+ | 1584.1990 | 1584.1790 | 12.62 | | | AYDTEVHNVWATHACVPTDP NPQEIVLENVTENFNMWK | | 59-96 | [Hex]9[HexNAc]2 |
| | | | | | | | | | | |
| 39-40 | 2+ | 1336.1443 | 1336.1406 | 2.77 | | | EINNYTDIIYSLIEESQNQQEK | | | non glycosylated, |

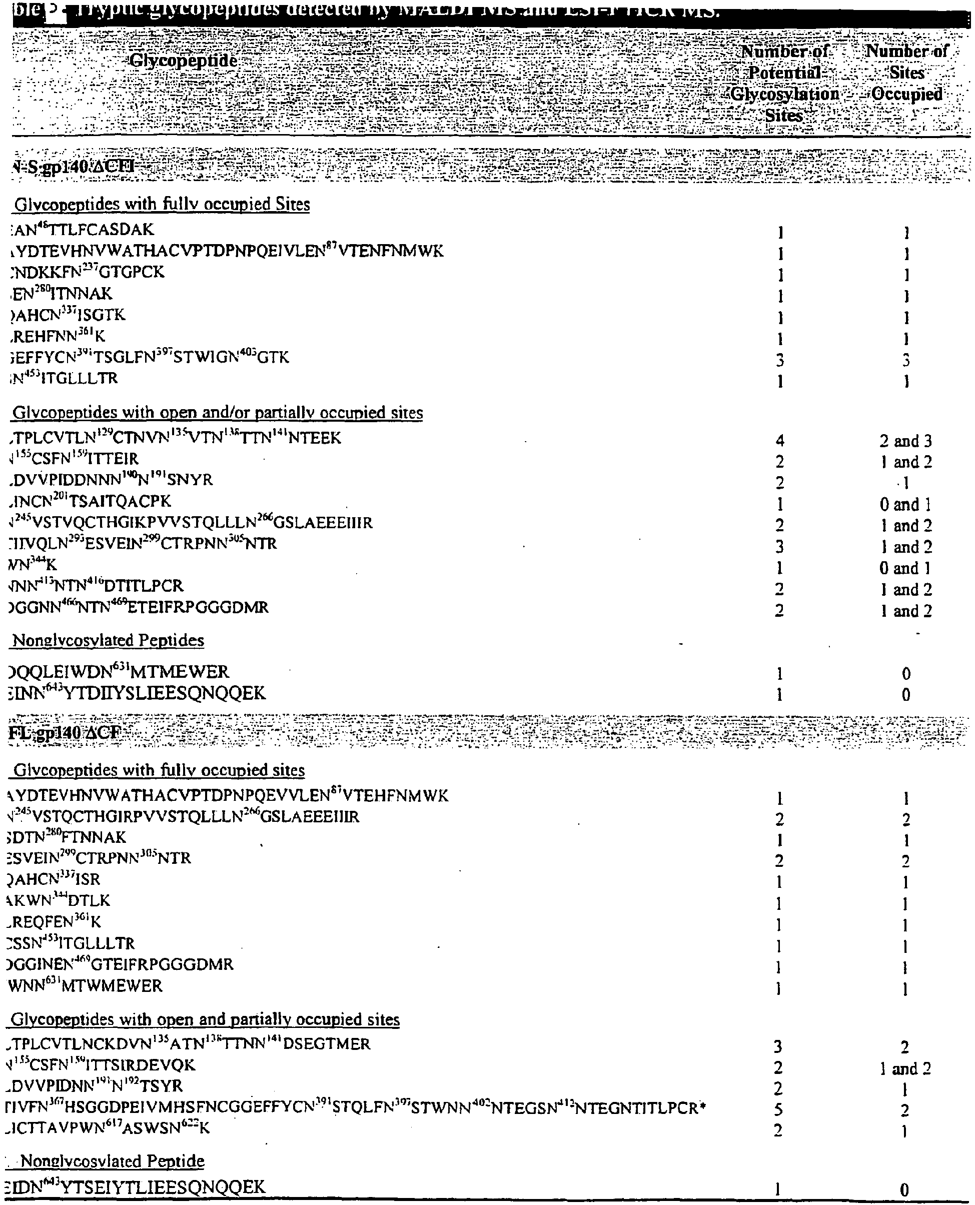

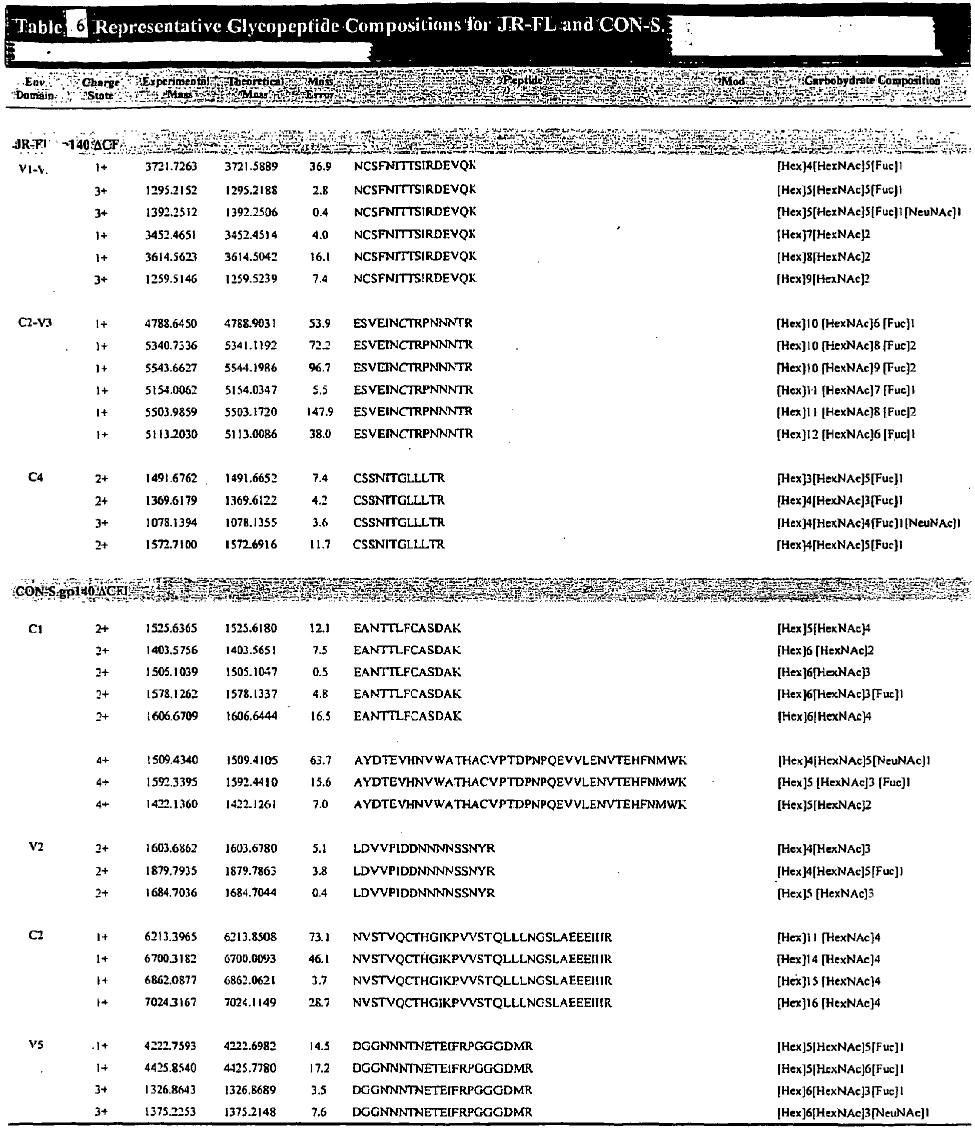

**Table 7. MALDI MS Glycopeptide Composition for CON-S gp140 ΔCF1**

| **Env Domain** | **Charge State** | **Experimental Mass** | **Theoretical Mass** | **Mass Error** | **Peptide** | **Mod*** | **Carbohydrate Composition** |
|---|---|---|---|---|---|---|---|
| C1 | 1+ | 2644.1221 | 2644.0699 | 19.7 | EANTTLFCASDAK | | [Hex]3[HexNAc]2 |
| | 1+ | 2888.3347 | 2888.1759 | 55.0 | EANTTLFCASDAK | | [Hex]4[HexNAc]4 |
| | 1+ | 2929.3943 | 2929.2025 | 65.5 | EANTTLFCASDAK | | [Hex]3[HexNAc]5 |
| | 1+ | 2993.3850 | 2993.2072 | 59.4 | EANTRLFCASDAK | | [Hex]5[HexNAc]3[Fuc]1 |
| | | | | | | | |
| C1-V1 | 1+ | 5041.5824 | 5041.1539 | 85.0 | LTPLCVTLNCTNVNVTNTTNNTEEK | | [Hex]6[HexNAc]6 |
| | 1+ | 5738.9521 | 5739.4285 | 83.0 | LTPLCVTLNCTNVNVTNTTNNTEEK | | [Hex]6[HexNAc]8[Fuc]2 |
| | 1+ | 5406.7663 | 5406.2861 | 88.8 | LTPLCVTLNCTNVNVTNTTNNTEEK | | [Hex]7[HexNAc]7 |
| | 1+ | 5609.9220 | 5609.3655 | 99.2 | LTPLCVTLNCTNVNVTNTTNNTEEK | | [Hex]7[HexNAc]8 |
| | 1+ | 5901.6890 | 5901.4813 | 35.2 | LTPLCVTLNCTNVNVTNTTNNTEEK | | [Hex]7[HexNAc]8[Fuc]2 |
| | 1+ | 5365.5875 | 5365.2596 | 61.1 | LTPLCVTLNCTNVNVTNTTNNTEEK | | [Hex]8[HexNAc]6 |
| | 1+ | 5771.8938 | 5771.4183 | 82.4 | LTPLCVTLNCTNVNVTNTTNNTEEK | | [Hex]8[HexNAc]8 |
| | 1+ | 6063.2075 | 6063.5341 | 53.9 | LTPLCVTLNCTNVNVTNTTNNTEEK | | [Hex]8[HexNAc]8 [Fuc]2 |
| | 1+ | 6266.7359 | 6266.6135 | 19.5 | LTPLCVTLNCTNVNVTNTTNNTEEK | | [Hex]8[HexNAc]9 [Fuc]2 |
| | 1+ | 5528.0909 | 5527.3124 | 140.8 | LTPLCVTLNCTNVNVTNTTNNTEEK | | [Hex]9[HexNAc]6 |
| | 1+ | 5730.6665 | 5730.3918 | 47.9 | LTPLCVTLNCTNVNVTNTTNNTEEK | | [Hex]9[HexNAc]7 |
| | 1+ | 5933.8848 | 5933.4711 | 69.7 | LTPLCVTLNCTNVNVTNTTNNTEEK | | [Hex]9[HexNAc]8 |
| | 1+ | 6225.4019 | 6225.5870 | 29.7 | LTPLCVTLNCTNVNVTNTTNNTEEK | | [Hex]9[HexNAc]8 [Fuc]2 |
| | 1+ | 6428.2896 | 6428.6663 | 58.6 | LTPLCVTLNCTNVNVTNTTNNTEEK | | [Hex]9[HexNAc]9 [Fuc]2 |
| | 1+ | 6485.2627 | 6485.6878 | 65.5 | LTPLCVTLNCTNVNVTNTTNNTEEK | | [Hex]9[HexNAc]10 [Fuc]1 |
| | 1+ | 5689.1245 | 5689.3652 | 42.3 | LTPLCVTLNCTNVNVTNTTNNTEEK | | [Hex]10[HexNAc]6 |
| | 1+ | 5835.5916 | 5935.4231 | 28.9 | LTPLCVTLNCTNVNVTNTTNNTEEK | | [Hex]10 [HexNAc]6[Fuc]1 |
| | 1+ | 5892.6890 | 5892.4445 | 41.5 | LTPLCVTLNCTNVNVTNTTNNTEEK | | [Hex]10[HexNAc]7 |
| | 1+ | 6096.1835 | 6095.5240 | 108.3 | LTPLCVTLNCTNVNVTNTTNNTEEK | | [Hex]10[HexNAc]8 |
| | 1+ | 6388.2949 | 6387.6398 | 102.6 | LTPLCVTLNCTNVNVTNTTNNTEEK | | [Hex]10 [HexNAc]8 [Fuc]2 |
| | 1+ | 6647.8921 | 6647.7406 | 22.8 | LTPLCVTLNCTNVNVTNTTNNTEEK | | [Hex]10 [HexNAc]1 [Fuc]1 |
| | 1+ | 5851.4702 | 5851.4180 | 8.9 | LTPLCVTLNCTNVNVTNTTNNTEEK | | [Hex]11[HexNAc]6 |
| | 1+ | 5997.6035 | 5997.4760 | 21.3 | LTPLCVTLNCTNVNVTNTTNNTEEK | | [Hex]11 [HexNAc]6[Fuc]1 |
| | 1+ | 6054.9711 | 6054.4974 | 78.2 | LTPLCVTLNCTNVNVTNTTNNTEEK | | [Hex]11[HexNAc]7 |
| | 1+ | 6258.2036 | 6257.5768 | 100.2 | LTPLCVTLNCTNVNVTNTTNNTEEK | | [Hex]11[HexNAc]8 |
| | 1+ | 6013.8884 | 6013.4019 | 69.4 | LTPLCVTLNCTNVNVTNTTNNTEEK | | [Hex]12[HexNAc]6 |
| | 1+ | 6159.9634 | 6159.5288 | 70.6 | LTPLCVTLNCTNVNVTNTTNNTEEK | | [Hex]12 [HexNAc]6 [Fuc]1 |
| | 1+ | 6217.2973 | 6216.5502 | 120.2 | LTPLCVTLNCTNVNVTNTTNNTEEK | | [Hex]12[HexNAc]7 |
| | 1+ | 6321.9770 | 6321.5816 | 62.5 | LTPLCVTLNCTNVNVTNTTNNTEEK | | [Hex]13 [HexNAc]6 [Fuc]1 |
| | 1+ | 6483.9414 | 6483.6344 | 47.3 | LTPLCVTLNCTNVNVTNTTNNTEEK | | [Hex]14 [HexNAc]6 [Fuc]1 |
| | | | | | | | |
| | 1+ | 7412.6018 | 7413.0309 | 57.9 | LTPLCVTLNCTNVNVTNTTNNTEEKGEIK | | [Hex]18 [HexNAc]6 |
| | 1+ | 7578.0944 | 7575.0837 | 397.4 | LTPLCVTLNCTNVNVTNTTNNTEEKGEIK | | [Hex]19 [HexNAc]6 |
| | 1+ | 7738.5202 | 7737.1365 | 178.8 | LTPLCVTLNCTNVNVTNTTNNTEEKGEIK | | [Hex]20 [HexNAc]6 |
| | 1+ | 7900.7293 | 7899.1893 | 195.0 | LTPLCVTLNCTNVNVTNTTNNTEEKGEIK | | [Hex]21 [HexNAc]6 |
| | 1+ | 8063.1905 | 8061.2422 | 241.7 | LTPLCVTLNCTNVNVTNTTNNTEEKGEIK | | [Hex]22 [HexNAc]6 |
| | 1+ | 8222.8220 | 8223.2950 | 57.5 | LTPLCVTLNCTNVNVTNTTNNTEEKGEIK | | [Hex]23 [HexNAc]6 |
| | 1+ | 8384.1082 | 8385.3478 | 147.8 | LTPLCVTLNCTNVNVTNTTNNTEEKGEIK | | [Hex]24 [HexNAc]6 |
| | 1+ | 8549.4511 | 8547.4006 | 239.9 | LTPLCVTLNCTNVNVTNTTNNTEEKGEIK | | [HEx]25 [HexNAc]6 |
| | 1+ | 8710.6020 | 8709.4534 | 131.9 | LTPLCVTLNCTNVNVTNTTNNTEEKGEIK | | [Hex]26[HexNAc]6 |
| V2-V1 | 1+ | 2247.1045 | 2246.9592 | 64.3 | NCSFNITTEIR | | [Hex]3[HexNAc]2 |
| | 1+ | 2393.0872 | 2393.0171 | 29.3 | NCSFNITTEIR | | [Hex]3[HexNAc]2[Fuc]1 |
| | 1+ | 2450.0596 | 2450.0386 | 8.6 | NCSFNITTEIR | | [Hex]3[HexNAc]3 |
| | 1+ | 2596.0635 | 2596.0965 | 12.7 | NCSFNITTEIR | | [Hex]3[HexNAc]3[Fuc]1 |
| | 1+ | 2653.1636 | 2653.1179 | 17.2 | NCSFNITTEIR | | [Hex]3[HexNAc]4 |
| | 1+ | 2799.2029 | 2799.1758 | 9.7 | NCSFNITTEIR | | [Hex]3[HexNAc]4[Fuc]1 |
| | 1+ | 2856.2510 | 2856.1974 | 18.8 | NCSFNITTEIR | | [Hex]3[HexNAc]5 |
| | 1+ | 3002.2961 | 3002.2552 | 13.6 | NCSFNITTEIR | | [Hex]3[HexNAc]5[Fuc]1 |
| | 1+ | 3059.3750 | 3059.2767 | 32.1 | NCSFNITTEIR | | [Hex]3[HexNAc]6 |
| | 1+ | 3205.3550 | 3205.3347 | 6.3 | NCSFNITTEIR | | [Hex]3[HexNAc]6[Fuc]1 |
| | 1+ | 2409.0415 | 2409.0120 | 12.2 | NCSFNITTEIR | | [Hex]4[HexNAc]2 |
| | 1+ | 2612.1174 | 2612.0914 | 10.0 | NCSFNITTEIR | | [Hex]4 [HexNAc]3 |
| | 1+ | 2758.1653 | 2758.1493 | 5.8 | NCSFNITTEIR | | [Hex]4[HexNAc]3[Fuc]1 |
| | 1+ | 2815.2466 | 2815.1705 | 27.0 | NCSFNITTEIR | | [Hex]4[HexNAc]4 |
| | 1+ | 2961.2813 | 2961.2287 | 17.8 | NCSFNITTEIR | | [Hex]4 [HexNAc]4 [Fuc]1 |
| | 1+ | 3164.3542 | 3164.3080 | 14.5 | NCSFNITTEIR | | [Hex]4[HexNAc]5[Fuc]1 |
| | 1+ | 2571.0828 | 2571.0648 | 7.0 | NCSFNITTEIR | | [Hex]5[HexNAc]2 |
| | 1+ | 2774.1431 | 2774.1442 | 0.4 | NCSFNITTEIR | | [Hex]5 [HexNAc]3 |
| | 1+ | 2920.1741 | 2920.2021 | 9.6 | NCSFNITTEIR | | [Hex]5[HexNAc]3[Fuc]1 |
| | 1+ | 3123.3137 | 3123.2815 | 10.3 | NCSFNITTEIR | | [Hex]5 [HexNAc]4 [Fuc]1 |
| | 1+ | 3326.4493 | 3326.3609 | 26.6 | NCSFNITTEIR | | [Hex]5[HexNAc]5[Fuc]1 |
| | 1+ | 2733.1489 | 2733.1177 | 11.4 | NCSFNITTEIR | | [Hex]6 [HexNAc]2 |
| | 1+ | 2936.2502 | 2936.1970 | 18.1 | NCSFNITTEIR | | [Hex]6 [HexNAc]3 |
| | 1+ | 3691.6216 | 3691.4931 | 34.8 | NCSFNITTEIR | | [Hex]6[HexNAc]6[Fuc]1 |
| | 1+ | 2895.2109 | 2895.1705 | 14.0 | NCSFNITTEIR | | [Hex]7[HexNAc]2 |
| | 1+ | 3057.2703 | 3057.2233 | 15.4 | NCSFNITTEIR | | [Hex]8[HexNAc]2 |
| | 1+ | 3219.3145 | 3219.2761 | 11.9 | NCSFNITTEIR | | [Hex]9[HexNAc]2 |
| | | | | | | | |
| V2 | 1+ + | 3231.4648 | 3231.3803 | 26.1 | LDVVPIDDNNNNSSNYR | | [Hex]2[HexNAc]4[Fuc]1 |
| | 1+ | 2841.2253 | 2841.2164 | 3.1 | LDVVPIDDNNNNSSNYR | | [Hex]3[HexNAc]2 |
| | 1+ | 3044.2886 | 3044.2958 | 2.4 | LDVVPIDDNNNNSSNYR | | [Hex]3[HexNAc]3 |
| | 1+ | 3190.3647 | 3190.3537 | 3.4 | LDVVPIDDNNNNSSNYR | | [Hex]3[HexNAc]3[Fuc]1 |
| | + | 3247.4536 | 3247.3751 | 24.2 | LDVVPIDDNNNNSSNYR | | [Hex]3[HexNAc]4 |
| | 1+ | 3393.4331 | 3393.4330 | 0.0 | LDVVPIDDNNNNSSNYR | | [Hex]3[HexNAc]4[Fuc]1 |
| | 1+ | 3450.4529 | 3450.4546 | 0.5 | LDVVPIDDNNNNSSNYR | | [Hex]3[HexNAc]5 |
| | 1+ | 3596.5557 | 3596.5125 | 12.0 | LDVVPIDDNNNNSSNYR | | [Hex]3[HexNAc]5[Fuc]1 |
| | 1+ | 3799.6394 | 3799.5919 | 12.5 | LDVVPIDDNNNNSSNYR | | [Hex]3[HexNAc]6[Fuc]1 |
| | 1+ | 4002.7573 | 4002.6715 | 21.7 | LDVVPIDDNNNNSSNYR | | [Hex]3[HexNAc]7[Fuc]1 |
| | 1+ | 3003.2751 | 3003.2693 | 1.9 | LDVVPIDDNNNNSSNYR | | [Hex]4[HexNAc]2 |
| | 1+ | 3206.2073 | 3206.3486 | 44.1 | LDVVPIDDNNNNSSNYR | | [Hex]4[HexNAc]3 |
| | 1+ | 3352.3937 | 3352.4065 | 3.8 | LDVVPIDDNNNNSSNYR | | [Hex]4[HexNAc]3[Fuc]1 |
| | 1+ | 3555.5718 | 3555.4859 | 24.2 | LDVVPIDDNNNNSSNYR | | [Hex]4[HexNAc]4[Fuc]1 |
| | 1+ | 3758.6563 | 3758.5652 | 17.7 | LDVVPIDDNNNNSSNYR | | [Hex]4[HexNAc]5[Fuc]1 |
| | 1+ | 4164.7207 | 4164.7243 | 0.9 | LDVVPIDDNNNNSSNYR | | [Hex]4[HexNAc]7[Fuc]1 |
| | 1+ | 3165.3523 | 3165.3220 | 9.6 | LDVVPIDDNNNNSSNYR | | [Hex]5[HexNAc]2 |
| | 1+ | 3368.4202 | 3368.4014 | 5.6 | LDVVPIDDNNNNSSNYR | | [Hex]5 [HexNAc]3 |
| | 1+ | 3514.4844 | 3514.4593 | 7.1 | LDVVPIDDNNNNSSNYR | | [Hex]5[HexNAc]3[Fuc]1 |
| | 1+ | 3717.5706 | 3717.5387 | 8.6 | LDVVPIDDNNNNSSNYR | | [Hex]5[HexNAc]4[Fuc]1 |
| | 1+ | 3774.6531 | 3774.5602 | 24.6 | LDVVPIDDNNNNSSNYR | | [Hex]5[HexNAc]5 |
| | 1+ | 3920.6768 | 3920.6181 | 15.0 | LDVVPIDDNNNNSSNYR | | [Hex]5[HexNAc]5[Fuc]1 |
| | 1+ | 4123.7106 | 4123.6980 | 3.1 | LDVVPIDDNNNNSSNYR | | [Hex]5[HexNAc]6[Fuc]1 |
| | 1+ | 4326.8469 | 4326.7771 | 16.1 | LDVVPIDDNNNNSSNYR | | [Hex]5[HexNAc]7[Fuc]1 |
| | 1+ | 3327.3921 | 3327.3749 | 5.2 | LDVVPIDDNNNNSSNYR | | [Hex]6 [HexNAc]2 |
| | 1+ | 4082.7095 | 4082.6709 | 9.5 | LDVVPIDDNNNNSSNYR | | [Hex]6[HexNAc]5[Fuc]1 |
| | 1+ | 4285.8389 | 4285.8031 | 8.4 | LDVVPIDDNNNNSSNYR | | [Hex]6[HexNAc]6[Fuc]1 |
| | 1+ | 4488.9243 | 4488.8299 | 21.0 | LDVVPIDDNNNNSSNYR | | [Hex]6[HexNAc]7[Fuc]1 |
| | 1+ | 3489.4756 | 3489.4277 | 13.7 | LDVVPIDDNNNNSSNYR | | [Hex]7[HexNAc]2 |
| | 1+ | 3692.4224 | 3692.5070 | 22.9 | LDVVPIDDNNNNSSNYR | | [Hex]7[HexNAc]3 |
| | 1+ | 4447.8613 | 4447.8031 | 13.1 | LDVVPIDDNNNNSSNYR | | [Hex]7 [HexNAc]6 [Fuc]1 |
| | 1+ | 4650.9059 | 4650.8825 | 5.0 | LDVVPIDDNNNNSSNYR | | [Hex]7[HexNAc]7[Fuc]1 |
| | 1+ | 3651.5273 | 3651.4805 | 12.8 | LDVVPIDDNNNNSSNYR | | [Hex]8[HexNAc]2 |
| | 1+ | 3813.6113 | 3813.5333 | 20.5 | LDVVPIDDNNNNSSNYR | | [Hex]9[HexNAc]2 |
| | | | | | | | |
| V2-C2 | 1+ | 2583.2419 | 2583.1423 | 38.6 | LINCNTSAITQACPK | | [Hex]3[HexNAc]2 |
| | 1+ | 2744.1780 | 2744.1308 | 17.2 | LINCNTSAITQACPK | | [Hex]4[HexNAc]2 |
| | 1+ | 2907.4192 | 2907.2479 | 58.9 | LINCNTSAITQACPK | | [Hex]5[HexNAc]2 |
| | | | | | | | |
| | 1+ | 1690.7651 | 1690.8251 | 35.5 | LINCNTSAITQACPK | | Non-glycosylated |
| | | | | | | | |
| C2 | 1+ | 2905.1387 | 2905.1437 | 1.7 | FNGTGPCK | for | [Hex]3[HexNAc]6[Fuc]2 |
| | | | | | | | |
| | 1+ | 2688.2720 | 2688.0910 | 67.3 | CNDKKFNGTGPCK | PyroC,u | [Hex]2[HexNAc]4 |
| | 1+ | 2485.1846 | 2485.0166 | 67.6 | CNDKKFNGTGPCK | PyroC,u | [Hex]2[HexNAc]3 |
| | 1+ | 2400.9614 | 2400.9792 | 7.4 | CNDKKFNGTGPCK | PyroC | [Hex]3 [HexNAc]2 |
| | 1+ | 2750.1011 | 2750.1165 | 5.6 | CNDKKFNGTGPCK | PyroC | [Hex]3 [HexNAc]3 [Fuc]1 |
| | 1+ | 2953.3030 | 2953.1958 | 24.9 | CNDKKFNGTGPCK | PyroC | [Hex]3[HexNAc]4[Fuc]1 |
| | 1+ | 3156.2390 | 3156.2752 | 11.5 | CNDKKFNGTGPCK | PyroC | [Hex]3[HexNAc]5[Fuc]1 |
| | 1+ | 2606.0569 | 2606.0379 | 7.3 | CNDKKFNGTGPCK | | [Hex]4[HexNAc]2 |
| | 1+ | 2912.0967 | 2912.1693 | 24.9 | CNDKKFNGTGPCK | PyroC | [Hex]4 [HexNAc]3 [Fuc]1 |
| | 1+ | 2725.0557 | 2725.0848 | 10.7 | CNDKKFNGTGPCK | PyroC | [Hex]5 [HexNAc]2 |
| | 1+ | 2754.0549 | 2754.1114 | 20.5 | CNDKKFNGTGPCK | for | [Hex]4[HexNAc]2[Fuc]1 |
| | 1+ | 3049.1704 | 3049.1905 | 6.6 | CNDKKFNGTGPCK | PyroC | [Hex]7[HexNAc]2 |
| | 1+ | 3211.2683 | 3211.2767 | 2.6 | CNDKKFNGTGPCK | PyroC | [Hex]8[HexNAc]2 |
| | 1+ | 3373.3042 | 3373.3295 | 7.5 | CNDKKFNGTGPCK | PyroC | [Hex]9[HexNAc]2 |
| | | | | | | | |
| | 1+ | 4511.4214 | 4511.2628 | 35.2 | NVSTVQCTHGIKPVVSTQLLLNGSLAEEEIIIR | | [Hex]3[HexNAc]2 |
| | 1+ | 5063.5541 | 5063.4864 | 13.4 | NVSTVQCTHGIKPVVSTQLLLNGSLAEEEIIIR | | [Hex]3[HexNAc]4[Fuc]1 |
| | 1+ | 5266.7388 | 5266.5658 | 32.8 | NVSTVQCTHGIKPVVSTQLLLNGSLAEEEIIIR | | [Hex]3[HexNAc]5[Fuc]1 |
| | 1+ | 4673.3067 | 4673.3226 | 3.4 | NVSTVQCTHGIKPVVSTQLLLNGSLAEEEIIIR | | [Hex]4[HexNAc]2 |
| | 1+ | 4835.4373 | 4835.3754 | 12.8 | NVSTVQCTHGIKPVVSTQLLLNGSLAEEEIIIR | | [Hex]5[HexNAc]2 |
| | 1+ | 4997.4501 | 4997.4280 | 4.4 | NVSTVQCTHGIKPVVSTQLLLNGSLAEEEIIIR | | [Hex]6[HexNAc]2 |
| | 1+ | 5159.6187 | 5159.4811 | 26.7 | NVSTVQCTHGIKPVVSTQLLLNGSLAEEEIIIR | | [Hex]7[HexNAc]2 |
| | 1+ | 5321.7019 | 5321.5339 | 31.6 | NVSTVQCTHGIKPVVSTQLLLNGSLAEEEIIIR | | [Hex]8[HexNAc]2 |
| | 1+ | 5483.6548 | 5483.5867 | 12.4 | NVSTVQCTHGIKPVVSTQLLLNGSLAEEEIIIR | | [Hex]9[HexNAc]2 |
| | 1+ | 5727.7847 | 5727.6923 | 48.6 | NVSTVQCTHGIKPVVSTQLLLNGSLAEEEIIIR | | [Hex]8[HexNAc]4 |
| | 1+ | 5890.1123 | 5889.7452 | 62.3 | NVSTVQCTHGIKPVVSTQLLLNGSLAEEEIIIR | | [Hex]9[HexNAc]4 |
| | 1+ | 6051.9082 | 6051.7980 | 18.2 | NVSTVQCTHGIKPVVSTQLLLNGSLAEEEIIIR | | [Hex]10 [HexNAc]4 |
| | 1+ | 6213.3965 | 6213.8508 | 73.1 | NVSTVQCTHGIKPVVSTQLLLNGSLAEEEIIIR | | [Hex]11 [HexNAc]4 |
| | 1+ | 6375.6035 | 6375.9036 | 47.1 | NVSTVQCTHGIKPVVSTQLLLNGSLAEEEIIIR | | [Hex]12 [HexNAc]4 |
| | 1+ | 6537.7222 | 6537.9564 | 35.8 | NVSTVQCTHGIKPVVSTQLLLNGSLAEEEIIIR | | [Hex]13 [HexNAc]4 |
| | 1+ | 6700.3182 | 6700.0093 | 46.1 | NVSTVQCTHGIKPVVSTQLLLNGSLAEEEIIIR | | [Hex]14 [HexNAc]4 |
| | 1+ | 6862.0877 | 6862.0621 | 3.7 | NVSTVQCTHGIKPVVSTQLLLNGSLAEEEIIIR | | [Hex]15 [HexNAc]4 |
| | 1+ | 7024.3167 | 7024.1149 | 28.7 | NVSTVQCTHGIKPVVSTQLLLNGSLAEEEIIIR | | [Hex]16 [HexNAc]4 |
| | 1+ | 7186.4517 | 7186.1677 | 39.5 | NVSTVQCTHGIKPVVSTQLLLNGSLAEEEIIIR | | [Hex]17 [HexNAc]4 |
| | 1+ | 7348.2871 | 7348.2205 | 9.1 | NVSTVQCTHGIKPVVSTQLLLNGSLAEEEIIIR | | [Hex]18 [HexNAc]4 |
| | | | | | | | |
| | 1+ | 2044.8149 | 2044.8550 | 19.7 | SENITNNAK | | [Hex]4[HexNAc]2 |
| | 1+ | 2206.8865 | 2206.9080 | 9.7 | SENITNNAK | | [Hex]5[HexNAc]2 |
| | | | | | | | |
| C2-V3 | 1+ | 3477.7258 | 3477.6272 | 28.4 | TIIVQLNESVEINCTRPNNNTR | | [Hex]3[HexNAc]2 |
| | 1+ | 4030.0671 | 4029.8438 | 55.4 | TIIVQLNESVEINCTRPNNNTR | | [Hex]3[HexNAc]4[Fuc]1 |
| | 1+ | 4233.1758 | 4232.9232 | 59.7 | TIIVQLNESVEINCTRPNNNTR | | [Hex]3[HexNAc]5[Fuc]1 |
| | 1+ | 3639.7512 | 3639.6800 | 19.6 | TIIVQLNESVEINCTRPNNNTR | | [Hex]4[HexNAc]2 |
| | 1+ | 3842.6460 | 3842.7594 | 29.5 | TIIVQLNESVEINCTRPNNNTR | | [Hex]4[HexNAc]3 |
| | 1+ | 3801.6996 | 3801.7328 | 8.7 | TIIVQLNESVEINCTRPNNNTR | | [Hex]5[HexNAc]2 |
| | 1+ | 4004.9220 | 4004.8122 | 27.4 | TIIVQLNESVEINCTRPNNNTR | | [Hex]5[HexNAc]3 |
| | 1+ | 4817.3409 | 4817.1297 | 43.8 | TIIVQLNESVEINCTRPNNNTR | | [Hex]5[HexNAc]7 |
| | 1+ | 3963.7484 | 3963.7857 | 9.4 | TIIVQLNESVEINCTRPNNNTR | | [Hex]6[HexNAc]2 |
| | 1+ | 4979.7837 | 4979.1825 | 120.7 | TIIVQLNESVEINCTRPNNNTR | | [Hex]6[HexNAc]7 |
| | 1+ | 4126.0322 | 4125.8385 | 46.9 | TIIVQLNESVEINCTRPNNNTR | | [Hex]7[HexNAc]2 |
| | 1+ | 5142.3660 | 5141.2353 | 219.9 | TIIVQLNESVEINCTRPNNNTR | | [Hex]7[HexNAc]7 |
| | 1+ | 4450.2113 | 4449.9441 | 60.0 | TIIVQLNESVEINCTRPNNNTR | | [Hex]9[HexNAc]2 |
| | 1+ | 4694.2067 | 4694.0500 | 33.4 | TIIVQLNESVEINCTRPNNNTR | | [Hex]8[HexNAc]4 |
| | 1+ | 5246.4630 | 5246.2667 | 37.4 | TIIVQLNESVEINCTRPNNNTR | | [Hex]8[HexNAc]6[Fuc]1 |
| | 1+ | 5303.8301 | 5303.2882 | 102.2 | TIIVQLNESVEINCTRPNNNTR | | [Hex]8[HexNAc]7 |
| | 1+ | 5505.4528 | 5506.3675 | 166.1 | TIIVQLNESVEINCTRPNNNTR | | [Hex]8[HexNAc]8 |
| | 1+ | 5449.9218 | 5449.3461 | 105.7 | TIIVQLNESVEINCTRPNNNTR | | [Hex]8[HexNAc]7[Fuc]1 |
| | 1+ | 4855.9375 | 4856.1029 | 34.0 | TIIVQLNESVEINCTRPNNNTR | | [Hex]9[HexNAc]4 |
| | 1+ | 5465.6126 | 5465.3410 | 49.7 | TIIVQLNESVEINCTRPNNNTR | | [Hex]9[HexNAc]7 |
| | 1+ | 5667.5271 | 5668.4203 | 157.6 | TIIVQLNESVEINCTRPNNNTR | | [Hex]9[HexNAc]8 |
| | 1+ | 5205.4983 | 5205.2401 | 49.6 | TIIVQLNESVEINCTRPNNNTR | | [Hex]9[HexNAc]5[Fuc]1 |
| | 1+ | 5408.5939 | 5408.3195 | 50.7 | TIIVQLNESVEINCTRPNNNTR | | [Hex]9[HexNAc]6[Fuc]1 |
| | 1+ | 5018.1253 | 5018.1557 | 6.1 | TIIVQLNESVEINCTRPNNNTR | | [Hex]10 [HexNAc]4 |
| | 1+ | 5830.5752 | 5830.4732 | 17.5 | TIIVQLNESVEINCTRPNNNTR | | [Hex]10 [HexNAc]8 |
| | 1+ | 5180.5497 | 5180.2085 | 65.9 | TIIVQLNESVEINCTRPNNNTR | | [Hex]11 [HexNAc]4 |
| | 1+ | 5991.4027 | 5992.5260 | 187.5 | TIIVQLNESVEINCTRPNNNTR | | [Hex]11 [HexNAc]8 |
| | 1+ | 5342.6948 | 5342.2613 | 81.1 | TIIVQLNESVEINCTRPNNNTR | | [HEX]12 [HexNAc]4 |
| | 1+ | 6153.6869 | 6154.5778 | 144.9 | TIIVQLNESVEINCTRPNNNTR | | [Hex]12 [HexNAc]8 |
| | 1+ | 5894.4149 | 5894.4780 | 10.7 | TIIVQLNESVEINCTRPNNNTR | | [Hex]12[HexNAc]6[Fuc]1 |
| | 1+ | 6097.6776 | 6097.5573 | 19.7 | TIIVQLNESVEINCTRPNNNTR | | [Hex]12[HexNAc]7[Fuc]1 |
| | 1+ | 5504.7299 | 5504.3141 | 75.5 | TIIVQLNESVEINCTRPNNNTR | | [Hex]13 [HexNAc]4 |
| | 1+ | 6315.8974 | 6316.6316 | 116.2 | TIIVQLNESVEINCTRPNNNTR | | [Hex]13 [HexNAc]8 |
| | 1+ | 5666.1128 | 5666.3670 | 44.9 | TIIVQLNESVEINCTRPNNNTR | | [Hex]14 [HexNAc]4 |
| | 1+ | 5990.7121 | 5990.4726 | 40.0 | TIIVQLNESVEINCTRPNNNTR | | [Hex]16 [HexNAc]4 |
| | 1+ | 6152.6319 | 6152.5253 | 17.3 | TIIVQLNESVEINCTRPNNNTR | | [Hex]17 [HexNAc]4 |
| | 1+ | 6314.7820 | 6314.5782 | 32.3 | TIIVQLNESVEINCTRPNNNTR | | [Hex]18 [HexNAc]4 |
| | 1+ | 4817.3409 | 4817.1297 | 43.8 | TIIVQLNESVEINCTRPNNNTR | | [Hex]5 [HexNAc]7 |
| | 1+ | 4979.7837 | 4979.1825 | 120.7 | TIIVQLNESVEINCTRPNNNTR | | [Hex]6 [HexNAc]7 |
| | 1+ | 5142.3660 | 5141.2353 | 219.9 | TIIVQLNESVEINCTRPNNNTR | | [Hex]7 [HexNAc]7 |
| | 1+ | 5303.8301 | 5303.2882 | 102.2 | TIIVQLNESVEINCTRPNNNTR | | [Hex]8 [HexNAc]7 |
| | 1+ | 5465.6126 | 5465.3410 | 49.7 | TIIVQLNESVEINCTRPNNNTR | | [Hex]9 [HexNAc]7 |
| | 1+ | 5341.8901 | 5344.3147 | 453.7 | TIIVQLNESVEINCTRPNNNTR | | [Hex]7 [HexNAc]8 |
| | 1+ | 5505.4528 | 5506.4203 | 166.1 | TIIVQLNESVEINCTRPNNNTR | | [Hex]8 [HexNAc]8 |
| | 1+ | 5667.5271 | 5668.4203 | 157.6 | TIIVQLNESVEINCTRPNNNTR | | [Hex]9 [HexNAc]8 |
| | 1+ | 5830.5752 | 5830.4732 | 17.5 | TIIVQLNESVEINCTRPNNNTR | | [Hex]10 [HexNAc]8 |
| | 1+ | 5991.4027 | 5992.5260 | 187.5 | TIIVQLNESVEINCTRPNNNTR | | [Hex]11 [HexNAc]8 |
| | 1+ | 6153.6869 | 6154.5788 | 144.9 | TIIVQLNESVEINCTRPNNNTR | | [Hex]12 [HexNAc]8 |
| | 1+ | 6315.8974 | 6316.6316 | 116.2 | TIIVQLNESVEINCTRPNNNTR | | [Hex]13 [HexNAc]8 |
| | | | | | | | |
| V3-C3 | 1+ | 2152.9331 | 2152.8698 | 35.6 | QAHCNISGTK | PyroC | [Hex]4[HexNAc]2 |
| | 1+ | 2315.0049 | 2314.9225 | 35.6 | QAHCNISGTK | PyroC | [Hex]5[HexNAc]2 |
| | 1+ | 2477.0840 | 2476.9754 | 43.8 | QAHCNISGTK | PyroC | [Hex]6 [HexNAc]2 |
| | 1+ | 2639.1426 | 2639.0282 | 43.3 | QAHCNISGTK | PyroC | [Hex]7[HexNAc]2 |
| | 1+ | 2801.1992 | 2801.0810 | 42.2 | QAHCNISGTK | PyroC | [Hex]8[HexNAc]2 |
| | 1+ | 2963.2224 | 2963.1338 | 31.8 | QAHCNISGTK | PyroC | [Hex]9[HexNAc]2 |
| | | | | | | | |
| C3 | 1+ | 1961.9016 | 1961.8710 | 15.6 | LREHFNNK | for | [Hex]2[HexNAc]2[Fuc]1 I |
| | 1+ | 2136.9360 | 2136.9560 | 9.4 | LREHFNNK | | [Hex]2[HexNAc]3[Fuc]1 |
| | 1+ | 1949.8629 | 1949.8710 | 4.2 | LREHFNNK | | [Hex]3[HexNAc]2 |
| | 1+ | 2095.9573 | 2095.9289 | 13.6 | LREHFNNK | | [Hex]3[HexNAc]2[Fuc]1 |
| | 1+ | 2152.9226 | 2152.9504 | 12.9 | LREHFNNK | | [Hex]3[HexNAc]3 |
| | 1+ | 2298.9907 | 2299.0083 | 7.7 | LREHFNNK | | [Hex]3 [HexNAc]3 [Fuc]1 |
| | 1+ | 2356.0176 | 2356.0297 | 5.1 | LREHFNNK | | [Hex]3[HexNAc]4 |
| | 1+ | 2502.0662 | 2502.0876 | 8.6 | LREHFNNK | | [Hex]3[HexNAc]4[Fuc]1 |
| | 1+ | 2559.1252 | 2559.1091 | 6.3 | LREHFNNK | | [Hex]3[HexNAc]5 |
| | 1+ | 2705.1394 | 2705.1670 | 10.2 | LREHFNNK | | [Hex]3[HexNAc]5[Fuc]1 I |
| | 1+ | 2908.2629 | 2908.2464 | 5.7 | LREHFNNK | | [Hex]3[HexNAc]6[Fuc]1 I |
| | 1+ | 2111.9068 | 2111.9238 | 8.0 | LREHFNNK | | [Hex]4[HexNAc]2 |
| | 1+ | 2258.0251 | 2257.9817 | 19.2 | LREHFNNK | | [Hex]4[HexNAc]2[Fuc]1 |
| | 1+ | 2314.9868 | 2315.0032 | 7.1 | LREHFNNK | | [Hex]4[HexNAc]3 |
| | 1+ | 2461.0405 | 2461.0611 | 8.4 | LREHFNNK | | [Hex]4 [HexNAc]3 [Fuc]1 |
| | 1+ | 2518.0513 | 2518.0826 | 12.4 | LREHFNNK | | [Hex]4[HexNAc]4 |
| | 1+ | 2664.1150 | 2664.1405 | 9.6 | LREHFNNK | | [Hex]4[HexNAc]4[Fuc]1 |
| | 1+ | 2721.1809 | 2721.1620 | 6.9 | LREHFNNK | | [Hex]4[HexNAc]5 |
| | 1+ | 2867.2256 | 2867.2198 | 2.0 | LREHFNNK | | [Hex]4[HexNAc]5[Fuc]1 |
| | 1+ | 3070.3928 | 3070.2997 | 30.3 | LREHFNNK | | [Hex]4[HexNAc]6[Fuc]1 |
| | 1+ | 2273.9646 | 2273.9766 | 5.3 | LREHFNNK | | [Hex]5[HexNAc]2 |
| | 1+ | 2477.0398 | 2477.0560 | 6.5 | LREHFNNK | | [Hex]5[HexNAc]3 |
| | 1+ | 2623.0879 | 2623.1139 | 9.9 | LREHFNNK | | [Hex]5[HexNAc]3[Fuc]1 |
| | 1+ | 2680.0977 | 2680.1354 | 14.1 | LREHFNNK | | [Hex]5[HexNAc]4 |
| | 1+ | 2826.2041 | 2826.1933 | 3.8 | LREHFNNK | | [Hex]5[HexNAc]4[Fuc]1 |
| | 1+ | 2883.0730 | 2883.2148 | 49.2 | LREHFNNK | | [Hex]5[HexNAc]5 |
| | 1+ | 3029.2881 | 3029.2727 | 5.1 | LREHFNNK | | [Hex]5[HexNAc]5[Fuc]1 |
| | 1+ | 2436.0095 | 2436.0295 | 8.2 | LREHFNNK | | [Hex]6[HexNAc]2 |
| | 1+ | 2639.1279 | 2639.1088 | 7.2 | LREHFNNK | | [Hex]6[HexNAc]3 |
| | 1+ | 2785.1487 | 2785.1667 | 6.5 | LREHFNNK | | [Hex]6[HexNAc]3[Fuc]1 |
| | 1+ | 3191.2815 | 3191.3255 | 13.8 | LREHFNNK | | [Hex]6[HexNAc]5[Fuc]1 |
| | 1+ | 2598.1082 | 2598.0823 | 10.0 | LREHFNNK | | [Hex]7[HexNAc]2 |
| | 1+ | 2801.0750 | 2801.1616 | 30.9 | LREHFNNK | | [Hex]7[HexNAc]3 |
| | 1+ | 3453.1436 | 3453.4056 | 75.9 | LREHFNNK | u | [Hex]7[HexNAc]6 |
| | 1+ | 2760.1155 | 2760.1351 | 7.1 | LREHFNNK | | [Hex]8[HexNAc]2 |
| | 1+ | 2963.2173 | 2963.215 | 0.8 | LREHFNNK | | [Hex]8[HexNAc]3 |
| | 1+ | 2922.1624 | 2922.1879 | 8.7 | LREHFNNK | | [Hex]9[HexNAc]2 |
| | | | | | | | |
| C3-V4 | 1+ | 6800.9766 | 6801.7104 | 107.9 | GEFFYCNTSGLFNSTWIGNGTK | | [Hex]9 [HexNAc]14 |
| | 1+ | 6152.0557 | 6151.4457 | 99.2 | GEFFYCNTSGLFNSTWIGNGTK | | [Hex]10 [HexNAc]10 |
| | 1+ | 6962.1792 | 6963.7632 | 227.5 | GEFFYCNTSGLFNSTWIGNGTK | | [Hex]10 [HexNAc]14 |
| | 1+ | 7166.5439 | 7166.8428 | 41.7 | GEFFYCNTSGLFNSTWIGNGTK | | [Hex]10 [HexNAc]15 |
| | 1+ | 6314.0684 | 6313.4985 | 90.3 | GEFFYCNTSGLFNSTWIGNGTK | | [Hex]11 [HexNAc]10 |
| | 1+ | 6866.1265 | 6865.7152 | 59.9 | GEFFYCNTSGLFNSTWIGNGTK | | [Hex]11 [HexNAc]12 [Fuc]1 |
| | 1+ | 7069.6514 | 7068.7945 | 121.5 | GEFFYCNTSGLFNSTWIGNGTK | | [Hex]11 [HexNAc]13 [Fuc]1 |
| | 1+ | 7125.0083 | 7125.8160 | 113.3 | GEFFYCNTSGLFNSTWIGNGTK | | [Hex]11[HexNAc]14 |
| | 1+ | 7327.9819 | 7328.8954 | 124.6 | GEFFYCNTSGLFNSTWIGNGTK | | [Hex]11 [HexNAc]15 |
| | 1+ | 5663.5830 | 5663.2339 | 61.6 | GEFFYCNTSGLFNSTWIGNGTK | | [Hex]12 [HexNAc]6 |
| | 1+ | 6475.9736 | 6475.5513 | 65.2 | GEFFYCNTSGLFNSTWIGNGTK | | [Hex]12 [HexNAc]10 |
| | 1+ | 6824.7241 | 6824.6886 | 5.2 | GEFFYCNTSGLFNSTWIGNGTK | | [Hex]12 [HexNAc]11 [Fuc]1 |
| | 1+ | 7028.3403 | 7027.7680 | 81.4 | GEFFYCNTSGLFNSTWIGNGTK | | [Hex]12 [HexNAc]12 [Fuc]1 |
| | 1+ | 7231.2227 | 7230.8474 | 51.9 | GEFFYCNTSGLFNSTWGNGTK | | [Hex]12 [HexNAc]13 [Fuc]1 |
| | 1+ | 7287.7441 | 7287.8668 | 16.8 | GEFFYCNTSGLFNSTWIGNGTK | | [Hex]12 [HexNAc]14 |
| | 1+ | 7491.4077 | 7490.9482 | 61.3 | GEFFYCNTSGLFNSTWIGNGTK | | [Hex]12 [HexNAc]15 |
| | 1+ | 5826.4419 | 5825.2867 | 198.3 | GEFFYCNTSGLFNSTWIGNGTK | | [Hex]13 [HexNAc]6 |
| | 1+ | 6985.7554 | 6986.7414 | 141.1 | GEFFYCNTSGLFNSTWIGNGTK | | [Hex]13 [HexNAc]1 [Fuc]1 |
| | 1+ | 7190.4492 | 7189.8208 | 87.4 | GEFFYCNTSGLFNSTWIGNGTK | | [Hex]13 [HexNAc]12 [Fuc]1 |
| | 1+ | 7392.1875 | 7392.9002 | 96.4 | GEFFYCNTSGLFNSTWIGNGTK | | [Hex]13 [HexNAc]13 [Fuc]1 |
| | 1+ | 7449.9614 | 7449.9216 | 5.3 | GEFFYCNTSGLFNSTWIGNGTK | | [Hex]13 [HexNAc]14 |
| | 1+ | 7652.7634 | 7653.0010 | 31.0 | GEFFYCNTSGLFNSTWIGNGTK | | [Hex]13 [HexNAc]15 |
| | 1+ | 5987.4145 | 5987.3395 | 12.5 | GEFFYCNTSGLFNSTWIGNGTK | | [Hex]14 [HexNAc]6 |
| | 1+ | 7149.2563 | 7148.7943 | 64.6 | GEFFYCNTSGLFNSTWIGNGTK | | [Hex]14 [HexNAc]11 [Fuc]1 |
| | 1+ | 7351.7793 | 7351.8736 | 12.8 | GEFFYCNTSGLFNSTWIGNGTK | | [Hex]14 [HexNAc]12 [Fuc]1 |
| | 1+ | 7555.3291 | 7554.9530 | 49.8 | GEFFYCNTSGLFNSTWIGNGTK | | [Hex]14 [HexNAc]13 [Fuc]1 |
| | 1+ | 7612.7563 | 7611.9745 | 102.7 | GEFFYCNTSGLFNSTWIGNGTK | | [Hex]14 [HexNAc]14 |
| | 1+ | 8424.8809 | 8424.2919 | 69.9 | GEFFYCNTSGLFNSTWIGNGTK | | [Hex]14 [HexNAc]16 |
| | 1+ | 6148.8357 | 6149.3923 | 90.5 | GEFFYCNTSGLFNSTWIGNGTK | | [Hex]15 [HexNAc]6 |
| | 1+ | 6961.6298 | 6961.7098 | 11.5 | GEFFYCNTSGLFNSTWIGNGTK | | [Hex]15 [HexNAc]10 |
| | 1+ | 7310.8101 | 7310.8471 | 5.1 | GEFFYCNTSGLFNSTWIGNGTK | | [Hex]15 [HexNAc]11 [Fuc]1 |
| | 1+ | 7513.4927 | 7513.9264 | 57.7 | GEFFYCNTSGLFNSTWIGNGTK | | [Hex]15 [HexNAc]12 [Fuc]1 |
| | 1+ | 7717.5254 | 7717.0058 | 67.3 | GEFFYCNTSGLFNSTWIGNGTK | | [Hex]15 [HexNAc]13 [Fuc]1 |
| | 1+ | 7773.5708 | 7774.0273 | 58.7 | GEFFYCNTSGLFNSTWIGNGTK | | [Hex]15 [HexNAc]14 |
| | 1+ | 8586.8281 | 8586.3448 | 56.3 | GEFFYCNTSGLFNSTWIGNGTK | | [Hex]15 [HexNAc]16 |
| | 1+ | 6311.8320 | 6311.4451 | 61.3 | GEFFYCNTSGLFNSTWIGNGTK | | [Hex]16 [HexNAc]6 |
| | 1+ | 7124.6578 | 7123.7626 | 125.7 | GEFFYCNTSGLFNSTWIGNGTK | | [Hex]16 [HexNAc]10 |
| | 1+ | 7472.9624 | 7472.8999 | 8.4 | GEFFYCNTSGLFNSTWIGNGTK | | [Hex]16 [HexNAc]11 [Fuc]1 |
| | 1+ | 7675.3120 | 7375.9793 | 86.9 | GEFFYCNTSGLFNSTWIGNGTK | | [Hex]16 [HexNAc]12 [Fuc]1 |
| | 1+ | 7880.0347 | 7879.0586 | 123.9 | GEFFYCNTSGLFNSTWIGNGTK | | [Hex]16 [HexNAc]13 [Fuc]1 |
| | 1+ | 7936.1904 | 7936.0801 | 13.9 | GEFFYCNTSGLFNSTWIGNGTK | | [Hex]16 [HexNAc]14 |
| | 1+ | 6473.2277 | 6473.4980 | 41.8 | GEFFYCNTSGLFNSTWIGNGTK | | [Hex]17 [HexNAc]6 |
| | 1+ | 7286.0383 | 7285.8154 | 30.6 | GEFFYCNTSGLFNSTWIGNGTK | | [Hex]17 [HexNAc]10 |
| | 1+ | 7635.0415 | 7634.9257 | 15.2 | GEFFYCNTSGLFNSTWIGNGTK | | [Hex]17 [HexNAc]11 [Fuc]1 |
| | 1+ | 7837.9741 | 7838.0321 | 7.4 | GEFFYCNTSGLFNSTWIGNGTK | | [Hex]17 [HexNAc]12 [Fuc]1 |
| | 1+ | 8097.7425 | 8098.1329 | 48.2 | GEFFYCNTSGLFNSTWIGNGTK | | [Hex]17 [HexNAc]14 |
| | 1+ | 6635.3876 | 6635.5508 | 24.6 | GEFFYCNTSGLFNSTWIGNGTK | | [Hex]18 [HexNAc]6 |
| | 1+ | 7448.1455 | 7447.8683 | 37.2 | GEFFYCNTSGLFNSTWIGNGTK | | [Hex]18 [HexNAc]10 |
| | 1+ | 7796.8379 | 7797.0055 | 21.5 | GEFFYCNTSGLFNSTWIGNGTK | | [Hex]18 [HexNAc]11 [Fuc]1 |
| | 1+ | 8260.7666 | 8260.70.3 | 70.3 | GEFFYCNTSGLFNSTWIGNGTK | | [Hex]18 [HexNAc]14 |
| | 1+ | 6797.1307 | 6767.6036 | 69.6 | GEFFYCNTSGLFNSTWIGNGTK | | [Hex]19 [HexNAc]6 |
| | | | | | | | |
| V4 | 1+ | 2929.3943 | 2929.2361 | 54.0 | NNNNTNDTITLPCR | | [Hex]2[HexNAc]4[Fuc]1 |
| | 1+ | 2539.0361 | 2539.0722 | 14.2 | NNNNTNDTITLPCR | | [Hex]3[HexNAc]2 |
| | 1+ | 2888.4421 | 2888.2185 | 77.4 | NNNNTNDTITLPCR | | [Hex]3[HexNAc]3[Fuc]1 |
| | 1+ | 3091.2617 | 3091.2888 | 8.8 | NNNNTNDTITLPCR | | [Hex]3[HexNAc]4[Fuc]1 |
| | 1+ | 3294.3725 | 3294.3683 | 1.3 | NNNNTNDTITLPCR | | [Hex]3[HexNAc]5[Fuc]1 |
| | 1+ | 2701.1055 | 2701.1251 | 7.3 | NNNNTNDTITLPCR | | [Hex]4[HexNAc]2 |
| | 1+ | 2904.2659 | 2904.2044 | 21.2 | NNNNTNDTITLPCR | | [Hex]4[HexNAc]3 |
| | 1+ | 3050.3079 | 3050.2623 | 14.9 | NNNNTNDTITLPCR | | [Hex]4[HexNAc]3[Fuc]1 |
| | 1+ | 3135.5369 | 3135.2787 | 82.4 | NNNNTNDTITLPCR | for | [Hex]4[HexNAc]4 |
| | 1+ | 3253.4631 | 3253.3417 | 37.3 | NNNNTNDTITLPCR | | [Hex]4[HexNAc]4[Fuc]1 |
| | 1+ | 3338.6038 | 3338.3581 | 73.6 | NNNNTNDTITLPCR | | [Hex]4[HexNAc]5 |
| | 1+ | 3456.3638 | 3456.4210 | 16.5 | NNNNTNDTITLPCR | for | [Hex]4[HexNAc]5[Fuc]1 |
| | 1+ | 2863.1807 | 2863.1778 | 1.0 | NNNNTNDTITLPCR | | [Hex]5[HexNAc]2 |
| | 1+ | 3066.3567 | 3066.2572 | 32.4 | NNNNTNDTITLPCR | | [Hex]5[HexNAc]3 |
| | 1+ | 3212.4602 | 3212.3151 | 45.2 | NNNNTNDTITLPCR | | [Hex]5[HexNAc]3[Fuc]1 |
| | 1+ | 3312.3333 | 3312.3424 | 2.7 | NNNNTNDTITLPCR | U | Hex]5[HexNAc]4 |
| | 1+ | 3025.3657 | 3025.2307 | 44.6 | NNNNTNDTITLPCR | | [Hex]6[HexNAc]2 |
| | 1+ | 3954.5527 | 3954.5795 | 6.8 | NNNNTNDTITLPCR | for | [Hex]6[HexNAc]5[Fuc]2 |
| | 1+ | 3187.3948 | 3187.2835 | 34.9 | NNNNTNDTITLPCR | | [Hex]7[HexNAc]2 |
| | 1+ | 3349.4900 | 3349.3363 | 45.9 | NNNNTNDTITLPCR | | [Hex]2[HexNAc]2 |
| | 1+ | 3511.5010 | 3511.3891 | 31.9 | NNNNTNDTITLPCR | | [Hex]9[HexNAc]2 |
| | 1+ | 4890.0397 | 4889.8654 | 35.6 | NNNNTNDTITLPCR | | [Hex]15 [HexNAc]4 |
| | 1+ | 5051.9805 | 5051.9182 | 12.3 | NNNNTNDTITLPCR | | [Hex]16[HexNAc]4 |
| | 1+ | 5214.0788 | 5213.9710 | 20.7 | NNNNTNDTITLPCR | | [Hex]17 [HexNAc]4 |
| | 1+ | 5376.3584 | 5376.0239 | 62.2 | NNNNTNDTITLPCR | | [Hex]18 [HexNAc]4 |
| | | | | | | | |
| C4 | 1+ | 2532.4939 | 2532.1810 | 123.6 | SNITGLLLTR | | [Hex]3[HexNAc]4[Fuc]1 |
| | 1+ | 2631.7939 | 2632.2084 | 157.2 | SNITGLLLTR | u | [Hex]3[HexNAc]5 |
| | 1+ | 2735.2043 | 2735.2605 | 20.5 | SNITGLLLTR | | [Hex]3[HexNAc]5[Fuc]1 |
| | 1+ | 2938.6240 | 2938.3399 | 96.7 | SNITGLLLTR | | [Hex]3[HexNAc]6[Fuc]1 |
| | 1+ | 2142.0044 | 2142.0173 | 6.0 | SNITGLLLTR | | [Hex]4[HexNAc]2 |
| | 1+ | 2751.4282 | 2751.2554 | 62.8 | SNITGLLLTR | | [Hex]4[HexNAc]5 |
| | 1+ | 2304.0483 | 2304.0272 | 9.2 | SNITGLLLTR | | [Hex]5[HexNAc]2 |
| | 1+ | 2466.0999 | 2466.1229 | 9.3 | SNITGLLLTR | | [Hex]6[HexNAc]2 |
| | 1+ | 2915.7188 | 2915.2874 | 148.0 | SNITGLLLTR | u | [Hex]6[HexNAc]4 |
| | 1+ | 2628.1599 | 2628.1757 | 6.0 | SNITGLLLTR | | [Hex]7[HexNAc]2 |
| | 1+ | 2790.2070 | 2790.2285 | 7.7 | SNITGLLLTR | | [Hex]8[HexNAc]2 |
| | 1+ | 2952.2661 | 2952.2813 | 5.1 | SNITGLLLTR | | [Hex]9[HexNAc]2 |
| | | | | | | | |
| V5 | 1+ | 3143.2939 | 3143.2965 | 0.8 | DGGNNNTNETEIFRPGGGDMR | | [Hex]3[HexNAc]2 |
| | 1+ | 3289.3408 | 3289.3544 | 4.1 | DGGNNNTNETEIFRPGGGDMR | | [Hex]3[HexNAc]2[Fuc]1 |
| | 1+ | 3492.4683 | 3492.4683 | 0.0 | DGGNNNTNETEIFRPGGGDMR | | [Hex]3[HexNAc]3[Fuc]1 I |
| | 1+ | 3695.5459 | 3695.5132 | 8.8 | DGGNNNTNETEIFRPGGGDMR | | [Hex]3[HexNAc]4[Fuc]1 |
| | 1+ | 3898.6094 | 3898.5926 | 4.3 | DGGNNNTNETEIFRPGGGDMR | | [Hex]3[HexNAc]5Fuc]1 |
| | 1+ | 4101.7837 | 4101.6723 | 27.2 | DGGNNNTNETEIFRPGGGDMR | | [Hex]3[HexNAc]6Fuc]1 |
| | 1+ | 4304.3888 | 4304.7512 | 84.2 | DGGNNNTNETEIFRPGGGDMR | | [Hex]3[HexNAc]7Fuc]1 |
| | 1+ | 3305.3665 | 3305.3493 | 5.2 | DGGNNNTNETEIFRPGGGDMR | | [Hex]4[HexNAc]2 |
| | 1+ | 3654.5195 | 3654.4866 | 9.0 | DGGNNNTNETEIFRPGGGDMR | | [Hex]4[HexNAc]3[Fuc]1 |
| | 1+ | 3711.5139 | 3711.5081 | 1.6 | DGGNNNTNETEIFRPGGGDMR | | [Hex]4[HexNAc]4 |
| | 1+ | 3857.5933 | 3857.5660 | 7.1 | DGGNNNTNETEIFRPGGGDMR | | [Hex]4[HexNAc]4[Fuc]1 I |
| | 1+ | 3914.6484 | 3914.5875 | 15.6 | DGGNNNTNETEIFRPGGGDMR | | [Hex]4[HexNAc]5 |
| | 1+ | 4060.7559 | 4060.6453 | 27.2 | DGGNNNTNETEIFRPGGGDMR | | [Hex]4[HexNAc]5[Fuc]1 |
| | 1+ | 4263.7798 | 4263.7250 | 12.9 | DGGNNNTNETEIFRPGGGDMR | | [Hex]4[HexNAc]6[Fuc]1 |
| | 1+ | 3467.4426 | 3467.4021 | 11.7 | DGGNNNTNETEIFRPGGGDMR | | [Hex]5[HexNAc]2 |
| | 1+ | 3816.5886 | 3816.5394 | 12.9 | DGGNNNTNETEIFRPGGGDMR | | [Hex]5[HexNAc]3[Fuc]1 |
| | 1+ | 4019.6672 | 4019.6188 | 12.0 | DGGNNNTNETEIFRPGGGDMR | | [Hex]5[HexNAc]4[Fuc]1 |
| | 1+ | 4222.7593 | 4222.6982 | 14.5 | DGGNNNTNETEIFRPGGGDMR | | [Hex]5[HexNAc]5[Fuc]1 |
| | 1+ | 4425.8540 | 4425.7780 | 17.2 | DGGNNNTNETEIFRPGGGDMR | | [Hex]5[HexNAc]6[Fuc]1 |
| | 1+ | 3629.5090 | 3629.4550 | 14.9 | DGGNNNTNETEIFRPGGGDMR | | [Hex]6 [HexNAc]2 |
| | 1+ | 4384.9868 | 4384.7510 | 53.8 | DGGNNNTNETEIFRPGGGDMR | | [Hex]6[HexNAc]5[Fuc]1 |
| | 1+ | 4937.0005 | 4936.9675 | 6.7 | DGGNNNTNETEIFRPGGGDMR | | [Hex]6[HexNAc]7[Fuc]2 |
| | 1+ | 5140.1138 | 5140.0469 | 13.0 | DGGNNNTNETEIFRPGGGDMR | | [Hex]6[HexNAc]8[Fuc]2 |
| | 1+ | 5343.1846 | 5343.1263 | 10.9 | DGGNNNTNETEIFRPGGGDMR | | [Hex]6[HexNAc]9[Fuc]2 |
| | 1+ | 5546.2119 | 5546.2056 | 1.1 | DGGNNNTNETEIFRPGGGDMR | | [Hex]6[HexNAc]10[Fuc]2 |
| | 1+ | 5749.2845 | 5749.2850 | 0.1 | DGGNNNTNETEIFRPGGGDMR | | [Hex]6[HexNAc]11[Fuc]2 |
| | 1+ | 5952.7117 | 5952.3644 | 58.3 | DGGNNNTNETEIFRPGGGDMR | | [Hex]6[HexNAc]12[Fuc]2 |
| | 1+ | 3791.5891 | 3791.5078 | 21.4 | DGGNNNTNETEIFRPGGGDMR | | [Hex]7[HexNAc]2 |
| | 1+ | 5099.1850 | 5099.0204 | 32.3 | DGGNNNTNETEIFRPGGGDMR | | [Hex]7[HexNAc]7[Fuc]2 |
| | 1+ | 5505.0344 | 5505.1791 | 26.3 | DGGNNNTNETEIFRPGGGDMR | | [Hex]7[HexNAc]9[Fuc]2 |
| | 1+ | 5708.2237 | 5708.2585 | 6.1 | DGGNNNTNETEIFRPGGGDMR | | [Hex]7[HexNAc]10[Fuc]2 |
| | 1+ | 5911.4698 | 5911.3378 | 22.3 | DGGNNNTNETEIFRPGGGDMR | | [Hex]7[HexNAc]11 [Fuc]2 |
| | 1+ | 3953.6326 | 3953.5606 | 18.2 | DGGNNNTNETEIFRPGGGDMR | | [Hex]8[HexNAc]2 |
| | 1+ | 4359.9347 | 4359.7192 | 49.4 | DGGNNNTNETEIFRPGGGDMR | | [Hex]8[HexNAc]4 |
| | 1+ | 5058.1890 | 5057.9938 | 38.6 | DGGNNNTNETEIFRPGGGDMR | | [Hex]8[HexNAc]6[Fuc]2 |
| | 1+ | 5261.1964 | 5261.0732 | 23.4 | DGGNNNTNETEIFRPGGGDMR | | [Hex]8[HexNAc]7[Fuc]2 |
| | 1+ | 5464.1788 | 5464.1525 | 4.8 | DGGNNNTNETEIFRPGGGDMR | | [Hex]8[HexNAc]8[Fuc]2 |
| | 1+ | 4115.6401 | 4115.6134 | 6.5 | DGGNNNTNETEIFRPGGGDMR | | [Hex]9[HexNAc]2 |
| | 1+ | 4521.9683 | 4521.7721 | 43.4 | DGGNNNTNETEIFRPGGGDMR | | [Hex]9[HexNAc]4 |
| | 1+ | 4684.1528 | 4683.8429 | 70.0 | DGGNNNTNETEIFRPGGGDMR | | [Hex]10[HexNAc]4 |
| | 1+ | 4846.1432 | 4845.8777 | 54.8 | DGGNNNTNETEIFRPGGGDMR | | [Hex]11[HexNAc]4 |
| | 1+ | 5007.8786 | 5007.9305 | 10.4 | DGGNNNTNETEIFRPGGGDMR | | [Hex]12[HexNAc]4 |
| | 1+ | 5169.1245 | 5169.9833 | 166.1 | DGGNNNTNETEIFRPGGGDMR | | [Hex]13[HexNAc]4 |
| | 1+ | 5373.1152 | 5373.0627 | 9.8 | DGGNNNTNETEIFRPGGGDMR | | [Hex]13[HexNAc]5 |
| | 1+ | 5575.1899 | 5576.1421 | 170.8 | DGGNNNTNETEIFRPGGGDMR | | [Hex]13[HexNAc]6 |
| | | | | | | | |
| TM | 1+ | 2310.4268 | 2309.9954 | 186.9 | DQQLEIWDNMTWMEWER | | Non-glycosylated |

**Table 8. MALDI MS Glycopeptide Composition for JR-FL gp 140 ΔCF**

| **Env Domain** | **Charge State** | **Experimental Mass** | **Theoretical Mass** | **Mass Error** | **Peptide** | **Mod^{*}** | **Carbohydrate Competition** |
|---|---|---|---|---|---|---|---|
| C1-V1 | 1+ | 5840.2241 | 5840.4332 | 35.8 | LTPLCVTLNCKDVNATNTTNDSEGTMER | | [Hex]6 [HexNAc]7 [Fuc]2 |
| | 1+ | 6043.8494 | 6043.5126 | 55.7 | LTPLCVTLNCKDVNATNTTNDSEGTMER | | [Hex]6 [HexNAc]8 [Fuc]2 |
| | 1+ | 6247.0411 | 6246.5920 | 71.9 | LTPLCVTLNCKDVNATNTTNDSEGTMER | | [Hex]6 [HexNAc]9 [Fuc]2 |
| | 1+ | 6303.2070 | 6303.6154 | 64.8 | LTPLCVTLNCKDVNATNTTNDSEGTMER | | [Hex]6 [HexNAc]10 [Fuc]1 |
| | 1+ | 6449.8385 | 6449.6713 | 28.2 | LTPLCVTLNCKDVNATNTTNDSEGTMER | | [Hex]6 [HexNAc]10 [Fuc]2 |
| | 1+ | 6653.4891 | 6652.7507 | 111.0 | LTPLCVTLNCKDVNATNTTNDSEGTMER | | [Hex]6 [HexNAc]11 [Fuc]2 |
| | 1+ | 6856.5493 | 6855.8301 | 104.9 | LTPLCVTLNCKDVNATNTTNDSEGTMER | | [Hex]6 [HexNAc]12 [Fuc]2 |
| | 1+ | 6059.3135 | 6059.5075 | 32.0 | LTPLCVTLNCKDVNATNTTNDSEGTMER | | [Hex]7[HexNAc]8[Fuc]1 |
| | 1+ | 6206.1958 | 6205.5654 | 101.6 | LTPLCVTLNCKDVNATNTTNDSEGTMER | | [Hex]7 [HexNAc]8 [Fuc]2 |
| | 1+ | 6263.0366 | 6262.5869 | 71.8 | LTPLCVTLNCKDVNATNTTNDSEGTMER | | [Hex]7 [HexNAc]9 [Fuc]1 |
| | 1+ | 6409.1567 | 6408.6448 | 79.9 | LTPLCVTLNCKDVNATNTTNDSEGTMER | | [Hex]7 [HexNAc]9 [Fuc]2 |
| | 1+ | 6465.4558 | 6465.6662 | 32.5 | LTPLCVTLNCKDVNATNTTNDSEGTMER | | [Hex]7 [HexNAc]10 [Fuc]1 |
| | 1+ | 6612.4608 | 6611.7242 | 111.4 | LTPLCVLNCKDVNATNTTNDSEGTMER | | [Hex]7 (HexNAc]10 [Fuc]2 |
| | 1+ | 6669.9580 | 6668.7456 | 181.8 | LTPLCVTLNCKDVNATNTTNDSEGTMER | | [Hex]7 [HexNAc]11 [Fuc]1 |
| | 1+ | 6815.7756 | 6814.8035 | 142.6 | LTPLCVTLNCKDVNATNTTNDSEGTMER | | [Hex]7 [HexNAc]11 [Fuc]2 |
| | 1+ | 7018.3477 | 7017.8829 | 66.2 | LTPLCVTLNCKDVNATNTTNDSEGTMER | | [Hex]7 [HexNAc]12 [Fuc]2 |
| | 1+ | 5467.2055 | 5466.2643 | 172.2 | LTPLCVTLNCKDVNATNTTNDSEGTMER | | [Hex]8 [HexNAc]5 |
| | 1+ | 6018.2075 | 6018.4810 | 45.4 | LTPLCVTLNCKDVNATNTTNDSEGTMER | | [Hex]8 [HexNAc]7 [Fuc]1 |
| | 1+ | 6221.8198 | 6221.5603 | 41.7 | LTPLCVTLNCKDVNATN1TNDSEGTMER | | [Hex]8 [HexNAc]8 [Fuc]1 |
| | 1+ | 6368.3890 | 6367.6182 | 121.0 | LTPLCVTLNCKDVNATNTTNDSEGTMER | | [Hex]8 [HexNAc]8 [Fuc]2 |
| | 1+ | 6774.33.87 | 6773.7770 | 82.9 | LTPLCVTLNCKDVNATNTTNDSEGTMER | | [Hex]8 [HexNAc)12 [Fuc]2 |
| | 1+ | 6992.9269 | 6991.8513 | 10.8 | LTPLCVTLNCKDVNATNTTNDSEGTMER | | [Hex]9 [HexNAc]11 [Fuc]1 |
| | 1+ | 6951.7734 | 6951.8147 | 5.9 | LTPLCVTLNCKDVNATNTTNDSEGTMER | | [Hex]10 [HexNAc]10 [Fuc]1 |
| | 1+ | 7114.2993 | 7113.8775 | 59.3 | LTPLCVTLNCKDVNATNTTNDSEGTMER | | [Hex]11 [HexNAc]10 [Fue]1 |
| | 1+ | 7316.6846 | 7316.9590 | 37.5 | LTPLCVTLNCKDVNATNTTNDSEGTMER | | [Hex]11 [HexNAc]11 [Fue)1 |
| | 1+ | 7275.6885 | 7275.9304 | 33.2 | LTPLCVTLMCKDVNATNTTNDSEGTMER | | [Hex]12 [HexNAc]10 [Fuc]1 |
| | 1+ | 7422.9560 | 7421.9883 | 130.4 | LTPLCVTLNCKDVNATNTTNDSEGTMER | | [Hex]12 [HexNAc]10 [Fuc]2 |
| | 1+. | 7478.9316 | 7479.0097 | 10.4 | LTPLCVTLNCKDVNATNTTNDSEGTMER | | [Hex]12 [HexNAC]11 [Fue]1 |
| | 1+ | 7438.1411 | 7437.9832 | 21.2 | LTPLCVTLNCKDVNATNTTNDSEGTMER | | [Hex]13 [HexNAc]10 [Fuc]1 |
| | 1+ | 7641.0825 | 7641.0625 | 2.6 | LTPLCVTLNCKDVNATNTTNDSEGTMER | | [Hex]13 [HexNAc] [Fue]1 |
| | 1+ | 7599.9932 | 7600.0360 | 5.6 | LTPLCVTLNCKDVNATNTTNDSEGTMER | | [Hex]14[HexNAc]10[Fuc]1 |
| | 1+ | 7802.7280 | 7803.1154 | 49.6 | LTPLCVTLNCKDVNATNTTNDSEGTMER | | [Hex]14 [HexNAc]11 [Fuc]1 |
| | 1+ | 7761.9741 | 7762.0888 | 14.8 | LTPLCVTLNCKDVNATNTTNDSEGTMER | | [Hex]15[HexNAc]10[Fuc]1 |
| | 1+ | 7964.5063 | 7965.1682 | 83.1 | LTPLCVTLNCKDVNATNTTNDSEGTMER | | [Hex)15 [HexNAc]11 [Fuc]1 |
| | 1+ | 6559.6694 | 6559.6075 | 9.4 | LTPLCVTLNCKDVNATNTTNDSEGTMER | | [Hex]16[HexNAc]4 |
| | 1+ | 7923.7856 | 7924.1416 | 44.9 | LTPLCVTLNCKDVNATNTTNDSEGTMER | | [Hex]16[HexNAc]10 [Fuc]1 |
| | 1+ | 8127.2319 | 8127.2210 | 1.3 | LTPLCVTLNCKDVNATNTTNDSEGTMER | | [Hex]16 [HexNAc]1 [Fuc]1 |
| | 1+ | 6721.5526 | 6721.6603 | 15.5 | LTPLCVTLNCKDVNATNTTNDSEGTMER | | [Hex]17[HexNAc]4 |
| | 1+ | 8086.2433 | 8086.1945 | 6.0 | LTPLCVTLNCKDVNATNTTNDSEGTMER | | [Hex]17[HexNAc]10 [Fuc]1 |
| | 1+ | 6883.6978 | 6883.7131 | 2.2 | LTPLCVTLNCKDVNATNTTNDSEGTMER | | [Hex]18[HexNAc]4 |
| | | | | | | | |
| V1-V2 | 1+ | 3153.3216 | 3153.3778 | 17.8 | NCSFNITTSIRDEVQK | | [Hex]3[HexNAc]3[Fuc]1 |
| | 1+ | 3356.4980 | 3356.4598 | 11.4 | NCSFNITTSIRDEVQK | | [Hex]3[HexNAc]4[Fuc]1 |
| | 1+ | 3559.5686 | 3559.5362 | 9.1 | NCSFNITTSIRDEVQK | | [Hex]3[HexNAc]5[Fuc]1 |
| | 1+ | 3315.4333 | 3315.4302 | 0.9 | NCSFNITTSIRDEVQK | | [Hex]4[HexNAc]3[Fuc]1 |
| | 1+ | 3518.5540 | 3518.5096 | 12.6 | NCSFNITTSIRDEVQK | | [Hex]4[HexNAc]4[Fuc]1 |
| | 1+ | 3721.7263 | 3721.5889 | 36.9 | NCSFNITTSIRDEVQK | | [Hex]4[HexNAc]5[Fuc]1 |
| | 1+ | 3762.7498 | 3762.5414 | 55.4 | NCSFNITTSIRDEVQK | | [Hex]4[HexNAc]6[Fuc]1 |
| | 1+ | 3128.3633 | 3128.3457 | 5.6 | NCSFNITTSIRDEVQK | | [Hex]5[HexNAc]2 |
| | 1+ | 3477.5159 | 3477.4831 | 9.4 | NCSFNITTSIRDEVQK | | [Hex]5[HexNAc]3[Fuc]1 |
| | 1+ | 3680.6418 | 3680.5624 | 21.6 | NCSFNITTSIRDEVQK | | [Hex]5[HexNAc]4[Fuc]1 |
| | 1+ | 3883.5893 | 3883.6418 | 13.5 | NCSFNITTSIRDEVQK | | [Hex]5[HexNAc]5[Fuc]1 |
| | 1+ | 3290.4221 | 3290.3986 | 7.1 | NCSFNITTSIRDEVQK | | [Hex]6 [HexNAc]2 |
| | 1+ | 4045.9458 | 4045.6946 | 62.1 | NCSFNITTSIRDEVQK | | [Hex]6[HexNAc]5[Fuc]1 |
| | 1+ | 5228.5767 | 5228.2337 | 65.8 | NCSFNITTSIRDEVQK | | [Hex]6[HexNAc]8 [Fuc]2 |
| | 1+ | 5431.4263 | 5431.3131 | 20.8 | NCSFNITTSIRDEVQK | | [Hex]6[HexNAc]9 [Fuc]2 |
| | 1+ | 5634.1426 | 5634.3924 | 44.3 | NCSFNITTSIRDEVQK | | [Hex]6[HexNAc]10 [Fuc]2 |
| | 1+ | 5837.2295 | 5837.4718 | 41.5 | NCSFNITTSIDEVQK | | [Hex]6[HexNAc]11 [Fuc]2 |
| | 1+ | 3452.4651 | 3452.4514 | 4.0 | NCSFNITTSIRDEVQK | | [Hex]7[HexNAc]2 |
| | 1+ | 4578.5184 | 4577.9690 | 120.0 | NCSFNITTSIRDEVQK | | [Hex]7[HexNAc]4 [Fuc]2 |
| | 1+ | 4983.6496 | 4984.1278 | 95.9 | NCSFNITTSIRDEVQK | | [Hex]7[HexNAc]6 [Fuc]2 |
| | 1+ | 5796.1812 | 5796.4453 | 45.6 | NCSFNITTSIRDEVQK | | [Hex]7[HexNAc]10 [Fuc]2 |
| | 1+ | 5998.9331 | 5999.5246 | 98.6 | NCSFNITTSIRDEVQK | | [Hex]7[HexNAc]11 [Fuc]2 |
| | 1+ | 6040.9596 | 6040.5512 | 67.6 | NCSFNITTSIRDEVQK | | [Hex]7[HexNAc]12 [Fuc]2 |
| | 1+ | 3614.5623 | 3614.5042 | 16.1 | NCSFNITTSIRDEVQK | | [Hex]8[HexNAc]2 |
| | 1+ | 4740.0826 | 4740.0219 | 12.8 | NCSFNITTSIRDEVQK | | [Hex]8[HexNAc]4 [Fuc]2 |
| | 1+ | 5146.7193 | 5146.1806 | 104.7 | NCSFNITTSIRDEVQK | | [Hex]8[HexNAc]6 [Fuc]2 |
| | 1+ | 5348.8698 | 5349.2600 | 72.9 | NCSFNITTSIRDEVQK | | [Hex]8[HexNAc]7 [Fuc]2 |
| | 1+ | 5958.8901 | 5958.4981 | 65.8 | NCSFNITTSIRDEVQK | | [Hex]8[HexNAc]10 [Fuc]2 |
| | 1+ | 3776.6552 | 3776.5570 | 26.0 | NCSFNITTSIRDEVQK | | [Hex]9[HexNAc]2 |
| | 1+ | 6120.6161 | 6120.5509 | 10.7 | NCSFNITTSIRDEVQK | | [Hex]9[HexNAc]10 [Fuc]2 |
| | 1+ | 6283.1626 | 6282.6037 | 89.0 | NCSFNITTSIRDEVQK | | [Hex]10 [HexNAc]10 [Fuc]2 |
| | 1+ | 6444.1577 | 6444.6565 | 77.4 | NCSFNITTSIRDEVQK | | [Hex]11 [HexNAc]10 [Fuc]2 |
| | | | | | | | |
| V2 | 1+ | 2861.2158 | 2861.2567 | 14.3 | LDVVPIDNNNTSYR | | [Hex]3[HexNAc]3[Fuc]1 |
| | 1+ | 3064.3611 | 3064.3361 | 8.2 | LDVVPIDNNNTSYR | | [Hex]3[HexNAc]4[Fuc]1 |
| | 1+ | 3267.4178 | 3267.4155 | 0.7 | LDVVPIDNNNTSYR | | [Hex]3[HexNAc]5[Fuc]1 |
| | 1+ | 3470.4568 | 3470.4949 | 11.0 | LDVVPIDNNNTSYR | | [Hex]3[HexNAc]6[Fuc]1 |
| | 1+ | 3023.2849 | 3023.3095 | 8.1 | LDVVPIDNNNTSYR | | [Hex]4[FlexNAc]4[Fuc]1 |
| | 1+ | 3226.3701 | 3226.3889 | 5.8 | LDVVPIDNNNTSYR | | [Hex]4[HexNAc]4[Fuc]1 |
| | 1+ | 2836.1948 | 2836.2250 | 10.6 | LVVPIDNNNTSYR | | [Hex]5[HexNAc]2 |
| | 1+ | 3185.5374 | 3185.3623 | 55.0 | LDVVPIDNNNTSYR | | [Hex]5[HexNAc]3[Fuc]1 |
| | 1+ | 3388.4583 | 3388.4417 | 4.9 | LDVVPIDNNNTSYR | | [Hex]5[HexNAc]4[Fuc]1 |
| | 1+ | 3956.6758 | 3956.6533 | 5.7 | LDVVPIDNNNTSYR | | [Hex]6[HexNAc]6[Fuc]1 |
| | | | | | | | |
| C2 | 1+ | 6304.5221 | 6304.9155 | 62.4 | NVSTVQCTHGIRPVVSTQLLLNGSLAEEEVVIR | | [Hex]6[HexNAc]7[Fuc]2 |
| | 1+ | 7352.9316 | 7353.3729 | 60.0 | NVSTVQCTHGIRPVVSTQLLLNGSLAEEEVVIR | | [Hex]6 [HexNAc]8 [Fuc]1 |
| | 1+ | 6508.3501 | 6507.9949 | 54.6 | NVSTVQCTHGIRPVVSTQLLLNGSLAEEEVVIR | | [Hex]6 [HexNAc]8 [Fuc]2 |
| | 1+ | 6711.4565 | 6711.0743 | 57.0 | NVSTVQCTHGIRPVVSTQLLLNGSLAEEEVVIR | | [Hex]6 [HexNAc]9 [Fuc]2 |
| | 1+ | 6954.2759 | 6953.1268 | 165.8 | NVSTVQCTHGIRPVVSTQLLLNGSLAEEEVVIR | | [Hex]6 [HexNAc]10 [Fuc]2 |
| | 1+ | 7116.0029 | 7117.2330 | 172.8 | NVSTVQCTHGIRPVVSTQLLLNGSLAEEEVVIR | | [Hex]6 [HexNAc)11 [Fuc]2 |
| | 1+ | 6465.7764 | 6466.9834 | 184.3 | NVSTVQCTHGIRPVVSTQLLLNGSLAEEEVVIR | | [Hex]7[HexNAc]7 [Fuc]2 |
| | 1+ | 6581.1773 | 6581.0013 | 26.7 | NVSTVQCTHG IRPVVSTQLLLNGSLAEEEVVIR | | [Hex]7[HexNAc]9 |
| | 1+ | 6872.9424 | 6873.1271 | 26.9 | NVSTVQCTHGIRPVVSTQLLLNGSLAEEEVVIR | | [Hex]7[HexNAc]9 [Fuc]2 |
| | 1+ | 6279.2788 | 6279.8839 | 96.4 | NVSTVQCTHGIRPVVSTQLLLNGSLAEEEVVIR | | [Hex]8[HexNAc]9 |
| | 1+ | 6482.6450 | 6482.8785 | 36.0 | NVSTVQCTHGIRPVVSTQLLLNGSLAEEEVVIR | | [Hex]8 [HexNAc]6 [Fuc]1 |
| | 1+ | 6742.7813 | 6743.0641 | 41.9 | NVSTVQCTHGIRPVVSTQLLLNGSLAEEEVVIR | | [Hex]8 [HexNAc]7 [Fuc]1 |
| | 1+ | 6791.4130 | 6791.0740 | 49.9 | NVSTVQCTHGIRPVVSTQLLLNGSLAEEEVVIR | | [Hex]9 [HexNAc]7 [Fuc]2 |
| | 1+ | 6904.8320 | 6905.1169 | 41.3 | NVSTVQCTHGIRPVVSTQLLLNGSLAEEEVVIR | | [Hex]9[HexNAc]9 |
| | 1+ | 7399.8169 | 7400.3121 | 66.9 | NVSTVQCHGIRPVVSTQLLLNGSLAEEEVVIR | | [Hex]9 [HexNAc]10 [Fuc]2 |
| | 1+ | 7603.9351 | 7603.3915 | 71.5 | NVSTVQCTHGIRPVVSTQLLLNGSLAEEEVVIR | | [Hex]9 [HexNAc]11 [Fuc]2 |
| | 1+ | 6052.2602 | 6051.7729 | 80.5 | NVSTVQCTHGIRPVVSTQLLLNGSLAEEEVVIR | | [Hex]10 [HeXNAc]4 |
| | 1+ | 6954.2759 | 6953.1268 | 165.3 | NVSTVQCTHGIRPVVSTQLLLNGSLAEEEVVIR | | [Hex]10 [HexNAc]7 [Fuc]2 |
| | 1+ | 7067.3115 | 7067.1697 | 20.1 | NVSTVQCTHGIRPVVSTQLLLNGSLAEEEVVIR | | [Hex]10 [HexNAc]9 |
| | 1+ | 6214.1226 | 6213.8257 | 47.8 | NVSTVQCTHGIRPVVSTQLLLNGSLAEEEVVIR | | [Hex]11 [HexNAc]4 |
| | 1+ | 6375.5576 | 6375.8785 | 50.3 | NVSTVQCTHGIRPVVSTQLLLNGSLAEEEVVIR | | [Hex]12 [HexNAc]4 |
| | 1+ | 6537.5483 | 6537.9313 | 58.6 | NVSTVQCTHGIRPVVSTQLLLNGSLAEEEVVIR | | [Nex]13 [HexNAc]4 |
| | 1+ | 6699.8198 | 6699.9842 | 24.5 | NVSTVQCTHGIRPVVSTQLLLNGSLAEEEVVIR | | [Hex]14 [HexNAc]4 |
| | 1+ | 6861.7544 | 6862.0370 | 41.2 | NVSTVQCTHGIRPVVSTQLLLNGSLAEEEVVIR | | [Hex]15 [HexNAc]4 |
| | 1+ | 7023.8594 | 7024.0898 | 32.8 | NVSTVQCTHGIPVVSTQLLLNGSLAEEEVVIR | | [Hex]16 [HexNAc]4 |
| | 1+ | 7186.5855 | 7186.1426 | 61.6 | NVSTVQCTHGIRPVVSTQLLLNGSLAEEEVVIR | | [Hex]17 [HexNAc]4 |
| | 1+ | 7348.7136 | 7348.1954 | 70.5 | NVSTVQCTHGIRPVVSTQLLLNGSLAEEEVVIR | | [Hex]18 [HexNAc]4 |
| | | | | | | | |
| | 1+ | 2430.1475 | 2429.9559 | 78.8 | SDNFTNNAK | | [Flex]3 [HexNAc]5 |
| | 1+ | 2454.8904 | 2454.9875 | 39.6 | SDNFTNNAK | | [Hex]3 [HexNAc]4 [Fuc]1 |
| | 1+ | 2779.3523 | 2779.0932 | 93.2 | SDNFTNNAK | | [Hex]5 [HexNAc]3 [Fuc]1 |
| | | | | | | | |
| C2-V3 | 1+ | 4490.0210 | 4489.8285 | 42.9 | ESVEINCTRPNNNTR | | [Hex]6 [HexNAc]7 [Fuc]2 |
| | 1+ | 4693.1523 | 4692.9084 | 52.0 | ESVEINCTRPNNNTR | | [Hex]6 [HexNAc]8 [Fuc]2 |
| | 1+ | 4896.2964 | 4895.9878 | 63.0 | ESVEINCTRPNNNTR | | [Hex]6 [HexNAc]9 [Fuc]2 |
| | 1+ | 5099.1667 | 5099.0666 | 19.6 | ESVEINCTRPNNNTR | | [Hex]6 [HexNAc]10 [Fuc]2 |
| | 1+ | 4652.0552 | 4651.8814 | 37.4 | ESVEINCTRPNNNTR | | [Hex]7 [HexNAc]7 [Fuc]2 |
| | 1+ | 4855.7248 | 4854.9612 | 157.3 | ESVEINCTRPNNNTR | | [Hex]7 [HexNAc]8 [Fuc]2 |
| | 1+ | 5261.1938 | 5261.1195 | 14.1 | ESVEINCTRPNNNTR | | [Hex]7 [HexNAc]1 [Fuc]2 |
| | 1+ | 4464.9632 | 4464.7973 | 37.2 | ESVEINCTRPNNNTR | | [Hex]8 [HexNAc]6 [Fuc]1 |
| | 1+ | 4667.9463 | 4667.8763 | 15.0 | ESVEINCTRPNNNTR | | [Hex]8 [HexNAc]7[Fuc]1 |
| | 1+ | 4814.1397 | 4813.9342 | 42.7 | ESVEINCTRPNNNTR | | [Hex]8 [HexNAc]7[Fuc]2 |
| | 1+ | 4627.2714 | 4626.8501 | 91.1 | ESVEINCTRPNNNTR | | [Hex]9 [HexNAc]6 [Fuc]1 |
| | 1+ | 4829.8747 | 4829.9291 | 11.3 | ESVEINCTRPNNNTR | | [Hex]9 [HexNAc]7 [Fuc]1 |
| | 1+ | 4585.4624 | 4585.8232 | 78.7 | ESVEINCTRPNNNTR | | [Hex]10[HexNAc]5[Fuc]1 |
| | 1+ | 4788.6450 | 4788.9031 | 53.9 | ESVEINCTRPNNNTR | | [Hex]10 [HexNAc]6 [Fuc]1 |
| | 1+ | 5340.7336 | 5341.1192 | 72.2 | ESVEINCTRPNNNTR | | [Hex]10 [HexNAc]8 [Fuc]8 |
| | 1+ | 5543.6627 | 5544.1986 | 96.7 | ESVEINCTRPNNNTR | | [Hex]10 [HexNAc]9 [Fuc]2 |
| | 1+ | 4398.6553 | 4398.7387 | 19.0 | ESVEINCTRPNNNTR | | [Hex]11 [HexNAc]4 |
| | 1+ | 4748.0782 | 4747.8764 | 42.6 | ESVEINCTRPNNNTR | | [Hex]11 [HexNAc]5 [Fuc]1 |
| | 1+ | 4951.0351 | 4950.9558 | 16.0 | ESVEINCTRPNNNTR | | [Hex]11 [HexNAc]6 [Fuc]1 |
| | 1+ | 5154.0062 | 5154.0347 | 5.5 | ESVEINCTRPNNNTR | | [Hex]11 [HexNAc]7 [Fuc]1 |
| | 1+ | 5503.9859 | 5503.1720 | 147.9 | ESVEINCTRPNNNTR | | [Hex]11 [HexNAc]8 [Fuc]2 |
| | 1+ | 5560.2095 | 5560.1935 | 2.9 | ESVEINCTRPNNNTR | | [Hex]11 [HexNAc]9 [Fuc]1 |
| | 1+ | 5762.6011 | 5763.2728 | 116.5 | ESVEINCTRPNNNTR | | [Hex]11 [HexNAc]10 [Fuc]1 |
| | 1+ | 4560.9717 | 4560.7915 | 39.5 | ESVEINCTRPNNNTR | | [Hex]12 [HexNAc]4 |
| | 1+ | 4909.4232 | 4909.9288 | 103.0 | ESVEINCTRPNNNTR | | [Hex]12 [HexNAc]5 [Fuc]1 |
| | 1+ | 5113.2030 | 5113.0086 | 38.0 | ESVEINCTRPNNNTR | | [Hex]12 [HexNAc]6 [Fuc]1 |
| | 1+ | 5316.3879 | 5316.0875 | 56.5 | ESVEINCTRPNNNTR | | [Hex]12 [HexNAc]7 [Fuc]1 |
| | 1+ | 6199.6883 | 6199.4786 | 33.8 | ESVEINCTRPNNNTR | | [Hex]12 [HexNAc]10 [Fuc]2 |
| | 1+ | 4722.5962 | 4722.8443 | 52.5 | ESVEINCTRPNNNTR | | [Hex]13 [HexNAc]4 |
| | 1+ | 5275.0105 | 5275.0610 | 9.6 | ESVEINCTRPNNNTR | | [Hex]13 [RexNAc]6 [Fuc]1 |
| | 1+ | 4885.0040 | 4884.8972 | 21.9 | ESVEINCTRPNNNTR | | [Hex]14 [HexNAc]4 |
| | 1+ | 5437.1445 | 5437.1138 | 5.6 | ESVEINCTRPNNNTR | | [Hex]14 [HexNAc]6 [Fuc]1 |
| | 1+ | 5639.9912 | 5640.1932 | 35.8 | ESVEINCTRPNNNTR | | [Hex]14 [HexNAc]7 [Fuc]1 |
| | 1+ | 5046.6255 | 5046.9500 | 64.3 | ESVEINCTRPNNNTR | | [Hex]15 [HexNAc]4 |
| | 1+ | 5208.8913 | 5209.0028 | 21.4 | ESVEINCTRPNNNTR | | [Hex]16 [HexNAc]4 |
| | 1+ | 5370.0668 | 5533.1084 | 60.4 | ESVEINCTRPNNNTR | | [Hex]17 [HexNAc]4 |
| | 1+ | 5533.0668 | 5533.1084 | 7.5 | ESVEINCTRPNNNTR | | [Hex]18 [HexNAc]4 |
| | | | | | | | |
| V3-C3 | 1+ | 2147.9563 | 2147.8656 | 42.2 | QAHCNISR | PyroQ, u | [Hex]2[HexNAc]4 |
| | 1+ | 1860.7773 | 1860.7538 | 12.6 | QAHCNISR | PyroQ | [Nex]3[HexNAc]2 |
| | 1+ | 2080.8608 | 2080.8598 | 0.5 | QAHCNISR | | [Hex]3[HexNAc]3 |
| | 1+ | 2226.9297 | 2226.9177 | 5.4 | QAHCNISR | | [Hex]3[HexNAc]3[Fuc]1 |
| | 1+ | 2266.9385 | 2266.9125 | 11.5 | QAHCNISR | PyroQ | [Hex]3[HexNAc]4 |
| | 1+ | 2429.9922 | 2429.9971 | 2.0 | QAHCNISR | | [Hex]3[HexNAc]4[Fuc]1 |
| | 1+ | 2469.9956 | 2469.9920 | 1.5 | QAHCNISR | PyroQ | [Hex]3[HexNAc]5 |
| | 1+ | 2633.0708 | 2633.0765 | 2.2 | QAHCNISR | | [Hex]3[HexNAc]5[Fuc]1 |
| | 1+ | 2732.9641 | 2733.1038 | 51.1 | QAHCNISR | u | [Hex]3[HexNAc]6 |
| | 1+ | 2819.1650 | 2819.1293 | 12.7 | QAHCNISR | PyroQ | [Hex]3[HexNAc]6[Fuc]1 |
| | 1+ | 2039.8585 | 2039.8333 | 12.4 | QAHCNISR | | [Hex]4[HexNAc]2 |
| | 1+ | 2225.9060 | 2225.8860 | 9.0 | QAHCNISR | PyroQ | [Hex]4[HexNAc]3 |
| | 1+ | 2388.9639 | 2388.9705 | 2.8 | QAHCNISR | | [Hex]4[HexNAc]3[Fuc]1 |
| | 1+ | 2533.9917 | 2534.0080 | 6.4 | QAHCNISR | | [Hex]4[HexNAc]3[NeuNAc]1 |
| | 1+ | 2592.0488 | 2592.0499 | 0.4 | QAHCNISR | | [Hex]4[HexNAc]4[Fuc]1 |
| | 1+ | 2632.0984 | 2632.0448 | 20.4 | QAHCNISR | PyroQ | [Hex]4 [HexNAc]5 |
| | 1+ | 2795.1240 | 2795.1293 | 1.9 | QAHCNISR | | [Hex]4[HexNAc]5[Fuc]1 |
| | 1+ | 2201.8879 | 2201.8860 | 0.9 | QAHCNISR | | [Hex]5[HexNAc]2 |
| | 1+ | 2404.9954 | 2404.9654 | 12.5 | QAHCNISR | | [Hex]5[HexNAc]3 |
| | 1+ | 2551.0503 | 2551.0233 | 10.6 | QAHCNISR | | [Hex]5[HexNAc]3[Fuc]1 |
| | 1+ | 2591.0437 | 2591.0182 | 9.8 | QAHCNISR | PyroQ | [Hex]5[HexNAc]4 |
| | 1+ | 2754.0999 | 2754.1027 | 1.0 | QAHCNISR | | [Hex]5[HexNAc]4[Fuc]1 |
| | 1+ | 2794.1653 | 2794.0976 | 24.2 | QAHCNISR | PyroQ | [Hex]5[HexNAc]5 |
| | 1+ | 2957.1902 | 2957.1821 | 2.7 | QAHCNISR | | [Hex]5[HexNAc]5[Fuc]1 |
| | 1+ | 2363.9373 | 2363.9389 | 0.7 | QAHCNISR | | [Hex]6[HexNAc]2 |
| | 1+ | 2567.0396 | 2567.0182 | 8.3 | QAHCNISR | | [Hex]6[HexNAc]3 |
| | 1+ | 2696.1089 | 2696.0495 | 22.0 | QAHCNISR | PyroQ | [Hex]6[HexNAc]3[Fuc]1 |
| | 1+ | 2956.2246 | 2956.1504 | 25.1 | QAHCNISR | PyroQ | [Hex]6 [HexNAc]5 |
| | 1+ | 3119.2397 | 3119.2349 | 1.6 | QAHCNISR | | [Hex]5[HexNAc]5[Fuc]1 |
| | 1+ | 2525.9885 | 2525.9917 | 1.3 | QAHCNISR | | [Hex]7[HexNAc]2 |
| | 1+ | 3467.5456 | 3467.3405 | 59.2 | QAHCNISR | PyroQ | [Hex]7[HexNAc]6[Fuc]1 |
| | 1+ | 2688.0483 | 2688.0445 | 1.4 | QAHCNISR | | [Hex]8[HexNAc]2 |
| | 1+ | 3077.3538 | 3077.1766 | 57.6 | QAHCNISR | PyroQ | [Hex]8[HexNAc]4 |
| | 1+ | 2850.1138 | 2850.0973 | 5.8 | QAHCNISR | | [Hex]9[HexNAc]2 |
| | | | | | | | |
| C3 | 1+ | 2013.8879 | 2013.9010 | 6.5 | AKWNDTLK | | [Hex]3[HexNAc]2[Fuc]1 |
| | 1+ | 2216.9792 | 2216.9804 | 0.5 | AKWNDTLK | | [Hex]3[HexNAc]3[Fuc]1 |
| | 1+ | 2420.0789 | 2420.0597 | 7.9 | AKWNDTLK | | [Hex]3[HexNAc]4[Fuc]1 |
| | 1+ | 2623.1213 | 2623.1392 | 6.8 | AKWNDTLK | | [Hex]3[HexNAc]5[Fuc]1 |
| | 1+ | 2233.0652 | 2232.9753 | 40.3 | AKWNDTLK | | [Hex]4[HexNAc]3 |
| | 1+ | 2379.0356 | 2379.0332 | 1.0 | AKWNDTLK | | [Hex]4[HexNAc]3[Fuc]1 |
| | 1+ | 2785.2178 | 2785.1919 | 9.3 | AKWNDTLK | | [Hex]4[HexNAc]5[Fuc]1 |
| | 1+ | 2191.9480 | 2191.9487 | 0.3 | AKWNDTLK | | [Hex]5[HexNAc]2 |
| | 1+ | 2395.0344 | 2395.0281 | 2.6 | AKWNDTLK | | [Hex]5 [HexNAc]3 |
| | 1+ | 2541.1064 | 2541.0860 | 8.0 | AKWNDTLK | | [Hex]5[HexNAc]3[Fuc]1 |
| | 1+ | 2598.0649 | 2598.1075 | 16.4 | AKWNDTLK | | [Hex]5[HexNAc]4 |
| | 1+ | 2744.2117 | 2744.1654 | 16.9 | AKWNDTLK | | [Hex]5[HexNAc]4[Fuc]1 |
| | 1+ | 2947.225 | 2947.2448 | 6.7 | AKWNDTLK | | [Hex]5[HexNAc]5[Fuc]1 |
| | 1+ | 2353.9919 | 2354.0016 | 4.1 | AKWNDTLK | | [Hex]6[HexNAc]2 |
| | 1+ | 2557.1262 | 2557.0809 | 17.7 | AKWNDTLK | | [Hex]6 [HexNAc]3 |
| | 1+ | 2703.1860 | 2703.1388 | 17.5 | AKWNDTLK | | [Hex]6[HexNAc]3[Fuc]1 |
| | 1+ | 2516.0298 | 2516.0544 | 9.8 | AKWNDTLK | | [Hex]7[HexNAc]2 |
| | 1+ | 2678.0605 | 2678.1072 | 17.4 | AKWNDTLK | | [Hex]8[HexNAc]2 |
| | 1+ | 2840.3750 | 2840.1600 | 75.7 | AKWNDTLK | | [Hex]9[HexNAc]2 |
| | | | | | | | |
| | 1+ | 2346.0696 | 2346.0343 | 15.0 | LREQFENK | | [Hex]3[HexNAc]4[Fuc]1 |
| | 1+ | 2101.9580 | 2101.9283 | 14.1 | LREQFENK | | [Hex]3[HexNAc]2[Fuc]1 |
| | 1+ | 2158.9602 | 2158.9498 | 4.8 | LREQFENK | | [Hex]3[HexNAc]3 |
| | 1+ | 2305.0049 | 2305.0082 | 1.4 | LREQFENK | | [Hex]3[HexNAc]3[Fuc]1 |
| | 1+ | 2362.0161 | 2362.0291 | 5.5 | LREQFENK | | [Hex]3[HexNAc]4 |
| | 1+ | 2508.0801 | 2508.0870 | 2.8 | LREQFENK | | [Hex]3[HexNAc]4[Fuc]1 |
| | 1+ | 2565.1423 | 2565.1086 | 13.1 | LREQFENK | | [Hex]3[HexNAc]5 |
| | 1+ | 2711.1494 | 2711.1664 | 6.3 | LREQFENK | | [Hex]3[HexNAc]5[Fuc]1 |
| | 1+ | 2914.2271 | 2914.2459 | 6.5 | LREQFENK | | [Hex]3[HexNAc]6[Fuc]1 |
| | 1+ | 2117.9783 | 2117.9233 | 26.0 | LREQFENK | | [Hex]4[HexNAc]2 |
| | 1+ | 2321.0354 | 2321.0026 | 14.1 | LREQFENK | | [Hex]4[HexNAc]3 |
| | 1+ | 2467.0479 | 2467.0605 | 5.1 | LREQFENK | | [Hex]4[HexNAc]3[Fuc]1 |
| | 1+ | 2670.2852 | 2670.1399 | 54.4 | LREQFENK | | [Hex]4[HexNAc]4[Fuc]1 |
| | 1+ | 2873.2473 | 2873.2192 | 9.8 | LREQFENK | | [Hex]4[HexNAc]5[Fuc]1 |
| | 1+ | 3119.3691 | 3119.3049 | 20.6 | LREQFENK | u | [Hex]4[HexNAc]6[Fuc]1 |
| | 1+ | 2280.0222 | 2279.9760 | 20.3 | LREQFENK | | [Hex]5[HexNAc]2 |
| | 1+ | 2469.1606 | 2469.0403 | 48.7 | LREQFENK | u | [Hex]5[HexNAc]2[Fuc]1 |
| | 1+ | 2483.1245 | 2483.0554 | 27.8 | LREQFENK | | [Hex]5[HexNAc]3 |
| | 1+ | 2629.1079 | 2629.1133 | 2.1 | LREQFENK | | [Hex]5[HexNAc]3[Fuc]1 |
| | 1+ | 2832.1821 | 2832.1921 | 3.7 | LREQFENK | | [Hex]5[HexNAc]4[Fuc]1 |
| | 1+ | 3035.2500 | 3035.2721 | 7.3 | LREQFENK | | [Hex]5[HexNAc]5[Fuc]1 |
| | 1+ | 2442.0735 | 2442.0289 | 18.3 | LREQFENK | | [Hex]6 [HexNAc]2 |
| | 1+ | 2645.1660 | 2645.1082 | 21.9 | LREQFENK | | [Hex]6[HexNAc]3 |
| | 1+ | 2791.1648 | 2791.1927 | 10.0 | LREQFENK | | [Hex]6[HexNAc]3[Fuc]1 |
| | 1+ | 3197.3093 | 3197.3249 | 4.9 | LREQFENK | | [Hex]6[HexNAc]5[Fuc]1 |
| | 1+ | 3282.4448 | 3282.3413 | 31.5 | LREQFENK | for | [Hex]6[HexNAc]6 |
| | 1+ | 3400.3762 | 3400.4048 | 8.4 | LREQFENK | | [Hex]6[HexNAc]6[Fuc]1 |
| | 1+ | 2604.1636 | 2604.0817 | 31.5 | LREQFENK | | [Hex]7[HexNAc]2 |
| | 1+ | 3416.5291 | 3416.3992 | 38.0 | LREQFENK | | [Hex]7[HexNAc]6 |
| | 1+ | 3562.4316 | 3562.4571 | 7.2 | LREQFENK | | [Hex]7[HexNAc]6[Fuc]1 |
| | 1+ | 2809.1265 | 2809.1403 | 4.9 | LREQFENK | u | [Hex]8[HexNAc]2 |
| | 1+ | 2971.1519 | 2971.1931 | 13.9 | LREQFENK | u | [Hex]9[HexNAc]2 |
| | | | | | | | |
| C3-V4 | 1+ | 9381.8677 | 9380.8710 | 106.2 | TIVFNHSGGDPEIVMIISFNCGGEFFYCNSTQLFNSTWNN NTEGSNNTEGNTITLPCR | | [Hex]8[HexNAc]6[Fuc]2 |
| | 1+ | 9584.1865 | 9583.9504 | 24.6 | TIVFNHSGGDPEIVMHSFNCGGEFFYCNSTQLFNSTWNN NTEGSNNTEGNTITLPCR | | [Hex]8[HexNAc]7[Fuc]2 |
| | 1+ | 9788.1772 | 9787.0297 | 117.2 | TIVFNHSGGDPEIVMHSFNCGGEFFYCNSTQLFNSTWNN NTEGSNNTEGNTITLPCR | | [Hex]8[HexNAc]8[Fuc]2 |
| | 1+ | 10193.2184 | 10193.1885 | 2.9 | TIVFNHSGGDPEIVMHSFNCGGEFFYCNSTQLFNSTWNN NTEGSNNTEGNTITLPCR | | [Hex]8[HexNAc]10 [Fuc]2 |
| | 1+ | 9746.2392 | 9746.0032 | 24.2 | TIVFNHSGGDPEIVMHSFNCGGEFFYCNSTQLFNSTWNN NTEGSNNTEGNTITLPCR | | [Hex]9[HexNAc]7 [Fuc]2 |
| | 1+ | 9948.1348 | 9949.0826 | 95.3 | TIVFNHSGGDPEIVMHSFNCGGEFFYCNSTQLFNSTWNN NTEGSNNTEGNTITLPCR | | [Hex]9[HexNAc]8 [Fuc]2 |
| | 1+ | 10150.6719 | 10152.1619 | 146.0 | TIVFNHSGGDPEIVMHSFNCGGEFFYCNSTQLFNSTWNN NTEGSNNTEGNTITLPCR | | [Hex]9[HexNAc]9 [Fuc]2 |
| | 1+ | 10331.6562 | 10331.2097 | 140.0 | TIVFNHSGGDPEIVMHSFNCGGEFFYCNSTQLFNSTWNN NTEGSNNTEGNTITLPCR | | [Hex]11[HexNAc]9[Fuc]1 |
| | | | | | | | |
| C4 | 1+ | 2576.1475 | 2516.1643 | 6.5 | CSSNITGLLLTR | | [Hex]3[NexNAc]3[Fuc]1 |
| | 1+ | 2779.2349 | 2779.2439 | 3.2 | CSSNITGLLLTR | | [Hex]3[HexNAc]4[Fuc]1 |
| | 1+ | 2982.3120 | 2982.3231 | 3.7 | CSSNITGLLLTR | | [Hex]3[HexNAc]5[Fuc]1 |
| | 1+ | 2721.1565 | 2721.1905 | 12.5 | CSSNITGLLLTR | PyroC | [Hex]4[HexNAc]3[Fuc]1 |
| | 1+ | 2941.2930 | 2941.2965 | 1.2 | CSSNITGLLLTR | | [Hex]4[HexNAc]4[Fuc]1 |
| | 1+ | 3127.4250 | 3127.3493 | 24.2 | CSSNITGLLLTR | PyroC | [Hex]4[HexNAc]5[Fuc]1 |
| | 1+ | 2551.1184 | 2551.1326 | 5.6 | CSSNITGLLLTR | | [Hex]5[HexNAc]2 |
| | 1+ | 2754.2078 | 2754.2120 | 1.5 | CSSNITGLLLTR | | [Hex]5 [HexNAc]3 |
| | 1+ | 2883.2478 | 2883.2433 | 1.6 | CSSNITGLLLTR | PyroC | [Hex]5 [HexNAc]3 [Fuc]1 |
| | 1+ | 3103.3445 | 3103.3493 | 1.5 | CSSNITGLLLTR | | [Hex]5[HexNAc)4[Fuc]1 |
| | 1+ | 3306.4746 | 3306.4287 | 13.9 | CSSNITGLLLTR | | [Hex]5[HexNAc]5[Fuc]1 |
| | 1+ | 2713.1985 | 2713.1855 | 4.8 | CSSNITGLLLTR | | [Hex]6[HexNAc]2 |
| | 1+ | 2859.3179 | 2859.2439 | 25.9 | CSSNITGLLLTR | PyroC,for | [Hex]6[HexNAc]2[Fuc]1 |
| | 1+ | 2899.2617 | 2899.2382 | 8.1 | CSSNITGLLLTR | PyroC | [Hex]6 [HexNAc]3 |
| | 1+ | 3451.4756 | 3451.4549 | 6.0 | CSSNITGLLLTR | | [Hex]6[HexNAc]5[Fuc]1 |
| | 1+ | 2875.2400 | 2875.2383 | 0.6 | CSSNITGLLLTR | PyroC | [Hex]7[HexNAc]2 |
| | 1+ | 3037.2922 | 3037.2911 | 0.4 | CSSNITGLLLTR | | [Hex]8[HexNAc]2 |
| | 1+ | 3199.3481 | 3199.3439 | 1.3 | CSSNITGLLLTR | | [Hex]9[HexNAc]2 |
| V5 | 1+ | 3333.5847 | 3333.4059 | 53.6 | DGGINENGTEIFRPGGGDMR | | [Hex]3[HexNAc]3[Fuc]1 |
| | 1+ | 3536.4839 | 3536.4852 | 0.4 | DGGINENGTEIFRPGGGDMR | | [Hex]3[NexNAc]4[Fuc]1 |
| | 1+ | 3593.7019 | 3593.5068 | 54.3 | DGGINENGTEIFRPGGGDMR | | [Hex]3[HexNAc]5 |
| | 1+ | 3739.5930 | 3739.5647 | 7.6 | DGGINENGTEIFRPGGGDMR | | [Hex]3[HexNAc]5[Fuc]1 |
| | 1+ | 3812.7542 | 3812.5811 | 45.4 | DGGINENGTEIFRPGGGDMR | mox | [Hex]3 [HexNAc]6 |
| | 1+ | 3942.6719 | 3942.6440 | 7.1 | DGGPIENGTEIFRPGGGDMR | | [Hex]3[HexNAc]6[Fuc]1 |
| | 1+ | 3292.5959 | 3292.3793 | 65.8 | DGGINENGTEIFRPGGGDMR | | [Hex]4[HexNAc]2[Fuc]1 |
| | 1+ | 3495.4312 | 3495.4587 | 7.9 | DGGINENGTEIFRPGGGDMR | | [Hex]4[HexNAc]3[Fuc]1 |
| | 1+ | 3698.5691 | 3698.5381 | 8.4 | DGGINENGTEIFRPGGGDMR | | [Hex]4[HexNAc]4[Fuc]1 |
| | 1+ | 3901.6543 | 3901.6174 | 9.5 | DGGINENGTEIFRPGGGDMR | | [Flex]4[HexNAc]5[Fuc]1 |
| | 1+ | 4104.9648 | 4104.6943 | 65.9 | DGGINENGTEIFRPGGGDMR | | [Hex]4[HexNAc]6[Fuc]1 |
| | 1+ | 3308.5476 | 3308.3742 | 52.4 | DGGINENGTEIFRPGGGDMR | | [Hex]5[HexNAc]2 |
| | 1+ | 3657.7288 | 3657.5115 | 59.4 | DGGINENGTEIFRPGGGDMR | | [Hex]5[HexNAc]3[Fuc]1 |
| | 1+ | 3860.6372 | 3860.5909 | 12.0 | DGGINENGTEIFRPGGGDMR | | [Hex]5[HexNAc]4[Fuc]1 |
| | 1+ | 3917.8411 | 3917.6124 | 58.4 | DGGINENGTEIFRPGGGDMR | | [Hex]5[HexNAc]5 |
| | 1+ | 4063.6707 | 4063.6703 | 0.1 | DGGINENGTEIFRPGGGDMR | | [Hex]5[HexNAc]5[Fuc]1 |
| | 1+ | 4267.0376 | 4266.7502 | 67.4 | DGGINENGTEIFRPGGGDMR | | [Hex]5[HexNAc]6[Fuc]1 |
| | 1+ | 4225.7739 | 4225.7231 | 12.0 | DGGINENGTEIFRPGGGDMR | | [Hex]6[HexNAc]5[Fuc]1 |
| | 1+ | 4428.9495 | 4428.8030 | 33.1 | DGGINENGTEIFRPGGGDMR | | [Hex]6[HexNAc]6[Fuc]1 |
| | 1+ | 3632.3776 | 3632.4798 | 28.1 | DGGINENGTEIFRPGGGDMR | | [Hex]7[HexNAc]2 |
| | 1+ | 4590.9377 | 4590.8553 | 17.9 | DGGINENGTEIFRPGGGDMR | | [Hex]7[HexNAc]6[Fuc]1 |
| | 1+ | 4794.4761 | 4793.9345 | 113.0 | DGGINENGTEIFRPGGGDMR | | [Hex]7[HexNAc]7[Fuc]1 |
| | 1+ | 3794.5559 | 3794.5327 | 6.1 | DGGINENGTEIFRPGGGDMR | | [Hex]8[HexNAc]2 |
| | 1+ | 3956.6758 | 3956.5855 | 22.8 | DGGINENGTEIFRPGGGDMR | | [Hex]9[HexNAc]2 |
| | | | | | | | |
| TM | 1+ | 2937.3625 | 2937.1952 | 57.0 | IWNNMTWMEWER | | [Hex]3[HexNAc]3[Fuc]1 |
| | 1+ | 3140.3572 | 3140.2745 | 26.3 | IWNNMTWMEWER | | [Hex]3[HexNAc]4[Fuc]1 |
| | 1+ | 3343.4673 | 3343.3539 | 33.9 | IWNNMTWMEWER | | [Hex]3[HexNAc]5[Fuc]1 |
| | 1+ | 3546.4897 | 3546.4333 | 15.9 | IWNNMTWMEWER | | [Hex]3[HexNAc]6[Fuc]1 |
| | 1+ | 3156.4517 | 3156.2695 | 57.7 | IWNNMTWMEWER | | [Hex]4[HexNAc]4 |
| | 1+ | 3359.4663 | 3359.3488 | 25.0 | IWNNMTWMEWER | | [Hex]4[HexNAc]5 |
| | 1+ | 3505.5205 | 3505.4067 | 32.5 | IWNNMTWMEWER | | [Hex]4[HexNAc]5[Fuc]1 |
| | 1+ | 3708.6440 | 3708.4583 | 50.1 | IWNNMTWMEWER | | [Hex]4[HexNAc]6[Fuc]1 |
| | 1+ | 2912.2561 | 2912.1635 | 31.8 | IWNNMTWMEWER | | [Hex]5[HexNAc]2 |
| | 1+ | 3464.4841 | 3464.3802 | 30.0 | IWNNMTWMEWER | | [Hex]5[HexNAC]4[Fuc]1 |
| | 1+ | 3667.4997 | 3667.4596 | 10.9 | IWNNMTWMEWER | | [Hex]5[HexNAc]5[Fuc]1 |
| | 1+ | 3480.6052 | 3480.3323 | 78.4 | IWNNMTWMEWER | | [Hex]6[HexNAc]4 |
| | 1+ | 3829.6699 | 3829.5124 | 41.1 | IWNNMTWMEWER | | [Hex]6[HexNAc]5[Fuc]1 |
| | 1+ | 4032.5845 | 4032.5918 | 1.8 | IWNNMTWMEWER | | [Hex]6[HexNAc]6[Fuc]1 |
| | 1+ | 3236.4509 | 3236.2692 | 56.1 | IWNNMTWMEWER | | [Hex]7[HexNAc]2 |
| | 1+ | 4194.6055 | 4194.6446 | 9.3 | IWNNMTWMEWER | | [Hex]7[HexNAc]6[Fuc]1 |
| | 1+ | 3398.4519 | 3398.3220 | 38.2 | IWNNMTWMEWER | | [Hex]8[HexNAc]2 |
| | | | | | | | |

**Table 9. LC/MS Glycopeptide Composition for CON-S gp 140 ΔCFI**

| **Env Domain** | **Charge State** | **Experimental Mass** | **Theoretical Mass** | **Mass Error** | **Peptide** | **Mod*** | **Carbohydrate Composition** |
|---|---|---|---|---|---|---|---|
| CI | 2+ | 1160.4872 | 1160.4858 | 1.2 | EANTTLFCASDAK | | [Hex]3[HexNAc]2 |
| | 2+ | 1262.0140 | 1262.0255 | 9.1 | EANTTLFCASDAK | | [Hex]3[HexNAc]3 |
| | 2+ | 1335.0390 | 1335.0545 | 11.6 ' | EANTTLFCASDAK | | [Hex]3[HexNAc]3[Fuc]1 |
| | 2+ | 1363.5621 | 1363.5652 | 2.3 | EANTTLFCASDAK | | [Hex]3[HexNAc]4 |
| | 2+ | 1436.5968 | 1436.5941 | 1.9 | EANTTLFCASDAK | | [Hex]3[HexNAc]4[Fuc]1 |
| | 2+ | 1465.1025 | 1465.1049 | 1.6 | EANTTLFCASDAK | | [Hex]3[HexNAc]5 |
| | 2+ | 1538.1244 | 1538.1339 | 6.2 | EANTTLFCASDAK | | [Hex]3[HexNAc]5[Fuc]1 |
| | 2+ | 1639.6930 | 1639.6736 | 11.8 | EANTTLFCASDAK | | [Hex]3[HexNAc]6[Fuc]1 |
| | 2+ | 1241.5155 | 1241.5123 | 2.6 | EANTTLFCASDAK | | [Hex]4[HexNAc]2 |
| | 2+ | 1343.0704 | 1343.0519 | 13.8 | EANTTLFCASDAK | | [Hex]4[HexNAc]3 |
| | 2+ | 1416.0768 | 1416.0809 | 2.9 | EANTTLFCASDAK | | [Hex]4[HexNAc]3[Fuc]1 |
| | 2+ | 1424.0856 | 1424.0783 | 5.1 | EANTTLFCASDAK | | [Hex]5[HexNAc]3 |
| | 2+ | 1322.5446 | 1322.5386 | 4.5 | EANTTLFCASDAK | | [Hex]5[HexNAc]2 |
| | 2+ | 1497.1163 | 1497.1073 | 6.0 | EANTTLFCASDAK | | [Hex]5[HexNAc]3[Fuc]1 |
| | 2+ | 1464.0842 | 1464.0567 | 18.8 | EANTTLFCASDAK | | [Hex]5[HexNAc]3[SO3]1 |
| | 2+ | 1525.6365 | 1525.6180 | 12.1 | EANTTLFCASDAK | | [Hex]5[HexNAc]4 |
| | 2+ | 1403.5756 | 1403.5651 | 7.5 | EANTTLFCASDAK | | [Hex]6 [HexNAc]2 |
| | 2+ | 1505.1039 | 1505.1047 | 0.5 | EANTTLFCASDAK | | [Hex]6[HexNAc]3 |
| | 2+ | 1578.1262 | 1578.1337 | 4.8 | EANTTLFCASDAK | | [Hex]6[HexNAc]3[Fuc]1 |
| | 2+ | 1606.6709 | 1606.6444 | 16.5 | EANTTLFCASDAK | | [Hex]6[HexNAc]4 |
| | 2+ | 1484.6057 | 1484.5915 | 9.6 | EANTTLFCASDAK | | [Hex]7[HexNAc]2 |
| | 2+ | 1565.6242 | 1565.6179 | 4.0 | EANTTLFCASDAK | | [Hex]8[HexNAc]2 |
| | 2+ | 1646.6523 | 1646.6443 | 4.7 | EANTTLFCASDAK | | [Hex]9[HexNAc]2 |
| | 2+ | 1517.6222 | 1517.6206 | 1.1 | EANTTLFCASDAK | | [Hex]4[HexNAc]5 |
| | 2+ | 1686.6788 | 1686.6631 | 9.3 | EANTTLFCASDAK | | [Hex]5[HexNAc]3[NeuNAc]2 |
| | 2+ | 1086.7249 | 1086.7223 | 2.4 | EANTTLFCASDAK | | [Hex]6[HexNAc]3[Fuc]1[NeuGc]2 |
| | 2+ | 1659.1649 | 1659.1601 | 2.9 | EANTTLFCASDAK | | [Hex]7[HexNAc]4 |
| | | | | | | | |
| | 4+ | 1479.4066 | 1479.3881 | 8.5 | AYDTEVHNVWATHACVPTDPNPQEVVLENVTEHFNMWK | u,ox | [Hex]3[HexNAc]3[Fuc]2 |
| | 4+ | 1428.3833 | 1428.3841 | 0.5 | AYDTEVHNVWATHACVPTDPNPQEVVLENVTEHFNMWK | | [Hex]3[HexNAc]4 |
| | 4+ | 1493.4064 | 1493.403B | 2.2 | AYDTEVHNVWATHACVPTDPNPQEVVLENVTEHFNMWK | | [Hex]3[HexNAc]5 |
| | 4+ | 1529.9330 | 1529.9238 | 6.0 | AYDTEVHNVWATHACVPTDPNPQEVVLENVTEHFNMWK | | [Hex]3[HexNAc]6 |
| | 4+ | 1468.8833 | 1468.8973 | 9.5 | AYDTEVHNVWATHACVPTDPNPQEVVLENVTEHFNMWK | | [Hex]4 [HexNAc]4 |
| | 4+ | 1570.4200 | 1570.4369 | 10.8 | AYDTEVHNVWATHACVPTDPNPQEVVLENVTEHFNMWK | | [Hex]4[HexNAc]5[Fuc]1 |
| | 4+ | 1509.4340 | 1509.4105 | 63.7 | AYDTEVHNVWATHACVPTDPNPQEVVLENVTEHFNMWK | | [Hex]4[HexNAc]5[NeuNAc]1 |
| | 4+ | 1592.3395 | 1592.4410 | 15.6 | AYDTEVHNVWATHACVPTDPNPQEVVLENVTEHFNMWK | | [Hex]5 [HexNAc]3 [Fuc]1 |
| | 4+ | 1422.1360 | 1422.1261 | 7.0 | AYDTEVHNVWATHACVPTDPNPQEVVLENVTEHFNMWK | | [fiex]5[HexNAc]2 |
| | 4+ | 1560.2100 | 1560.1803 | 19.0 | AYDTEVHNVWATHACVPTDPNPQEVVLENVTEHFNMWK | | [Hex]5[HexNAc]5 |
| | 4+ | 1632.7999 | 1632.9542 | 94,5 | AYDTEVHNVWATHACVPTDPNPQEVVLENVTEHFNMWK | | [Hex]5[HexNAc]5[NeuNAc]1 |
| | 4+ | 1462.6240 | 1462.6394 | 10.5 | AYDTEVHNVWATHACVPTDPNPQEVVLENVTEHFNMWK | | [Hex]6 [HexNAc]2 |
| | 4+ | 1503.1770 | 1503.1526 | 16.2 | AYDTEVHNVWATHACVPTDPNPQEVVLENVTEHFNMWK | | [Hex]7[HexNAc]2 |
| | 4+ | 1543.6690 | 1543.6658 | 2.1 | AYDTEVHNVWATHACVPTDPNPQEVVLENVTEHFNMWK | | [Hex]8[HexNAc]2 |
| | 4+ | 1584,1990 | 1584.1790 | 12.6 | AYDTEVHNVWATHACVPTDPNPQEVVLENVTEHFNMWK | | [Hex]9[HexNAc]2 |
| | | | | | | | |
| V1-V2 | 2+ | 1123,9866 | 1123.9833 | 2.9 | NCSFNITTEIR | | [Hex]3[NexNAc]2 |
| | 2+ | 1298.5541 | 1298.5519 | 1.7 | NCSFNITTEIR | | [Hex]3[HexNAc]3[Fuc]1 |
| | 2+ | 1327.0679 | 1327.0626 | 4.0 | NCSFNITTEIR | | [Hex]3[HexNAc]4 |
| | 2+ | 1400.1004 | 1400.0916 | 6.3 | NCSFNITTEIR | | [Hex]3[HexNAc]4[Fuc]1 |
| | 2+ | 1501.6420 | 1501.6313 | 7.1 | NCSFNITTEIR | | [Hex]3[HexNAc]5[Fuc]1 |
| | 2+ | 1603,1831 | 1603.1710 | 7.6 | NCSFNITTEIR | | [Hex]3[HexNAc]6[Fuc]1 |
| | 2+ | 1306.5547 | 1306.5494 | 4.1 | NCSFNITTEIR | | [Hex]4[HexNAc]3 |
| | 2+ | 1379.5812 | 1379.5783 | 2.1 | NCSFNITTEIR | | [Hex]4[HexNac]3 [Fuc]1 |
| | 2+ | 1408.0902 | 1408.0891 | 0.8 | NCSFNITTEIR | | [Hex]4[HexNAc]4 |
| | 2+ | 1481.1246 | 1481.1180 | 4.5 | NCSFNITTEIR | | [Hex]4[HexNAc]4 [Fuc]1 |
| | 2+ | 1205.0137 | 1205.0097 | 3.4 | NCSFNITTEIR | | [Hex]4[HexNAc]2 |
| | 2+ | 1582.6652 | 1582.6577 | 4.7 | NCSFNITTEIR | | [Hex[4[HexNAc]5[Fuc]1 |
| | 2+ | 1728.2005 | 1728.2054 | 2.8 | NCSFNITTEIR | | [Hex]4[HexNAc]5[Fuc]1[NeuNAc]1 |
| | 2+ | 1286.0405 | 1286.0361 | 3.4 | NCSFNITTEIR | | [Hex]5[HexNAc]2 |
| | 2+ | 1387.5846 | 1387.5758 | 6.4 | NCSFNITTEIR | | [Hex]5[HexNAc]3 |
| | 2+ | 1533.1154 | 1533.1235 | 5.3 | NCSFNITTEIR | | [Hex]5[HexNAc]3 [NeuNAc]1 |
| | 2+ | 1707.6869 | 1707.6921 | 3.1 | NCSFNITTEIR | | [Hex]5[HexNAc]4[Fuc]1[NeuNAc]1 |
| | 2+ | 1460.6116 | 1460.6047 | 4.7 | NCSFNITTEIR | | [Hex]5[HexNAc]3[Fuc]1 |
| | 2+ | 1489.1111 | 1489.1155 | 3.0 | NCSFNITTEIR | | [Hex]5[HexNAc]4 |
| | 2+ | 1707.6948 | 1707.6921 | 1.6 | NCSFNITTEIR | | [Hex]5[HexNAc]4[Fuc]1[NeuNAc]1 |
| | 2+ | 1367.0661 | 1367.0625 | 2.6 | NCSFNITTEIR | | [Hex]6[HexNAc]2 |
| | 2+ | 1468.6081 | 1468.6022 | 4.0 | NCSFNITTEIR | | [Hex]6[HexNAc]3 |
| | 2+ | 1671.6914 | 1671.6816 | 5.9 | NCSFNITTEIR | | [Hex]6[HexNAc]5 |
| | 2+ | 1541.6262 | 1541.6311 | 3.2 | NCSFNITTEIR | | [Hex]6[HexNAc]3[Fuc]1 |
| | 2+ | 1584.1572 | 1584.1393 | 11.3 | NCSFNITTEIR | for | [Hex]6[HexNAc]4 |
| | 2+ | 1448.0986 | 1448.0889 | 4.8 | NCSFNITTEIR | | [Hex]7[HexNAc]2 |
| | 2+ | 1563.6435 | 1563.6260 | 11.2 | NCSFNITTEIR | for | [Hex]7[HexNAc]3 |
| | 2+ | 1636.6431 | 1636.6555 | 7.6 | NCSFNITTEIR | | [Hex]7[HexNAc]3Fuc1 |
| | 2+ | 1636.6431 | 1636.6550 | 7.3 | NCSFNITTEIR | for | [Hex]7[HexNAc]4 |
| | 2+ | 1529.1250 | 1529.1153 | 6.3 | NCSFNITTEIR | | [Hex]8[HexNAc]2 |
| | 2+ | 1610.1519 | 1610.1417 | 6.3 | NCSFNITTEIR | | [Hex]9[HexNAc]2 |
| | | | | | | | |
| V2 | 2+ | 1522.6397 | 1522.6516 | 7.8 | LDVVPIDDNNNNSSNYR | | [Hex]3[HexNAc]3 |
| | 2+ | 1595.6768 | 1595.6805 | 2.3 | LDVVPIDDNNNNSSNYR | | [Hex]3[HexNAc]3[Fuc]1 |
| | 2+ | 1697.2305 | 1697.2202 | 6.1 | LDVVPIDDNNNNSSNYR | | [Hex]3[HexNAc]4[Fuc]1 |
| | 2+ | 1199.5139 | 1199.5090 | 4.1 | LDVVPIDDNNNNSSNYR | | [Hex]3[HexNAc]5[Fuc]1 |
| | 2+ | 1900.3074 | 1900.2996 | 4.1 | LDVVPIDDNNNNSSNYR | | [Hex]3[HexNAc]6[Fuc]1 |
| | 2+ | 1502.1424 | 1502.1383 | 2.7 | LDVVPIDDNNNNSSNYR | | [Hex]4[HexNAc]2 |
| | 2+ | 1603.6862 | 1603.6780 | 5.1 | LDVVPIDDNNNNSSNYR | | [Hex]4[HexNAc]3 |
| | 2+ | 1676.7151 | 1676.7069 | 4.9 | LDVVPIDDNNNNSSNYR | | [Hex]4[HexNAc]3[Fuc]1 |
| | 2+ | 1879.7935 | 1879.7863 | 3.8 | LDVVPIDDNNNNSSNYR | | [Hex]4[HexNAc]5[Fuc]1 |
| | 2+ | 1684.7036 | 1684.7044 | 0.4 | LDVVPIDDNNNNSSNYR | | [Hex]5 [HexNAc]3 |
| | 2+ | 1583.1708 | 1583.1647 | 3.9 | LDVVPIDDNNNNSSNYR | | [Hex]5[HexNAc]2 |
| | 3+ | 1172.1578 | 1172.1580 | 0.2 | LDVVPIDDNNNNSSNYR | | [Hex]5[HexNAc]3[Fuc]1 |
| | 2+ | 1960.7955 | 1960.8127 | 8.8 | LDVVPIDDNNNNSSNYR | | [Hex]5[HexNAc]5[Fuc]1 |
| | 2+ | 1664.1902 | 1664.1911 | 0.5 | LDVVPIDDNNNNSSNYR | | [Hex]6 [HexNAc]2 |
| | 2+ | 1838.7486 | 1838.7597 | 6.0 | LDVVPIDDNNNNSSNYR | | [Hex]6[HexNAc]3[Fuc]1 |
| | 2+ | 1274.5348 | 1274.5214 | 10.5 | LDVVPIDDNNNNSSNYR | | [Hex]6[HexNAc]3[NeuNAc]1 |
| | 2+ | 1745.2247 | 1745.2175 | 4.1 | LDVVPIDDNNNNSSNYR | | [Hex]7[HexNAc]2 |
| | 2+ | 1826.2494 | 1826.2439 | 3.0 | LDVVPIDDNNNNSSNYR | | [Hex]8[HexNAc]2 |
| | 3+ | 1353.2122 | 1353.2179 | 4.2 | LDVVPIDDNNNNSSNYR | | [Hex]8[HexNAc]4 |
| | 2+ | 1907.2739 | 1907.2703 | 1.9 | LDVVPIDDNNNNSSNYR | | [Hex]9[HexNAc]2 |
| | | | | | | | |
| V2-C2 | 2+ | 1292.0772 | 1292.0748 | 1.9 | LINCNTSAITQACPK | | [Hex]3[HexNAc]2 |
| | 2+ | 1393.6180 | 1393.6145 | 2.5 | LINCNTSAITQACPK | | [Hex]3[HexNAc]3 |
| | 2+ | 1466.6413 | 1466.6435 | 1.5 | LINCNTSAITQACPK | | [Hex]3[HexNAc]3[Fuc]1 |
| | 2+ | 1495.1548 | 1495.1542 | 0.4 | LINCNTSAITQACPK | | [Hex]3[HexNAc]4 |
| | 2+ | 1568.1874 | 1568.1831 | 2.7 | LINCNTSAITQACPK | | [Hex]3[HexNAc]4[Fuc]1 |
| | 2+ | 1669.7298 | 1669.7229 | 4.1 | LINCNTSAITQACPK | | [Hex]3[HexNAc]5[Fuc]1 |
| | 2+ | 1771.2772 | 1771.2626 | 8.2 | LINCNTSAITQACPK | | [Hex]3[HexNAc]6[Fuc]1 |
| | 2+ | 1373.1031 | 1373.1013 | 1.3 | LINCNTSAITQACPK | | [Hex]4[HexNAc]2 |
| | 2+ | 1474.6313 | 1474.6409 | 6.5 | LINCNTSAITQACPK | | [Hex]4[HexNAc]3 |
| | 2+ | 1547.6695 | 1547.6699 | 0.3 | LINCNTSAITQACPK | | [Hex]4[HexNAc]3[Fuc]1 |
| | 2+ | 1750.7448 | 1750.7492 | 2.5 | LINCNTSAITQACPK | | [Hex]4[HexNAc]5[Fuc]1 |
| | 3+ | 1361.5569 | 1361.5655 | 6.3 | LINCNTSAITQACPK | | [Hex]4[HexNAc]5[Fuc]1[NeuNAc]2 |
| | 2+ | 1555.6774 | 1555.6673 | 6.5 | LINCNTSAITQACPK | | [Hex]5 [HexNAc]2 |
| | 2+ | 1454.1300 | 1454.1276 | 1.7 | LINCNTSAITQACPK | | [Hex]5[HexNAc]3 |
| | 2+ | 1628.7037 | 1628.6963 | 4.6 | LINCNTSAITQACPK | | [Hex]5[HexNAc]3[Fuc]1 |
| | 2+ | 1730.2303 | 1730.2360 | 3.3 | LINCNTSAITQACPK | | [Hex]5[HexNAc]4[Fuc]1 |
| | 3+ | 1347.8951 | 1347.8900 | 3.8 | LINCNTSAITQACPK | | [Hex]5[HexNAc]4[Fuc]1 [NeuNAc]2 |
| | 2+ | 1831.7700 | 1831.7757 | 3.1 | LINCNTSAITQACPK | | [Hex]5[HexNAc]5[Fuc]1 |
| | 2+ | 1535.1602 | 1535.1541 | 4.0 | LINCNTSAITQACPK | | [Hex]6 [HexNAc]2 |
| | 2+ | 1636.6929 | 1636.6937 | 0.5 | LINCNTSAITQACPK | | [Hex]6[HexNAc]3 |
| | 2+ | 1709.7191 | 1709.7227 | 2.1 | LINCNTSAITQACPK | | [Hex]6[HexNAc]3[Fuc]1 |
| | 2+ | 1616.1805 | 1616.1805 | 0.0 | LINCNTSAITQACPK | | [Hex]7[HexNAc]2 |
| | 2+ | 1697.2128 | 1697.2069 | 3.5 | LINCNTSAITQACPK | | [Hex]8[HexNAc]2 |
| | 2+ | 1778.2159 | 1778.2333 | 9.8 | LINCNTSAITQACPK | | [Hex]9[HexNAc]2 |
| | | | | | | | |
| C2 | 2+ | 1156.4487 | 1156.4539 | 4.5 | FNGTGPCK | for | [Hex]4 [HexNAc]4 |
| | 2+ | 1587.6075 | 1587.6072 | 0.2 | FNGTGPCK | | [Hex]5[HexNAc]4[Fuc]1[NeuNAc]2 |
| | 2+ | 1291.9885 | 1291.9934 | 3.8 | FNGTGPCK | | [Hex]8[HexNAc]2 |
| | 2+ | 1373.0222 | 1373.0198 | 1.8 | FNGTGPCK | | [Hex]9[HexNAc]2 |
| | | | | | | | |
| | 2+ | 1319.5503 | 1319.5529 | 2.0 | SENITNNAK | | Hex]3[HexNAc]5[Fuc]1 |
| | 2+ | 1205.5023 | 1205.4973 | 4.2 | SENITNNAK | | [Hex]5[HexNAc]3 |
| | 2+ | 1372.5452 | 1372.5479 | 2.0 | SENITNNAK | u | [Hex]5 [HexNAc]3 [NeuNAc]1 |
| | 2+ | 1103.9567 | 1103.9576 | 0.8 | SENITNNAK | | [Hex]5[HexNAc]2 |
| | 2+ | 1184.9899 | 1184.9841 | 4.9 | SENITNNAK | | [Nex]6[HexNAc]2 |
| | 2+ | 1409.5882 | 1409.5663 | 15.5 | SENITNNAK | u | [Hex]6[HexNAc]4 |
| | 2+ | 1584.1332 | 1584.1350 | 1.1 | SENITNNAK | u | [Hex]6[HexNAc]5[Fuc]1 |
| | 2+ | 1266.0109 | 1266.0105 | 0.4 | SENITNNAK | | [Hex]7[HexNAc]2 |
| | 2+ | 1490.5868 | 1490.5927 | 4.0 | SENITNNAK | u | [Hex]7[HexNAc]4 |
| | 2+ | 1347.0394 | 1347.0369 | 1.9 | SENITNNAK | | [Hex]8[HexNAc]2 |
| | 2+ | 1428.0684 | 1428.0633 | 3.6 | SENITNNAK | | [Hex]9[HexNAc]2 |
| | | | | | | | |
| V3-C3 | 2+ | 1166.4753 | 1166.4782 | 2.5 | QAHCNISGTK | | [Hex]5[HexNAc]2 |
| | 2+ | 1587.6075 | 1587.6184 | 6.9 | QAHCNISGTK | PyroC | [Hex]6[HexNAc]4[NeuNAc]1 |
| | 2+ | 1409.5582 | 1409.5574 | 0.5 | QAHCNISGTK | | [Hex]8[HexNAc]2 |
| | 2+ | 1490.5868 | 1490.5839 | 2.0 | QAHCNISGTK | | [Hex]9[HexNAc]2 |
| | 3+ | 1218.8466 | 1218.8505 | 3.2 | QAHCNISGTKWNK | | [Hex]3[HexNAc]8 |
| | 3+ | 1305.1849 | 1305.1852 | 0.2 | QAHCNISGTKWNK | for | [Hex]6[HexNAc]3[Fuc]1[NeuGc]2 |
| | | | | | | | |
| C3 | 2+ | 1442.5788 | 1442.6577 | 54.7 | WNKTLQQVAKK | | [Hex]7[HexNAc]2 |
| | 2+ | 1156.4409 | 1156.4383 | 2.3 | WNK | | [Hex]9[HexNAc]2 |
| | | | | | | | |
| | 2+ | 1116.9586 | 1116.9549 | 3.3 | EHFNNK | | [Hex]3[HexNAc]4[Fuc]1 |
| | 2+ | 1218.5059 | 1218.4946 | 9.3 | EHFNNK | | [Hex]3[HexNAc]5[Fuc]1 |
| | 2+ | 1164.9294 | 1164.9308 | 1.1 | EHFNNK | | [Hex]4[HexNAc]4[SO3]1 |
| | 2+ | 1002.9005 | 1002.8994 | 1.1 | EHFNNK | | [Hex]5[HexNAc]2 |
| | 2+ | 1177.4673 | 1177.4680 | 0.6 | EHFNNK | | [Hex]5[HexNAc]3[Fuc]1 |
| | 2+ | 1245.9790 | 1245.9786 | 0.3 | EHFNNK | | [Hex]8[HexNAc]2 |
| | 2+ | 1327.0049 | 1327.0050 | 0.1 | EHFNNK | | [Hex]9[HexNAc]2 |
| | | | | | | | |
| | 2+ | 1231.0430 | 1231.0342 | 7.2 | LREHFNNK | | [Hex]4[HexNAc]3[Fuc]1 |
| V4 | 2+ | 1270.0439 | 1270.0398 | 3.2 | NNNNTNDTITLPCR | | [Hex]3[HexNAc]2 |
| | 2+ | 1444.5985 | 1444.6084 | 6.8 | NNNNTNDTITLPCR | | [Hex]3[HexNAc]3[Fuc]1 |
| | 2+ | 1546.1551 | 1546.1481 | 4.5 | NNNNTNDTITLPCR | | [Hex]3[HexNAc]4[Fuc]1 |
| | 2+ | 1618.6795 | 1618.6669 | 7.8 | NNNNTNDTITLPCR | | [Hex]3[HexNAc]4[NeuNAc]1 |
| | 2+ | 1647.6877 | 1647.6878 | 1.0 | NNNNTNDTTTLPCR | | [Hex]3[HexNAc]5[Fuc]1 |
| | 2+ | 1351.0603 | 1351.0662 | 4.4 | NNNNTNDTITLPCR | | [Hex]4[HexNAc]2 |
| | 2+ | 1424.0871 | 1424.0951 | 5.6 | NNNNTNDTITLPCR | | [Hex]4[HexNAc]2[Fuc]1 |
| | 2+ | 1452.6028 | 1452.6059 | 2.1 | NNNNTNDTITLPCR | | [Hex]4[HexNAc]3 |
| | 2+ | 1525.6392 | 1525.6348 | 2.9 | NNNNTNDTITLPCR | | [Hex]4[HexNAc]3[Fuc]1 |
| | 2+ | 1655.6876 | 1655.6853 | 1.4 | NNNNTNDTITLPCR | | [Hex]4[HexNAc]5 |
| | 2+ | 1728.7193 | 1728.7142 | 3.0 | NNNNTNDTITLPCR | | [Hex]4[HexNAc]5[Fuc]1 |
| | 3+ | 1346.8753 | 1346.8755 | 0.2 | NNNNTNDTITLPCR | | [Hex]4[HexNAc]5[Fuc]1[NeuNAc]2 |
| | 3+ | 1268.8394 | 1268.8509 | 9.1 | NNNNTNDTITLPCR | | [Hex]4[HexNAc]6[Fuc]1[NeuNAc]1 I |
| | 2+ | 1533.6464 | 1533.6323 | 9.2 | NNNNTNDTITLPCR | | [Hex]5 [HexNAc]3 |
| | 2+ | 1432.0954 | 1432.0926 | 2.0 | NNNNTNDTITLPCR | | [Hex]5[HexNAc]2 |
| | 2+ | 1606.6708 | 1606.6612 | 6.0 | NNNNTNDTITLPCR | | [Hex]5[HexNAc]3[Fuc]1 |
| | 2+ | 1635.1774 | 1635.1720 | 3.3 | NNNNTNDTITLPCR | | [Hex]5[HexNAc]4 |
| | 2+ | 1708.2052 | 1708.2009 | 2.5 | NNNNTNDTITLPCR | | [Hex]5[HexNAc]4[Fuc]1 |
| | 3+ | 1206.8378 | 1206.8295 | 6.9 | NNNNTNDTITLPCR | | [Hex]5[HexNAc]5[Fuc]1 |
| | 2+ | 1513.1252 | 1513.1190 | 4.1 | NNNNTNDTITLPCR | | [Hex]6 [HexNAc]2 |
| | 3+ | 1260.8585 | 1260.8471 | 9.0 | NNNNTNDTITLPCR | | [Hex]6[HexNAc]5[Fuc]1 |
| | 3+ | 1309.5245 | 1309.5331 | 6.6 | NNNNTNDTITLPCR | | [Hex]6[HexNAc]6[Fuc]1 |
| | 2+ | 1594.1540 | 1594.1454 | 5.4 | NNNNTNDTITLPCR | | [Hex]7[HexNAc]2 |
| | 3+ | 1314.8659 | 1314.8647 | 0.9 | NNNNTNDTITLPCR | | [Hex]7[HexNAc]6 |
| | 3+ | 1382.5632 | 1382.5578 | 3.9 | NNNNTNDTITLPCR | | [Hex]7[HexNAc]6[Fuc]1 |
| | 2+ | 1675.1782 | 1675.1718 | 3.8 | NNNNTNDTITLPCR | | [Hex]8[HexNAc]2 |
| | 2+ | 1756.2094 | 1756.1982 | 6.4 | NNNNTNDTITLPCR | | [Hex]9[HexNAc]2 |
| | | | | | | | |
| C4 | 2+ | 1165.0431 | 1165.0545 | 9.8 | SNITGLLLTR | | [Hex]3[HexNAc]3[Fuc]1 |
| | 2+ | 1193.5674 | 1193.5652 | 1.8 | SNITGLLLTR | | [Hex]3[HexNAc]4 |
| | 2+ | 1266.5962 | 1266.5942 | 1.7 | SNITGLLLTR | | [Hex]3[HexNAc]4[Fuc]1 |
| | 2+ | 1295.1151 | 1295.1050 | 7.9 | SNITGLLLTR | | [Hex]3[HexNAc]5 |
| | 2+ | 1368.1382 | 1368.1339 | 3.1 | SNITGLLLTR | | [Hex]3[HexNAc]5[Fuc]1 |
| | 2+ | 1267.5791 | 1267.5838 | 3.7 | SNITGLLLTR | u | [Hex]4 [HexNAc]3 [Fuc]1 |
| | 2+ | 1274.5985 | 1274.5917 | 5.3 | SNITGLLLTR | | [Hex]4 [HexNAc]4 |
| | 2+ | 1507.1642 | 1507.1658 | 1.1 | SNITGLLLTR | for | Hex]4 [HexNAc]4 [Fuc]1 [NeuNAc]1 |
| | 2+ | 1071.5125 | 1071.5123 | 0.2 | SNITGLLLTR | | [Hex]4[HexNAc]2 |
| | 2+ | 1173.0544 | 1173.0520 | 2.1 | SNITGLLLTR | | [Hex]4[HexNAc]3 |
| | 2+ | 1246.0837 | 1246.0809 | 2.3 | SNITGLLLTR | | [Hex]4[HexNAc]3[Fuc]1 |
| | 2+ | 1489.1458 | 1489.1387 | 4.8 | SNITGLLLTR | | [Hex]4[HexNAc]5[Fuc]1[SO3]1 |
| | 2+ | 1254.0812 | 1254.0784 | 2.2 | SNITGLLLTR | | [Hex]5 [HexNAc]3 |
| | 2+ | 1327.1130 | 1327.1073 | 4.3 | SNITGLLLTR | | [Hex]5[HexNAc]3[Fuc]1 |
| | 2+ | 1355.6198 | 1355.6181 | 1.3 | SNITGLLLTR | | [Hex]5[HexNAc]4 |
| | 2+ | 1559.6943 | 1559.6871 | 4.6 | SNITGLLLTR | u | [Hex]6[HexNAc]5 |
| | 2+ | 1233.5686 | 1233.5651 | 2.8 | SNITGLLLTR | | [Hex]6[HexNAc]2 |
| | 2+ | 1429.6647 | 1429.6366 | 19.7 | SNITGLLLTR | | [Hex]6[HexNAc]3[Fuc]1 |
| | 2+ | 1314.5969 | 1314.5915 | 4.1 | SNITGLLLTR | u | [Hex]7[HexNAc]2 |
| | 2+ | 1395.6220 | 1395.6179 | 2.9 | SNITGLLLTR | | [Hex]8[HexNAc]2 |
| | 2+ | 1476.6450 | 1476.6443 | 0.5 | SNITGLLLTR | | [Hex]9[HexNAc]2 |
| | | | | | | | |
| V5 | 3+ | 1232.5150 | 1232.5092 | 4.7 | DGGNNNTNETEIFRPGGGDMR | | [Hex]3[HexNAc]4[Fuc]1 |
| | 3+ | 1300.2075 | 1300.2024 | 3.9 | DGGNNNTNETEIFRPGGGDMR | | [Hex]3[HexNAc]5[Fuc]1 |
| | 3+ | 1367.8986 | 1367.8955 | 2.3 | DGGNNNTNETEIFRPGGGDMR | | [Hex]3[HexNAc]6[Fuc]1 |
| | 3+ | 1102.4567 | 1102.4547 | 1.8 | DGGNNNTNETEIFRPGGGDMR | | [Hex]4[HexNAc]2 |
| | 3+ | 1218.8466 | 1218.8337 | 10.6 | DGGNNNTNETEIFRPGGGDMR | | [Hex]4[HexNAc]3[Fuc]1 |
| | 3+ | 1305.5218 | 1305.5340 | 9.3 | DGGNNNTNETEIFRPGGGDMR | | [Hex]4[HexNAc]5 |
| | 3+ | 1354.2230 | 1354.2200 | 2.2 | DGGNNNTNETEIFRPGGGDMR | | [Hex]4[HexNAc]5[Fuc]1 |
| | 3+ | 1451.2552 | 1451.2518 | 2.3 | DGGNNNTNETEIFRPGGGDMR | | [Hex]4[HexNAc]5[Fuc]1[NeuNAc]1 |
| | 3+ | 1518.9468 | 1518.9449 | 1.3 | DGGNNNTNETEIFRPGGGDMR | | [Hex]4[HexNAc]6[Fuc]1[NeuNAc]1 |
| | 3+ | 1156.4763 | 1156.4722 | 3.6 | DGGNNNTNETEIFRPGGGDMR | | [Hex]5[HexNAc]2 |
| | 3+ | 1272.8628 | 1272.8513 | 9.0 | DGGNNNTNETEIFRPGGGDMR | | [Hex]5[HexNAc]3[Fuc]1 |
| | 3+ | 1408.2524 | 1408.2376 | 10.5 | DGGNNNTNETEIFRPGGGDMR | | [Hex]5[HexNAc]5[Fuc]1 |
| | 3+ | 1505.2666 | 1505.2694 | 1.9 | DGGNNNTNETEIFRPGGGDMR | | [Hex]5[HexNAc]5[Fuc]1[NeuNAc]1 |
| | 3+ | 1210.4972 | 1210.4899 | 6.0 | DGGNNNTNETEIFRPGGGDMR | | [Hex]6 [HexNAc]2 |
| | 3+ | 1326.8643 | 1326.8689 | 3.5 | DGGNNNTNETEIFRPGGGDMR | | [Hex]6[HexNAc]3[Fuc]1 |
| | 3+ | 1375.2253 | 1375.2148 | 7.6 | DGGNNNTNETEIFRPGGGDMR | | [Hex]6[HexNAc]3[NeuNAc]1 |
| | 3+ | 1437.5853 | 1437.5763 | 6.3 | DGGNNNTNETEIFRPGGGDMR | | [Hex]6[HexNAc]4[NeuNAc]1 |
| | 3+ | 1462.2479 | 1462.2552 | 5.0 | DGGNNNTNETEIFRPGGGDMR | | [Hex]6[HexNAc]5[Fuc]1 |
| | 3+ | 1559.3010 | 1559.2870 | 9.0 | DGGNNNTNETEIFRPGGGDMR | | [Hex]6[HexNAc]5[Fuc]1[NeuNAc]1 |
| | 3+ | 1264.5123 | 1264.5075 | 3.8 | DGGNNNTNETEIFRPGGGDMR | | [Hex]7[HexNAc]2 |
| | 3+ | 1372.5427 | 1372.5427 | 0.0 | DGGNNNTNETEIFRPGGGDMR | | [Hex]9[HexNAc]2 |
| | | | | | | | |
| TM | 2+ | 1336.1443 | 1336.1406 | 2.8 | EINNYTDIIYSLIEESQNQQEK | | Non-glycosylated |

**Table 10. LC/MS Glycopeptide Composition for JR-FL gp140 ΔCF**

| **Env Domain** | **Charge State** | **Experimental Mass** | **Theoretical Mass** | **Mass Error** | **Peptide** | **Mod*** | **Carbohydrate Composition** |
|---|---|---|---|---|---|---|---|
| Cl | 5+ | 1266.5685 | 1266.5564 | 9.6 | AYDTEVHNVWATHACVPTDPNPQEVVLENVTEHFNMWK | | [Hex]3[HexNAc]4[Fuc]1 |
| | 4+ | 1532.1911 | 1532.1738 | 11.3 | AYDTEVHNVWATHACVPTDPNPQEVVLENVTEHFNMWK | | [Hex]3[HexNAc]5[Fuc]1 |
| | 5+ | 1258.3565 | 1258.3511 | 4.3 | AYDTEVHNVWATHACVPTDPNPQEVVLENVTEHFNMWK | | [Hex]3[HexNAc]6 |
| | 4+ | 1582.9747 | 1582.9437 | 19.6 | AYDTEVHNVWATHACVPTDPNPQEVVLENVTEHFNMWK | | [Hex]3[HexNAc]6[Fuc]1 |
| | 5+ | 1269.3493 | 1269.3275 | 17.2' | AYDTEVHNVWATHACVPTDPNPQEVVLENVTEHFNMWK | | [Hex]4 [HexNAc]3 [Fuc]1 |
| | 4+ | 1481.4264 | 1481.4040 | 15.1 | AYDTEVHNVWATHACVPTDPNPQEVVLENVTEHFNMWK | | [Hex]4 [HexNAc]4 [Fuc]1 |
| | 5+ | 1229.1332 | 1229.1395 | 5.1 | AYDTEVHNVWATHACVPTDPNPQEVVLENVTEHFNMWK | | [Hex]4[HexNAc]5 |
| | 5+ | 1258.3565 | 1258.3511 | 4.3 | AYDTEVHNVWATHACVPTDPNPQEVVLENVTEHFNMWK | | [Hex]4[HexNAc]5[Fuc]1 |
| | 5+ | 1396.2154 | 1396.1986 | 12.0 | AYDTEVHNVWATHACVPTDPNPQEVVLENVTEHFNMWK | | [Hex]5[HexNAc]2 |
| | 5+ | 1225.9579 | 1225.9405 | 14.2 | AYDTEVHNVWATHACVPTDPNPQEVVLENVTEHFNMWK | | [Hex]5[HexNAc]3[Fuc]1 |
| | 4+ | 1471.1580 | 1471.1473 | 7.3 | AYDTEVHNVWATHACVPTDPNPQEVVLENVTEHFNMWK | | [Hex]5 [HexNAc]4 [Fuc]1 |
| | 5+ | 1290.7546 | 1290.7616 | 5.4 | AYDTEVHNVWATHACVPTDPNPQEVVLENVTEHFNMWK | | [Hex]5 [HexNAc]5 [Fuc]1 |
| | 5+ | 1323.1835 | 1323.1722 | 8.5 | AYDTEVHNVWATHACVPTDPNPQEVVLENVTEHFNMWK | | [Nex]6[HexNAc]5 [Fuc]1 |
| | 5+ | 1229.1332 | 1229.1395 | 5.1 | AYDTEVHNVWATHACVPTDPNPQEVVLENVTEHFNMWK | | [Hex]6[HexNAc]6[Fuc]1 |
| | 5+ | 1396.2154 | 1396.1986 | 12.0 | AYDTEVHNVWATHACVPTDPNPQEVVLENVTEHFNMWK | | [Hex]7[HexNAc]6[Fuc]1 |
| | | | | | | | |
| V1-V2 | 3+ | 1119.4877 | 1119.4904 | 2.4 | NCSFNITTSIRDEVQK | | [Hex]3[HexNAc]4[Fuc]1 |
| | 3+ | 1187.1908 | 1187.1836 | 6.1 | NCSFNITTSIRDEVQK | | [Hex]3[HexNAc]5[Fuc]1 |
| | 3+ | 1241.1953 | 1241.2012 | 4.8 | NCSFNITTSIRDEVQK | | [Hex]4[HexNAc]5[Fuc]1 |
| | 3+ | 1227.5392 | 1227.5256 | 11.0 | NCSFNITTSIRDEVQK | | [Hex]5[HexNAc]4[Fuc]1 |
| | 3+ | 1246.5354 | 1246.5328 | 2.1 | NCSFNITTSIRDEVQK | | [Hex]5[HexNAc]5 |
| | 3+ | 1295.2152 | 1295.2188 | 2.8 | NCSFNITTSIRDEVQK | | [Hex]5[HexNAc]5[Fuc]1 |
| | 3+ | 1392.2512 | 1392.2506 | 0.4 | NCSFNITTSIRDEVQK | | [Hex]5[HexNAc]5[Fuc]1[NeuNAc]1 |
| | 3+ | 1262.2046 | 1262.1960 | 6.8 | NCSFNITTSIRDEVQK | | [Hex]6[HexNAc]3[NeuNAc]1 |
| | 3+ | 1259.5146 | 1259.5096 | 4.0 | NCSFNITTSIRDEVQK | | [Hex]6[HexNAc]4[SO3]1 |
| | 3+ | 1205.5102 | 1205.5063 | 3.2 | NCSFNITTSIRDEVQK | | [Hex]8[HexNAc]2 |
| | 3+ | 1259.5146 | 1259.5239 | 7.4 | NCSFNITTSIRDEVQK | | [Hex]9[HexNAc]2 |
| | | | | | | | |
| V2 | 2+ | 1431.1325 | 1431.1320 | 0.3 | LDVVPIDNNNTSYR | | [Hex]3[HexNAc]3[Fuc]1 |
| | 2+ | 1532.6792 | 1532.6716 | 4.9 | LDVVPIDNNNTSYR | | [Hex]3[HexNAc]4[Fuc]1 |
| | 2+ | 1634.2266 | 1634.2114 | 9.3 | LDVVPIDNNNTSYR | | [Hex]3[HexNAc]5[Fuc]1 |
| | 2+ | 1735.7579 | 173557511 | 3.9 | LDVVPIDNNNTSYR | | [Hex]3[HexNAc]6[Fuc]1 |
| | 2+ | 1512.1611 | 1512.1584 | 1.8 | LDVVPIDNNNTSYR | | [Hex]4[HexNAc]3[Fuc]1 |
| | 2+ | 1642.1907 | 1642.2088 | 11.0 | LDVVPTDNNNTSYR | | [Hex]4[HexNAc]5 |
| | 3+ | 1337.8800 | 1337.8912 | 8.4 | LDVVPIDNNNTSYR | | [Hex]4[HexNAc]5[Fuc]1[NeuNAc]2 |
| | 2+ | 1418.6325 | 1418.6161 | 11.5 | LDVVPIDNNNTSYR | | [Hex]5[HexNAc]2 |
| | 3+ | 1062.4683 | 1062.4589 | 8.8 | LDVVPIDNNNTSYR | | [Hex]5[HexNAc]3[Fuc]1 |
| | 2+ | 1694.7371 | 1694.7245 | 7.4 | LDVVPIDNNNTSYR | | [Hex]5[HexNAc]4[Fuc]1 |
| | 3+ | 1197.8553 | 1197.8452 | 8.4 | LDVVPIDNNNTSYR | | [Hex]5[HexNAc]5[Fuc]1 |
| | 2+ | 1499.6545 | 1499.6426 | 7.9 | LDVVPIDNNNTSYR | | [Hex]6[HexNAc]2 |
| | 2+ | 1674.1967 | 1674.2112 | 8.7 | LDVVPIDNNNTSYR | | [Hex]6[HexNAc]3[Fuc]1 |
| | 3+ | 1270.8585 | 1270.8700 | 9.0 | LDVVPIDNNNTSYR | | [Hex]6[HexNAc]6 |
| | 2+ | 1319.5512 | 1319.5559 | 3.6 | LDVVPIDNNNTSYR | | [Hex]6[HexNAc]6[Fuc]1 |
| | 3+ | 1580.6923 | 1580.6690 | 14.7 | LDVVPIDNNNTSYR | | [Hex]7[HexNAc]2 |
| | 2+ | 1373.5710 | 1373.5735 | 1.9 | LDVVPIDNNNTSYR | | [Hex]7[HexNAc]6[Fuc]1 |
| | 3+ | 1661.7095 | 1661.6954 | 8.5 | LDVVPIDNNNTSYR | | [Hex]8[HexNAc]2 |
| | 2+ | 1468.2872 | 1468.2650 | 15.1 | LDVVPIDNNNTSYR | | [Hex]8[HexNAc]5[Fuc]1[NeuGc]1 |
| | 3+ | 1742.7289 | 1742.7218 | 4.1 | LDVVPIDNNNTSYR | | [Hex]9[FHexNAc]2 |
| | | | | | | | |
| C2 | 3+ | 1132.5155 | 1132.5206 | 0.6 | SDNFTNNAKTIIVQLK | | [Hex]3[HexNAc]4[NeuNAc]1 |
| | 3+ | 1118.8391 | 1118.8450 | 5.3 | SDNFTNNAKTIIVQLK | | [Hex]4[HexNAc]3[NeuNAc]1 |
| | 3+ | 1186.5345 | 1186.5382 | 3.1 | SDNFTNNAKTIIVQLK | | [Hex]4[HexNAc]4[NeuNAc]1 |
| | 3+ | 1254.2295 | 1254.2313 | 1.4 | SDNFTNNAKTIIVQLK | | [Hex]4[HeXNAc]5[NeuNAc]1 |
| | 3+ | 1172.8591 | 1172.8626 | 3.0 | SDNFTNNAKTIIVQLK | | [Hex]5(HexNAc)3[NeuNAc]1 |
| | 3+ | 1240.5576 | 1240.5558 | 1.5 | SDNFTNNAKTIIVQLK | | [Hex]5[HexNAc]4[NeuNAc]1 |
| | 3+ | 1124.5074 | 1124.5168 | 8.4 | SDNFTNNAKTIIVQLK | | [Hex]5[HexNAc]3[Fuc]1 |
| | 3+ | 1337.6003 | 1337.5876 | 9.5 | SDNFTNNAKTIIVQLK | | [Hex]5[HexNAc]4[NeuNAc]2 |
| | 3+ | 1405.2883 | 1405.2807 | 5.4 | SDNFTNNAKTIIVQLK | | [Hex]5[HexNAc]5[NeuNAc]2 |
| | 3+ | 1129.8552 | 1129.8484 | 4.5 | SDNFTNNAKTIIVQLK | | [Hex]6[HexNAc]3 |
| | 3+ | 1362.2695 | 1362.2665 | 2.2 | SDNFTNNAKTIIVQLK | | [Hex]6[HexNAc]5[NeuNAc]1 |
| | 3+ | 1459.2782 | 1459.2983 | 13.8 | SDNFTNNAKTIIVQLK | | [Hex]6[HexNAc]5[NeuNAc]2 |
| | | | | | | | |
| V3-C3 | 2+ | 1276.0527 | 1276.0153 | 29.3 | QAHCNISR | | [Hex]5[HexNAc]3[Fuc]1 |
| | 2+ | 1377.5928 | 1377.5550 | 27.4 | QAHCNISR | | [Hex]5[HexNAc]4[Fuc]1 |
| | 2+ | 1487.0876 | 1487.0921 | 3.0 | QAHCNISR | | [Hex]6[HexNAc]5 |
| | | | | | | | |
| C3 | 2+ | 1108.9988 | 1108.9938 | 4.5 | AKWNDTLK | | [Hex]3[HexNAc]3[Fuc]1 |
| | 2+ | 1210.5391 | 1210.5335 | 4.6 | AKWNDTLK | | [Hex]3[HexNAc]4[Fuc]1 |
| | 2+ | 1304.5245 | 1304.5552 | 23.5 | AKWNDTLK | u | [Hex]3[HexNAc]4[NeuNAc]1 |
| | 2+ | 1312.0836 | 1312.0732 | 7.9 | AKWNDTLK | | [Hex]3[HexNAc]5[Fuc]1 |
| | 2+ | 1413.6098 | 1413.6129 | 2.3 | AKWNDTLK | | [Hex]3[HexNAc]6[Fuc]1 |
| | 2+ | 1218.5409 | 1218.5310 | 8.1 | AKWNDTLK | | [Hex]4[HexNAc]4 |
| | 2+ | 1211.5251 | 1211.5231 | 1.7 | AKWNDTLK | u | [Hex]4[HexNAc]3[Fuc]1 |
| | 2+ | 1357.0678 | 1357.0708 | 2.3 | AKWNDTLK | u | [Hex]4[HexNAc]3[Fuc]1[NeuNAc]1 |
| | 2+ | 1385.574 | 1385.5816 | 5.5 | AKWNDTLK | u | [Hex]4[HexNAc]4[NeuNAc]1 |
| | 3+ | 929.0752 | 929.0688 | 6.9 | AKWNDTLK | | [Hex]4[HexNAc]5[Fuc]1 |
| | 2+ | 1096.4889 | 1096.4780 | 9.9 | AKWNDTLK | | [Hex]5[HexNAc]2 |
| | 3+ | 847.7045 | 847.7002 | 5.1 | AKWNDTLK | | [Hex]5[HexNAc]3[Fuc]1 |
| | 3+ | 915.3974 | 915.3933 | 4.5 | AKWNDTLK | | [Hex]5[HexNAc]4[Fuc]1 |
| | 3+ | 1012.4247 | 1012.4251 | 0.4 | AKWNDTLK | | [Hex]5[HexNAc]4[Fuc]1[NeuNAc]1 |
| | 3+ | 983.0925 | 983.0865 | 6.1 | AKWNDTLK | | [Hex]5[HexNAc]5[Fuc]1 |
| | 2+ | 1177.5095 | 1177.5044 | 4.3 | AKWNDTLK | | [Hex]6[HexNAc]2 |
| | 3+ | 853.0334 | 853.0318 | 1.9 | AKWNDTLK | | [Hex]6[HexNAc]3 |
| | 3+ | 901.7213 | 901.7178 | 3.9 | AKWNDTLK | | [Hex]6[HexNAc]3[Fuc]1 |
| | 3+ | 950.0626 | 950.0636 | 1.1 | AKWNDTLK | | [Hex]6[HexNAc]3[NeuNAc]1 |
| | 2+ | 1394.5787 | 1394.5812 | 1.8 | AKWNDTLK | for | [Hex]6[HexNAc]4 |
| | 3+ | 839.3596 | 839.3563 | 3.9 | AKWNDTLK | | [Hex]7[HexNAc]2 |
| | 3+ | 893.3776 | 893.3739 | 4.1 | AKWNDTLK | | [Hex]8[HexNAc]2 |
| | 3+ | 947.3965 | 947.3915 | 5.3 | AKWNDTLK | | [Hex]9[HexNAc]2 |
| | | | | | | | |
| C4 | 2+ | 1215.5448 | 1215.5569 | 10.0 | CSSNITGLLLTR | | [Hex]3[HexNAc]3 |
| | 2+ | 1288.5958 | 1288.5858 | 7.8 | CSSNITGLLLTR | | [Hex]3[HexNAc]3[Fuc]1 |
| | 2+ | 1317.1068 | 1317.0965 | 7.8 | CSSNITGLLLTR | | [Hex]3[HexNAc]4 |
| | 2+ | 1390.1245 | 1390.1255 | 0.7 | CSSNITGLLLTR | | [Hex]3[HexNAc]4[Fuc]1 |
| | 2+ | 1491.6762 | 1491.6652 | 7.4 | CSSNITGLLLTR | | [Hex]3[HexNAc]5[Fuc]1 |
| | 2+ | 1296.5939 | 1296.5833 | 8.2 | CSSNITGLLLTR | | [Hex]4[HexNAc]3 |
| | 2+ | 1369.6179 | 1369.6122 | 4.2 | CSSNITGLLLTR | | [Hex]4[HexNAc]3[Fuc]1 |
| | 3+ | 1078.1394 | 1078.1355 | 3.6 | CSSNITGLLLTR | | [Hex]4[HexNAc]4[Fuc]1[NeuNAc]1 |
| | 2+ | 1572.7100 | 1572.6916 | 11.7 | CSSNITGLLLTR | | [Hex]4[HexNAc]5[Fuc]1 |
| | 2+ | 1377.6164 | 1377.6097 | 4.9 | CSSNITGLLLTR | | [Hex]5[HexNAc]3 |
| | 2+ | 1276.0779 | 1276.0700 | 6.2 | CSSNITGLLLTR | | [Hex]5[HexNAc]2 |
| | 2+ | 1450.6507 | 1450.6386 | 8.3 | CSSNITGLLLTR | | [Hex]5[HexNAc]3[Fuc]1 |
| | 2+ | 1479.1699 | 1479.1494 | 13.9 | CSSNITGLLLTR | | [Hex]5[HexNAc]4 |
| | 3+ | 1035.1253 | 1035.1213 | 3.9 | CSSNITGLLLTR | | [Hex]5[HexNAc]4[Fuc]1 |
| | 3+ | 1132.1535 | 1132.1531 | 0.4 | CSSNITGLLLTR | | [Hex]5[HexNAc]4[Fuc]1[NeuNAc]1 |
| | 2+ | 1653.7465 | 1653.7180 | 17.2 | CSSNITGLLLTR | | [Hex]5[HexNAc]5[Fuc]1 |
| | 2+ | 1357.1039 | 1357.0964 | 5.5 | CSSNITGLLLTR | | [Hex]6[HexNAc]2 |
| | 2+ | 1458.6430 | 1458.6361 | 4.7 | CSSNITGLLLTR | | [Hex]6[HexNAc]3 |
| | 2+ | 1531.6806 | 1531.6650 | 10.2 | CSSNITGLLLTR | | [Hex]6[HexNAc]3[Fuc]1 |
| | 2+ | 1604.1660 | 1604.1838 | 11.1 | CSSNITGLLLTR | | [Hex]6[HexNAc]3[NeuNAc]1 |
| | 2+ | 1438.1321 | 1438.1228 | 6.5 | CSSNITGLLLTR | | [Hex]7[HexNAc]2 |
| | 3+ | 1013.0974 | 1013.1019 | 4.4 | CSSNITGLLLTR | | [Hex]8[HexNAc]2 |
| | 3+ | 1067.1 183 | 1067.1195 | 1.1 | CSSNITGLLLTR | | [Hex]9[HexNAc]2 |
| | | | | | | | |
| V5 | 3+ | 1111.8127 | 1111.8068 | 5.3 | DGGINENGTEIFRPGGGDMR | | [Hex]3[HexNAc]3[Fuc]1 |
| | 3+ | 1169.4996 | 1169.4843 | 13.1 | DGGINENGTEIFRPGGGDMR | | [Hex]3[HexNAc]3[NeuNAc]1 |
| | 3+ | 1179.5052 | 1179.4999 | 4.5 | DGGINENGTEIFRPGGGDMR | | [Hex]3[HexNAc]4[Fuc]1 |
| | 3+ | 1198.4897 | 1198.5071 | 14.5 | DGGPIENGTEIFRPGGGDMR | | [Hex]3[HexNAc]5 |
| | 3+ | 1247.1980 | 1247.1931 | 3.9 | DGGINENGTEIFRPGGGDMR | | [Nex]3[HexNAc]5[Fuc]1 |
| | 3+ | 1314.8981 | 1314.8862 | 9.1 | DGGINENGTEIFRPGGGDMR | | [Hex]3[HexNAc]6[Fuc]1 |
| | 3+ | 1368.9120 | 1368.9038 | 6.0 | DGGINENGTEIFRPGGGDMR | mox | [Hex]3[HexNAc]6[Fuc]2 |
| | 3+ | 1184.8352 | 1184.8316 | 3.0 | DGGINENGTEIFRPGGGDMR | | [Hex]4[HexNAc]4 |
| | 3+ | 1107.4579 | 1107.4629 | 4.5 | DGGINENGTEIFRPGGGDMR | for | [Hex]4[HexNAc]2[Fuc]1 |
| | 3+ | 1165.8293 | 1165.8244 | 4.2 | DGGINENGTEIFRPGGGDMR | | [Hex]4[HexNAc][Fuc]1 |
| | 3+ | 1262.8588 | 1262.8560 | 2.1 | DGGINENGTEIFRPGGGDMR | | [Hex]4[HexNAc]3[Fuc]1[NeuNAc]1 |
| | 3+ | 1330.5602 | 1330.5493 | 8.1 | DGGINENGTEIFRPGGGDMR | | [Hex]4[HexNAc]4[Fuc]1[NeuNAc]1 |
| | 3+ | 1252.5317 | 1252.5247 | 5.6 | DGGINENGTEIFRPGGGDMR | | [Hex]4[HexNAc]5 |
| | 3+ | 1301.2222 | 1301.2106 | 8.8 | DGGINENGTEIFRPGGGDMR | | [Hex]4[HcxNAc]5[Fuc]1 |
| | 3+ | 1398.2534 | 1398.2425 | 7.8 | DGGINENGTEIFRPGGGDMR | | [Hex]4[HexNAc]5[Fuc]1 [NeuNAc]1 |
| | 3+ | 1417.2489 | 1417.2496 | 0.6 | DGGINENGTEIFRPGGGDMR | | [Hex]4[HexNAc]6[NeuNAc]1 |
| | 3+ | 1465.9532 | 1465.9356 | 12.0 | DGGINENGTEIFRPGGGDMR | | [Hex]4[HexNAc]6[Fuc]1[NeuNAc]1 |
| | 3+ | 1171.1581 | 1171.1560 | 1.7 | DGGINENGTEIFRPGGGDMR | | [Hex]5[HexNAc]3 |
| | 3+ | 1268.2010 | 1268.1878 | 10.3 | DGGINENGTEIFRPGGGDMR | | [Hex]5[HexNAc]3 [NeuNAc]1 |
| | 3+ | 1103.4695 | 1103.4629 | 6.0 | DGGINENGTEIFRPGGGDMR | | [Hex]5[HexNAc]2 |
| | 3+ | 1219.8537 | 1219.8420 | 9.6 | DGGINENGTEIFRPGGGDMR | | [Hex]5[HexNAc]3[Fuc]1 |
| | 3+ | 1287.5434 | 1287.5351 | 6.4 | DGGINENGTEIFRPGGGDMR | | [Hex]5[HexNAc]4[Fuc]1 |
| | 3+ | 1384.5786 | 1384.5669 | 8.4 | DGGINENGTEIFRPGGGDMR | | [Hex]5[HexNAc]4[Fuc]1[NeuNAc]1 |
| | 3+ | 1481.6149 | 1481.5987 | 10.9 | DGGINENGTEIFRPGGGDMR | | [Hex]5[HexNAc]4[Fuc]1[NeuNAc]2 |
| | 3+ | 1306.5448 | 1306.5423 | 1.9 | DGGINENGTEIFRPGGGDMR | | [Hex]5[HexNAc]5 |
| | 3+ | 1355.2366 | 1355.2283 | 6.1 | DGGINENGTEIFRPGGGDMR | | [Hex]5[HexNAc]5[Fuc]1 |
| | 3+ | 1452.2683 | 1452.2601 | 5.6 | DGGINENGTEIFRPGGGDMR | | [Hex]3[HexNAc]5[Fuc]1[NeuNAc]1 |
| | 3+ | 1403.5745 | 1403.5741 | 0.3 | DGGINENGTEIFRPGGGDMR | | [Hex]5[HexNAc]5[NeuNAc]1 |
| | 3+ | 1333.2109 | 1333.1946 | 12.2 | DGGINENGTEIFRPGGGDMR | | [Hex]5[HexNAc]5[SO3]1 |
| | 3+ | 1157.4879 | 1157.4805 | 6.3 | DGGINENGTEIFRPGGGDMR | | [Hex]6[HexNAc]2 |
| | 3+ | 1360.5682 | 1360.5599 | 6.1 | DGGINENGTEIFRPGGGDMR | | [Hex]6[HexNAc]5 |
| | 3+ | 1225.1788 | 1225.1736 | 4.2 | DGGINENGTEIFRPGGGDMR | | [Hex]6[HexNAc]3 |
| | 3+ | 1273.8745 | 1273.8596 | 11.7 | DGGINENGTEIFRPGGGDMR | | [Hex]6[HexNAc]3[Fuc]1 |
| | 3+ | 1322.2201 | 1322.2054 | 11.0 | DGGINENGTEIFRPGGGDMR | | [Hex]6[HexNAc]3[NeuNAc]1 |
| | 3+ | 1389.9101 | 1389.8986 | 8.3 | DGGINENGTEIFRPGGGDMR | | [Hex]6[HexNAc]4[NeuNAc]1 |
| | 3+ | 1409.2587 | 1409.2459 | 9.1 | DGGINENGTEIFRPGGGDMR | | [Hex]6[HexNAc]5[Fuc]1 |
| | 3+ | 1506.2964 | 1506.2777 | 12.4 | DGGINENGTEIFRPGGGDMR | | [Hex]6[HexNAc]5[Fuc]1[NeuNAc]1 |
| | 3+ | 1603.3206 | 1603.3095 | 6.9 | DGGINENGTEIFRPGGGDMR | | [Hex]6[HexNAc]5[Fuc]1[NeuNAc]2 |
| | 3+ | 1428.2686 | 1428.2530 | 10.9 | DGGINENGTEIFRPGGGDMR | | [Hex]6[HexNAc]6 |
| | 3+ | 1476.9544 | 1476.9390 | 10.4 | DGGINENGTEIFRPGGGDMR | | [Hex]6[HexNAc]6[Fuc]1 |
| | 3+ | 1525.2770 | 1525.2848 | 5.2 | DGGINENGTEIFRPGGGDMR | | [Hex]6[HexNAc]6[NeuNAc]1 |
| | 3+ | 1211.5025 | 1211.4981 | 3.5 | DGGINENGTEIFRPGGGDMR | | [Hex]7[HexNAc]2 |
| | 3+ | 1279.1816 | 1279.1912 | 7.6 | DGGINENGTEIFRPGGGDMR | | [Hex]7[HexNAc]3 |
| | 3+ | 1530.9761 | 1530.9566 | 12.7 | DGGINENGTEIFRPGGGDMR | | [Hex]7[HexNAc]6[Fuc]1 |
| | 3+ | 1633.3537 | 1633.3215 | 19.7 | DGGINENGTEIFRPGGGDMR | | [Hex]7[HexNAc]6[Fuc]1[NeuGc]1 |
| | 3+ | 1628.0097. | 1627.9884 | 13.1 | DGGINENGTEIFRPGGGDMR | | [Hex]7[HexNAc]6[Fuc]1[NeuNAc]1 |
| | 3+ | 1725.0450 | 1725.0202 | 14.4 | DGGINENGTEIFRPGGGDMR | | [Hex]7[HexNAc]6[Fuc]1[NeuNAc]2 |
| | 3+ | 1265.5192 | 1265.5157 | 2.7 | DGGINENGTEIFRPGGGDMR | | [Hex]8[HexNAc]2 |
| | 3+ | 1319.5459 | 1319.5333 | 9.5 | DGGINENGTEIFRPGGGDMR | | [Hex]9[HexNAc]2 |
| | | | | | | | |
| TM | 2+ | 1545.1895 | 1545.1863 | 2.1 | LICTTAVPWNASWSNK | | [Hex]3[HexMAc]3[Fuc]1 |
| | 3+ | 1098.1575 | 1098.1531 | 4.0 | LICTTAVPWNASWSNK | | [Hex]3[HexNAc]4[Fuc]1 |
| | 2+ | 1748.2660 | 1748.2657 | 0.2 | LICTTAVPWNASWSNK | | [Hex]3[HexNAc]5[Fuc]1 |
| | 3+ | 1233.5400 | 1233.5394 | 0.5 | LICTTAVPWNASWSNK | | [Hex]3[HexNAc]6[Fuc]1 |
| | 3+ | 1238.1741 | 1238.1579 | 13.1 | LICTTAVPWNASWSNK | | [Hex]3[HexNAc]6[SO3]2 |
| | 2+ | 1626.2239 | 1626.2127 | 6.9 | LICTTAVPWNASWSNK | | [Hex]4[HexNAc]3[Fuc]1 |
| | 3+ | 1181.4943 | 1181.5094 | 12.8 | LICTTAVPWNASWSNK | | [Hex]4[HexNAc]3[Fuc]1[NeuNAc]1 |
| | 3+ | 1219.8777 | 1219.8638 | 11.4 | LICTTAVPWNASWSNK | | [Hex]4[HexNAc]5[Fuc]1 |
| | 3+ | 1384.5927 | 1384.5888 | 2.8 | LICTTAVPWNASWSNK | | [Hex]4[HexNAc]6[Fuc]1[NeuNAc]1 |
| | 2+ | 1532.6864 | 1532.6705 | 10.4 | LICTTAVPWNASWSNK | | [Hex]5[HexNAc]2 |
| | 3+ | 1206.1957 | 1206.1883 | 6.1 | LICTTAVPWNASWSNK | | [Hex]5[HexNAc]4[Fuc]1 |
| | 3+ | 1303.2216 | 1303.2201 | 1.2 | LICTTAVPWNASWSNK | | [Hex]5[HexNAc]4[Fuc]1[NeuNAc]1 |
| | 3+ | 1273.8855 | 1273.8814 | 3.2 | LICTTAVPWNASWSNK | | [Hex]5[HexNAc]5[Fuc]1 |
| | 2+ | 1613.6986 | 1613.6969 | 1.1 | LICTTAVPWNASWSNK | | [Hex]6[HexNAc]2 |
| | 3+ | 1192.5289 | 1192.5128 | 13.5 | LICTTAVPWNASWSNK | | [Hex]6[HexNAc]3[Fuc]1 |
| | 3+ | 1238.1741 | 1238.1722 | 1.5 | LICTTAVPWNASWSNK | | [Hex]6[HexNAc]4[SO3]1 , |
| | 3+ | 1327.8999 | 1327.8990 | 0.7 | LICTTAVPWNASWSNK | | [Hex]6[HexNAc]5[Fuc]1 |
| | 2+ | 1694.7447 | 1694.7233 | 12.6 | LICTTAVPWNASWSNK | | [Hex]7[HexNAc]2 |
| | 3+ | 1314.2352 | 1314.2235 | 8.9 | LICTTAVPWNASWSNK | | [Hex]7[HexNAc]4[Fuc]1 |
| | 3+ | 1184.1662 | 1184.1689 | 2.3 | LICTTAVPWNASWSNK | | [Hex]8[HexNAc]2 |
| | 3+ | 1238.1741 | 1238.1865 | 10.0 | LICTTAVPWNASWSNK | | [Hex]9[HexNAc]2 |
| | 3+ | 1079.4228 | 1079.4279 | 4.8 | IWNNMTWMEWER | | [Hex]7[HexNAc]2 |
| | | | | | | | |
| | 2+ | 1337.6222 | 1337.6335 | 8.4 | EIDNYTSEIYTLIEESQNQQEK | | Non glycosylated |

| | | | | | | | |
|---|---|---|---|---|---|---|---|
| *mod = peptide modification For = formylation u = carbamylation PyroC5= Cysteine Deamidation PyroQ = Glutamic Acid Deamidation | | | | | | | |

## Claims

1. A composition comprising carbohydrates of the V1 or V2 region of HIV Env rendered immunogenic by derivatization and a carrier.

2. The composition according to claim 1 wherein said composition further comprises V3 peptides, whole consensus or wild type Env gp140s that induce anti-V3 antibodies, or derivatized carbohydrates from non-V1 or V2 loops of HIV Env, optionally wherein said peptides comprise 62.19 clade B V3.

3. The composition according to claim 1 wherein said carbohydrates are derivatized with keyhole limpet hemocyanin, CRM 197, tetanus toxoid or an HIV gag p24 helper region, optionally wherein said helper region is the GTH1 sequence (YKRWIILGLNKIVRMYS).

4. The composition according to claim 1 wherein said composition comprises HIV Env wherein all carbohydrates on the surface thereof are derivatized so as to be rendered immunogenic, optionally wherein said carbohydrates on the surface of said HIV Env are rendered immunogenic by derivatization with tetanus toxoid.

5. A composition according to claim 1 for use in a method of inducing the production of neutralizing antibodies against HIV, the method comprising administering said composition to a patient, optionally wherein said patient is a human or non-human mammal.

## Patentansprüche

1. Zusammensetzung, umfassend Kohlenhydrate der V1- oder V2-Region des HIV-Env, durch Derivatisierung immunogen gemacht, und einen Träger.

2. Zusammensetzung gemäß Anspruch 1, wobei die Zusammensetzung des Weiteren V3-Peptide, gesamtes Konsensus- oder Wildtyp-Env-gp140s, das Anti-V3-Antikörper induziert, oder derivatisierte Kohlenhydrate aus Nicht-V1- oder -V2-Schleifen des HIV-Env umfasst, wobei gegebenenfalls die Peptide 62.19-Clade-B-V3 umfassen.

3. Zusammensetzung gemäß Anspruch 1, wobei die Kohlenhydrate mit Schlitzschnecken-Hämocyanin, CRM 197, Tetanus-Toxoid oder einer HIV-gag-p24-Helferregion derivatisiert sind, wobei gegebenenfalls die Helferregion die GTH1-Sequenz ist (YKRWIILGLNKIVRMYS).

4. Zusammensetzung gemäß Anspruch 1, wobei die Zusammensetzung HIV-Env umfasst, wobei alle Kohlenhydrate auf dessen Oberfläche derivatisiert sind, so dass sie immunogen gemacht sind, wobei gegebenenfalls die Kohlenhydrate auf der Oberfläche des HIV-Env durch Derivatisierung mit Tetanus-Toxoid immunogen gemacht sind.

5. Zusammensetzung gemäß Anspruch 1 zur Verwendung in einem Verfahren zum Induzieren der Produktion neutralisierender Antikörper gegen HIV, wobei das Verfahren das Verabreichen der Zusammensetzung an einen Patienten umfasst, wobei gegebenenfalls der Patient ein menschlicher oder nicht-menschlicher Säuger ist.

## Revendications

1. Composition comprenant des glucides de la région V1 ou V2 de la protéine Env de VIH, rendus immunogènes par dérivation, et un véhicule.

2. Composition conforme à la revendication 1, laquelle composition comprend en outre des peptides de la région V3, des glycoprotéines gp140 d'enveloppe de type sauvage ou à consensus global qui induisent la production d'anticorps anti-V3, ou des glucides résultant d'une dérivation à partir de boucles non-V1 et non-V2 de la protéine Env de VIH, lesquels peptides comprennent, en option, le clade B 62.19 de V3.

3. Composition conforme à la revendication 1, pour laquelle la dérivation desdits glucides a été opérée avec de l'hémocyanine de fissurelle, de la protéine CRM197, de l'anatoxine tétanique ou une région auxiliaire p24 de la protéine Gag de VIH, laquelle région auxiliaire est, en option, la séquence GTH1 (YKRWIILGLNKIVRMYS).

4. Composition conforme à la revendication 1, laquelle composition comprend une protéine Env de VIH dans laquelle tous les glucides présents à la surface de celle-ci ont subi une dérivation qui les a rendus immunogènes, et en option, dans laquelle lesdits glucides présents à la surface de ladite protéine Env de VIH ont été rendus immunogènes par dérivation avec de l'anatoxine tétanique.

5. Composition conforme à la revendication 1, pour utilisation dans un procédé d'induction de la production d'anticorps neutralisants dirigés contre un VIH, lequel procédé comporte le fait d'administrer ladite composition à un patient, lequel patient est, en option, un mammifère humain ou non-humain.
